# EUROPEAN PATENT APPLICATION

(11) **EP 4 067 499 A1**
(43) Date of publication of application: **05.10.2022**
(21) Application number: 21166743.1
(22) Date of filing: 01.04.2021
(51) Int. Cl.: C12P 19/26, C12N 9/88

(54) **HYALURONIC ACID-PRODUCING RECOMBINANT CELLS**

(71) Applicant: Givaudan SA, 1214 Vernier (CH)
(72) Inventor: BEVILACQUA, Valentina, 91400 Orsay (FR); DURANT, Lionel, 91400 Orsay (FR); JAILLARDON, Karine, 91400 Orsay (FR); LOUIS, Dominique, 91400 Orsay (FR); THOMAS, Dominique, 95100 Argenteuil (FR)
(74) Representative: Nony

(57) **Abstract**

The present invention relates to the field of bio-production of hyaluronic acid.

There is a need in the art for hyaluronic acid production methods allowing its highly efficient synthesis and secretion.

The solution proposed in the present invention is the use of a genetically modified cell comprising many modifications as described in the present text.

The present invention further proposes methods allowing the bio-production of hyaluronic acid having a controlled molecular weight using the genetically modified cells of the invention.

## Description

### FIELD OF THE INVENTION

The present invention relates to the field of bio-production of hyaluronic acid.

### BACKGROUND OF THE INVENTION

Hyaluronic acid, also known as hyaluronan or HA, is a naturally ocurring high molecular weight polysaccharide composed of D-glucuronic acid and N-acetyl-D-glucosamine, linked via alternating β-(1→4) and β-(1→3) glycosidic bonds and having the chemical formula (C₁₄H₂₁NO₁₁)n. Hyaluronic acid can be 25,000 disaccharide repeats in length. Polymers of hyaluronic acid can range in size from 5,000 to 20,000,000 Da *in vivo.*

HA has many applications in the medical and cosmetic fields including scaffolding for tissue engineering, dermatological fillers, and viscosupplementation for osteoarthritis treatment. In particular, a reduction in HA mass or molecular weight via degradation or slowing of synthesis affects physical and chemical properties such as tissue volume, viscosity, and elasticity. There is therefore a constant need for HA production.

Today's main known sources of HA are human ombilical cords, rooster combs and fermentation of certain microorganisms.

Fermentation methods for preparing HA from microorganisms are particularly of interest since they facilitate the production of a large quantity of HA through possible scale-up, at a reduced cost. Contrary to isolating HA from animal sources which provide hyaluronic acid of very high molecular weights, microbial fermentation allows to control, to a certain extent, the size of the starting molecular weight. This avoids the need for further fractionation steps by mechanical, physical or chemical means.

In this aim, bacterial cultures such as group A and C hemolytic streptococci have been shown to represent good sources of HA (US5316926, US4801539, JP2009011315). However, such bacteria, and in particular *Streptococcus zooepidemicus* which is mainly used in the art, is not generally recognized as safe. Recombinant *Bacillus* host cells have also shown the ability to produce HA in the range of 20 to 800 KDa (US2008038780). It has also been demonstrated in US2006168690 that transforming plant cells to include a DNA encoding hyaluronic acid synthase successfully allowed for the production of HA. Finally, production of small molecules of HA has also been demonstrated in yeasts such as in a recombinant *Pichia pastoris* in CN104263666.

Contrary to other microorganisms that are commonly used for the production of biological molecules, yeasts are generally recognised as safe. They can grow rapidly and be cultivated at higher density, compared to bacteria, and do not require an aseptic environment. Furthermore, yeast cells can be more easily separated from the culture medium than bacteria, greatly simplifying the process for product extraction and purification. Finally, yeasts present the advantage of being more resistant to pH changes in the culture medium, and as such represent stronger fermentation systems.

However, among yeasts, distinguishing features between species may also present certain challenges. This is mainly due to the difference in metabolisms between the species. For example, genome integrations in *P. pastoris* are stable but often a high variability of clones from one transformation is encountered, displaying various productivity characteristics or changes in their physiology. This requires a time-consuming screening process so as to find the clone with the optimal features for the desired application. This clonal variability is an important drawback for the further development of *P. pastoris* as a platform for producing value-added chemicals. Another drawback of using *P. pastoris* is that it is a methanotrophic organism.

On the contrary, *Saccharomyces cerevisiae* is particularly useful as a tool for the production of molecules of interest since it has a long safe history when it comes to being used by humans (such as in wine, beer, or bread) and as such is a well established model whose genetic information is well known in the art. *S. cerevisiae* further presents the advantage of being generally recognised as safe for humans and animals. What's more, the acidification of the medium which occurs when cultivating this yeast reduces the possibility of contamination of the bio fermenters, and therefore eliminates the need to add antibiotics to the medium. Finally, many genetic tools have been developed allowing for stable modifications of its genome (integration within the chromosome).

Accordingly, there is still a need in the art for further hyaluronic acid production methods allowing its highly efficient synthesis and secretion. In particular, there is still a need to provide production methods that are cost-efficient and supply hyaluronic acid which is safe for human application.

In a particular case, there is still a need in the art for hyaluronic acid methods allowing to obtain large amounts of hyaluronic acid of a particular and controlled size.

### SUMMARY OF THE INVENTION

The present invention accordingly relates to the following items:
**Item 1:** a recombinant yeast cell producing hyaluronic acid (HA) wherein the recombinant cell comprises:
   (a) one or more nucleic acids encoding a polypeptide having hyaluronan synthase activity;
   (b) one or more nucleic acids encoding a polypeptide having UDP-Glucose dehydrogenase (UDP-GlcDH or HASB) activity; and
   (c) one or more recombinant nucleic acids encoding a polypeptide having hyaluronidase activity wherein the polypeptide having hyaluronidase activity comprises a secretion signal so that hyaluronic acid, in particular of a desired molecular weight (HAMW) is produced by the recombinant yeast cell,
   wherein said recombinant yeast cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.
   As illustrated in the examples, the recombinant yeasts of the invention allow for the production of hyaluronic acid in a yeast cell which is not naturally able to produce hyaluronic acid. It is further demonstrated in the examples that the size of the hylaruonic acid produced by the recombinant yeast may be controlled.
   Said advantageous properties can be further increased by recombining the yeast with additional modifications described here-after.
**Item 2:** A recombinant host cell producing hyaluronic acid (HA) wherein the recombinant host cell comprises:
   (a) one or more recombinant nucleic acids encoding a polypeptide having hyaluronan synthase activity;
   (b) one or more recombinant nucleic acids encoding a polypeptide having UDP-Glucose dehydrogenase (UDP-GlcDH or HASB) activity; and
   (c) one or more recombinant nucleic acids encoding a polypeptide having hyaluronidase activity wherein the polypeptide having hyaluronidase activity comprises a secretion signal and an anchoring signal so that hyaluronic acid, in particular of a desired molecular weight (HAMW) is produced by the host cell.
**Item 3:** The recombinant cell according to item 1 or 2, wherein the molecular weight of the HA is in the range of less than 50kDa, preferably in the range of about 20kDa to about 50kDa.
**Item 4:** The recombinant cell according to item 1 or 2, wherein the molecular weight of the HA is in the range of greater than 50kDa, preferably in the range of about 50kDa to about 250kD.
**Item 5:** The recombinant cell according to item 1 or 2, wherein the molecular weight of the HA is in the range of greater than 100kDa, preferably in the range of about 100kDa to about 1500kDa.
**Item 6:** The recombinant cell according to any one of items 1 to 5, wherein the nucleic acid encoding a polypeptide having hyaluronidase activity is obtained or derived from at least one of *Cupiennius salei, Loxosceles intermedia, Hirudo nipponia, Bothrops atrox* or *Tityus serrulatus.*
**Item 7:** The recombinant cell according to any one of items 1 to 6, wherein the nucleic acid encoding a polypeptide having hyaluronan synthase activity is obtained or derived from at least one of *Streptococcus zooepidemicus, Chlorella virus PBCV1, Xenopus laevis* or *Pasteurella multocida,* and is in particular obtained or derived from at least one of *Streptococcus zooepidemicus, Chlorella virus PBCV1* or *Xenopus laevis.*
**Item 8:** The recombinant cell according to any one of items 1 to 7, wherein the nucleic acid encoding a polypeptide having UDP-Glucose dehydrogenase activity is obtained or derived from at least one of *Arabidopsis thaliana, Chlorella virus PBCV1* or *Streptococcus zooepidemicus,* and in particular from *Arabidopsis thaliana* or *Chlorella virus PBCV1.*
**Item 9**: The recombinant cell according to any one of items 1 to 8, wherein the recombinant cell comprises a recombinant nucleic acid encoding one or more of:
   (i) a polypeptide having glutamine-fructose-6-phosphate amidotransferase (GFA1) activity; and/or
   (ii) a polypeptide having UDP-N-acetylglucosamine pyrophosphorylase (QRI1) activity.
**Item 10:** The recombinant cell according to any one of items 1 to 9, wherein the recombinant cell comprises at least one recombinant nucleic acid encoding one or more of:
   (i) a polypeptide having Phosphoglucomutase-1 (PGM1) activity; and/or
   (ii) a polypeptide having UTP-glucose-1-phosphate uridylyltransferase (UGP1) activity; and/or
   (iii) a polypeptide having Glucosamine-6-phosphate N-acetyltransferase (GNA1) activity; and/or
   (iv) a polypeptide having phosphoacetylglucosamine mutase (PCM1) activity.
**Item 11:** The recombinant yeast cell according to any one of items 1 and 3 to 10, wherein the recombinant yeast cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens Ogataea polymorpha, Yarrowia lypolytica, Debaryomyces hansenii,* and *Ashbya gossypii,* and is preferably *Saccharomyces cerevisiae.*
**Item 12:** The recombinant host cell according to any one of items 2 to 10 wherein the recombinant host cell belongs to the *Saccharomycetales* order, in particular to the *Saccharomycetaceae* family, and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens Ogataea polymorpha, Yarrowia lypolytica, Debaryomyces hansenii,* and *Ashbya gossypii,* and is preferably *Saccharomyces cerevisiae.*
**Item 13**: A method of producing hyaluronic acid (HA) of a desired molecular weight (HAMW) comprising:
   (a) cultivating a recombinant cell as defined in any one of items 1 to 12 in a cultivation medium for a time sufficient to produce hyaluronic acid (HA) of the desired molecular weight; and
   (b) optionally isolating or recovering the hyaluronic acid (HA) from the recombinant cell and/or from the cultivation medium.
**Item 14:** The method according to item 13, wherein the HA has a molecular weight of from about 20kDa to about 50kDA, and preferably from about 20 kDa to about 30 kDa.
**Item 15:** The method according to item 14, wherein the HA has a molecular weight of from about about 30 kDa to about 50 kDa.
**Item 16:** The method according to item 13, wherein the molecular weight of the HA is of from about 50kDa to about 150 kDa.
**Item 17:** The method according to item 13, wherein the molecular weight of the HA is of from about 150kDa to about 1500 kDa.
**Item 18:** The method according to any one of items 13 to 17 wherein the recombinant cell comprises at least one recombinant nucleic acid encoding one or more of:
   (i) a polypeptide having glutamine-fructose-6-phosphate amidotransferase (GFA1) activity; and/or
   (ii) a polypeptide having UDP-N-acetylglucosamine pyrophosphorylase (QRI1) activity.
**Item 19:** The method according to any one of items 13 to 18, wherein the recombinant cell comprises at least one recombinant nucleic acid encoding one or more of:
   (i) a polypeptide having Phosphoglucomutase-1 (PGM1) activity;
   (ii) a polypeptide having UTP-glucose-1-phosphate uridylyltransferase (UGP1) activity;
   (iii) a polypeptide having Glucosamine-6-phosphate N-acetyltransferase (GNA1) activity; and/or
   (iv) a polypeptide having phosphoacetylglucosamine mutase (PCM1) activity.
**Item 20:** The method according to any one of items 13 to 19, wherein the recombinant cell is a member of the genus *Saccharomyces,* and in particular is *Saccharomyces cerevisiae.*
**Item 21:** The method according according to any one of items 13 to 20, wherein the time sufficient to produce hyaluronic acid (HA) of the desired molecular weight is a period of from about 35 hours to about 50 hours, preferably from about 40 hours to about 50 hours, preferably about 48 hours.
**Item 22:** The method according according to any one of items 13 to 21, wherein the molecular weight of the hyaluronic acid is controlled by regulating the pH of the cultivation medium.
**Item 23:** The method according to any one of idems 13 to 22, wherein the method is carried out on an industrial scale, preferably where the cultivation medium is at least about 100L, more preferably in the range of about 1000L to about 3000L, even more preferably about 10,000L or even more preferably about 100,000L or even about 250,000L.
**Item 24:** Hyaluronic acid (HA) obtainable from a recombinant cell of any one of items 1 to 10 or from the method according to any one of items 13 to 23.
**Item 25:** A cultivation medium comprising the HA according to item 24.
**Item 26:** A composition comprising the Hyaluronic acid (HA) according to item 24.
**Item 27:** An industrial product or a consumer product or a consumable comprising (i) the HA according to item 24, (ii) the cultivation medium of item 25 or (iii) the composition of item 26.
**Item 28:** The industrial product or the consumer product or the consumable of item 27, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product and/or a dental and/or an oral hygiene composition.
**Item 29:** Use of a recombinant cell as defined in any one of items 1 to 12 for the production of hyaluronic acid (HA) having a molecular weight in the range of from about 20kDa to about 50kDa or from about 50kDa to about 1000 kDa.
**Item 30:** A method for producing hyaluronic acid comprising the steps of:
   (a) culturing a recombinant yeast as defined in any one of items 1 and 3 to 11 in a culture medium; and
   (b) recovering the hyaluronic acid from said culture medium,
   wherein the hyaluronic acid recovered in step (b) has a molecular weight controlled through the selection of:
   - the nature and origin of the nucleic acid encoding the hyaluronidase of the recombinant yeast,
   - the nature and origin of the promoter controlling the expression of the nucleic acid encoding the hyaluronidase(s) of the recombinant yeast,
   - the presence of an anchoring and/or of a secretion signal associated to the encoded hyaluronidase(s) of the recombinant yeast,
   - the pH of the culture medium during the step of culturing the recombinant yeast, and/or
   - the duration of the culturing of the recombinant yeast.

Certain embodiments of the present invention may provide one or more of the following advantages:
- a process for producing Hyaluronic acid of controlled molecular weight;
- a process for producing Hyaluronic acid of controlled molecular weight in a *Saccharomyces* yeast cell; and
- a process for producing Hyaluronic acid of controlled molecular weight by varying genetic parameters (eg regulatory sequences) and/or process parameters (pH or fermentation time).

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### BRIEF DESCRIPTION OF THE FIGURES

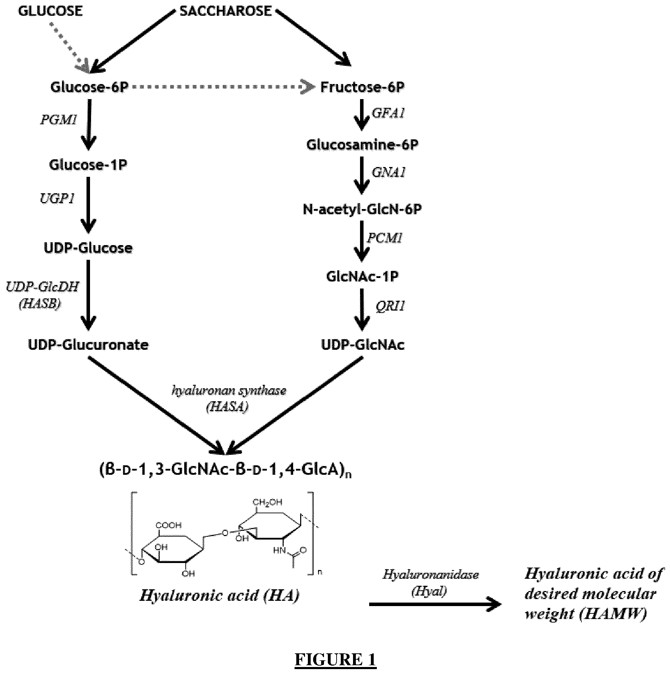

Figure 1 shows a schematic pathway for the production of hyaluronic acid
Figures 2A and 2B show agarose gels used to determine the molecular weight of the hyaluronic acid produced by strains according to the invention, (for example strain YA5235-1 (Figure 2A) and strain YA5359-10 (Figure 2B)). The agarose gels are obtained after running an aliquot of the supernatant of the considered strain and staining it with "stains all" (CAS Number 7423-31-6). HA molecular weight standards are present in each gel for reference.

### SUMMARY OF THE SEQUENCES

**SEQ ID NO: 1** is a reencoded nucleic acid sequence of hyaluronan synthase (HASA) originating from *Chlorella virus PBCV-1*
**SEQ ID NO: 2** is a reencoded nucleic acid sequence of hyaluronan synthase (HASA) originating from *Chlorella virus PBCV-1*
**SEQ ID NO: 3** is a reencoded nucleic acid sequence of hyaluronan synthase (HASA) originating from *Pasteurella multocida*
**SEQ ID NO: 4** is a reencoded nucleic acid sequence of hyaluronan synthase (HASA) originating from *Pasteurella multocida*
**SEQ ID NO: 5** is a reencoded nucleic acid sequence of hyaluronan synthase (HASA) originating from *Pasteurella multocida*
**SEQ ID NO: 6** is a reencoded nucleic acid sequence of hyaluronan synthase (HASA) originating from *Xenopus laevis*
**SEQ ID NO: 7** is a reencoded nucleic acid sequence of hyaluronan synthase (HASA) originating from *Streptococcus zooepidemicus*
**SEQ ID NO: 8** is an amino acid sequence of hyaluronan synthase (HASA) originating from *Chlorella virus PBCV-1*
**SEQ ID NO: 9** is an amino acid sequence of hyaluronan synthase (HASA) originating from *Pasteurella multocida*
**SEQ ID NO: 10** is an amino acid sequence of hyaluronan synthase (HASA) originating from *Xenopus laevis*
**SEQ ID NO: 11** is an amino acid sequence of hyaluronan synthase (HASA) originating from *Streptococcus zooepidemicus*
**SEQ ID NO: 12** is a nucleic acid sequence of UDP-Glucose dehydrogenase (HASB) originating from *Arabidopsis thaliana*
**SEQ ID NO: 13** is a reencoded nucleic acid sequence of UDP-Glucose dehydrogenase (HASB) originating from *Chlorella virus PBCV-1*
**SEQ ID NO: 14** is a reencoded nucleic acid sequence of UDP-Glucose dehydrogenase (HASB) originating from *Chlorella virus PBCV-1*
**SEQ ID NO: 15** is a reencoded nucleic acid sequence of UDP-Glucose dehydrogenase (HASB) originating from *Streptococcus zooepidemicus*
**SEQ ID NO: 16** is an amino acid sequence of UDP-Glucose dehydrogenase (HASB) originating from *Arabidopsis thaliana*
**SEQ ID NO: 17** is an amino acid sequence of UDP-Glucose dehydrogenase (HASB) originating from *Chlorella virus PBCV1*
**SEQ ID NO: 18** is an amino acid sequence of UDP-Glucose dehydrogenase (HASB) originating from *Streptococcus zooepidemicus*
**SEQ ID NO: 19** is a reencoded nucleic acid sequence of hyaluronidase (HYAL) originating from *Bothrops atrox* with a N-terminal secretion signal
**SEQ ID NO: 20** is a reencoded nucleic acid sequence of hyaluronidase (HYAL) originating from *Bothrops atrox* with a N-terminal secretion signal and a C-terminal anchoring signal
**SEQ ID NO: 21** is a reencoded nucleic acid sequence of hyaluronidase (HYAL) originating from *Cupiennius salei* with a N-terminal secretion signal
**SEQ ID NO: 22** is a reencoded nucleic acid sequence of hyaluronidase (HYAL) originating from *Cupiennius salei* with a N-terminal secretion signal and a C-terminal anchoring signal
**SEQ ID NO: 23** is a reencoded nucleic acid sequence of hyaluronidase (HYAL) originating from *Hirudo Nipponia* with a N-terminal secretion signal
**SEQ ID NO: 24** is a reencoded nucleic acid sequence of hyaluronidase (HYAL) originating from *Hirudo Nipponia* with a N-terminal secretion signal and a C-terminal anchoring signal
**SEQ ID NO: 25** is a reencoded nucleic acid sequence of hyaluronidase (HYAL) originating from *Loxosceles intermedia* with a N-terminal secretion signal
**SEQ ID NO: 26** is a reencoded nucleic acid sequence of hyaluronidase (HYAL) originating from *Loxosceles intermedia* with a N-terminal secretion signal and a C-terminal anchoring signal
**SEQ ID NO: 27** is a reencoded nucleic acid sequence of hyaluronidase (HYAL) originating from *Tityus serrulatus* with a N-terminal secretion signal
**SEQ ID NO: 28** is a reencoded nucleic acid sequence of hyaluronidase (HYAL) originating from *Tityus serrulatus* with a N-terminal secretion signal and a C-terminal anchoring signal
**SEQ ID NO: 29** is a reencoded nucleic acid sequence of hyaluronidase (HYAL) originating from *Vespa magnifica* with a N-terminal secretion signal
**SEQ ID NO: 30** is a reencoded nucleic acid sequence of hyaluronidase (HYAL) originating from *Vespa magnifica* with a N-terminal secretion signal and a C-terminal anchoring signal
**SEQ ID NO: 31** is an amino acid sequence of hyaluronidase (HYAL) originating from *Bothrops atrox* with a N-terminal secretion signal
**SEQ ID NO: 32** is an amino acid sequence of hyaluronidase (HYAL) originating from *Bothrops atrox* with a N-terminal secretion signal and a C-terminal anchoring signal
**SEQ ID NO: 33** is an amino acid sequence of hyaluronidase (HYAL) originating from *Cupiennius salei* with a N-terminal secretion signal
**SEQ ID NO: 34** is an amino acid sequence of hyaluronidase (HYAL) originating from *Cupiennius salei* with a N-terminal secretion signal and a C-terminal anchoring signal
**SEQ ID NO: 35** is an amino acid sequence of hyaluronidase (HYAL) originating from *Hirudo Nipponia* with a N-terminal secretion signal
**SEQ ID NO: 36** is an amino acid sequence of hyaluronidase (HYAL) originating from *Hirudo Nipponia* with a N-terminal secretion signal and a C-terminal anchoring signal
**SEQ ID NO: 37** is an amino acid sequence of hyaluronidase (HYAL) originating from *Loxosceles intermedia* with a N-terminal secretion signal
**SEQ ID NO: 38** is an amino acid sequence of hyaluronidase (HYAL) originating from *Loxosceles intermedia* with a N-terminal secretion signal and a C-terminal anchoring signal
**SEQ ID NO: 39** is an amino acid sequence of hyaluronidase (HYAL) originating from *Tityus serrulatus* with a N-terminal secretion signal
**SEQ ID NO: 40** is an amino acid sequence of hyaluronidase (HYAL) originating from *Tityus serrulatus* with a N-terminal secretion signal and a C-terminal anchoring signal
**SEQ ID NO: 41** is an amino acid sequence of hyaluronidase (HYAL) originating from *Vespa magnifica* with a N-terminal secretion signal
**SEQ ID NO: 42** is an amino acid sequence of hyaluronidase (HYAL) originating from *Vespa magnifica* with a N-terminal secretion signal and a C-terminal anchoring signal
**SEQ ID NO: 43** is a nucleic acid sequence of the secretion sequence added in 5'
**SEQ ID NO: 44** is an amino acid sequence of the secretion sequence added in N-ter
**SEQ ID NO: 45** is a nucleic acid sequence of the anchoring sequence added in 3'
**SEQ ID NO: 46** is an amino acid sequence of the anchoring sequence added in C-ter
**SEQ ID NO: 47** is a nucleic acid sequence of glutamine-fructose-6-phosphate amidotransferase (GFA1) originating from *Saccharomyces cerevisiae*
**SEQ ID NO: 48** is a reencoded nucleic acid sequence of glutamine-fructose-6-phosphate amidotransferase (GFA1) originating from *Chlorella virus 1* (PBCV-1)
**SEQ ID NO: 49** is a reencoded nucleic acid sequence of glutamine-fructose-6-phosphate amidotransferase (GFA1) originating from *Chlorella virus 1* (PBCV-1)
**SEQ ID NO: 50** is an amino acid sequence of glutamine-fructose-6-phosphate amidotransferase (GFA1) originating from *Saccharomyces cerevisiae*
**SEQ ID NO: 51** is an amino acid sequence of glutamine-fructose-6-phosphate amidotransferase (GFA1) originating from *Chlorella virus 1* (PBCV-1)
**SEQ ID NO: 52** is a nucleic acid sequence of UDP-N-acetylglucosamine pyrophosphorylase (QRI1) originating from *Saccharomyces cerevisiae*
**SEQ ID NO: 53** is an amino acid sequence of UDP-N-acetylglucosamine pyrophosphorylase (QRI1) originating from *Saccharomyces cerevisiae*
**SEQ ID NO: 54** is a nucleic acid sequence of Phosphoglucomutase-1 (PGM1) originating from *Saccharomyces cerevisiae*
**SEQ ID NO: 55** is an amino acid sequence Phosphoglucomutase-1 (PGM1) originating from *Saccharomyces cerevisiae*
**SEQ ID NO: 56** is a nucleic acid sequence of UTP--glucose- 1 -phosphate uridylyltransferase (UGP1) originating from *Saccharomyces cerevisiae*
**SEQ ID NO: 57** is an amino acid sequence of UTP--glucose-1-phosphate uridylyltransferase (UGP1) originating from *Saccharomyces cerevisiae*
**SEQ ID NO: 58** is a nucleic acid sequence of Glucosamine 6-phosphate N-acetyltransferase (GNA1) originating from *Saccharomyces cerevisiae*
**SEQ ID NO: 59** is an amino acid sequence of Glucosamine 6-phosphate N-acetyltransferase (GNA1) originating from *Saccharomyces cerevisiae*
**SEQ ID NO: 60** is a nucleic acid sequence of phosphoacetylglucosamine mutase (PCM1) originating from *Saccharomyces cerevisiae*
**SEQ ID NO: 61** is an amino acid sequence of phosphoacetylglucosamine mutase (PCM1) originating from *Saccharomyces cerevisiae*
**SEQ ID NO: 62** is the nucleic acid sequence of promotor pTDH3
**SEQ ID NO:63** is the nucleic acid sequence of promotor pTDH3.Sk
**SEQ ID NO: 64** is the nucleic acid sequence of promotor pTDH3-1.sba
**SEQ ID NO: 65** is the nucleic acid sequence of promotor pTDH3.Sar
**SEQ ID NO: 66** is the nucleic acid sequence of promotor pENO2
**SEQ ID NO: 67** is the nucleic acid sequence of promotor pTEF3
**SEQ ID NO: 68** is the nucleic acid sequence of promotor pTEF1
**SEQ ID NO: 69** is the nucleic acid sequence of promotor pTEF1.ago
**SEQ ID NO: 70** is the nucleic acid sequence of promotor pTEF1.Sba
**SEQ ID NO: 71** is the nucleic acid sequence of promotor pPDC1
**SEQ ID NO: 72** is the nucleic acid sequence of promotor pCCW12
**SEQ ID NO: 73** is the nucleic acid sequence of promotor pCCW12.Sm
**SEQ ID NO: 74** is the nucleic acid sequence of promotor pCCW12.Sk
**SEQ ID NO: 75** is the nucleic acid sequence of promotor pCCW12.Sba
**SEQ ID NO: 76** is the nucleic acid sequence of promotor pCCW12.Sar
**SEQ ID NO: 77** is the nucleic acid sequence of promotor pNUP57
**SEQ ID NO: 78** is the nucleic acid sequence of promotor pCCW10.ago
**SEQ ID NO: 79** is the nucleic acid sequence of promotor pCWP2
**SEQ ID NO: 80** is the nucleic acid sequence of promotor pRPLA1
**SEQ ID NO: 81** is the nucleic acid sequence of promotor pCUP1
**SEQ ID NO: 82** is the nucleic acid sequence of promotor pMET6
**SEQ ID NO: 83** is the nucleic acid sequence of promotor pMET25
**SEQ ID NO: 84** is the nucleic acid sequence of promotor pSAM1
**SEQ ID NO: 85** is the nucleic acid sequence of terminator tTPI1
**SEQ ID NO: 86** is the nucleic acid sequence of terminator tMET25
**SEQ ID NO:87** is the nucleic acid sequence of terminator tDIT1
**SEQ ID NO: 88** is the nucleic acid sequence of terminator tRPL3
**SEQ ID NO: 89** is the nucleic acid sequence of terminator tRPL3.sm
**SEQ ID NO: 90** is the nucleic acid sequence of terminator tRPL3.sba
**SEQ ID NO: 91** is the nucleic acid sequence of terminator tRPL41B
**SEQ ID NO: 92** is the nucleic acid sequence of terminator tRPL15A
**SEQ ID NO: 93** is the nucleic acid sequence of terminator tRPL15A.sba
**SEQ ID NO: 94** is the nucleic acid sequence of terminator tIDPl
**SEQ ID NO:95** is the nucleic acid sequence of terminator tTEF1.sba

### DETAILED DESCRIPTION OF THE INVENTION

The inventors have conceived genetically modified cells, and especially genetically modified yeasts, having the ability to produce hyaluronic acid, as compared to the parent cells, and especially as compared to the parent yeasts which are not naturally capable of doing so.

These genetically modified cells are described throughout the present specification.

### Definitions

The term "Hyaluronic acid" also known as hyaluronan or HA, is polysaccharide composed of D-glucuronic acid and N-acetyl-D-glucosamine, linked via alternating β-(1→4) and β-(1→3) glycosidic bonds and having the chemical formula (C₁₄H₂₁NO₁₁)n.

Hyalyuronic acid may be produced in a recombinant cell.

As used herein, the term "recombinant", when used in reference to a cell, indicates that the cell has been modified by the introduction of an endogenous and/or heterologous nucleic acid or protein into the cell or the alteration of a native cell or that the cell is derived from a cell so modified. Thus, for example, recombinant cells express genes or nucleic acid that are not found within the native (non-recombinant) form of the cell or express native (eg endogenous) genes at a different level than their native level or express additional or supplementary copies of native (eg endogenous) at a different level than their native level.

As used herein, the term "recombinant", when used in reference to a nucleic acid or vector, are sequences formed/obtained by technics of genetic engineering well known to the man skilled in the art. The guidelines of the US National Institute for Health (NIH) accordingly indicate that:

### "recombinant [...] nucleic acids are defined as:

### i. molecules that (a) are constructed by joining nucleic acid molecules and (b) that can replicate in a living cell, i.e., recombinant nucleic acids;",

which reflects the conventional use of the word "recombinant" attached to nucleic acid sequences to mean recombined following the insertion of, or joining together with, another nucleic acid.

Proteins that result from the expression of recombinant DNA or recombinant vector within living cells are also termed recombinant proteins.

The term "recombinant" is accordingly synonymous with the term "genetically modified". The term "gene" is synonymous with the term "nucleic acid" or "nucleotide sequence".
A recombinant nucleic acid sequence for use in a recombinant cell, and in particular a recombinant yeast, of the invention may be provided in the form of a nucleic acid construct. The term "nucleic acid construct" refers to as a nucleic acid molecule, either single-or doublestranded, which is isolated or derived from a native (eg endogenous) naturally occurring gene or is a heterologous nucleic acid or which has been modified to contain segments of nucleic acid which are combined and juxtaposed in a manner which would not otherwise exist in nature. The term "nucleic acid construct" is synonymous with the term "expression cassette" or "heterologous nucleic acid expression cassette" when the nucleic acid construct contains one or more regulatory elements required for expression of a coding sequence, wherein said control sequences are operably linked to said coding sequence. Non-limiting examples of regulatory elements include promoters, enhancers, silencers, terminators, and poly-A signals.

A recombinant nucleic acid sequence for use in a recombinant cell of the invention may be provided in the form of an expression vector, wherein the polynucleotide sequence is operably linked to at least one control sequence for the expression of the polynucleotide sequence in a recombinant cell.

The terms "obtained from" or "originate from" or "originating from" a microorganism or animal generally means that a substance (e.g., a nucleic acid molecule or polypeptide) originating from the microorganism or animal is native to that microorganism or animal.

The terms "derived from" a microorganism or animal generally means that a substance (e.g., a nucleic acid molecule or polypeptide) derived from the microorganism or animal is the result of modifications brought to the substance native to (i.e. present as such) in that microorganism or animal. For example, regarding a nucleic acid sequence derived from a microorganism or animal, said nucleic acid sequence can correspond to a reencoded and/or truncated version of the native nucleic acid sequence from this microorganism or animal. Other modifications well known to the man skilled in the art can be brought to the native substance of a microorganism or animal leading to the substance used being "derived from" said microorganism or animal.

As used herein, the term "polypeptide" refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity may be referred to as enzymes. It will be understood that, as a result of the degeneracy of the genetic code, a multitude of nucleotide sequences encoding a given polypeptide may be produced.

A polypeptide encoded by a recombinant nucleic acid for use in a recombinant cell, and in particular in a recombinant yeast, of the invention may comprise a signal peptide and/or a propeptide sequence. In the event that a polypeptide expressed by a recombinant cell, and in particular a recombinant yeast, of the invention comprises a signal peptide and/or a propeptide, sequence identity may be calculated over the mature polypeptide sequence.

The term "operably linked" as used herein refers to two or more nucleic acid sequence elements that are physically linked and are in a functional relationship with each other. For instance, a promoter is operably linked to a coding sequence if the promoter is able to initiate or regulate the transcription or expression of the coding sequence, in which case the coding sequence should be understood as being "under the control of" the promoter. Generally, when two nucleic acid sequences are operably linked, they will be in the same orientation and usually also in the same reading frame. They usually will be essentially contiguous, although this may not be required.

The term "native" or "endogenous" as used herein with reference to molecules, and in particular enzymes and nucleic acids, indicates molecules that are expressed in the organism in which they originated or are found in nature.

The term "endogenous gene" means that the gene was present in the cell before any genetic modification, in the wild-type strain. Endogenous genes may be overexpressed by introducing heterologous sequences in addition to, or to replace endogenous regulatory elements, or by introducing one or more additional or supplementary copies of the gene into the chromosome or a plasmid (said additional or supplementary copies being designated "exogenous or heterologous genes" or "heterologous nucleotide sequences" or "heterologous nucleic acids" as defined herein). Endogenous genes may also be modified to modulate their expression and/or activity. For example, mutations may be introduced into the coding sequence to modify the gene product or heterologous sequences may be introduced in addition to or to replace endogenous regulatory elements. Modulation of an endogenous gene may result in the up-regulation and/or enhancement of the activity of the gene product, or alternatively, in the down-regulation and/or attenuation of the activity of the endogenous gene product. Another way to enhance expression of endogenous genes is to introduce one or more additional or supplementary copies of the gene onto the chromosome or a plasmid (said supplementary copies being designated "exogenous or heterologous genes" or "heterologous nucleotide sequences" or heterologous nucleic acids" as defined herein).

By "one or more additional or supplementary copies of a gene" according to the invention, it is for example understood in the present invention from 1 to 50 copies, in particular from 1 to 30 copies, more particularly from 1 to 20 copies, and preferably from 1 to 10 copies. Said copies may be inserted into the same locus or into different loci of a recombinant cell of the invention.

The term "exogenous gene" means that the gene was introduced into a cell, by means well known to the man skilled in the art, whereas this gene is or is not naturally occurring in the wild-type cell. Cells can express exogenous genes if these genes are introduced into the cell with all the elements allowing their expression in the cell. Transforming cells with exogenous DNA is a routine task for the man skilled in the art. Exogenous genes may be integrated into the host chromosome, or be expressed extra-chromosomally from plasmids or vectors. A variety of plasmids, which differ with respect to their origin of replication and their copy number in the cell, are all known in the art. The sequence of exogenous genes may be adapted for its expression in the cell. Indeed, the man skilled in the art knows the notion of codon usage bias and how to adapt nucleic sequences for a particular codon usage bias without modifying the deduced protein. In particular embodiments, codon optimized genes express native enzymes.

The term "heterologous gene" or heterologous nucleic acid sequence" refers to a gene or nucleic acid sequence not normally found in a given cell in nature. As such, a heterologous nucleic acid sequence may be: (a) foreign to its host cell (i.e. is"exogenous" to the cell); (b) naturally found in the host cell (i.e. "endogenous") but present at an unnatural quantity in the cell (i.e., greater or lesser quantity than naturally found in the host cell); or (c) be naturally found in the host cell but positioned outside of its natural locus.

In the present application, all genes are referenced with their common names and with references to their nucleotide sequences and, the case arising, to their amino acid sequences. Using the references given in accession number for known genes, those skilled in the art are able to determine the equivalent genes in other organisms, bacterial strains, yeast, fungi, mammals, plants, etc. This routine work is advantageously done using consensus sequences that can be determined by carrying out sequence alignments with genes derived from other cells and designing degenerated probes to clone the corresponding gene in another organism.

The man skilled in the art knows different means to modulate, and in particular up-regulate or down-regulate, the expression of endogenous genes. For example, a way to enhance expression of, or over express, endogenous genes is to introduce one or more additional or supplementary copies of the gene onto the chromosome or a plasmid.

Another way is to replace the endogenous promoter of a gene with a stronger promoter. These promoters may be homologous or heterologous. Promoters particularly interesting in the present invention are described in more detail elsewhere in the present specification.

The nucleic acid expression construct may further comprise 5' and/or 3' recognition sequences and/or selection markers.

The term "inducible promoter" is used to qualify a promoter whose activity is induced, i.e. increased:
- in the presence of one or more particular metabolite(s). The higher the metabolite concentration in the medium, the stronger the promoter activity; or
- in the presence of a low concentration, or in the absence, of one or more metabolite(s). These metabolites are different from those whose increasing presence induces the activity of the promoter. The lower the metabolite concentration in the medium, the stronger the promoter activity.

The term "repressible promoter" is used to qualify a promoter whose activity is repressed, i.e. reduced:
- in the presence of one or more particular metabolite(s). The higher the metabolite concentration in the medium, the weaker the promoter activity; or
- in the presence of a low concentration, or in the absence, of one or more metabolite(s). These metabolites are different from those whose increasing presence represses the activity of the promoter. The lower the metabolite concentration in the medium, the weaker the promoter activity.

As used herein, the term "anchoring signal" when used in conjunction with a protein or polypeptide such as an enzyme (such as, for example hyaluronidase) means for example a first nucleic acid encoding a protein that is operably linked to a second nucleic acid encoding a protein or a polypeptide, or a first protein or polypeptide that is operably linked to a second protein or polypeptide, such as an enzyme (such as, for example hyaluronidase to form, for example a fusion protein), and that enables the cellular transport machinery of a cell, in particular of a S. *cerevisiae* cell, to correctly anchor and/or position in the membrane of the cell the second protein operably linked to the first protein.

As used herein, the term "secretion signal" when used in conjunction with a protein or polypeptide such as an enzyme (such as, for example hyaluronidase) means for example a first nucleic acid encoding a peptide or protein that is operably linked to a second nucleic acid encoding a protein, or a first protein that is linked to a second protein, such as an enzyme (such as, for example hyaluronidase to form, for example a fusion protein), and that enables the cellular transport machinery of a cell, in particular of a S. *cerevisiae* cell, to locate at least the second protein to the membrane of the cell and to secrete the second protein outside of the cell, after the first protein has, for example, been cleaved from the second protein.

As used herein the terms "secretion signal" and "anchoring signal" when used in conjunction with a protein or polypeptide such as an enzyme (such as, for example, hyaluronidase), mean for example a first nucleic acid encoding a peptide or protein that is operably linked to a second nucleic acid encoding a protein, or a first protein that is operably linked to a second protein, such as an enzyme (such as, for example hyaluronidase), and that enables the cellular transport machinery of a cell, in particular of a *S. cerevisiae* cell, to locate at least the second protein to the membrane of the cell wherein if the second protein is also operably linked to an "anchoring signal" the second protein is not secreted but remains attached to the membrane of the cell. In some cases, a secretion-anchoring signal may provide a dual secretion signal and anchoring signal function.

Sequences of secretion and anchoring signals, methods for the expression, anchoring and/or secretion of heterologous proteins, such as enzymes (such as, for example, hyaluronidase) on the surface of a cell (such, as for example, a yeast cell) are well known in the art (see for example, Ast et al (2013) Cell 152: 1134-1145, Ast and Schuldiner (2013) Crit Rev Biochem Mol Biol 48(3) 273-288, Van der Vaart et al (1997) Applied Environmental Microbiology 63(2) 615-620 and the entire contents of each of these publications are incorporated herein by reference).

The "activity" of an enzyme is used interchangeably with the term "function" and designates, in the context of the invention, the capacity of an enzyme to catalyze a desired reaction. The amount of an enzyme in a host cell may be altered by modifying the transcription of the gene that encodes the enzyme. This can be achieved for example by modifying the copy number of the nucleotide sequence encoding the enzyme (e.g., by using a higher or lower copy number expression vector comprising the nucleotide sequence, or by introducing additional copies of the nucleotide sequence into the genome of the host cell or by deleting or disrupting the nucleotide sequence in the genome of the host cell), by changing the order of coding sequences on a polycistronic mRNA of an operon or breaking up an operon into individual genes each with its own control elements, or by increasing the strength of the promoter or operator to which the nucleotide sequence is operably linked.

Alternatively or in addition, the copy number of an enzyme in a host cell may be altered by modifying the level of translation of an mRNA that encodes the enzyme. This can be achieved for example by modifying the stability of the mRNA, modifying the sequence of the ribosome binding site, modifying the distance or sequence between the ribosome binding site and the start codon of the enzyme coding sequence, modifying the entire intercistronic region located "upstream of" or adjacent to the 5' side of the start codon of the enzyme coding region, stabilizing the 3'-end of the mRNA transcript using hairpins and specialized sequences, modifying the codon usage of enzyme, altering expression of rare codon tRNAs used in the biosynthesis of the enzyme, and/or increasing the stability of the enzyme, as, for example, via mutation of its coding sequence.

The activity of an enzyme in a host cell can be altered in a number of ways, including, but not limited to, expressing a modified form of the enzyme that exhibits increased or decreased solubility in the host cell, expressing an altered form of the enzyme that lacks a domain through which the activity of the enzyme is inhibited, expressing a modified form of the enzyme that has a higher or lower Kcat or a lower or higher Km for the substrate, or expressing an altered form of the enzyme that is more or less affected by feed back or feed-forward regulation by another molecule in the pathway.

The terms "encoding" or "coding for" refer to the process by which a polynucleotide, through the mechanisms of transcription and translation, produces an amino-acid sequence.

The gene(s) encoding the enzyme(s) considered in the present invention can be exogenous or endogenous.

The methods implemented in the present invention preferably require the use of one or more chromosomal integration constructs for the stable introduction of a heterologous nucleotide sequence into a specific location on a chromosome or for the functional disruption of one or more target genes in a genetically modified cell. In some embodiments, disruption of a target gene prevents the expression of the related functional protein. In some embodiments, disruption of a target gene results in the expression of a non-functional protein from the disrupted gene.

Parameters of chromosomal integration constructs that may be varied in the practice of the present invention include, but are not limited to, the lengths of the homologous sequences; the nucleotide sequence of the homologous sequences; the length of the integrating sequence; the nucleotide sequence of the integrating sequence; and the nucleotide sequence of the target locus. In some embodiments, an effective range for the length of each homologous sequence is 20 to 5,000 base pairs, preferentially 50 to 100 base pairs. In particular embodiments, the length of each homologous sequence is about 50 base pairs. For more information on the length of homology required for gene targeting, see D. Burke et al., Methods in yeast Genetics - A cold spring harbor laboratory course Manual (2000).

In some embodiments, (a) disrupted gene(s) in which the above-mentioned DNA construct(s) is/are intended to be inserted may advantageously comprises one or more selectable markers useful for the selection of transformed cells. Preferably, said selectable marker(s) are comprised in the DNA construct(s) according to the present invention.

In some embodiments, the selectable marker is an antibiotic resistance marker. Illustrative examples of antibiotic resistance markers include, but are not limited to the, NAT1, AUR1-C, HPH, DSDA, KAN<R>, and SH BLE gene products. The NAT 1 gene product from *S*. *noursei* confers resistance to nourseothricin; the AUR1-C gene product from *Saccharomyces cerevisiae* confers resistance to Auerobasidin A (AbA); the HPH gene product of *Klebsiella pneumoniae* confers resistance to Hygromycin B; the DSDA gene product of *E. coli* allows cells to grow on plates with D-serine as the sole nitrogen source; the KAN<R> gene of the Tn903 transposon confers resistance to G418; and the SH BLE gene product from *Streptoalloteichus hindustanus* confers resistance to Zeocin (bleomycin).

In some embodiments, the antibiotic resistance marker is deleted after the genetically modified cell of the invention is isolated. The man skilled in the art is able to choose suitable marker in specific genetic context.

In a particular embodiment, a recombinant cell according to the invention is devoid of any antibiotic resistance marker. This advantageously prevents the necessity to add antibiotics in the selection medium.

In some embodiments, the selectable marker rescues an auxotrophy (e.g., a nutritional auxotrophy) in the genetically modified cell. In such embodiments, a parent cell, and in particular a parent yeast, comprises a functional disruption in one or more gene products that function in an amino acid or nucleotide biosynthetic pathway, such as, for example, the HIS3, LEU2, LYS1, LYS2, MET15, TRP1, ADE2, and URA3 gene products in yeast, which renders the parent cell incapable of growing in media without supplementation with one or more nutrients (auxotrophic phenotype). The auxotrophic phenotype can then be rescued by transforming the parent cell with a chromosomal integration encoding a functional copy of the disrupted gene product (in some embodimentsthe functional copy of the gene may originate from close species, such as *Kluveromyces, Candida* etc.), and the genetically modified cell generated can be selected based on the loss of the auxotrophic phenotype of the parent microbial cell.

For each of the nucleic acid sequences comprising a promoter sequence, a coding sequence (e.g. an enzyme coding sequence), or a terminator sequence, reference sequences are described herein. The present description also encompasses nucleic acid sequences having specific percentages of nucleic acid identity with a reference nucleic acid sequence.

For each or the amino acid sequences of interest, reference sequences are described herein. The present description also encompasses amino acid sequences (e.g. enzyme amino acid sequences), having specific percentages of amino acid identity with a reference amino acid sequence.

For obvious reasons, in all the present description, a specific nucleic acid sequence or a specific amino acid sequence which complies with, respectively, the considered nucleotide or amino acid identity, should further lead to obtaining a protein (or enzyme) which displays the desired biological activity. As used herein, the "percentage of identity" between two nucleic acid sequences or between two amino acid sequences is determined by comparing both optimally aligned sequences through a comparison window.

The portion of the nucleotide or amino-acid sequence in the comparison window may thus include additions or deletions (for example "gaps") as compared to the reference sequence (which does not include these additions or these deletions) so as to obtain an optimal alignment between both sequences.

The terms "sequence homology" or "sequence identity" or "homology" or "identity" are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

The "fermentation" or "culture" is generally conducted in fermenters with an appropriate culture medium adapted to the cell being cultivated, containing at least one simple carbon source, and if necessary co-substrates.

The term "fermentation composition" refers to a composition which comprises genetically modified host cells and products or metabolites produced by the genetically modified host cells. An example of a fermentation composition is a whole cell broth, which can be the entire contents of a vessel (e.g., a flasks, plate, or fermentor), including cells, aqueous phase, and compounds produced from the genetically modified host cells.

The term "medium" refers to a culture medium or cultivation medium or fermentation medium.

For maximal production of hyaluronic acid, the recombinant cells used as production hosts preferably have a high rate of carbohydrate utilization. These characteristics may be conferred by mutagenesis and selection, genetic engineering, or may be natural. Fermentation media, or "culture medium" or "cultivation medium", for the present cells may contain at least about 10 g/L of glucose and/or sucrose. Additional carbon substrates may include but are not limited to monosaccharides such as fructose, mannose, xylose and arabinose; oligosaccharides such as lactose, maltose, galactose, or sucrose; polysaccharides such as starch or cellulose or mixtures thereof and unpurified mixtures from renewable feedstocks such as cheese whey permeate cornsteep liquor, sugar beet molasses, and barley malt. Other carbon substrates may include glycerol, acetate and/or ethanol.

Hence, it is contemplated that the source of carbon utilized in the present invention may encompass a wide variety of carbon containing substrates and will only be limited by the choice of cell, and in particular of yeast.

Although it is contemplated that all of the above-mentioned carbon substrates and mixtures thereof are suitable in the present invention, preferred carbon substrates are glucose, fructose, and sucrose, or mixtures of these with C5 sugars such as xylose and/or arabinose for cells, and in particular yeasts, modified to use C5 sugars, and more particularly glucose.

Preferred carbon substrates are glucose or sucrose.

In addition to an appropriate carbon source, fermentation media may contain suitable minerals, salts, cofactors, buffers and other components, known to those skilled in the art, suitable for the growth of the cultures and promotion of the enzymatic pathway necessary for the production of the desired product.

Besides, additional genetic modifications suitable for the growth of recombinant cells according to the invention may be considered.

The terms "Aerobic conditions" refers to concentrations of oxygen in the culture medium that are sufficient for an aerobic or facultative anaerobic cell, and in particular yeast, to use di-oxygene as a terminal electron acceptor.

"Microaerobic condition" refers to a culture medium in which the concentration of oxygen is less than that in air, i.e. oxygen concentration up to 6% O₂.

An "appropriate culture medium" designates a medium (e.g. a sterile, liquid medium) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like. The term "carbon source" or "carbon substrate" or "source of carbon" according to the present invention denotes any source of carbon that can be used by those skilled in the art to support the normal growth of a cell, including hexoses (such as glucose, galactose or lactose), pentoses, monosaccharides, oligosaccharides, disaccharides (such as sucrose, cellobiose or maltose), molasses, starch or its derivatives, cellulose, hemicelluloses and combinations thereof.

Culture mediums that are particularly appropriate to produce recombinant cells of the invention, and in particular recombinant yeasts of the invention, are described in greater details further below in the text.

As used herein, the term "about" refers to a reasonable range about a value as determined by the practitioner of skill. In certain embodiments, the term about refers to ± one, two, or three standard deviations. In certain embodiments, the term about refers to ± 5%, 10%, 20%, or 25%. In certain embodiments, the term about refers to ± 0.1, 0.2, or 0.3 logarithmic units, e.g. pH units.

### General features of genetic modifications introduced according to the invention

- All the genome modifications are inserted in recombinant cells, and in particular recombinant yeasts, according to known genetic engineering techniques:
- The successive nucleic acid sequences included in a gene construct that is introduced in the recombinant cell genome according to the invention are of the following structure:
   Prom₁-ORF₁-term₁-ORF₂-gene₂-term₂ - .../... -Promₙ-ORFₙ-termₙ, wherein:
   - Prom1 is a sequence regulating the expression of the coding sequence ORF1,
   - ORF1 is a nucleic acid sequence encoding a desired protein PROT1, and especially a desired enzyme PROT1,
   - Term1 is a transcription terminator sequence that mediates transcriptional termination by providing signals in the newly synthesized mRNA that trigger processes which release the mRNA from the transcriptional complex, and
   - "1", "2", .../... "n" may or may not describe the same ORF (Open Reading Frame), promoter or terminator. The order of the nucleic acid sequences does not matter. "n" is an integer usually ranging from 5 and 20. These constructs are inserted in one of the recombinant cell chromosome at a controlled location. In some embodiments, the insertion site is neither essential for the functionality of the inserted construct, nor for the viability of the resulting genetically modified cell.

As will be understood by those of skill in the art, it can be advantageous to modify a coding sequence to enhance its expression in a particular host. The genetic code is redundant with 64 possible codons, but most organisms typically use a subset of these codons. The codons that are utilized most often in a species are called optimal codons, and those not utilized very often are classified as rare or low-usage codons. Codons can be substituted to reflect the preferred codon usage of the host, in a process sometimes called "codon optimization" or"controlling for species codon bias." Codon optimization for other host cells can be readily determined using codon usage tables or can be performed using commercially available software, such as CodonOp (www.idtdna.com/CodonOptfrom) from Integrated DNA Technologies. Optimized coding sequences containing codons preferred by a particular prokaryotic or eukaryotic host (Murray et al, 1989 , Nucl Acids Res . 17: 477-508) can be prepared, for example, to increase the rate of translation or to produce recombinant RNA transcripts having desirable properties, such as a longer half-life, as compared with transcripts produced from a non-optimized sequence. Translation stop codons can also be modified to reflect host preference. For example, typical stop codons for S. cerevisiae and mammals are UAA and UGA, respectively. The typical stop codon for monocotyledonous plants is UGA, whereas insects and E. coli commonly use UAA as the stop codon (Dalphin et al, 1996, Nucl Acids Res. 24: 216-8).
- When the recombinant cell is a yeast cell, and in particular is *Saccharomyces cerevisiae* yeast cell, nucleic acid sequences introduced in the yeast genome and originating from other organisms than *Saccharomyces cerevisiae* are generally "transcoded" (generally "codon-optimized"), meaning that these nucleic acid sequences are synthesized with an optimal codon usage for expression in *S. cerevisiae.* The nucleotide sequence (and not the protein sequence) of some nucleic acid sequences from *S. cerevisiae* has also been modified ("transcoded") to minimize recombination with an endogenous copy of the said gene.
- Genes may be deleted through standard procedures used in cell genetic engineering. In some embodiments, the genes targeted for deletion may be interrupted by insertion of one of the above described gene constructs, or alternatively the genes targeted for deletion are replaced by a short stretch of nucleotide.
- A nucleic acid sequences may be rendered "inducible or repressible" by deleting an endogenous copy of the nucleic acid sequences (if necessary) and placing a new copy of the ORF under the control of an inducible or repressible promoter. An inducible or repressible promoter is a promoter which activity is modulated or controlled, i.e. either increased or decreased, upon a change in the environmental conditions or external stimuli. Induction or repression may be artificially controlled, which encompasses induction or repression by abiotic factors such as chemical compounds not found naturally in the cell, and in particular yeast, of interest, light, oxygen levels, heat or cold. A list and sequences of inducible or repressible promoters are described elsewhere in the present specification.

### Recombinant cells according to the invention

The inventors have conceived recombinant cells, in particular recombinant yeasts, having an ability of producing hyaluronic acid.

The present invention relates to recombinant cells, and in particular recombinant yeasts, having the ability to produce hyaluronic acid, and wherein this ability to produce hyaluronic acid is obtained through a plurality of alterations that have been introduced in the genome thereof, by genetic engineering methods.

The present invention pertains to a recombinant yeast cell producing hyaluronic acid (HA), wherein the recombinant cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity; and
(c) one or more recombinant nucleic acids encoding a polypeptide having hyaluronidase activity wherein the polypeptide having hyaluronidase activity comprises a secretion signal so that hyaluronic acid, in particular of a desired molecular weight (HAMW), is produced by the recombinant yeast cell,
wherein said recombinant yeast cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus, and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens Ogataea polymorpha, Yarrowia lypolytica, Debaryomyces hansenii,* and *Ashbya gossypii,* and is preferably *Saccharomyces cerevisiae.*

The present invention further relates to a recombinant host cell producing hyaluronic acid (HA) wherein the recombinant host cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity; and
(c) one or more recombinant nucleic acids encoding a polypeptide having hyaluronidase activity wherein the polypeptide having hyaluronidase activity comprises a secretion signal and an anchoring signal so that hyaluronic acid, in particular of a desired molecular weight (HAMW) is produced by the host cell.

The inventors have found that an ability to produce hyaluronic acid by cells, and in particular yeast cells, may be reached by introducing in the genome of these cells a plurality of genetic alterations.

The production of hyaluronic acid by cells of the invention, and in particular by yeast cells of the invention, has been achieved by optimizing the endogenous metabolism of UDP-Glucose, and optionally UDP-N-Acetyl-glucosamine, and direct the subsequent artificially modified metabolic pathway mainly towards hyaluronic acid production while in the same time maintaining an optimal viability of the resulting genetically modified cells.

It has been determined that a hyaluronic acid production by recombinant cells according to the invention may be increased by increasing the conversion of glucose-6-phosphate into the successive intermediate metabolites (i) glucose-1-phosphate, UDP-glucose, UDP-glucuronate and hyaluronic acid and (ii) fructose-6-phosphate, glucosamine-6-phosphate, N-acetyl-glucosamine-6-phosphate, N-acetyl-glucosamine-1-phosphate, UDP-N-acetyl-glucosamine and hyaluronic acid, while maintaining a metabolic balance allowing a good viability of the resulting recombinant cells.

Indeed, in order to obtain a viable recombinant cell of the invention, many different constructs were tested in order to obtain a viable and efficient recombinant cell, and in particular a viable recombinant yeast. In particular such recombinant yeast were difficult to obtain because the temporary accumulation of some intermediates appeared to be toxic for yeasts.

Unexpected technical difficulties were encountered in establishing the conditions suitable for the preparation of a recombinant cell able to produce hyaluronic acid, and in particular to produce hyaluronic acid with a controlled molecular weight.

By "controlled" molecular weight of a hyaluronic acid of the invention is intended to mean that at least 80%, in particular at least 85%, of the hyaluronic acid produced by a method of the invention has a molecular weight comprised within a certain molecular weight's range through the regulation of at least one parameter of the method and/or of the recombinant cell of the invention, such as for example:
- the nature and origin of the nucleic acid encoding the hyaluronidase of the recombinant cell,
- the nature and origin of the promoter controlling the expression of the nucleic acid encoding the hyaluronidase(s) of the recombinant cell,
- the presence of an anchoring and/or of a secretion signal associated to the encoded hyaluronidase(s) of the recombinant cell,
- the pH of the culture medium during the step of culturing the recombinant cell, and/or
- the duration of the culturing of the recombinant cell.

Indeed, after much research and experimental trial, the inventors discovered that it was possible to cultivate a recombinant cell, and in particular a recombinant yeast cell, more particularly a recombinant *Saccharomyces cerevisiae* yeast cell, able to produce hyaluronic acid having a controlled molecular weight controlled using the following parameters:
- the selection of the nature and origin of the nucleic acid sequences encoding the polypeptide having hyaluronidase activity of the recombinant cell, in particular the recombinant yeast, of the invention; and/or
- the nature and origin of the promoter controlling the expression of the nucleic acid sequences encoding the polypeptide having hyaluronidase activity of the recombinant cell, in particular the recombinant yeast, of the invention; and/or
- the optional presence of an anchoring signal, in addition to a secretion signal, associated to the encoded polypeptide having hyaluronidase activity of the recombinant cell, in particular the recombinant yeast, according to the invention; and/or
- the pH of the culture medium during the step of culturing the recombinant cell, in particular the recombinant yeastc cell, according to the invention; and/or
- the duration of the culturing of the recombinant cell, in particular the recombinant yeast cell, according to the invention.

To the inventors' knowledge, this has never been achieved before.

The molecular weight of the hyaluronic acid (HA) can be in the range of less than 50kDa, preferably in the range of about 20kDa to about 50kDa.

Alternatively, the molecular weight of the hyaluronic acid (HA) can be in the range of greater than 50kDa, preferably in the range of about 50kDa to about 250kD.

In another alternative, the molecular weight of the HA can be in the range of greater than 100kDa, preferably in the range of about 100kDa to about 1500kDa.

The nucleic acid encoding a polypeptide having hyaluronidase activity in a recombinant cell of the invention is obtained or derived from at least one of *Cupiennius salei, Loxosceles intermedia, Hirudo nipponia, Bothrops atrox* or *Tityus serrulatus.*

The nucleic acid encoding a polypeptide having hyaluronan synthase activity in a recombinant cell of the invention may be obtained or derived from at least one of *Streptococcus zooepidemicus, Chlorella virus PBCV1, Xenopus laevis* or *Pasteurella multocida,* and is in particular obtained or derived from *Streptococcus zooepidemicus, Chlorella virus PBCV1* or *Xenopus laevis.*

The nucleic acid encoding a polypeptide having UDP-Glucose dehydrogenase activity in a recombinant cell of the invention may be obtained or derived from at least one of *Arabidopsis thaliana, Chlorella virus PBCV1* or *Streptococcus zooepidemicus,* and in particular from *Arabidopsis thaliana* or *Chlorella virus PBCV1.*

The recombinant cell according to the invention can comprise a recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

The recombinant cell according to the invention can comprise at least one recombinant nucleic acid encoding one or more of:
(i) a polypeptide having Phosphoglucomutase-1 (PGM1) activity; and/or
(ii) a polypeptide having UTP-glucose-1-phosphate uridylyltransferase (UGP1) activity; and/or
(iii) a polypeptide having Glucosamine-6-phosphate N-acetyltransferase (GNA1) activity; and/or
(iv) a polypeptide having phosphoacetylglucosamine mutase (PCM1) activity.

The recombinant yeast cell according to the invention can be in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

The recombinant host cell according to the invention can belong to the *Saccharomycetales* order and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens Ogataea polymorpha, Yarrowia lypolytica, Debaryomyces hansenii,* and *Ashbya gossypii,* and is preferably *Saccharomyces cerevisiae.*

Another object of the invention relates to a method of producing hyaluronic acid (HA) of a desired molecular weight (HAMW) comprising:
(a) cultivating a recombinant cell as defined above, i.e. a recombinant yeast cell or a recombinant host cell of the invention, in a cultivation medium for a time sufficient to produce hyaluronic acid (HA) of the desired molecular weight; and
(b) optionally isolating or recovering the hyaluronic acid (HA) from the recombinant cell and/or from the cultivation medium.

In the method of the invention, the HA may have a molecular weight of from about 20 kDa to about 50 kDA, and preferably from 20 kDa to 30 kDa.

In the method of the invention, the HA may alternatively have a molecular weight of from 30 kDa to 50 kDa.

In the method of the invention, the HA may alternatively have a molecular weight of from about 50 kDa to about 150 kDa.

In the method of the invention, the HA may alternatively have a molecular weight of from about 150 kDa to about 1500 kDa.

In a method of the invention, the nucleic acid encoding a polypeptide having hyaluronidase activity may be obtained or derived from *Cupiennius salei, Loxosceles intermedia, Hirudo nipponia, Bothrops atrox* or *Tityus serrulatus.*

In a method of the invention, the nucleic acid encoding a polypeptide having hyaluronan synthase activity may be obtained or derived from at least one of *Streptococcus zooepidemicus, Chlorella virus PBCV1, Xenopus laevis* or *Pasteurella multocida,* and is in particular obtained or derived from at least one of *Streptococcus zooepidemicus, Chlorella virus PBCV1* or *Xenopus laevis.*

In a method of the invention, the nucleic acid encoding a polypeptide having UDP-Glucose dehydrogenase activity may be obtained or derived from at least one of *Arabidopsis thaliana, Chlorella virus PBCV1* or *Streptococcus zooepidemicus,* and in particular obtained or derived from *Arabidopsis thaliana* or *Chlorella virus PBCV1.*

In the method of the invention, the recombinant cell may comprise at least one recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

In the method of the invention, the recombinant cell may comprise at least one recombinant nucleic acid encoding one or more of:
(i) a polypeptide having Phosphoglucomutase-1 (PGM1) activity;
(ii) a polypeptide having UTP-glucose-1-phosphate uridylyltransferase (UGP1) activity;
(iii) a polypeptide having Glucosamine-6-phosphate N-acetyltransferase (GNA1) activity; and/or
(iv) a polypeptide having phosphoacetylglucosamine mutase (PCM1) activity.

In the method of the invention, the recombinant cell may be a member of the genus *Saccharomyces,* and in particular is *Saccharomyces cerevisiae.*

In the method of the invention, the molecular weight of the hyaluronic acid may be controlled by the fermentation time.

In the method of the invention, the time sufficient to produce hyaluronic acid (HA) of a desired molecular weight may be a period of from about 35 hours to about 50 hours, preferably from about 40 hours to about 50 hours, preferably about 48 hours.

In the method of the invention, the molecular weight of the hyaluronic acid may be controlled by the pH of the cultivation medium.

In the method of the invention, the molecular weight of the hyaluronic acid may be controlled by regulating the pH of the cultivation medium during the cultivation step (a) of a method of the invention.

In the method of the invention, the molecular weight of the hyaluronic acid may be controlled by removing the biomass from the cultivation medium.

The method of the invention can be carried out on an industrial scale, preferably where the cultivation medium is at least about 100L, more preferably in the range of about 1000L to about 3000L, even more preferably about 10,000L or even more preferably about 100,000L, or even about 250,000L.

Another object of the invention relates to hyaluronic acid (HA) obtained or obtainable from a recombinant cell of the invention or from the method of the invention.

A further object of the invention relates to a cultivation medium comprising the hyaluronic acid (HA) according to the invention.

The present invention further relates to a composition comprising the hyaluronic acid (HA) according to the invention.

The present invention moreover relates to an industrial product or a consumer product or a consumable comprising (i) the HA having a molecular weight in the range of greater than 100kDa, preferably in the range of about 100kDa to about 1500kDa, (ii) the cultivation medium of the invention or (iii) the composition of the invention.

The present invention also relates to use of a recombinant cell according to the invention for the production of hyaluronic acid (HA) having a molecular weight in the range of from about 20kDa to about 50kDa or from about 50kDa to about 1000 kDa.

Another object of the present invention relates to a method for producing hyaluronic acid comprising the steps of:
(a) culturing a recombinant yeast according to the invention in a culture medium; and
(b) recovering the hyaluronic acid from said culture medium,
   wherein the hyaluronic acid recovered in step (b) has a molecular weight controlled through the selection of:
   - the nature and origin of the nucleic acid encoding the hyaluronidase of the recombinant yeast,
   - the nature and origin of the promoter controlling the expression of the nucleic acid encoding the hyaluronidase(s) of the recombinant yeast,
   - the presence of an anchoring and/or of a secretion signal associated to the encoded hyaluronidase(s) of the recombinant yeast,
   - the pH of the culture medium during the step of culturing the recombinant yeast, and/or
   - the duration of the culturing of the recombinant yeast.

More particularly, the invention pertains to a hyaluronic acid-producing recombinant yeast, wherein the recombinant yeast comprises:
(A) one or more recombinant nucleic acids encoding a polypeptide having glutamine-fructose-6-phosphate amidotransferase (GFA1) activity;
(B) one or more recombinant nucleic acids encoding a polypeptide having a UDP-N-acetylglucosamine pyrophosphorylase (QRI1) activity;
(C) one or more recombinant nucleic acids encoding a polypeptide having a UDP-Glucose dehydrogenase (UDP-GlcDH or HASB) activity;
(D) one or more recombinant nucleic acids encoding a polypeptide having a hyaluronan synthase (HASA) activity; and
(E) one or more recombinant nucleic acids encoding a polypeptide having a hyaluronidase activity wherein the polypeptide having hyaluronidase activity comprises a secretion signal so that hyaluronic acid, in particular of a desired molecular weight (HAMW), is produced by the recombinant yeast cell,
said recombinant yeast being *Saccharomyces cerevisiae.*

In a particular embodiment, the recombinant cell according to the invention, and in particular the recombinant yeast according to the invention comprises only one recombinant nucleic acid encoding a polypeptide having glutamine-fructose-6-phosphate amidotransferase (GFA1) activity.

In a particular embodiment, the recombinant cell according to the invention, and in particular the recombinant yeast comprises between 5 and 10 recombinant nucleic acids encoding a polypeptide having UDP-N-acetylglucosamine pyrophosphorylase (QRI1) activity.

In a particular embodiment, the recombinant cell according to the invention, and in particular the recombinant yeast comprises between 3 and 7 recombinant nucleic acids encoding a polypeptide having UDP-Glucose dehydrogenase (UDP-GlcDH or HASB) activity.

In another embodiment, the one or more nucleic acids encoding a polypeptide having a UDP-Glucose dehydrogenase (UDP-GlcDH or HASB) activity is obtained or derived from at least one of *Arabidopsis thaliana, Chlorella virus PBCV1* or *Streptococcus zooepidemicus,* and in particular obtained or derived from *Arabidopsis thaliana* or *Chlorella virus PBCV1.*

In a particular embodiment, the recombinant cell according to the invention, and in particular the recombinant yeast comprises between 4 and 8 recombinant nucleic acids encoding a polypeptide having hyaluronan synthase (HASA) activity.

In another embodiment, the one or more recombinant nucleic acids encoding a polypeptide having a hyaluronan synthase (HASA) activity is obtained or derived from at least one of *Streptococcus zooepidemicus, Chlorella virus PBCV1, Xenopus laevis* or *Pasteurella multocida,* and is in particular obtained or derived from *Streptococcus zooepidemicus, Chlorella virus PBCV1* or *Xenopus laevis.*

In a particular embodiment, the recombinant cell according to the invention, and in particular the recombinant yeast of the invention only comprises one recombinant nucleic acid encoding a polypeptide having hyaluronidase activity.

In another embodiment, the one or more recombinant nucleic acids encoding a polypeptide having a hyaluronidase activity is obtained or derived from *Cupiennius salei, Loxosceles intermedia, Hirudo nipponia, Bothrops atrox* or *Tityus serrulatus.*

In another embodiment, the recombinant cell according to the invention, and in particular the recombinant yeast cell may comprise at least one recombinant nucleic acid encoding one or more of:
(A) a polypeptide having Phosphoglucomutase-1 (PGM1) activity; and/or
(B) a polypeptide having UTP-glucose-1-phosphate uridylyltransferase (UGP1) activity; and/or
(C) a polypeptide having Glucosamine-6-phosphate N-acetyltransferase (GNA1) activity; and/or
(D) a polypeptide having phosphoacetylglucosamine mutase (PCM1) activity.

In particular, the recombinant cell according to the invention, and in particular the recombinant yeast cell of the invention comprises at least two, in particular at least three, and more particularly all of the modifications indicated above.

In a particular embodiment, the nucleic acid encoding a polypeptide having Phosphoglucomutase-1 (PGM1) activity, the nucleic acid encoding a polypeptide having UTP-glucose-1-phosphate uridylyltransferase (UGP1) activity, the nucleic acid encoding a polypeptide having glutamine-fructose-6-phosphate amidotransferase (GFA1) activity, the nucleic acid encoding a polypeptide having Glucosamine-6-phosphate N-acetyltransferase (GNA1) activity, the nucleic acid encoding a polypeptide having phosphoacetylglucosamine mutase (PCM1) activity and the nucleic acid encoding a polypeptide having UDP-N-acetylglucosamine pyrophosphorylase (QRI1) activity are nucleic acids originating or derivating from a yeast, preferably from *Saccharomyces cerevisiae.*

In a particular embodiment, the one or more recombinant nucleic acids encoding a polypeptide defined above and comprised in a recombinant cell according to the invention, and in particular comprised in the recombinant yeast of the invention are under the control of a promoter selected from the group consisting of pPDC1, pTDH3, pCCW12, pCCW12.Sm, pCCW12.sk, pCCW12.sba, pCCW12.sar, pTEF1, pENO2, pRPLA1, pNUP57 and pTEF3.

In a particular embodiment, the inducible or repressible promoters mentioned in the present specification are selected from the group consisting of promoters inducible or repressible with copper or promoters inducible or repressible with methionine, in particular selected from the group consisting of pMET6, pMET25 and pSAM1.

The invention further relates to a method for producing hyaluronic acid as previously described, and comprising the steps of:
(a) culturing a recombinant cell according to the invention, and in particular a recombinant yeast as defined herein in a culture medium; and
(b) recovering the hyaluronic acid from said culture medium,
   wherein the hyaluronic acid recovered in step (b) has a molecular weight controlled through the selection of:
   - the nature and origin of the nucleic acid encoding the hyaluronidase of the recombinant cell according to the invention, and in particular the recombinant yeast according to the invention, and/or
   - the nature and origin of the promoter controlling the expression of the nucleic acid encoding the hyaluronidase(s) of the recombinant cell according to the invention, and in particular the recombinant yeast, and/or
   - the presence of an anchoring and/or of a secretion signal associated to the encoded hyaluronidase(s) of the recombinant cell according to the invention, and in particular the recombinant yeast, and/or
   - the pH of the culture medium during the step of culturing the recombinant cell according to the invention, and in particular the recombinant yeast; and/or
   - the duration of the culturing of the recombinant cell according to the invention, and in particular the recombinant yeast.

### Recombinant nucleic acids encoding a polypeptide having hyaluronan synthase activity

A recombinant cell according to the invention, and in particular a recombinant yeast of the invention, comprises one or more recombinant nucleic acids encoding a polypeptide having hyaluronan synthase activity.

A polypeptide having hyaluronan synthase activity according to the invention means a polypeptide that converts the intermediate metabolites UDP-Glucoronate and UDP-N-acetylglucosamine (UDP-GlcNAc) into hyaluronic acid ((β-D-1,3-GlcNAc-β-D-1,4-GlcA)n).

In an embodiment, one or more of the recombinant nucleic acids encoding a polypeptide having hyaluronan synthase activity are under the control of an inducible or repressible promoter that is functional in the recombinant cells of the invention, such as for example the inducible or repressible promoters pCUP1 or pMET25.

One or more of the recombinant nucleic acids encoding a polypeptide having hyaluronan synthase activity may be under the control of a promoter selected from the group consisting of pCCW12, pCCW12.Sm, pTDH3-1.Sba and pTDH3.Sar.

The said one or more recombinant nucleic acids encoding a polypeptide having hyaluronan synthase activity may originate or be derived from at least one of *Streptococcus zooepidemicus (sz), Chlorella virus PBCV1 (Vir), Xenopus laevis (xl)* or *Pasteurella multocida (pm),* and may originate or derive in particular from *Streptococcus zooepidemicus, Chlorella virus PBCV1* or *Xenopus laevis,* as shown in the examples herein.

A recombinant cell according to the invention, and in particular a recombinant yeast according to the invention, may comprise between 2 and 8, in particular between 4 and 8 recombinant nucleic acids encoding a polypeptide having hyaluronan synthase (HASA) activity. A recombinant cell according to the invention, and in particular a recombinant yeast according to the invention, may for example comprise 2 or 6, and in particular 6, recombinant nucleic acids encoding a polypeptide having hyaluronan synthase (HASA) activity.

Illustratively, the one or more recombinant nucleic acids encoding a polypeptide having hyaluronan synthase activity may be inserted within the JLP1 gene and/or within the SAM3 gene and/or within the TRP1 gene and/or within the LYP1 gene of the recombinant cell, and in particular of the recombinant yeast, as it is shown in the examples herein.

In an embodiment of the invention, a recombinant cell, and in particular a recombinant yeast, of the invention comprises:
- between 2 and 8, in particular between 4 and 8 recombinant nucleic acids encoding a polypeptide having hyaluronan synthase (HASA) activity;
- said one or more recombinant nucleic acids encoding a polypeptide having hyaluronan synthase activity originating or being derived from at least one of *Streptococcus zooepidemicus (sz), Chlorella virus PBCV1 (Vir), Xenopus laevis (xl)* or *Pasteurella multocida (pm),* and originating or being derived in particular from at least one of *Streptococcus zooepidemicus, Chlorella virus PBCV1* or *Xenopus laevis; and*
- said one or more recombinant nucleic acids encoding a polypeptide having hyaluronan synthase activity being under the control of an inducible or repressible promoter that is functional in the recombinant cells of the invention, such as for example the inducible or repressible promoters pCUP or pMET25 and/or under the control of a promoter selected from the group consisting of pCCW12, pCCW12.Sm, pTDH3-1.Sba and pTDH3.Sar.

### Recombinant nucleic acids encoding a polypeptide having UDP-Glucose dehydrogenase activity

A recombinant cell according to the invention, and in particular a recombinant yeast according to the invention, comprises one or more recombinant nucleic acids encoding a polypeptide having UDP-Glucose dehydrogenase (UDP-GlcDH or HASB) activity.

A polypeptide having UDP-Glucose dehydrogenase (UDP-GlcDH or HASB) activity according to the invention means a polypeptide that converts the intermediate metabolite Uridine-Diphosphate-Glucose (UDP-Glucose) into UDP-Glucuronate.

In an embodiment, one or more of the recombinant nucleic acids encoding a polypeptide having UDP-Glucose dehydrogenase (UDP-GlcDH or HASB) activity are under the control of an inducible or repressible promoter that is functional in recombiant cells of the invention, such as for example the inducible or repressible promoters pMET25 or pMET6.

One or more of the recombinant nucleic acids encoding a polypeptide having UDP-Glucose dehydrogenase activity may be under the control of a promoter selected from the group consisting of pCCW12, in particular the pCCW12.sk and the pCCW12.sba promoters; pTEF1.Sba and pTDH3.Sk.

The one or more recombinant nucleic acids encoding a polypeptide having UDP-Glucose dehydrogenase activity may originate or be derived from at least one of *Arabidopsis thaliana, Chlorella virus PBCV1* or *Streptococcus zooepidemicus,* and in particular may originate or derive from *Arabidopsis thaliana* or *Chlorella virus PBCV1.*

A recombinant cell according to the invention, and in particular a recombinant yeast according to the invention, may comprise between 2 and 7, in particular between 3 and 7 recombinant nucleic acids encoding a polypeptide having UDP-Glucose dehydrogenase activity. A recombinant cell according to the invention, and in particular a recombinant yeast according to the invention, may for example comprise 2 or 5 recombinant nucleic acids encoding a polypeptide having UDP-Glucose dehydrogenase activity.

Illustratively, the one or more recombinant nucleic acids encoding a polypeptide having UDP-Glucose dehydrogenase activity may be inserted within the JLP1 gene and/or within the TRP1 gene, and/or within the LYP1 gene of the recombinant cell, and in particular of the recombinant yeast, as it is shown in the examples herein.

In an embodiment of the invention, a recombinant cell, and in particular a recombinant yeast, of the invention comprises:
- between 2 and 7, and in particular between 3 and 7 recombinant nucleic acids encoding a polypeptide having UDP-Glucose dehydrogenase activity;
- said one or more recombinant nucleic acids encoding a polypeptide having UDP-Glucose dehydrogenase activity originating or being derived from at least one of *Arabidopsis thaliana, Chlorella virus PBCV1* or *Streptococcus zooepidemicus,* and originating or being derived in particular from *Arabidopsis thaliana* or *Chlorella virus PBCV1*; and
- said one or more recombinant nucleic acids encoding a polypeptide having UDP-Glucose dehydrogenase activity being under the control of an inducible or repressible promoter that is functional in the recombinant cells of the invention, such as for example the inducible or repressible promoters pMET25 or pMET6 and/or under the control of a promoter selected from the group consisting of pCCW12, in particular the pCCW12.sk and the pCCW12.sba promoters; pTEF1.Sba and pTDH3.Sk.

### Recombinant nucleic acids encoding a polypeptide having Hyaluronidase activity

A recombinant cell according to the invention, and in particular a recombinant yeast according to the invention, comprises one or more recombinant nucleic acids encoding a polypeptide having hyaluronidase activity.

A polypeptide having hyaluronidase activity according to the invention means a polypeptide that degrades hyaluronic acid, i.e. that converts a hyaluronic acid of a given molecular weight into a hyaluronic acid of a lower molecular weight.

As previously indicated, polypeptides having hyaluronidase activity of the present invention comprise a secretion signal.

In an embodiment, polypeptide having hyaluronidase activity comprise both a secretion signal and an anchoring signal.

In an embodiment, the said one or more recombinant nucleic acids encoding a polypeptide having hyaluronidase activity are under the control of an inducible or repressible promoter that is functional in the recombinant cells of the invention.

One or more of the recombinant nucleic acids encoding a polypeptide having hyaluronidase activity may be under the control of a promoter selected from the group consisting of pTEF1, pCCW12, pCCW12.sba, pCCW12.Sar, pPDC1, pTEF3, pTDH3, pNUP57, pCWP2 and pCCW10.ago.

The said one or more recombinant nucleic acids encoding a polypeptide having hyaluronidase activity may originate or be derived from at least one of *Cupiennius salei (Csa), Loxosceles intermedia (Li), Hirudo nipponia (Hn), Bothrops atrox (Ba)* or *Tityus serrulatus (Ts)* as shown in the examples herein.

A recombinant cell according to the invention, and in particular a recombinant yeast according to the invention, may comprise only one recombinant nucleic acid encoding a polypeptide having hyaluronidase activity.

Illustratively, the one or more recombinant nucleic acids encoding a polypeptide having hyaluronidase activity may be inserted within the JLP1 gene and/or within the LYP1 gene, as it is shown in the examples herein.

In an embodiment of the invention, a recombinant cell, and in particular a recombinant yeast, of the invention comprises:
- only one recombinant nucleic acid encoding a polypeptide having hyaluronidase activity;
- said recombinant nucleic acid encoding a polypeptide having hyaluronidase activity originating or being derived from *Cupiennius salei (Csa), Loxosceles intermedia (Li), Hirudo nipponia (Hn), Bothrops atrox (Ba)* or *Tityus serrulatus (Ts);*
- said recombinant nucleic acid encoding a polypeptide having hyaluronidase activity comprising either (i) a secretion signal and no anchoring signal or (ii) a secretion signal and an anchoring signal; and
- said recombinant nucleic acid encoding a polypeptide having hyaluronidase activity being under the control of a promoter selected from the group consisting of pTEF1, pCCW12, pCCW12.sba, pCCW12.Sar, pPDC1, pTEF3, pTDH3, pNUP57, pCWP2 and pCCW10.ago.

### Recombinant nucleic acids encoding a polypeptide having Glutamine-fructose-6-phosphate amidotransferase activity

A recombinant cell according to the invention, and in particular a recombinant yeast cell according to the invention, may comprise one or more recombinant nucleic acids encoding a polypeptide having glutamine-fructose-6-phosphate amidotransferase activity.

A polypeptide having glutamine-fructose-6-phosphate amidotransferase activity according to the invention means a polypeptide that converts fructose-6-phosphate into glucosamine-6-phosphate.

In an embodiment, one or more of the recombinant nucleic acids encoding a polypeptide having glutamine-fructose-6-phosphate amidotransferase activity are under the control of an inducible or repressible promoter that is functional in the recombinant cells of the invention.

One or more of the recombinant nucleic acids encoding a polypeptide having glutamine-fructose-6-phosphate amidotransferase activity may be under the control of a promoter selected from the group consisting of pTEF1 and pTEF1.Ago.

The said one or more recombinant nucleic acids encoding a polypeptide having glutamine-fructose-6-phosphate amidotransferase activity can originate or be derived from *Saccharomyces cerevisiae,* as shown in the examples herein.

A recombinant cell according to the invention, and in particular a recombinant yeast cell according to the invention, may comprise only one recombinant nucleic acid encoding a polypeptide having glutamine-fructose-6-phosphate amidotransferase activity.

Illustratively, the one or more recombinant nucleic acids encoding a polypeptide having glutamine-fructose-6-phosphate amidotransferase activity may be inserted within the SAM3 gene of the recombinant cell, and in particular of the recombinant yeast cell, as it is shown in the examples herein.

In an embodiment of the invention, a recombinant cell, and in particular a recombinant yeast cell, of the invention comprises:
- only one recombinant nucleic acid encoding a polypeptide having glutamine-fructose-6-phosphate amidotransferase activity;
- said recombinant nucleic acid encoding a polypeptide having glutamine-fructose-6-phosphate amidotransferase activity originating or being derived from *Saccharomyces cerevisiae;* and
- said recombinant nucleic acid encoding a polypeptide having glutamine-fructose-6-phosphate amidotransferase activity being under the control of a promoter selected from the group consisting of pTEF1 and pTEF1.Ago.

### Recombinant nucleic acids encoding a polypeptide having UDP-N-acetylglucosamine pyrophosphorylase activity

A recombinant cell according to the invention, and in particular a recombinant yeast cell according to the invention, can comprise one or more recombinant nucleic acids encoding a polypeptide having UDP-N-acetylglucosamine pyrophosphorylase activity.

A polypeptide having UDP-N-acetylglucosamine pyrophosphorylase activity according to the invention means a polypeptide that can convert N-acetyl-glucosamine into UDP-N-acetyl-glucosamine.

In an embodiment, one or more of the recombinant nucleic acids encoding a polypeptide having UDP-N-acetylglucosamine pyrophosphorylase activity are under the control of an inducible or repressible promoter that is functional in the recombinant cells of the invention, such as for example the inducible or repressible promoters pMET6 or pCUP1.

One or more of the recombinant nucleic acids encoding a polypeptide having UDP-N-acetylglucosamine pyrophosphorylase activity may be under the control of the promoter pTDH3.

The said one or more recombinant nucleic acids encoding a polypeptide having UDP-N-acetylglucosamine pyrophosphorylase activity can originate or be derived from *Saccharomyces cerevisiae,* as shown in the examples herein.

A recombinant cell according to the invention, and in particular a recombinant yeast according to the invention, may comprise between 5 and 10 recombinant nucleic acids encoding a polypeptide having UDP-N-acetylglucosamine pyrophosphorylase activity. A recombinant cell according to the invention, and in particular a recombinant yeast cell according to the invention, may for example comprise 5, 7 or 8 recombinant nucleic acids encoding a polypeptide having UDP-N-acetylglucosamine pyrophosphorylase activity.

Illustratively, the one or more recombinant nucleic acids encoding a polypeptide having UDP-N-acetylglucosamine pyrophosphorylase activity may be inserted within the HIS3 gene and/or within the JLP1 gene and/or within the SAM3 gene of the recombinant cell, and in particular of the recombinant yeast, as it is shown in the examples herein.

In an embodiment of the invention, a recombinant cell, and in particular a recombinant yeast cell, of the invention comprises:
- between 5 and 10 recombinant nucleic acids encoding a polypeptide having UDP-N-acetylglucosamine pyrophosphorylase activity;
- said one or more recombinant nucleic acids encoding a polypeptide having UDP-N-acetylglucosamine pyrophosphorylase activity originating or being derived from *Saccharomyces cerevisiae;*
- said one or more recombinant nucleic acids encoding a polypeptide having UDP-N-acetylglucosamine pyrophosphorylase activity being under the control of an inducible or repressible promoter that is functional in the recombinant cells of the invention, such as for example the inducible or repressible promoters pMET6 or pCUP1 and/or under the control of the promoter pTDH3.

### Recombinant nucleic acids encoding a polypeptide having Phosphoglucomutase-1 activity

A recombinant cell according to the invention, and in particular a recombinant yeast cell according to the invention, may comprise one or more recombinant nucleic acids encoding a polypeptide having Phosphoglucomutase-1 (PGM1) activity.

A polypeptide having Phosphoglucomutase-1 (PGM1) activity according to the invention means a polypeptide that converts Glucose-6-phosphate into the intermediate metabolite Glucose-1-phosphate.

In an embodiment, one or more of the recombinant nucleic acids encoding a polypeptide having Phosphoglucomutase-1 (PGM1) activity are under the control of an inducible or repressible promoter that is functional in recombiant cells of the invention, such as for example the inducible or repressible promoters.

One or more of the recombinant nucleic acids encoding a polypeptide having Phosphoglucomutase-1 (PGM1) activity may be under the control of the promoter pPDC1.

The one or more recombinant nucleic acids encoding a polypeptide having Phosphoglucomutase-1 (PGM1) activity may originate or be derived from *Saccharomyces cerevisiae,* as shown in the examples herein.

A recombinant cell according to the invention, and in particular a recombinant yeast cell according to the invention, may comprise only one recombinant nucleic acid encoding a polypeptide having Phosphoglucomutase-1 (PGM1) activity.

Illustratively, the one or more recombinant nucleic acids encoding a polypeptide having Phosphoglucomutase-1 activity may be inserted within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having Phosphoglucomutase-1 activity;
- said recombinant nucleic acid encoding a polypeptide having Phosphoglucomutase-1 activity originating or being derived from *Saccharomyces cerevisiae;* and
- said recombinant nucleic acid encoding a polypeptide having Phosphoglucomutase-1 activity being under the control of the promoter pPDC1.

### Recombinant nucleic acids encoding a polypeptide having UTP-glucose-1-phosphate uridylyltransferase activity

A recombinant cell according to the invention, and in particular a recombinant yeast cell according to the invention, comprises one or more recombinant nucleic acids encoding a polypeptide having UTP-glucose-1-phosphate uridylyltransferase (UGP1) activity.

A polypeptide having UTP-glucose-1-phosphate uridylyltransferase (UGP1) activity according to the invention means a polypeptide that converts the intermediate metabolite Glucose-1-Phosphate into the intermediate metabolite UDP-glucose.

In an embodiment, one or more of the recombinant nucleic acids encoding a polypeptide having UTP-glucose-1-phosphate uridylyltransferase activity are under the control of an inducible or repressible promoter that is functional in recombiant cells of the invention, such as for example the inducible or repressible promoters pSAM1 or pCUP1.

One or more of the recombinant nucleic acids encoding a polypeptide having UTP-glucose-1-phosphate uridylyltransferase activity may be under the control of a promoter selected from the group consisting of pPDC1 and pENO2.

The one or more recombinant nucleic acids encoding a polypeptide having UTP-glucose-1-phosphate uridylyltransferase activity can originate or be derived from *Saccharomyces cerevisiae,* as shown in the examples herein.

A recombinant cell according to the invention, and in particular a recombinant yeast cell according to the invention, can comprise between 5 and 10 recombinant nucleic acids encoding a polypeptide having UTP-glucose-1-phosphate uridylyltransferase activity. A recombinant cell according to the invention, and in particular a recombinant yeast cell according to the invention, may for example comprise 5, 7 or 8 recombinant nucleic acids encoding a polypeptide having UTP-glucose-1-phosphate uridylyltransferase activity.

Illustratively, the one or more recombinant nucleic acids encoding a polypeptide having UTP-glucose-1-phosphate uridylyltransferase activity gene may be inserted within the HIS3 gene and/or within the JLP1 gene and/or within the SAM3 gene of the recombinant cell, and in particular of the recombinant yeast, as it is shown in the examples herein.

In an embodiment of the invention, a recombinant cell, and in particular a recombinant yeast cell, of the invention comprises:
- between 5 and 10 recombinant nucleic acids encoding a polypeptide having UTP-glucose-1-phosphate uridylyltransferase activity;
- said one or more recombinant nucleic acids encoding a polypeptide having UTP-glucose-1-phosphate uridylyltransferase activity originating or being derived from *Saccharomyces cerevisiae;* and
- said one or more recombinant nucleic acids encoding a polypeptide having being under the control of an inducible or repressible promoter that is functional in the recombinant cells of the invention, such as for example the inducible or repressible promoters pSAM1 or pCUP1 and/or under the control of a promoter selected from the group consisting of pPDC1 and pENO2.

### Recombinant nucleic acids encoding a polypeptide having Glucosamine-6-phosphate N-acetyltransferase activity

A recombinant cell according to the invention, and in particular a recombinant yeast cell according to the invention, can comprise one or more recombinant nucleic acids encoding a polypeptide having Glucosamine-6-phosphate N-acetyltransferase (GNA1) activity.

A polypeptide having Glucosamine-6-phosphate N-acetyltransferase (GNA1) activity according to the invention means a polypeptide that can convert Glucosamine-6-phosphate into N-acetyl-glucosamine-6-phosphate.

In an embodiment, one or more of the recombinant nucleic acids encoding a polypeptide having Glucosamine-6-phosphate N-acetyltransferase activity are under the control of an inducible or repressible promoter that is functional in the recombinant cells of the invention.

One or more of the recombinant nucleic acids encoding a polypeptide having Glucosamine-6-phosphate N-acetyltransferase activity may be under the control of the promoter pCWP2.

The said one or more recombinant nucleic acids encoding a polypeptide having Glucosamine-6-phosphate N-acetyltransferase activity may originate or be derived from *Saccharomyces cerevisiae,* as shown in the examples herein.

A recombinant cell according to the invention, and in particular a recombinant yeast cell according to the invention, can comprise only one recombinant nucleic acid encoding a polypeptide having Glucosamine-6-phosphate N-acetyltransferase activity.

Illustratively, the one or more recombinant nucleic acids encoding a polypeptide having Glucosamine-6-phosphate N-acetyltransferase activity may be inserted within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having Glucosamine-6-phosphate N-acetyltransferase activity;
- said recombinant nucleic acid encoding a polypeptide having Glucosamine-6-phosphate N-acetyltransferase activity originating or being derived from *Saccharomyces cerevisiae;* and
- said recombinant nucleic acid encoding a polypeptide having Glucosamine-6-phosphate N-acetyltransferase activity being under the control of the promoter pCWP2.

### Recombinant nucleic acids encoding a polypeptide having Phosphoacetylglucosamine mutase activity

A recombinant cell according to the invention, and in particular a recombinant yeast cell according to the invention, can comprise one or more recombinant nucleic acids encoding a polypeptide having phosphoacetylglucosamine mutase (PCM1) activity.

A polypeptide having phosphoacetylglucosamine mutase (PCM1) activity according to the invention means a polypeptide that can convert N-acetyl-glucosamine-6-phosphate N-acetyl-glucosamine-1-phosphate.

In an embodiment, one or more of the recombinant nucleic acids encoding a polypeptide having phosphoacetylglucosamine mutase activity are under the control of an inducible or repressible promoter that is functional in the recombinant cells of the invention.

One or more of the recombinant nucleic acids encoding a polypeptide having phosphoacetylglucosamine mutase activity may be under the control a promoter selected from the group consisting of pTEF3 and pTEF1.

The said one or more recombinant nucleic acids encoding a polypeptide having phosphoacetylglucosamine mutase activity can originate or be derived from *Saccharomyces cerevisiae,* as shown in the examples herein.

A recombinant cell according to the invention, and in particular a recombinant yeast cell according to the invention, may comprise only one recombinant nucleic acid encoding a polypeptide having phosphoacetylglucosamine mutase activity.

Illustratively, the one or more recombinant nucleic acids encoding a polypeptide having phosphoacetylglucosamine mutase activity may be inserted within the SAM3 gene of the recombinant cell, and in particular of the recombinant yeast, as it is shown in the examples herein.

In an embodiment of the invention, a recombinant cell, and in particular a recombinant yeast cell, of the invention comprises:
- only one recombinant nucleic acid encoding a polypeptide having phosphoacetylglucosamine mutase activity;
- said recombinant nucleic acid encoding a polypeptide having phosphoacetylglucosamine mutase activity originating or being derived from *Saccharomyces cerevisiae;* and
- said recombinant nucleic acid encoding a polypeptide having phosphoacetylglucosamine mutase activity being under the control of the promoter a promoter selected from the group consisting of pTEF3 and pTEF1.

### HYALURONAN SYNTHASE (HASA)

The hyaluronan synthase enzyme is a protein which is described in the art for catalyzing the conversion of UDP-glucuronate or UDP-N-acetyl-glucose into hyaluronic acid. The hyaluronan synthase originating from *Streptococcus zooepidemicus, Chlorella virus PBCV1, Xenopus laevis* or *Pasteurella multocida* may be termed HASA.

A method implemented to measure the activity level of a polypeptide having hyaluronan synthase activity belongs to the general knowledge of the one skilled in the art.

In this regard, the one skilled in the art may advantageously refer to the method of colorimetric determination after treatment with concentrated sulfuric acid and carbazole described by Bitter and Muir (Analytical Biochemistry, 4, 330-334, 1962).

Preferred polypeptide having hyaluronan synthase activity in the present invention is an enzyme having an EC number of n° 2.4.1.212.

According to a preferred embodiment, the one or more recombinant nucleic acids encoding a polypeptide having hyaluronan synthase activity may originate or be derived from organisms preferably selected in a group consisting of prokaryotic organisms and eukaryotic organisms. In some embodiments, the one or more recombinant nucleic acids encoding a polypeptide having hyaluronan synthase activity may originate or be derived from archaebacteria. In some embodiments, the one or more recombinant nucleic acids encoding a polypeptide having hyaluronan synthase activity may originate or be derived from bacteria, and especially from *Streptococcus zooepidemicus (Sz), Chlorella virus PBCV1 (Vir), Xenopus laevis* (X1) or *Pasteurella multocida (Pm).*

According to a yet preferred embodiment, the one or more recombinant nucleic acids encoding a polypeptide having hyaluronan synthase activity may be selected from the group consisting of nucleic acid sequences having (i) at least 65%, advantageously at least 70%, preferably at least 80%, nucleic acid identity with a nucleic acid sequence as set forth as sequence SEQ ID NO: 1 (Vir), SEQ ID NO: 2 (Vir), SEQ ID NO: 3 (Pm), SEQ ID NO: 4 (Pm), SEQ ID NO: 5 (Pm), SEQ ID NO: 6 (X1) or SEQ ID NO: 7 (sz) and (ii) a biological activity of the same nature as the nucleic acid sequence having, respectively, the nucleic acid sequence as set forth as sequence SEQ ID NO: 1 (Vir), SEQ ID NO: 2 (Vir), SEQ ID NO: 3 (Pm), SEQ ID NO: 4 (Pm), SEQ ID NO: 5 (Pm), SEQ ID NO: 6 (X1) or SEQ ID NO: 7 (sz).

A biological activity of the same nature regarding this sequence is, as previously explained, the capacity to code for a polypeptide that converts UDP-glucuronate or UDP-N-acetyl-glucose into hyaluronic acid.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having hyaluronan synthase activity originating from *Streptococcus zooepidemicus, Chlorella virus PBCV1, Xenopus laevis* or *Pasteurella multocida,* the one skilled in the art may refer, respectively, to the accession numbers B4U0D4, Q84419, P13563 and Q7BLV3 in the UniProt database, or to SEQ ID NO: 8 (Vir), SEQ ID NO: 9 (Pm) SEQ ID NO: 10 (X1) or SEQ ID NO:11 (Sz) described herein.

According to another particular embodiment, the one or more recombinant nucleic acids encoding a polypeptide having hyaluronan synthase activity may be nucleic acid(s) encoding polypeptide having an amino acid sequence selected from the group consisting of sequences having at least 50%, advantageously at least 65%, preferably at least 80%, amino acid identity with the amino acid sequence set forth as SEQ ID NO: 8 (Vir), SEQ ID NO: 9 (Pm), SEQ ID NO: 10 (X1) or SEQ ID NO:11 (Sz), and also a biological activity of the same nature as the amino acid sequence set forth as SEQ ID NO: 8 (Vir), SEQ ID NO: 9 (Pm), SEQ ID NO: 10 (X1) or SEQ ID NO:11 (Sz).

A biological activity of the same nature regarding this sequence is as described previously, i.e. the capacity to catalyze the conversion of UDP-glucuronate or UDP-N-acetyl-glucose into hyaluronic acid.

As described herein, an amino acid sequence having at least 50% amino acid identity with a reference amino acid sequence encompasses amino acid sequences having at least 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having hyaluronan synthase activity in the present invention is regulated by at least one promoter and at least one terminator, such as herein after defined more in details, which are present in 5' and 3' position respectively of the one or more recombinant nucleic acids encoding a polypeptide having hyaluronan synthase activity.

### UDP-GLUCOSE DEHYDROGENASE (UDP-GlcDH OR HASB)

The UDP-Glucose dehydrogenase is a protein which is known in the art to catalyze the conversion of UDP-glucose into UDP-glucuronate. The UDP-Glucose dehydrogenase originating from the genome of *Arabidopsis thaliana, Chlorella virus PBCV1* or *Streptococcus zooepidemicus* may be termed HASB.

A method implemented to measure the activity level of a polypeptide having UDP-Glucose dehydrogenase activity belongs to the general knowledge of the one skilled in the art.

In this regard, the one skilled in the art may advantageously refer to the method described by Oka and Jigami (FEBS Journal 273, 2645-2657, 2006).

Preferred polypeptide having UDP-Glucose dehydrogenase activity in the present specification is an enzyme having an EC number 1.1.1.22.

According to a preferred embodiment, the one or more recombinant nucleic acids encoding a polypeptide having UDP-Glucose dehydrogenase activity may originate or be derived from organisms preferably selected in a group comprising prokaryotic organisms and eukaryotic organisms. In some embodiments, the one or more recombinant nucleic acids encoding a polypeptide having UDP-Glucose dehydrogenase activity may originate or be derived from archaebacteria. In some preferred embodiments, the one or more recombinant nucleic acids encoding a polypeptide having UDP-Glucose dehydrogenase activity may originate or be derived from yeast, and especially from *Arabidopsis thaliana, Chlorella virus PBCV1* or *Streptococcus zooepidemicus haromyces.*

According to a yet preferred embodiment, the one or more recombinant nucleic acids encoding a polypeptide having UDP-Glucose dehydrogenase activity may be selected from the group consisting of nucleic acid sequences having (i) at least 65%, advantageously at least 70%, preferably at least 80%, nucleic acid identity with a nucleic acid as set forth as sequence SEQ ID NO: 12 (At), SEQ ID NO. 13 (Vir), SEQ ID NO. 14 (Vir) or SEQ ID NO: 15 (Sz), and (ii) a biological activity of the same nature as the nucleic acid as set forth as sequence SEQ ID NO: 12 (At), SEQ ID NO. 13 (Vir), SEQ ID NO. 14 (Vir) or SEQ ID NO: 15 (Sz). The nucleic acids set forth as sequences SEQ ID NO: 12 (At), SEQ ID NO: 13 (Vir), SEQ ID NO. 14 (Vir) and SEQ ID NO: 15 (Sz) encode a polypeptide having UDP-Glucose dehydrogenase activity originating, respectively, from *Arabidopsis thaliana (At), Chlorella virus PBCV1 (Vir)* or *Streptococcus zooepidemicus (Sz),* that may herein also be collectively termed HASB.

A biological activity of the same nature regarding this sequence is, as previously explained, the capacity to code for a polypeptide that converts UDP-glucose into UDP-glucuronate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequences.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequences.

For the amino acid sequence of the polypeptide having UDP-Glucose dehydrogenase activity from *Arabidopsis thaliana, Chlorella virus PBCV1* or *Streptococcus zooepidemicus,* the one skilled in the art may refer to the accession numbers NP_173979.1, NP_048965 or KIS19289, respectively, in the UniProt database, or to the sequences as set forth in SEQ ID NO: 16 (At), SEQ ID NO. 17 (Vir) and SEQ ID NO: 18 (Sz) described herein.

According to another particular embodiment, the one or more recombinant nucleic acids encoding a polypeptide having UDP-Glucose dehydrogenase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 16 (At), SEQ ID NO. 17 (Vir) and SEQ ID NO: 18 (Sz), and also a biological activity of the same nature as the amino acid sequence of SEQ ID NO: 16 (At), SEQ ID NO. 17 (Vir) and SEQ ID NO: 18 (Sz).

A biological activity of the same nature regarding this sequence is as described previously, i.e. the capacity to catalyze the conversion of UDP-glucose into UDP-glucuronate.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UDP-Glucose dehydrogenase activity in the present invention is regulated by at least one promoter and at least one terminator, such as herein after defined more in details, which are present in 5' and 3' position respectively of the one or more recombinant nucleic acids encoding a polypeptide having UDP-Glucose dehydrogenase activity.

### HYALURONIDASE (HYAL)

The hyaluronidase enzyme is a protein which is described in the art for catalyzing the degradation of hyaluronic acid molecules into smaller hyaluronic acid molecules. The hyaluronidase originating from *Cupiennius salei, Loxosceles intermedia, Hirudo nipponia, Bothrops atrox, Tityus serrulatus* or *Vespa magnifica* may be termed HYAL.

The polypeptide having hyaluronidase activity of the invention may possess both a secretion signal and an anchoring signal or a secretion signal and no anchoring signal or a secretion-anchor signal with a dual secretion and anchoring function. When the encoded polypeptide having hyaluronidase activity possesses both a secretion signal and an anchoring signal, it may be termed HYAL-31 as represented in the examples. When the encoded polypeptide having hyaluronidase activity possesses a secretion signal and no anchoring signal, it may be termed HYAL-3 as represented in the examples.

A method implemented to measure the activity level of a polypeptide having hyaluronidase activity belongs to the general knowledge of the one skilled in the art.

In this regard, the one skilled in the art may advantageously monitor the molecular weight of the obtained hyaluronic acid on an agarose gel.

Preferred polypeptide having hyaluronidase activity in the present specification is an enzyme having an EC number of n° EC 3.2.1.35.

According to a preferred embodiment, the one or more recombinant nucleic acids encoding a polypeptide having hyaluronidase activity may originate or be derived from organisms preferably selected in a group comprising prokaryotic organisms and eukaryotic organisms. In some embodiments, the one or more recombinant nucleic acids encoding a polypeptide having hyaluronidase activity may originate or be derived from archaebacteria. In some embodiments, the one or more recombinant nucleic acids encoding a polypeptide having hyaluronidase activity may originate or be derived from organisms preferably selected from yeasts. In some other preferred embodiments, the one or more recombinant nucleic acids encoding a polypeptide having hyaluronidase activity may originate or be derived from *Cupiennius salei (Csa), Loxosceles intermedia (Li), Hirudo nipponia (Hn), Bothrops atrox (Ba), Tityus serrulatus (Ts) or Vespa magnifica (Vm), and in particular from Cupiennius salei (Csa), Loxosceles intermedia (Li), Hirudo nipponia (Hn), Bothrops atrox (Ba) and Tityus serrulatus (Ts).*

According to a yet preferred embodiment, the one or more recombinant nucleic acids encoding a polypeptide having hyaluronidase activity may be selected from the group consisting of nucleic acid sequences having at least 65%, advantageously at least 70%, preferably at least 80%, nucleic acid identity with a nucleic acid of SEQ ID NO: 19 (Ba), SEQ ID NO: 25 (Li), SEQ ID NO: 21 (Csa), SEQ ID NO: 27 (Ts), SEQ ID NO: 23 (Hn), or SEQ ID NO: 29 (Vm) and also a biological activity of the same nature as the nucleic acid of SEQ ID NO: 19 (Ba), SEQ ID NO: 25 (Li), SEQ ID NO: 21 (Csa), SEQ ID NO: 27 (Ts), SEQ ID NO: 23 (Hn), or SEQ ID NO: 29 (Vm). The nucleic acid of SEQ ID NO: 19 (Ba), SEQ ID NO: 25 (Li), SEQ ID NO: 21 (Csa), SEQ ID NO: 27 (Ts), SEQ ID NO: 23 (Hn) or SEQ ID NO: 29 (Vm) encode a polypeptide having hyluronidase activity, comprise a secretion signal and no anchoring signal, and originate or derive from *Bothrops atrox, Loxosceles intermedia, Cupiennius salei, Tityus serrulatus, Hirudo nipponia* or *Vespa magnifica,* respectively.

According to another preferred embodiment, the one or more recombinant nucleic acids encoding a polypeptide having hyaluronidase activity may be selected from the group consisting of nucleic acid sequences having at least 65%, advantageously at least 70%, preferably at least 80%, nucleic acid identity with a nucleic acid of SEQ ID NO: 22 (Csa), SEQ ID NO: 26 (Li), SEQ ID NO: 24 (Hn), SEQ ID NO: 20 (Ba), SEQ ID NO: 28 (Ts) or SEQ ID NO: 30 (Vm), and also a biological activity of the same nature as the nucleic acid of SEQ ID NO: 22 (Csa), SEQ ID NO: 26 (Li), SEQ ID NO: 24 (Hn), SEQ ID NO: 20 (Ba), SEQ ID NO: 28 (Ts) or SEQ ID NO: 30 (Vm). The nucleic acid of SEQ ID NO: 22 (Csa), SEQ ID NO: 26 (Li), SEQ ID NO: 24 (Hn), SEQ ID NO: 20 (Ba) SEQ ID NO: 28 (Ts) or SEQ ID NO: 30 (Vm) encode a polypeptide having hyluronidase activity, comprise a secretion signal and an anchoring signal, and originate or derive from *Cupiennius salei, Loxosceles intermedia, Hirudo nipponia, Bothrops atrox, Tityus serrulatus* or *Vespa magnifica,* respectively.

In a particular embodiment, the one or more recombinant nucleic acids encoding a polypeptide having hyaluronidase activity may be selected from the group consisting of nucleic acid sequences (i) having at least 65%, advantageously at least 70%, preferably at least 80%, nucleic acid identity with a nucleic acid of SEQ ID NO: 19 (Ba), SEQ ID NO: 25 (Li), SEQ ID NO: 21 (Csa), SEQ ID NO: 27 (Ts), or SEQ ID NO: 23 (Hn) and (ii) having a biological activity of the same nature as the nucleic acid of SEQ ID NO: 19 (Ba), SEQ ID NO: 25 (Li), SEQ ID NO: 21 (Csa), SEQ ID NO: 27 (Ts), or SEQ ID NO: 23 (Hn).

In a particular embodiment, the one or more recombinant nucleic acids encoding a polypeptide having hyaluronidase activity may be selected from the group consisting of nucleic acid sequences having (i) at least 65%, advantageously at least 70%, preferably at least 80%, nucleic acid identity with a nucleic acid of SEQ ID NO: 22 (Csa), SEQ ID NO: 26 (Li), SEQ ID NO: 24 (Hn), SEQ ID NO: 20 (Ba), or SEQ ID NO: 28 (Ts) and (ii) having a biological activity of the same nature as the nucleic acid of SEQ ID NO: 22 (Csa), SEQ ID NO: 26 (Li), SEQ ID NO: 24 (Hn), SEQ ID NO: 20 (Ba), or SEQ ID NO: 28 (Ts).

A biological activity of the same nature regarding this sequence is, as previously explained, the capacity to code for a polypeptide that catalyzes the degradation of hyaluronic acid molecules into smaller hyaluronic acid molecules.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the one or more recombinant nucleic acids encoding a polypeptide having hyaluronidase activity from *Cupiennius salei, Loxosceles intermedia, Hirudo nipponia, Bothrops atrox* or *Tityus serrulatus,* the one skilled in the art may refer to the accession numbers A0A0S4JYH2, R4J7Z9, X4Y2L4, A0A2H4Z8F4 or P85841, respectively in the UniProt database, or to SEQ ID NO: 33 (Csa), SEQ ID NO: 37 (Li), SEQ ID NO: 35 (Hn), SEQ ID NO: 31 (Ba) SEQ ID NO: 39 (Ts) or SEQ ID NO: 41 (Vm), described herein.

According to another particular embodiment, the one or more recombinant nucleic acids encoding a polypeptide having hyaluronidase activity may be nucleic acid(s) encoding polypeptide having an amino acid sequence selected from the group consisting of sequences having at least 50%, advantageously at least 65%, preferably at least 80%, amino acid identity with the amino acid sequence of SEQ ID NO: 33 (Csa), SEQ ID NO: 37 (Li), SEQ ID NO: 35 (Hn), SEQ ID NO: 31 (Ba), SEQ ID NO: 39 (Ts) or SEQ ID NO: 41 (Vm) which comprise a secretion signal and no anchoring signal, and also a biological activity of the same nature as the amino acid sequence SEQ ID NO: 33 (Csa), SEQ ID NO: 37 (Li), SEQ ID NO: 35 (Hn), SEQ ID NO: 31 (Ba), SEQ ID NO: 39 (Ts) or SEQ ID NO: 41 (Vm).

According to another particular embodiment, the one or more recombinant nucleic acids encoding a polypeptide having hyaluronidase activity may be nucleic acid(s) encoding polypeptide having an amino acid sequence selected from the group consisting of sequences having at least 55%, advantageously at least 65%, preferably at least 80%, amino acid identity with the amino acid sequence of SEQ ID NO: 34 (Csa), SEQ ID NO: 38 (Li), SEQ ID NO: 36 (Hn), SEQ ID NO: 32 (Ba), SEQ ID NO: 40 (Ts) or SEQ ID NO: 42 (Vm) which comprise a secretion signal and an anchoring signal, and also a biological activity of the same nature as the amino acid sequence of SEQ ID NO: 34 (Csa), SEQ ID NO: 38 (Li), SEQ ID NO: 36 (Hn), SEQ ID NO: 32 (Ba), SEQ ID NO: 40 (Ts) or SEQ ID NO: 42 (Vm).

In a particular embodiment, the one or more recombinant nucleic acids encoding a polypeptide having hyaluronidase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 33 (Csa), SEQ ID NO: 37 (Li), SEQ ID NO: 35 (Hn), SEQ ID NO: 31 (Ba), or SEQ ID NO: 39 (Ts), and also a biological activity of the same nature as the amino acid sequence of SEQ ID NO: 33 (Csa), SEQ ID NO: 37 (Li), SEQ ID NO: 35 (Hn), SEQ ID NO: 31 (Ba), or SEQ ID NO: 39 (Ts).

In another particular embodiment, the one or more recombinant nucleic acids encoding a polypeptide having hyaluronidase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 34 (Csa), SEQ ID NO: 38 (Li), SEQ ID NO: 36 (Hn), SEQ ID NO: 32 (Ba) or SEQ ID NO: 40 (Ts), and also a biological activity of the same nature as the amino acid sequence of SEQ ID NO: 34 (Csa), SEQ ID NO: 38 (Li), SEQ ID NO: 36 (Hn), SEQ ID NO: 32 (Ba) or SEQ ID NO: 40 (Ts).

A biological activity of the same nature regarding this sequence is as described previously, i.e. the capacity to catalyze the degradation of hyaluronic acid molecules into smaller hyaluronic acid molecules.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having hyaluronidase activity in the present invention is regulated by at least one promoter and at least one terminator, such as herein after defined more in details, which are present in 5' and 3' position respectively of the one or more recombinant nucleic acids encoding a polypeptide having hyaluronidase activity.

### GLUTAMINE-FRUCTOSE-6-PHOSPHATE AMIDOTRANSFERASE (GFA1)

The glutamine-fructose-6-phosphate amidotransferase enzyme is a protein which is described in the art for catalyzing the conversion of fructose-6-phosphate into glucosamine-6-phosphate. The glutamine-fructose-6-phosphate amidotransferase originating from *Saccharomyces cerevisiae* may be termed GFA1.

A method implemented to measure the activity level of a polypeptide having glutamine-fructose-6-phosphate amidotransferase activity belongs to the general knowledge of the one skilled in the art.

In this regard, the one skilled in the art may advantageously refer to the method described by Shiga Shibatan and Hiroaki Kitazawa (Plant Biotechnology 26, 149-152, 2009).

Preferred polypeptide having glutamine-fructose-6-phosphate amidotransferase activity in the present invention is an enzyme having an EC number of n° EC 2.6.1.16.

According to a preferred embodiment, the one or more recombinant nucleic acids encoding a polypeptide having glutamine-fructose-6-phosphate amidotransferase activity may originate or be derived from organisms preferably selected in a group comprising prokaryotic organisms and eukaryotic organisms. In some embodiments, the one or more recombinant nucleic acids encoding a polypeptide having glutamine-fructose-6-phosphate amidotransferase activity may originate or be derived from archaebacteria. In some embodiments, the one or more recombinant nucleic acids encoding a polypeptide having glutamine-fructose-6-phosphate amidotransferase activity may originate or be derived from organisms preferably selected from *Bacillus subtilis,* and yeasts. In some other preferred embodiments, the one or more recombinant nucleic acids encoding a polypeptide having glutamine-fructose-6-phosphate amidotransferase activity may originate or be derived from a yeast, and especially from *Saccharomyces cerevisiae.*

According to a yet preferred embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 47 (Sc), and (ii) a biological activity of the same nature as the nucleic acid sequence as set forth as sequence SEQ ID NO: 47 (Sc). The nucleic acid as set forth as sequence SEQ ID NO: 47 encodes a polypeptide having glutamine-fructose-6-phosphate amidotransferase activity originating from *Saccharomyces cerevisiae,* that may also be termed GFA1.

According to yet another embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 48 or SEQ ID NO: 49, and (ii) a biological activity of the same nature as the nucleic acid sequence as set forth as sequence SEQ ID NO: 48 or SEQ ID NO: 49. The nucleic acid sequence as set forth as sequence SEQ ID NO: 48 or SEQ ID NO: 49 encodes a polypeptide having glutamine-fructose-6-phosphate amidotransferase activity originating from *Chlorella virus PBCV1.*

A biological activity of the same nature regarding this sequence is, as previously explained, the capacity to code for a polypeptide that converts fructose-6-phosphate into glucosamine-6-phosphate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having glutamine-fructose-6-phosphate amidotransferase activity originating from *Saccharomyces cerevisiae,* the one skilled in the art may refer to the accession number NP8012818 in the UniProt database, or to the sequence SEQ ID NO: 50 described herein.

For the amino acid sequence of the polypeptide having glutamine-fructose-6-phosphate amidotransferase activity originating from *Chlorella virus PBCV1*, the one skilled in the art may also refer to the accession number NP_048448 in the UniProt database, or to the sequence SEQ ID NO: 51 described herein.

According to another particular embodiment, the one or more recombinant nucleic acids encoding a polypeptide having glutamine-fructose-6-phosphate amidotransferase activity may be nucleic acid(s) encoding polypeptide having an amino acid sequence selected from the group consisting of sequences having at least 35%, advantageously at least 65%, preferably at least 80%, amino acid identity with the amino acid sequence of SEQ ID NO: 50 or with SEQ ID NO: 51, and also a biological activity of the same nature as the amino acid sequence of SEQ ID NO: 50 or with SEQ ID NO: 51.

A biological activity of the same nature regarding this sequence is as described previously, i.e. the capacity to catalyze the conversion of of fructose-6-phosphate into glucosamine-6-phosphate.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the polypeptide having glutamine-fructose-6-phosphate amidotransferase activity in the present invention is regulated by at least one promoter and at least one terminator, such as herein after defined more in details, which are present in 5' and 3' position respectively of one or more recombinant nucleic acids encoding a polypeptide having glutamine-fructose-6-phosphate amidotransferase activity.

### UDP-N-ACETYLGLUCOSAMINE PYROPHOSPHORYLASE (QRI1)

The UDP-N-acetylglucosamine pyrophosphorylase enzyme is a protein which is described in the art for catalyzing the conversion of N-acetyl-glucosmine-6-phosphate into UDP-N-acetyl-glucose. The UDP-N-acetylglucosamine pyrophosphorylase originating from *Saccharomyces cerevisiae* may be termed QRI1.

A method implemented to measure the activity level of a polypeptide having UDP-N-acetylglucosamine pyrophosphorylase activity belongs to the general knowledge of the one skilled in the art.

In this regard, the one skilled in the art may advantageously refer to the method described by Mio et al. (The Journal of Biological Chemistry, Col. 273, No 23, June 5, 1998, 14392-14397) except that UDP-N-acetyl-glucosamine is detected by LC MS/MS using a Synergi RP Fusion column.

Preferred polypeptide having UDP-N-acetylglucosamine pyrophosphorylase activity in the present invention is an enzyme having an EC number of n° EC 2.7.7.23

According to a preferred embodiment, the one or more recombinant nucleic acids encoding a polypeptide having UDP-N-acetylglucosamine pyrophosphorylase activity may originate or be derived from organisms preferably selected in a group consisting of prokaryotic organisms and eukaryotic organisms. In some embodiments, the one or more recombinant nucleic acids encoding a polypeptide having UDP-N-acetylglucosamine pyrophosphorylase activity may originate or be derived from archaebacteria. In some embodiments, the one or more recombinant nucleic acids encoding a polypeptide having UDP-N-acetylglucosamine pyrophosphorylase activity may originate or be derived from organisms preferably selected from *Bacillus subtilis*, and yeasts. In some other preferred embodiments, the one or more recombinant nucleic acids encoding a polypeptide having UDP-N-acetylglucosamine pyrophosphorylase activity may originate or be derived from yeasts, and especially from *Saccharomyces cerevisiae.*

According to a yet preferred embodiment, the one or more recombinant nucleic acids encoding a polypeptide having UDP-N-acetylglucosamine pyrophosphorylase activity may be selected from the group consisting of nucleic acid sequences having (i) at least 65%, advantageously at least 70%, preferably at least 80%, nucleic acid identity with a nucleic acid sequence as set forth as SEQ ID NO: 52, and (ii) a biological activity of the same nature as the nucleic acid sequence as set forth as SEQ ID NO: 52. The nucleic acid sequence set forth as SEQ ID NO: 52 encodes a polypeptide having UDP-N-acetylglucosamine pyrophosphorylase activity originating from *Saccharomyces cerevisiae,* that may also be termed QRI1.

A biological activity of the same nature regarding this sequence is, as previously explained, the capacity to code for a polypeptide that converts N-acetyl-glucosmine-6-phosphate into UDP-N-acetyl-glucose.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having UDP-N-acetylglucosamine pyrophosphorylase activity originating from *Saccharomyces cerevisiae,* the one skilled in the art may refer to the accession number NP_010180 in the UniProt database, or to SEQ ID NO. 53 described herein.

According to another particular embodiment, the one or more recombinant nucleic acids encoding a polypeptide having UDP-N-acetylglucosamine pyrophosphorylase activity may be nucleic acid(s) encoding polypeptide having an amino acid sequence selected from the group consisting of sequences having at least 35%, advantageously at least 45%, preferably at least 80%, amino acid identity with the amino acid sequence set forth as sequence SEQ ID NO. 53, and also a biological activity of the same nature as the amino acid sequence set forth as sequence SEQ ID NO. 53.

A biological activity of the same nature regarding this sequence is as described previously, i.e. the capacity to catalyze the conversion of of N-acetyl-glucosmine-6-phosphate into UDP-N-acetyl-glucose.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 45% amino acid identity with a reference amino acid sequence encompasses amino acid sequences having at least 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UDP-N-acetylglucosamine pyrophosphorylase activity in the present invention is regulated by at least one promoter and at least one terminator, such as herein after defined more in details, which are present in 5' and 3' position respectively of the one or more recombinant nucleic acids encoding a polypeptide having UDP-N-acetylglucosamine pyrophosphorylase activity.

### PHOSPHOGLUCOMUTASE-1 (PGM1)

The Phosphoglucomutase-1 enzyme is a protein which is described in the art for catalyzing the conversion of Glucose-6-phosphate into Glucose-1-phosphate. The Phosphoglucomutase-1 originating from *Saccharomyces cerevisiae* may be termed PGM1.

A method implemented to measure the activity level of a polypeptide facing Phosphoglucomutase-1 activity belongs to the general knowledge of the one skilled in the art.

In this regard, the one skilled in the art may advantageously refer to the method described by Tiwari and Bhat (Biochemical and Biophysical Research Communications 366, 340-345, 2008).

Preferred polypeptide having Phosphoglucomutase-1 activity in the present invention is an enzyme having an EC number of n° 5.4.2.2.

According to a preferred embodiment, the one or more recombinant nucleic acids encoding a polypeptide having Phosphoglucomutase-1 activity may originate from organisms preferably selected in a group consisting of prokaryotic organisms and eukaryotic organisms. In some embodiments, the one or more recombinant nucleic acids encoding a polypeptide having Phosphoglucomutase-1 activity may originate or be derived from archaebacteria. In some embodiments, the one or more recombinant nucleic acids encoding a polypeptide having Phosphoglucomutase-1 activity may originate or be derived from organisms preferably selected from bacteria. In a preferred embodiment, the one or more recombinant nucleic acids encoding a polypeptide having Phosphoglucomutase-1 activity may orginate or be derived from *Saccharomyces cerevisiae.*

According to an embodiment, the one or more recombinant nucleic acids encoding a polypeptide having Phosphoglucomutase-1 activity may be selected from the group consisting of nucleic acid sequences having (i) at least 80% nucleic acid identity with the nucleic acid sequence set forth as sequence SEQ ID NO: 54, which originates from *Saccharomyces cerevisiae,* and (ii) a biological activity of the same nature as the nucleic acid sequence set forth as sequence SEQ ID NO: 54.

A biological activity of the same nature regarding this sequence is, as previously explained, the capacity to code for a polypeptide that converts Glucose-6-phosphate into Glucose-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the peptide having Phosphoglucomutase-1 activity from *Saccharomyces cerevisiae,* the one skilled in the art may refer to the accession number NP33401 in the UniProt database, or to SEQ ID NO: 55 described herein.

According to another particular embodiment, the one or more recombinant nucleic acids encoding a polypeptide having Phosphoglucomutase-1 activity may be nucleic acid(s) encoding polypeptide having an amino acid sequence selected from the group consisting of sequences having at least least 80% amino acid identity with the amino acid sequence set forth as sequence SEQ ID NO: 55, and also a biological activity of the same nature as the amino acid sequence set forth as sequence SEQ ID NO: 55.

A biological activity of the same nature regarding this sequence is as described previously, i.e. the capacity to catalyze the conversion of Glucose-6-phosphate into Glucose-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having Phosphoglucomutase-1 activity in the present invention is regulated by at least one promoter and at least one terminator, such as herein after defined more in details, which are present in 5' and 3' position respectively of the one or more recombinant nucleic acids encoding a polypeptide having Phosphoglucomutase-1 activity.

### UTP-GLUCOSE-1-PHOSPHATE URIDYLYLTRANSFERASE (UGP1)

The UTP-glucose-1-phosphate uridylyltransferase enzyme is a protein which is described in the art for catalyzing the conversion of Glucose-1-Phosphate into UDP-glucose. The UTP-glucose-1-phosphate uridylyltransferase originating from *Saccharomyces cerevisiae* may be termed UGP1.

A method implemented to measure the activity level of a polypeptide having UTP-glucose-1-phosphate uridylyltransferase activity belongs to the general knowledge of the one skilled in the art.

In this regard, the one skilled in the art may advantageously refer to the method described by Roeben (J. Mol. Biol 364, 551-560, 2006).

Preferred polypeptide having UTP-glucose-1-phosphate uridylyltransferase activity in the present invention is an enzyme having an EC number of n° 2.7.7.9.

According to a preferred embodiment, the one or more recombinant nucleic acids encoding a polypeptide having UTP-glucose-1-phosphate uridylyltransferase activity mayoriginate or be derived from organisms preferably selected in a group consisting of prokaryotic organisms and eukaryotic organisms. In some embodiments, the one or more recombinant nucleic acids encoding a polypeptide having UTP-glucose-1-phosphate uridylyltransferase activity may originate or be derived from archaebacteria. In some embodiments, the one or more recombinant nucleic acids encoding a polypeptide having UTP-glucose-1-phosphate uridylyltransferase activity may originate or be derived from organisms preferably selected from bacteria. In a preferred embodiment, the one or more recombinant nucleic acids encoding a polypeptide having UTP-glucose-1-phosphate uridylyltransferase activity may originate or be derived from *Saccharomyces cerevisiae.*

According to a particular embodiment, the one or more recombinant nucleic acids encoding a polypeptide having UTP-glucose-1-phosphate uridylyltransferase activity may be selected from the group consisting of nucleic acid sequences having (i) at least 80% nucleic acid identity with a nucleic acid as set forth as sequence SEQ ID NO: 56, originating from *Saccharomyces cerevisiae,* and (ii) a biological activity of the same nature as the nucleic acid as set forth as sequence SEQ ID NO: 56.

A biological activity of the same nature regarding this sequence is, as previously explained, the capacity to code for a polypeptide that converts Glucose-1-phosphate into UDP-glucose.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having UTP-glucose-1-phosphate uridylyltransferase activity originating from *Saccharomyces cerevisiae,* the one skilled in the art may refer to the accession number NP_32861 in the UniProt database, or to the sequence set forth as sequence SEQ ID NO: 57 described herein.

According to another particular embodiment, the one or more recombinant nucleic acids encoding a polypeptide having UTP-glucose-1-phosphate uridylyltransferase activity may be nucleic acid(s) encoding polypeptide having an amino acid sequence selected from the group consisting of sequences having at least 80% amino acid identity with the amino acid sequence set forth as SEQ ID NO: 57, and also a biological activity of the same nature as the amino acid sequence set forth as SEQ ID NO: 57.

A biological activity of the same nature regarding this sequence is as described previously, i.e. the capacity to catalyze the conversion of Glucose-1-phosphate into UDP-glucose.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UTP-glucose-1-phosphate uridylyltransferase activity in the present invention is regulated by at least one promoter and at least one terminator, such as herein after defined more in details, which are present in 5' and 3' position respectively of the one or more recombinant nucleic acids encoding a polypeptide having UTP-glucose-1-phosphate uridylyltransferase activity.

### GLUCOSAMINE-6-PHOSPHATE N-ACETYLTRANSFERASE (GNA1)

The Glucosamine-6-phosphate N-acetyltransferase enzyme is a protein which is described in the art for catalyzing the conversion of Glucosamine-6-phosphate into N-acetyl-glucosamine-6-phosphate. The Glucosamine-6-phosphate N-acetyltransferase originating from *Saccharomyces cerevisiae* may be termed GNA1.

A method implemented to measure the activity level of a polypeptide having Glucosamine-6-phosphate N-acetyltransferase activity belongs to the general knowledge of the one skilled in the art.

In this regard, the one skilled in the art may advantageously refer to the method described by Li et al. (Anal. Biochem. 370, 142-146, 2007).

Preferred polypeptide having Glucosamine-6-phosphate N-acetyltransferase activity in the present invention is an enzyme having an EC number of n° 2.3.1.4.

According to a preferred embodiment, the one or more recombinant nucleic acids encoding a polypeptide having Glucosamine-6-phosphate N-acetyltransferase activity may originate or be derived from organisms preferably selected in a group consisting of prokaryotic organisms and eukaryotic organisms. In some preferred embodiments, the one or more recombinant nucleic acids encoding a polypeptide having Glucosamine-6-phosphate N-acetyltransferase activity may originate or be derived from a yeast, and especially from *Saccharomyces cerevisiae.*

According to a particular embodiment, the one or more recombinant nucleic acids encoding a polypeptide having Glucosamine-6-phosphate N-acetyltransferase activity may be selected from the group consisting of nucleic acid sequences having (i) at least 80% nucleic acid identity with a nucleic acid sequence as set forth as sequence SEQ ID NO: 58, and (ii) a biological activity of the same nature as the nucleic acid sequence as set forth as sequence SEQ ID NO: 58. The nucleic acid sequence as set forth as sequence SEQ ID NO: 58 encodes a polypeptide having Glucosamine-6-phosphate N-acetyltransferase activity originating from *Saccharomyces cerevisiae,* that may also be termed GNA1.

A biological activity of the same nature regarding this sequence is, as previously explained, the capacity to code for a polypeptide that converts Glucosamine-6-phosphate into N-acetyl-glucosamine-6-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having Glucosamine-6-phosphate N-acetyltransferase activity originating from *Saccharomyces cerevisiae,* the one skilled in the art may refer to the accession number NP_116637 in the UniProt database, or to SEQ ID NO. 59 described herein.

According to another particular embodiment, the one or more recombinant nucleic acids encoding a polypeptide having Glucosamine-6-phosphate N-acetyltransferase activity may be nucleic acid(s) encoding polypeptide having an amino acid sequence selected from the group consisting of sequences having at least 80% amino acid identity with the amino acid sequence set forth as SEQ ID NO. 59, and also a biological activity of the same nature as the amino acid sequence set forth as SEQ ID NO. 59.

A biological activity of the same nature regarding this sequence is as described previously, i.e. the capacity to catalyze the conversion of Glucosamine-6-phosphate into N-acetyl-glucosamine-6-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having Glucosamine-6-phosphate N-acetyltransferase activity in the present invention is regulated by at least one promoter and at least one terminator, such as herein after defined more in details, which are present in 5' and 3' position respectively of the one or more recombinant nucleic acids encoding a polypeptide having Glucosamine-6-phosphate N-acetyltransferase activity.

### PHOSPHOACETYLGLUCOSAMINE MUTASE (PCM1)

The phosphoacetylglucosamine mutase enzyme is a protein which is described in the art for catalyzing the conversion of N-acetyl-glucosamine-6-phosphate into N-acetyl-glucosamine-1-phosphate. The phosphoacetylglucosamine mutase originating from *Saccharomyces cerevisiae* may be termed PCM1.

A method implemented to measure the activity level of a polypeptide having phosphoacetylglucosamine mutase activity belongs to the general knowledge of the one skilled in the art.

In this regard, the one skilled in the art may advantageously refer to the method described by Bandini et al. (Molecular Microbiology 85(3), 513-534, 2012).

Preferred polypeptide having phosphoacetylglucosamine mutase activity in the present invention is an enzyme having an EC number of n° 5.4.2.3.

According to a preferred embodiment, the one or more recombinant nucleic acids encoding a polypeptide having phosphoacetylglucosamine mutase activity mayoriginate or be derived from organisms preferably selected in a group consisting of prokaryotic organisms and eukaryotic organisms. In some preferred embodiments, the one or more recombinant nucleic acids encoding a polypeptide having phosphoacetylglucosamine mutase activity may originate or be derived from a yeast, and especially from *Saccharomyces cerevisiae.*

According to a particular embodiment, the one or more recombinant nucleic acids encoding a polypeptide having phosphoacetylglucosamine mutase activity may be selected from the group consisting of nucleic acid sequences having (i) at least 80% nucleic acid identity with a nucleic acid sequence set forth as SEQ ID NO: 60, and (ii) a biological activity of the same nature as the nucleic acid sequence set forth as SEQ ID NO: 60. The nucleic acid set forth as SEQ ID NO: 60 encodes a polypeptide having phosphoacetylglucosamine mutase activity originating from *Saccharomyces,* that may also be termed PCM1.

A biological activity of the same nature regarding this sequence is, as previously explained, the capacity to code for a polypeptide that converts N-acetyl-glucosamine-6-phosphate into N-acetyl-glucosamine-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having phosphoacetylglucosamine mutase activity originating from *Saccharomyces cerevisiae,* the one skilled in the art may refer to the accession number NP_010856 in the UniProt database, or to SEQ ID NO. 61 described herein.

According to another particular embodiment, the one or more recombinant nucleic acids encoding a polypeptide having phosphoacetylglucosamine mutase activity may be nucleic acid(s) encoding polypeptide having an amino acid sequence selected from the group consisting of sequences having at least 80% amino acid identity with the amino acid sequence set forth as SEQ ID NO. 61, and also a biological activity of the same nature as the amino acid sequence set forth as SEQ ID NO. 61.

A biological activity of the same nature regarding this sequence is as described previously, i.e. the capacity to catalyze the conversion of N-acetyl-glucosamine-6-phosphate into N-acetyl-glucosamine-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having phosphoacetylglucosamine mutase activity in the present invention is regulated by at least one promoter and at least one terminator, such as herein after defined more in detail, which are present in 5' and 3' position respectively of the one or more recombinant nucleic acids encoding a polypeptide having phosphoacetylglucosamine mutase activity.

### PROMOTERS

As disclosed herein, the expression of the genes of interest that have been genetically engineered for obtaining a recombinant cell according to the invention comprise appropriate regulatory sequences that are functional in the recombinant cells of the invention, and in particular in recombinant yeast cells of the invention, including in particular *Saccharomyces cerevisiae.*

Various promoters may be used for the desired expression of the coding sequences of interest.

Promoters according to the invention can be selected from the group consisting of the following promoters:
- pTDH3 (SEQ ID NO: 62),
- pTDH3.sk (SEQ ID NO: 63),
- pTDH3-1.Sba (SEQ ID NO: 64),
- pTDH3.Sar (SEQ ID NO: 65),
- pENO2 (SEQ ID NO: 66),
- pTEF3 (SEQ ID NO: 67),
- pTEFl (SEQ ID NO: 68),
- pTEFl.ago (SEQ ID NO: 69),
- pTEF1.sba (SEQ ID NO: 70),
- pPDC1 (SEQ ID NO: 71),
- pCCW12 (SEQ ID NO: 72),
- pCCW12.Sm (SEQ ID NO: 73),
- pCCW12.sk (SEQ ID NO: 74),
- pCCW12.sba (SEQ ID NO: 75),
- pCCW12.sar (SEQ ID NO: 76),
- pNUP57 (SEQ ID NO: 77),
- pCCW10.ago (SEQ ID NO: 78),
- pCWP2 (SEQ ID NO: 79), and
- pRPLA1 (SEQ ID NO: 80).

Promoters more particularly interesting in the present invention may be selected from the group consisting of:
- pTDH3 (SEQ ID NO: 62),
- pTDH3.sk (SEQ ID NO: 63),
- pTDH3-1.Sba (SEQ ID NO: 64),
- pTDH3.Sar (SEQ ID NO: 65),
- pENO2 (SEQ ID NO: 66),
- pTEF3 (SEQ ID NO: 67),
- pTEFl (SEQ ID NO: 68),
- pTEFl.ago (SEQ ID NO: 69),
- pTEFl.sba (SEQ ID NO: 70),
- pPDC1 (SEQ ID NO: 71),
- pCCW12 (SEQ ID NO: 72),
- pCCW12.Sm (SEQ ID NO: 73),
- pCCW12.sk (SEQ ID NO: 74),
- pCCW12.sba (SEQ ID NO: 75), and
- pCCW12.sar (SEQ ID NO: 76),

Said promoters can in particular be selected from the group consisting of pTDH3, pTDH3-1.Sba, pTDH3.Sar, pENO2, pTEF3, pTEF1, pPDC1, pCCW12, pCCW12.Sm, pCCW12.sk, pCCW12.sba, and pCCW12.sar.

Alternatively, promoters interesting in the present invention may be selected from the group consisting of:
- pNUP57 (SEQ ID NO: 77), and
- pCCW10.ago (SEQ ID NO: 78).

Other promoters of interest in the present invention may be:
- pCWP2 (SEQ ID NO: 79); and
- pRPLA1 (SEQ ID NO: 80).

As previously mentioned, inducible or repressible promoters are promoters whose activity is controlled by the presence or absence of biotic or abiotic factors and also by the quantity of said factor. Accordingly, for some promoters, their activity will in particular be induced and thus increased when the quantity of a given factor increases or is increased, and, accordingly, the activity of these same promoters can be repressed and thus reduced when the quantity of said factor diminishes or is reduced. The quantity of said factor(s) in the culture medium of a recombinant yeast cell of the invention comprising inducible or repressible promoters can be decided and thus controlled by the man skilled in the art.

For example, increasing the quantity of copper in a culture medium of a recombinant yeast cell according to the invention comprising a pCUP-1 promoter will induce and thus increase transcription of the gene under the control of this promoter. On the contrary, reducing the quantity of copper in said culture medium will lead to a repression, and thus a reduced, transcription of the gene under the control of this promoter.

In another example, increasing the quantity of methionine in a culture medium of a recombinant yeast cell according to the invention comprising a pMET6 promoter will repress and thus decrease transcription of the gene under the control of this promoter. On the contrary, reducing the quantity of methionine in said culture medium will lead to an induced, and thus an increased, transcription of the gene under the control of this promoter.

For this reason, the following promoters are referred to in the present text as being inducible or repressible promoters.

According to a first embodiment, inducible or repressible promoters according to the invention may be selected from the group comprising promoters inducible or repressible with copper, promoters inducible or repressible with methionine and promoters inducible or repressible with threonine, and are in particular is CUP1 - copper inducible or repressible (SEQ ID NO: 81).

According to this embodiment, the inducible or repressible promoter according to the invention can in particular be pCUP1.

The activity of these promoters is thus induced by the increasing presence of methionine, copper or threonine as indicated above, and their activity diminishes, i.e. is repressed, when the quantity of methionine, copper or threonine is reduced.

According to a second embodiment, inducible or repressible promoters according to the invention may be selected from the group comprising promoters inducible or repressible with copper, promoters inducible or repressible with lysine and promoters inducible or repressible with methionine, and in particular selected from the group consisting of:
- pMET6 - methionine inducible or repressible (SEQ ID NO: 82),
- pMET25 - methionine inducible or repressible (SEQ ID NO: 83), and
- pSAM1 - methionine inducible or repressible (SEQ ID NO: 84),

According to this particular embodiment, the inducible or repressible promoter according ot the invention may, be selected from the group consisting of pMET6, pMET25 and pSAM1.

The activity of these promoters is thus repressed by the increasing presence of methionine, copper, lysine or glucose as indicated above, and their activity increases, i.e. is induced, when the quantity of methionine, copper, lysine or glucose is reduced.

In a particular embodiment, inducible or repressible promoters according to the invention may be selected from the group comprising promoters inducible or repressible with copper, promoters inducible or repressible with glucose, promoters inducible or repressible with lysine, promoters inducible or repressible with methionine and promoters inducible or repressible with threonine.

In a more particular embodiment, the inducible or repressible promoter according to the invention may be selected from the group consisting of pCUP1, pMET6, pSAM1, and pMET25.

Synthetic promoters as described in Blazeck & Alper (2013) Biotechnol. J. 8 46-58 can also be used.

The promoters of the invention can originate from any organism from the Saccharomycetes class and can in particular originate from an organism selected from the group consisting of at least one of *Saccharomyces cerevisiae, Saccharomyces boulardii, Saccharomyces castelii, Saccharomyces bayanus, Saccharomyces arboricola, Saccharomyces kudriavzevii, Saccharomyces mikatae, Ashbya gossypii, Kluveromyces lactis, Pichia pastoris, Candida glabrata, Candida tropicalis, Debaryomyces castelii, Yarrowia lipolitica,* and *Cyberlindnera jadinii,.*

The promoters of the invention can preferably originate from an organism selected from the group consisting of *Saccharomyces cerevisiae (sc), Saccharomyces mikatae (Sm), Saccharomyces kudriavzevii (sk), Saccharomyces bayanus (sba), and Saccharomyces arboricola (Sar).*

### TERMINATORS

As it is disclosed herein, the expression of the genes of interest that have been genetically engineered for obtaining a recombinant cell according to the invention, and in particular a recombinant yeast according to the invention, comprise appropriate transcription terminator sequences that are functional in recombinant cells of the invention, and in particular in recombinant yeast cells of the invention, in particular in *Saccharomyces cerevisiae.*

Said transcription terminators, identical or different, may be found in literature Yamanishi et al., (2013) ACS synthetic biology 2, 337-347.

Terminators more particularly interesting in the present invention may be selected from the group comprising:
- tTPI1 from the gene encoding for the Triose Phosphate Isomerase (SEQ ID NO: 85),
- tMET25 from the gene encoding for the O-acetyl homoserine-O-acetyl serine sulfhydrylase (SEQ ID NO: 86),
- tDIT1 (SEQ ID NO: 87),
- tRPL3 (SEQ ID NO: 88),
- tRPL3.sm (SEQ ID NO: 89),
- tRPL3.sba (SEQ ID NO: 90),
- tRPL41B (SEQ ID NO: 91),
- tRPL15A (SEQ ID NO: 92),
- tRPL15A.sba (SEQ ID NO: 93),
- tIDP1 (SEQ ID NO: 94), and
- tTEF1.sba (SEQ ID NO: 95).

In particular, said terminator may be selected from the group consisting of tTPI1, tMET25, tDIT1, tRPL3, tRPL3.sm, tRPL3.sba, tRPL41B, tRPL15A, tRPL15A.sba, tIDP1 and tTEF1.sba.

The terminators of the invention can originate from any organism from the Saccharomycetes class and can in particular originate from an organism selected from the group consisting of *Saccharomyces cerevisiae* and *Saccharomyces Bayanus.*

### RECOMBINANT CELLS

Recombinant cells of the invention can be selected from the group consisting of yeasts and bacteria.

Recombinant cells of the invention, such as a recombinant host cell of the invention, are preferably recombinant yeast cells.

Generally, yeast can grow rapidly and can be cultivated at higher density as compared with bacteria, and does not require an aseptic environment in the industrial setting. Furthermore, yeast cells can be more easily separated from the culture medium compared to bacterial cells, greatly simplifying the process for product extraction and purification.

A recombinant cell of the invention, and in particular a recombinant yeast cell of the invention is preferably a *Saccharomycetales* cell.

A recombinant cell of the invention, and in particular a recombinant yeast of the invention can in particular belong to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.

A recombinant cell of the invention belonging to the *Saccharomyces* genus can be selected from the group consisting of *Saccharomyces cerevisiae, Saccharomyces boulardii, Saccharomyces bayanus, Saccharomyces paradoxus, Saccharomyces mikatae, Saccharomyces castelli, Saccharomyces cariocanus, Saccharomyces kudriavzevii, Saccharomyces arboricolus, Saccharomyces pastorianus, Saccharomyces uvarum* and *Saccharomyces delbrueckii.*

A recombinant cell of the invention belonging to the *Candida* genus can be selected from the group consisting of *Candida albicans, Candida glabrata, Candida tropicalis, Candida dubliniensis, Candida parapsilosis, Candida lusitaniae* and *Candida guilliermondii.*

A recombinant cell of the invention belonging to the *Kluyveromyces* genus can be selected from the group consisting of *Kluyveromyces lactis, Kluyveromyces marxianus, Kluyveromyces polysporus, Kluyveromyces thermotoleren, Kluyveromyces dobzhanskii* and *Kluyveromyces wickerhamii.*

A recombinant cell of the invention belonging to the *Ogataea* genus can be selected from the group consisting of *Ogataea polymorpha, Ogataea histrianica, Ogataea deakii, Ogataea kolombanensis, Ogataea philodendra, Ogataea siamensis, Ogataea angusta, Ogataea parapolymorpha, Ogataea minuta, Ogataea nonfermentans* and *Ogataea kodamae.*

A recombinant cell of the invention belonging to the *Yarrowia* genus can be selected from the group consisting of *Yarrowia lypolytica, Yarrowia parophonii, Yarrowia galli, Yarrowia oslonensis, Yarrowia alimentaria, Yarrowia hollandica* and *Yarrowia yakushimensis.*

A recombinant cell of the invention belonging to the *Debaryomyces* genus can be selected from the group consisting of *Debaryomyces hansenii, Debaryomyces carsonii, Debaryomyces castellii, Debaryomyces marama, Debaryomyces occidentalis, Debaryomyces oviformis, Debaryomyces nepalensis, Debaryomyces coudertii, Debaryomyces udenii, Debaryomyces psychrosporus* and *Debaryomyces yamadae.*

A recombinant cell of the invention belonging to the *Ashbya* genus can be selected from the group consisting of *Ashbya gossypii* and *Ashbya aceri.*

A recombinant cell of the invention, and in particular a recombinant yeast cell of the invention, can in particular be selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

In a particular embodiment, the recombinant yeast cell according to the invention is of the genus *Saccharomyces, Kluyveromyces,* or *Eremothecium,* more particularly is of the species selected from the group consisting of *Saccharomyces cerevisiae, Kluyveromyces Marxianus, Ogataea polymorpha* and *Ashbya gossypii.*

In an embodiment, a recombinant host cell of the invention is a yeast selected belonging to the *Saccharomycetales* order, in particular to the *Saccharomycetaceae* family, and is in particular selected from the group consisting of *Yarrowia lipolitica, Kluyveromyces Marxianus, Ogataea polymorpha, Ashbya gossypii and Saccharomyces cerevisiae.*

A recombinant cell of the invention can most preferably be a *Saccharomyces cerevisiae* cell.

As above-mentioned, a recombinant cell according to the invention has the ability to produce hyaluronic acid by insertion of the one or more recombinant nucleic acids according to the present invention. In certain embodiments, a recombinant yeast according to the invention has the ability to produce hyaluronic acid having a controlled size (controlled molecular weight) by insertion of the one or more recombinant nucleic acids according to the present invention.

Methods implemented to insert a specific DNA construct within a gene belong to the general knowledge of a man skilled in the art. A related method is described in more details in the herein after examples.

However, unexpected technical difficulties were encountered because the consequences of the insertion of DNA constructs within the genome of a cell, and in particular within the genome of a yeast, such as for example in the genome of *Saccharomyces cerevisiae,* are unpredictable. In particular, the survival rate of the cells, and in particular of the yeasts, and their ability to grow and produce the desired hyaluronic acid is also unpredictable.

In order to obtain a recombinant cell, and in particular a recombinant yeast, of the invention, many different constructs were tested by the inventors in order to obtain a viable and efficient recombinant cell, and in particular yeast.

### CULTURE CONDITIONS

The present invention also relates to the use of a recombinant cell of the invention, for the production of hyaluronic acid, in particular of hyaluronic acid of controlled molecular weight.

The present invention further relates to a method of producing hyaluronic acid (HA) of a desired molecular weight (HAMW) comprising:
(a) cultivating a recombinant cell of the present invention in a cultivation medium for a time sufficient to produce hyaluronic acid (HA) of the desired molecular weight; and
(b) optionally isolating or recovering the hyaluronic acid (HA) from the recombinant cell and/or from the cultivation medium.

Typically, cells of the invention, and in particular yeasts of the invention, are grown at a temperature in the range of about 20°C to about 37°C, preferably at a temperature ranging from 27 to 34°C, in an appropriate culture medium.

Suitable growth media for cells of the invention, and in particular for yeasts of the invention, are common commercially prepared media such as broth that includes yeast nitrogen base, ammonium sulfate, and dextrose as the carbon/energy source or YPD Medium, a blend of peptone, yeast extract, and dextrose in optimal proportions for growing most. Other defined or synthetic growth media may also be used and the appropriate medium for growth of the particular cell, and in particular yeast, will be known by one skilled in the art of microbiology or fermentation science.

A particular medium that is suitable herein is the SY medium which comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeC13: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM ; Myo-Inositol (C6H12O6): 555 µM ; Nicotinic acid (C6H5NO2): 29 µM; D-Biotine: 0,82 µM; Ammonium citrate tribasic: 33 mM; and glucose or sucrose 2-30%.

Carbon sources that may be used in the culture medium include fructose, mannose, xylose and arabinose; oligosaccharides such as lactose, maltose, galactose, or sucrose; polysaccharides such as starch or cellulose or mixtures thereof; and unpurified mixtures from renewable feedstocks such as cheese whey permeate cornsteep liquor, sugar beet molasses, and barley malt.

Nitrogen sources that may be included in the culture medium include peptone, yeast extracts, meat extracts, malt extracts, urea, ammonium sulfate, ammonium chloride, ammonium nitrate, ammonium phosphate, ammonium citrate and combinations thereof.

The culture medium may further comprise trace elements (e.g., metal salts), for example magnesium salts, cobalt salts and/or manganese salts; as well as growth factors such as amino acids, vitamins, growth promoters, and the like.

Examples of vitamins that may be included are Panthotenic Acids hemicalcium, Thiamin hydrochloride; Pyridoxin hydrochloride; Myo-Inositol; Nicotinic acid; D-Biotine, folic acid, p-aminobenzoique acid, riboflavin.

A culture medium of the invention may further include rare elements, such as CuSO₄· 5H₂O, KI, FeCl₃, ZnSO₄ ·7H₂O, MnSO₄ ·H₂O, or H₂SO₄, MgCl2, CaCl2, NaCl, K2HPO4, KH2PO4, ZnCl, H3BO3, MnSO4, Na2MoO4.

The term "appropriate culture medium" is above-defined.

Examples of known culture media for a recombinant cell according to the present invention are known to the person skilled in the art, and are presented in the following publication D. Burke et al., Methods in yeast Genetics - A cold spring harbor laboratory course Manual (2000).

Suitable pH ranges for the fermentation may be between pH 3.0 to pH 7.5, where pH 4 to pH 6 is preferred as the initial condition.

As mentioned elsewhere in this specification, the pH value of the culture medium can be regulated during the cultivation step of a method of the invention in order to modulate the activity of the polypeptide facing hyaluronidase activity, which will impact the molecular weight of the hyaluronic acid molecules produced by the recombinant cell of the invention, and in particular by the recombinant yeast of the invention.

In particular, the pH of the culture medium can be modified according to the hyaluronic acid that is meant to be produced by the recombinant yeast. For example, the pH of the culture medium may remain at a pH of 4, of 5,5 or of 6 during the culture time.

In a particular embodiment, the pH of the culture medium may change or be changed during the time length of the culture of the recombinant cell of the invention, in particular of the recombinant yeast of the invention. As previously mentioned, *Saccharomyces cerevisiae* acidifies the medium in which it is cultivated and thus reduces the pH of its culture medium. For example, the pH of the culture medium may begin at 6, may be taken down to 4 and then brought back up to 6. In another example, the pH of the culture medium may begin at 6, remain or be maintained at 6, and then may be lowered to 4.

In a particular embodiment, the pH of the culture medium may be modulated during the cultivating step (a) of a method of the invention so that at the end of the cultivating step of a method of the invention, the pH of the cultivation medium is the same as the pH at the beginning of said cultivation step (a).

In another embodiment, the pH of the culture medium may remain the same during the time length of the culture of the recombinant cell of the invention, and in particular the recombinant yeast cell of the invention.

Said time length of the culture of a recombinant cell according to the invention, and in particular of the recombinant yeast of the invention, can vary depending on the molecular weight of the hyaluronic acid of interest. The longer said time length, the lower the molecular weight of hyaluronic acid in a given culture medium of a recombinant cell of the invention, and in particular a recombinant yeast cell of the invention.

The time length of the culture time of a recombinant cell of the invention, and in particular a recombinant yeast cell in the present invention can be a period of from about 35 hours to about 50 hours, preferably from about 40 hours to about 50 hours, and is in particular about 48 hours.

Fermentations may be performed under aerobic conditions or micro-aerobic conditions.

The amount of hyaluronic acid product in the fermentation medium can be determined using a number of methods known in the art, for example, high performance liquid chromatography (HPLC) or gas chromatography (GC).

The present process may employ a batch method of fermentation. A classical batch fermentation is a closed system where the composition of the medium is set at the beginning of the fermentation and not subject to artificial alterations during the fermentation. Thus, at the beginning of the fermentation, the medium is inoculated with the desired organism or organisms, and fermentation is permitted to occur without adding anything to the system. Typically, however, a "batch" fermentation method or system is batch with respect to the addition of carbon source and attempts are often made at controlling factors such as temperature, pH and oxygen concentration. In batch systems, the metabolite and biomass compositions of the system change constantly up to the time when the fermentation is stopped. Within batch cultures cells progress through a static lag phase to a high growth log phase and finally to a stationary phase where growth rate is diminished or halted. If untreated, cells in the stationary phase will eventually die. Cells in log phase generally are responsible for the bulk of production of end product or intermediate.

A Fed-Batch system may also be used in the present invention. A Fed-Batch system is similar to a typical batch system with the exception that the carbon source substrate is added in increments as the fermentation progresses. Fed-Batch systems are useful when catabolite repression (e.g. glucose repression) is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂.

Batch and Fed-Batch culturing methods are common and well known in the art and examples may be found in Biotechnology: A Textbook of Industrial Microbiology, Crueger, Crueger, and Brock, Second Edition (1989) Sinauer Associates, Inc., Sunderland, MA , or Deshpande, Mukund V., Appl. Biochem. Biotechnol., 36, 227, (1992). Although the present invention is performed in batch mode it is contemplated that the method would be adaptable to continuous fermentation.

Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to vary. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology.

It is contemplated that the present invention may be practiced using either batch, fed- batch or continuous processes and that any known mode of fermentation would be suitable. Additionally, it is contemplated that cells may be immobilized on a substrate as whole cell catalysts and subjected to fermentation conditions for production.

In order to still improve the hyaluronic acid production, a particular embodiment may consist of culturing the recombinant cells of the invention, in particular the recombinant yeast cells of the invention, in an appropriate culture medium, such as above-mentioned, wherein the said culture medium comprises an optimal amount of carbon source, especially glucose or sucrose.

In preferred embodiments, the carbon source comprised in said optimal culture medium consists of glucose and/or sucrose. In preferred embodiments, the said optimal culture medium comprises 1% w/w or more glucose and/or sucrose, in particular comprises 5% w/w or more glucose and/or sucrose, in particular comprises 10% w/w or more glucose and/or sucrose, in particular comprises 15% w/w or more glucose and/or sucrose. In preferred embodiments, the said optimal culture medium comprises at most 40% w/w glucose, which includes at most 35% w/w glucose.

In a preferred embodiment, the method of the invention is carried out on an industrial scale.

More particularly, the cultivation medium of the method according to the invention can be at least about 100L, more preferably in the range of about 1000L to about 3000L, even more preferably about 10,000L, even more preferably 100,000L, or even about 250,000L.

The invention further relates to a method for producing hyaluronic acid as previously described, and comprising the steps of:
(a) culturing a recombinant cell of the invention in a culture medium; and
(b) recovering the hyaluronic acid from said culture medium,
   wherein the hyaluronic acid recovered in step (b) has a molecular weight controlled through the selection of:
   - the nature and origin of the one or more recombinant nucleic acids encoding a polypeptide having hyaluronidase activity of the recombinant cell of the invention, and in particular of the recombinant yeast of the invention,
   - the nature and origin of the promoter controlling the expression of the one or more recombinant nucleic acids encoding a polypeptide having hyaluronidase activity of the recombinant cell of the invention, and in particular of the recombinant yeast of the invention,
   - the presence or absence of an anchoring signal associated to the one or more recombinant nucleic acids encoding a polypeptide having hyaluronidase activity of the recombinant cell of the invention, and in particular of the recombinant yeast of the invention,
   - the pH of the culture medium during the step of culturing the recombinant cell of the invention, and in particular of the recombinant yeast of the invention, and/or
   - the duration of the culturing of the recombinant cell of the invention, and in particular of the recombinant yeast of the invention.

The molecular weight of the hyaluronic acid can be a particular molecular weight or more preferably a specific range of molecular weights such as, for example, less than 50 kDa, in the range of about 20 kDa to about 50 kDa, greater than or equal to 50 kDa, in the range of about 50 kDa to about 150kDa, in the range of about 50 kDa to about 250kDa, greater than or equal to 100 kDa, in the range of about 100kDa to about 1500 kDa, in the range of about 150kDa to about 1500 kDa, greater than 1000 kDa or greater than 1500kDa.

The present invention also relates to the use of a recombinant cell according to the invention, and in particular a recombinant yeast cell of the invention, for the production of hyaluronic acid (HA) having a molecular weight in the range of from about 20kDa to about 50kDa or from about 50kDa to about 1000 kDa.

The one or more recombinant nucleic acids encoding a polypeptide having hyaluronidase activity of a recombinant cell of the invention can for example be selected from those originating or being derived from at least one of *Cupiennius salei (Csa), Loxosceles intermedia (Li), Hirudo nipponia (Hn), Bothrops atrox (Ba), Tityus serrulatus (Ts) or Vespa magnifica (Vm), in particular from at least one of Cupiennius salei (Csa), Loxosceles intermedia (Li), Hirudo nipponia (Hn), Bothrops atrox (Ba)* or *Tityus serrulatus (Ts),* more particularly from those of sequences set forth as sequences SEQ ID NO: 19 (Ba), SEQ ID NO: 25 (Li), SEQ ID NO: 21 (Csa), SEQ ID NO: 27 (Ts) or SEQ ID NO: 23 (Hn) (in the presence of a secretion signal and in the absence of an anchoring signal).

The one or more recombinant nucleic acids encoding a polypeptide having hyaluronidase activity of a recombinant cell of the invention can for example be selected from those originating or being derived from at least one of *Cupiennius salei (Csa), Loxosceles intermedia (Li), Hirudo nipponia (Hn), Bothrops atrox (Ba), Tityus serrulatus (Ts) or Vespa magnifica (Vm), in particular from Cupiennius salei (Csa), Loxosceles intermedia (Li), Hirudo nipponia (Hn), Bothrops atrox (Ba)* or *Tityus serrulatus (Ts),* in particular from those of sequences set forth as sequences SEQ ID NO: 22 (Csa), SEQ ID NO: 26 (Li), SEQ ID NO: 24 (Hn), SEQ ID NO: 20 (Ba) or SEQ ID NO: 28 (Ts) (in the presence of both a secretion signal and an anchoring signal).

The one or more recombinant nucleic acids encoding a polypeptide having hyaluronidase activity of a recombinant cell of the invention can for example be placed under the control of a promoter selected from the group consisting of pTEF1, pCCW12, pCCW12.sba, pCCW12.Sar, pPDC1, pTEF3, pTDH3, pNUP57 and pCCW10.ago.

In particular, the promoters of the one or more recombinant nucleic acids encoding a polypeptide having hyaluronidase activity of a recombinant cell of the invention, and in particular of a recombinant yeast of the invention, may originate or be derived from *Saccharomyces Bayanus, Saccharomyces kudriavzevii, Saccharomyces mikatae, Saccharomyces arboricola* or other saccharomycetales, or *Abishia gossypii.*

The secretion signal of the present invention may for example have:
- the nucleic acid sequence set forth as SEQ ID NO: 43; and/or
- the amino acid sequence set forth as SEQ ID NO: 44.

The anchoring signal of the present invention may for example have:
- the nucleic acid sequence set forth as SEQ ID NO: 45; and/or
- the amino acid sequence set forth SEQ ID NO: 46.

The secretion signal may be fused to the polypeptide having hyluronidase activity by creating a chimeric nucleic acid starting by a nucleic acid sequence encoding the signal peptide followed by a recombinant nucleic acid encoding a polypeptide having hyaluronidase activity as previously defined.

The secretion signal and anchoring signal may be fused to the polypeptide having hyluronidase activity by creating a chimeric nucleic acid starting by a nucleic acid sequence encoding the signal peptide followed by a recombinant nucleic acid encoding a polypeptide having hyaluronidase activity as previously defined, followed by a nucleic acid sequence encoding for the anchoring signal.

Such chimeric nucleic acid sequences may be obtained by techniques known by the man skilled in the art such as chemical synthesis of nucleic acids or any recombination techniques such as cloning or PCR.

The invention further relates to a method as previously described, for producing hyaluronic acid having a molecular weight less than or equal to about 50 kDa, and in particular less than 50 kDa and greater than or equal to about 20 kDa, wherein:
(a) the pH of the medium is superior to 4, and is in particular in the range of from about 5 to about 7;
(b) the one or more recombinant nucleic acids encoding a polypeptide having hyaluronidase activity integrated in the genome of the recombinant cell of the invention originates or is derived:
   (i) from *Tityus serrulatus,* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar;
   (ii) from *Cupiennius salei,* possesses a secretion signal but is devoid of an anchoring signal, and is under the control of a promoter selected from the group consisting of pNUP57 and pCCW10.ago;
   (iii) from *Cupiennius salei,* possesses a secretion signal and an anchoring signal, and is under the control of the promoter pCWP2;
   (iv) from *Loxosceles intermedia* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; or
   (v) from *Hirudo nipponia*, possesses a secretion signal but is devoid of an anchoring signal, and is under the control of a promoter selected from the group consisting of pNUP57 and pCCW10.ago; or
   (vi) from *Hirudo nipponia*, possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; and
(c) the step of recovering the hyaluronic acid from the culture medium is performed about 48 hours after the beginning of the culturing of the recombinant cell of the invention, in particular the recombinant yeast cell of the invention;
the recombinant cell being in particular a recombinant yeast cell, and more particularly a *Saccharomyces cerevisiae* cell.

The invention further relates to a method as previously described, for producing hyaluronic acid having a molecular weight greater than or equal to about 50 kDa and less than or equal to about 1000 kDa, wherein:
(a) the pH of the medium is between about 4 and about 6 and is optionally varied from about 6 to about 4 during the culturing step of the recombinant cell, in particular the recombinant yeast cell, of the invention;
(b) the one or more recombinant nucleic acids encoding a polypeptide having hyaluronidase activity integrated in the genome of the recombinant cell of the invention originates or is derived:
   (i) from *Tityus serrulatus,* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar;
   (ii) from *Tityus serrulatus,* possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar;
   (iii) from *Loxosceles intermedia,* possesses both a secretion signal and an anchoring signal, and is under the control of a promoter selected from the group consisting of pNUP57 and pCCW10.ago;
   (iv) from *Loxosceles intermedia,* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar,
   (v) from *Cupiennius salei,* possesses both a secretion signal and an anchoring signal, and is under the control of a promoter selected from the group consisting of pNUP57 and pCCW10.ago,
   (vi) from *Cupiennius salei,* possesses a secretion signal but is devoid of an anchoring signal, and is under the control of a promoter selected from the group consisting of pNUP57 and pCCW10.ago, or
   (vii) from *Hirudo nipponia*, possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; and
(c) the step of recovering the hyaluronic acid from the culture medium is performed about 48 hours after the beginning of the culturing of the recombinant cell of the invention, in particular the recombinant yeast cell of the invention;
the recombinant cell being in particular a recombinant yeast cell, and more particularly a *Saccharomyces cerevisiae* cell.

The invention further relates to a method as previously described, for producing hyaluronic acid having a molecular weight greater than about 1000 kDa, and in particular having a molecular weight from about 1000 kDa to about 1.5 Million Da, wherein:
(a) the pH of the medium is from about 4 to about6 and is optionally varied from about6 to about 4 during the culturing step of the recombinant cell, in particular recombinant yeast, of the invention;
(b) the one or more recombinant nucleic acids encoding a polypeptide having hyaluronidase activity integrated in the genome of the recombinant cell of the invention originates or is derived:
   (i) from *Tityus serrulatus*, possesses a secretion signal but is devoid of an anchoring signal, and is under the control of a promoter selected from the group consisting of pNUP57 and pCCW10.ago;
   (ii) from *Loxosceles intermedia,* possesses a secretion signal but is devoid of an anchoring signal, and is under the control of a promoter selected from the group consisting of pNUP57 and pCCW10.ago;
   (iii) from *Bothrops atrox*, possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; or
   (iv) from *Bothrops atrox*, possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar;
      and
(c) the step of recovering the hyaluronic acid from the culture medium is performed about 48 hours after the beginning of the culturing of the recombinant cell of the invention, in particular the recombinant yeast cell of the invention;
the recombinant cell being in particular a recombinant yeast, and more particularly a *Saccharomyces cerevisiae* cell.

Another aspect of the present invention relates to hyaluronic acid (HA) obtained or obtainable from a recombinant cell of the invention or from a method according to the invention.

A further aspect of the invention is a cultivation medium comprising the hyaluronic acid of the invention.

The invention further relates to a composition comprising the hyaluronic acid according to the invention.

The invention also relates to an industrial product or a consumer product or a consumable comprising (i) the hyaluronic acid of the invention, (ii) the cultivation medium comprising hyaluronic acid of the invention or (iii) the composition comprising hyaluronic acid of the invention.

In particular, said industrial product or consumer product or consumable according to the invention can be a cosmetic product, a flavour product, a fragrance product, a food product, a food, a beverage, a texturant, a pharmaceutical composition, a dietary supplement, a nutraceutical, a cleaning product and/or a dental and/or an oral hygiene composition.

### PURIFICATION OF HYALURONIC ACID

According to a specific aspect of the invention, the fermentative production of hyaluronic acid preferably comprises a step of isolation of the hyaluronic acid produced from the culture medium. Recovering the hyaluronic acid from the culture medium is a routine task for a man skilled in the art. It may be achieved by a number of techniques well known in the art including but not limiting to pervaporation, selective precipitation, filtration, centrifugation, spray drying, lyophylisation or liquid extraction. The expert in the field knows how to adapt parameters of each technique dependant on the characteristics of the material to be separated.

The yeast as model of a cell in the present invention is preferred in that the synthesized hyaluronic acid is/are entirely exported outside the cells, thus simplifying the purification process.

Gas stripping is achieved with a stripping gas chosen among helium, argon, carbon dioxide, hydrogen, nitrogen or mixture thereof.

Liquid extraction is achieved with organic solvent as the hydrophobic phase such as pentane, hexane, heptane or dodecane. Renewal solvents may also be used.

Those of skill in the art will recognize that, due to the degenerate nature of the genetic code, a variety of DNA molecules differing in their nucleotide sequences can be used to encode a given enzyme of the disclosure. The native DNA sequence encoding the biosynthetic enzymes described above are referenced herein merely to illustrate an embodiment of the disclosure, and the disclosure includes DNA molecules of any sequence that encode the amino acid sequences of the polypeptides and proteins of the enzymes utilized in the methods of the disclosure. In similar fashion, a polypeptide can typically tolerate one or more amino acid substitutions, deletions, and insertions in its amino acid sequence without loss or significant loss of a desired activity. The disclosure includes such polypeptides with different amino acid sequences than the specific proteins described herein so long as the modified or variant polypeptides have the enzymatic anabolic or catabolic activity of the reference polypeptide. Furthermore, the amino acid sequences encoded by the DNA sequences shown herein merely illustrate embodiments of the disclosure.

Described herein are specific genes and proteins useful in the methods, compositions and organisms of the disclosure; however it will be recognized that absolute identity to such genes is not necessary. For example, changes in a particular gene or polynucleotide comprising a sequence encoding a polypeptide or enzyme can be performed and screened for activity. Typically such changes comprise conservative mutations and silent mutations. Such modified or mutated polynucleotides and polypeptides can be screened for expression of a functional enzyme using methods known in the art.

Due to the inherent degeneracy of the genetic code, other polynucleotides which encode substantially the same or functionally equivalent polypeptides can also be used to clone and express the polynucleotides encoding such enzymes

Techniques known to those skilled in the art may be suitable to identify additional homologous genes and homologous enzymes. Generally, analogous genes and/or analogous enzymes can be identified by functional analysis and will have functional similarities.

Techniques known to those skilled in the art may be suitable to identify analogous genes and analogous enzymes or any biosynthetic pathway genes, proteins, or enzymes, techniques may include, but are not limited to, cloning a gene by PCR using primers based on a published sequence of a gene/enzyme of interest, or by degenerate PCR using degenerate primers designed to amplify a conserved region among a gene of interest. Further, one skilled in the art can use techniques to identify homologous or analogous genes, proteins, or enzymes with functional homology or similarity. Techniques include examining a cell or cell culture for the catalytic activity of an enzyme through *in vitro* enzyme assays for said activity (e.g. as described herein or in Kiritani, K., Branched-Chain Amino Acids Methods Enzymology, 1970), then isolating the enzyme with said activity through purification, determining the protein sequence of the enzyme through techniques such as Edman degradation, design of PCR primers to the likely nucleic acid sequence, amplification of said DNA sequence through PCR, and cloning of said nucleic acid sequence. To identify homologous or similar genes and/or homologous or similar enzymes, analogous genes and/or analogous enzymes or proteins, techniques also include comparison of data concerning a candidate gene or enzyme with databases such as BRENDA, KEGG, or MetaCYC. The candidate gene or enzyme may be identified within the above mentioned databases in accordance with the teachings herein.

### DERIVATIVES

The term "Hyaluronic acid (HA)", as used herein, is also intended to cover derivatives of Hyaluronic acid, such as but not limited to acetylated or sulfated Hyaluronic acid useful for applications in a cosmetic product, flavor product a fragrance product, a food product, a food, a beverage, a texturant, a pharmaceutical composition, a dietary supplement, a nutraceutical, a cleaning product and/or a dental and/or an oral hygiene composition or combinations thereof

Hyaluronic acid may be prepared as a composition.

### FORMULATIONS AND PRODUCTS

A composition of the invention may be incorporated into a formulation/product, such as a nutraceutical, pharmaceutical, veterinary, oenological or cosmetic formulation/product.

Thus, the present invention provides a formulation comprising a composition of the invention. For example, the present invention may provide a cosmetic formulation comprising the composition of the invention.

The present invention also provides a product comprising a composition of the invention. For example, the present invention may provide a cosmetic product comprising the composition of the invention.

A "cosmetic product" is intended to mean any substance or mixture intended to be placed in contact with the external parts of the human body (epidermis, hair system, nails, lips and external genital organs) or with the teeth and the mucous membranes of the oral cavity with a view exclusively or mainly to cleaning them, perfuming them, changing their appearance, protecting them, keeping them in good condition, correcting body odours and/or combinations thereof.

A "substance" is intended to mean a chemical element and its compounds in the natural state or obtained by any manufacturing process, including any additive necessary to preserve its stability and any impurity deriving from the process used but excluding any solvent which may be separated without affecting the stability of the substance or changing its composition.

A "mixture" is intended to mean a mixture or solution composed of two or more substances.

The present invention also provides the use of a composition of the invention in a nutraceutical, pharmaceutical, veterinary, oenological or cosmetic formulation/product.

Such formulations or products are hereinafter referred to as the "formulations or products of the invention".

The nutraceutical, pharmaceutical, veterinary, oenological or cosmetic formulation/product may optionally further comprise pharmaceutically/veterinary/cosmetic (including cosmetic active) ingredients, such as excipients, carriers and mixtures thereof as appropriate.

"Cosmetic" or "Cosmetic Active Ingredients" means any and all natural, naturally occurring, nature identical, synthetic synthetically produced, biosynthetically produced, sustainable, renewable and/or biodegradable compounds, ingredients, intermediates, molecules, substances, raw materials or products individually or as part of a mixture of compounds, ingredients, intermediates, molecules, substances, raw materials or products, blends, compositions, formulations (including but not limited to skin moisturizers, creams, balms, serums, oils, eye, facial makeup, wash off hair products, leave-on hair products, hair colorants (including but not limited to natural hair colorants) and/or combinations thereof), finished products and related technologies including, but not limited to components, incorporated, for example, into a cosmetic formulation (such as but not limited to natural colorants, preservatives, emulsifiers, anti-oxidants and the like which do not, for example, have an activity on the skin, hair, scalp and the like but play a role in the formulation of the finished product), delivery systems, marketing aids (such as, for example, coloured unispheres applied to translucent formulations) and methods of making anything related thereto useful in/used for/intended for:
- application by rubbing, pouring, sprinkling, spraying or otherwise directly on or to a human or animal body and/or by placing in contact with the various external and/or on surface parts of a human or animal body (including but not limited to the skin, hair, body hair, the hair system, scalp, nails, lips, external genitalia, teeth, oral and/or nasal mucosa and the like); and/or application indirectly on or to a human or animal body such as for example, application as part of a textile or application to a textile as part of a delivery device (such as a capsule) or a delivery system (such as blends or formulations) applied to a textile; and/or
- cleansing, caring, cooling, beautifying, conditioning, treating, soothing, texturizing, promoting attractiveness, protecting, maintaining, improving, enhancing, altering and/or changing an external part and/or surface of a human or animal body (such as, but not limited to the scalp) or the aesthetic appearance of a human or animal body; and/or with a view to mainly cleaning or perfuming, or protecting or maintaining in good condition or combating body odour or changing the appearance of or correcting or repairing a state of imbalance in skin, oral mucosa, scalp or hair by providing a calming, healing, repairing, or revitalization, hydration of the skin or in order to provide relief to, lubricate, moisten, tone, heal, sterilize, relieve, correct and/or remedy states of dryness, irritation, injury or fatigue, and/or with a view to correcting pigmentation disorders or providing a non-pharmaceutical prevention and/or treatment of dandruff, acne, irritation and/or inflammation and the like and/or rebalancing the bacterial flora (such as, for example, the microbiome) on the surface of the skin (such as, for example, by promoting the level of beneficial bacterial flora on the skin surface) and/or for the purposes of keeping a human or animal body in good condition for health and/or wellbeing purposes and/or for improving the appearance of a human or animal body by, for example, improving the appearance of a product applied to a human or body; and/or
- providing a cosmetic and/or dermatological function and/or benefit with a biological activity benefit (but without affecting a body's structure or function. For the avoidance of doubt, a Cosmetic or Cosmetic Active Ingredient or any part thereof may also qualify as a Functional Ingredient and/or Nutraceutical.

"Functional Ingredient" means a food ingredient or part of a food that provides medicinal or health benefits, including any of the following: a carotenoid, dietary fiber, fatty acid, saponin, antioxidant, flavonoid, isothiocyanate, phenol, polyphenol (such as resveratrol), plant sterol or stanol (phytosterols and phytostanols), a polyol, a prebiotic, a phytoestrogen, soy protein, sulfides/thiol, a vitamin, glucosamine, preservatives, hydration agents, edible gelling ingredients, edible gel mixes and gel compositions, long chain primary aliphatic saturated alcohols, colour agents, texturizing agents, emulsifiers and combinations thereof.

"Nutraceutical" means any and all natural, naturally occurring, sustainable, synthetically-produced and bio-synthetically-produced compounds, mixtures of compounds, Functional Ingredients, molecules, compositions, raw materials, and intermediates (including components and delivery devices (such as capsules) related thereto, delivery systems thereof (such as blends or formulations) and methods of making the foregoing) that are associated with health and/or cosmetic benefits, as well as improving or maintaining the appearance of the human body. For the avoidance of doubt, Nutraceuticals includes compounds that can be used as supplements to food or beverage, whether a solid formulation, capsule, tablet, liquid formulation, solution or suspension.

Alternatively, the nutraceutical, pharmaceutical, veterinary, oenological or cosmetic formulation/product may consist or consist essentially of the composition of the invention.

The cosmetic formulation/product may be an anti-aging formulation.

As used herein, references to pharmaceutically, veterinary or cosmetically acceptable excipients may refer to pharmaceutically, veterinary or cosmetically acceptable adjuvants, diluents and/or carriers as known to those skilled in the art.

By "pharmaceutically/veterinary/cosmetically acceptable" we mean that the additional components of the composition are generally safe, non-toxic, and neither biologically nor otherwise undesirable. For example, the additional components may be generally sterile and pyrogen free. Such components must be "acceptable" in the sense of being compatible with the composition of the invention and not deleterious to the recipients thereof. Thus, "pharmaceutically acceptable excipients" includes any compound(s) used in forming a part of the formulation that is intended to act merely as an excipient, i.e. not intended to have biological activity itself.

The nutraceutical, pharmaceutical, veterinary, oenological or cosmetic formulation/product may be in the form of a liquid or a solid.

Liquid dosage formulations/products for oral administration include solutions, emulsions, aqueous or oily suspensions, syrups and elixirs.

Formulations and products (e.g. pharmaceutical, veterinary or cosmetic formulations/products) described herein, such as those intended for oral administration, may be prepared according to methods known to those skilled in the art, such as by mixing the components of the formulation/product together.

The formulation or product (e.g. pharmaceutical, veterinary or cosmetic formulation/product) may contain one or more additional ingredients, such as pharmaceutical ingredients and excipients, such as sweetening agents, flavouring agents, colouring agents and preserving agents.

The formulation or product (e.g. pharmaceutical, veterinary or cosmetic formulation/product) may also contain one or more additional active ingredients, such as cosmetic or pharmaceutical active ingredients, such as hyaluronic acid, centella asiatica extract, peptides such as Matrixyl^{®} and Argireline^{®}, and mixtures thereof.

The formulation or product of the invention may contain the active ingredient(s) in admixture with non-toxic pharmaceutically acceptable excipients (or ingredients). These excipients (or ingredients) may, for example, be: inert diluents, such as calcium carbonate, sodium carbonate, lactose, calcium phosphate or sodium phosphate; granulating and disintegrating agents, for example, corn starch, maltodextrin or alginic acid; binding agents, for example, starch, gelatine or acacia; or lubricating agents, for example magnesium stearate, stearic acid, talc and mixtures thereof.

Liquid formulations or products (e.g. pharmaceutical, veterinary or cosmetic formulations/products) may be contained within a capsule, which may be uncoated or coated as defined above.

Suitable pharmaceutical or veterinary carriers include inert solid diluents or fillers, sterile aqueous solutions and various organic solvents. Examples of liquid carriers are syrup, peanut oil, olive oil, phospholipids, fatty acids, fatty acid amines, polyoxyethylene and water.

Moreover, the carrier or diluent may include any sustained release material known in the art, such as glyceryl monostearate or glyceryl distearate, alone or mixed with a wax.

Suitable pharmaceutical carriers include inert sterile aqueous solutions and various organic solvents. Examples of liquid carriers are syrup, vegetables oils, phospholipids, fatty acids, fatty acid amines, polyoxyethylene and water. Moreover, the carrier or diluent may include any sustained release material known in the art, such as glyceryl monostearate or glyceryl distearate, alone or mixed with a wax.

Suitable cosmetic carriers are typically those that are suitable for topical administration to the outer surface of the human body, such as the skin and/or hair and/or scalp.

Typically, such carriers are dermatologically acceptable.

The phrase "dermatologically acceptable carrier" means that the carrier is suitable for topical application to the keratinous tissue, has good aesthetic properties, is compatible with the actives in the composition, and will not cause any unreasonable safety or toxicity concerns.

The carrier can be in a wide variety of forms. In some instances, the solubility or dispersibility of the components (e.g. extracts, sunscreen active, additional components) may dictate the form and character of the carrier. Non-limiting examples include simple solutions (e.g. aqueous or anhydrous), dispersions, emulsions, and solid forms (e.g. gels, sticks, flowable solids, or amorphous materials).

The dermatologically acceptable carrier may be in the form of an emulsion. An emulsion may be generally classified as having a continuous aqueous phase (e.g. oil-in-water and water-in-oil-in-water) or a continuous oil phase (e.g. water-in-oil or oil-in-water). The oil phase of the present invention may comprise silicone oils, non-silicone oils such as hydrocarbon oils, esters, ethers, and the like, and mixtures thereof. The aqueous phase typically comprises water and water-soluble ingredients (e.g. water-soluble moisturizing agents, conditioning agents, anti-microbials, humectants and/or other skin care actives). However, in some instances, the aqueous phase may comprise components other than water, including but not limited to watersoluble moisturizing agents, conditioning agents, antimicrobials, humectants and/or other watersoluble skin care actives. In some instances, the non-water component of the composition comprises a humectant, such as glycerin and/or other polyol(s). Emulsions may also contain an emulsifier. Emulsifiers may be non-ionic, anionic or cationic.

The carrier may contain one or more dermatologically acceptable, hydrophilic diluents. As used herein, "diluent" includes materials in which the composition of the invention can be dispersed, dissolved, or otherwise incorporated. Hydrophilic diluents include water, organic hydrophilic diluents, such as lower monovalent alcohols (e.g., C1-C4), and low molecular weight glycols and polyols, including propylene glycol, polyethylene glycol, polypropylene glycol, glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof.

The cosmetic formulation/product may optionally include one or more additional ingredients commonly used in cosmetic compositions (e.g., colorants, skin tone agents, skin anti-aging agents, anti-inflammatory agents, sunscreen agents, combinations of these and the like), provided that the additional ingredients do not undesirably alter the anti-glycation benefits provided by the composition.

In some instances, it may be desirable to select skin tone agents that function via different biological pathways so that the actives do not interfere with one another, which could reduce the efficacy of both agents. The additional ingredients, when incorporated into the composition, should be suitable for use in contact with human skin tissue without undue toxicity, incompatibility, instability, allergic response, and the like.

The term "carrier" as used herein, may also refer to a natural product or a product originating from nature that has been transformed or modified so that it is distinct from the natural product from which it originated, such as maltodextrin.

The amount of the composition of the invention present in nutraceutical, pharmaceutical, veterinary, oenological or cosmetic formulations or products will vary depending on the application.

Typically, the amount of composition of the invention that may be present in nutraceutical, pharmaceutical, veterinary, oenological or cosmetic formulations or products will be from about 0.001 to about 50% by weight, such as from about 0.01% to about 30% or from about 1% to about 20% of the nutraceutical, pharmaceutical, veterinary, oenological or cosmetic formulations or products, such as from about 0.01 to about 20%, or from about 0.1 to 10% or from about 1 to about 5% by weight of the formulation or product.

Those of skill in the art will recognize that, due to the degenerate nature of the genetic code, a variety of DNA molecules differing in their nucleotide sequences can be used to encode a given enzyme of the disclosure. The native DNA sequence encoding the biosynthetic enzymes described above are referenced herein merely to illustrate an embodiment of the disclosure, and the disclosure includes DNA molecules of any sequence that encode the amino acid sequences of the polypeptides and proteins of the enzymes utilized in the methods of the disclosure. In similar fashion, a polypeptide can typically tolerate one or more amino acid substitutions, deletions, and insertions in its amino acid sequence without loss or significant loss of a desired activity. The disclosure includes such polypeptides with different amino acid sequences than the specific proteins described herein so long as the modified or variant polypeptides have the enzymatic anabolic or catabolic activity of the reference polypeptide. Furthermore, the amino acid sequences encoded by the DNA sequences shown herein merely illustrate embodiments of the disclosure.

Described herein are specific genes and proteins useful in the methods, compositions and organisms of the disclosure; however it will be recognized that absolute identity to such genes is not necessary. For example, changes in a particular gene or polynucleotide comprising a sequence encoding a polypeptide or enzyme can be performed and screened for activity. Typically such changes comprise conservative mutations and silent mutations. Such modified or mutated polynucleotides and polypeptides can be screened for expression of a functional enzyme using methods known in the art.

Due to the inherent degeneracy of the genetic code, other polynucleotides which encode substantially the same or functionally equivalent polypeptides can also be used to clone and express the polynucleotides encoding such enzymes.

Techniques known to those skilled in the art may be suitable to identify additional homologous genes and homologous enzymes. Generally, analogous genes and/or analogous enzymes can be identified by functional analysis and will have functional similarities.

Techniques known to those skilled in the art may be suitable to identify analogous genes and analogous enzymes or any biosynthetic pathway genes, proteins, or enzymes, techniques may include, but are not limited to, cloning a gene by PCR using primers based on a published sequence of a gene/enzyme of interest, or by degenerate PCR using degenerate primers designed to amplify a conserved region among a gene of interest. Further, one skilled in the art can use techniques to identify homologous or analogous genes, proteins, or enzymes with functional homology or similarity. Techniques include examining a cell or cell culture for the catalytic activity of an enzyme through *in vitro* enzyme assays for said activity (e.g . as described herein or in Kiritani, K., Branched-Chain Amino Acids Methods Enzymology, 1970), then isolating the enzyme with said activity through purification, determining the protein sequence of the enzyme through techniques such as Edman degradation, design of PCR primers to the likely nucleic acid sequence, amplification of said DNA sequence through PCR, and cloning of said nucleic acid sequence. To identify homologous or similar genes and/or homologous or similar enzymes, analogous genes and/or analogous enzymes or proteins, techniques also include comparison of data concerning a candidate gene or enzyme with databases such as BRENDA, KEGG, or MetaCYC. The candidate gene or enzyme may be identified within the above-mentioned databases in accordance with the teachings herein.

The terms "between... and..." and "ranging from... to..." should be understood as being inclusive of the limits, unless otherwise specified. Throughout this specification and the claims which follow, unless the context requires otherwise, the word "comprise", and variations such as "comprises", "comprising" and "possesses", will be understood to imply the inclusion of a stated integer or step or group of integers or steps but not the exclusion of any other integer or step or group of integer or step. The term "comprising" also means "including" as well as "consisting" e.g. a composition "comprising" X may consist exclusively of X or may include something additional e.g. X + Y. It must be noted also that, as used in this specification and the appended claims, the singular forms "a", "an" and "the" include plural referents unless the content clearly dictates otherwise. By way of example, a reference to "a gene" or "an enzyme" is a reference to "one or more genes" or "one or more enzymes".

It is to be understood that this disclosure is not limited to the particular methodology, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by the person skilled in the art. In accordance with the present disclosure there may be conventional molecular biology, microbiology, and recombinant DNA techniques employed which are within the skill of the art.

This disclosure is not limited in its application to the details of construction and the arrangement of components set forth in the following description or illustrated in the drawings. The disclosure is capable of other embodiments and of being practiced or of being carried out in various ways. Also, the phraseology and terminology used herein is for the purpose of description and should not be regarded as limiting. Preferably, the terms used herein are defined as described in "A multilingual glossary of biotechnological terms: (IUPAC Recommendations)", Leuenberger, H.G.W, Nagel, B. and Kolbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety.

The examples and figures which follow are presented by way of illustration and without implied limitation of the invention.

### EXAMPLES

### Exemple 1: Protocol for making a recombinant Saccharomyces cerevisiae strain according to the invention

All the hereinafter implemented recombinant *Saccharomyces cerevisiae* strains were constructed from standard strains using standard yeast molecular genetics procedure (Methods in yeast Genetics - A cold spring harbor laboratory course Manual (2000) by D. Burke, D. Dawson, T. Stearns CSHL Press).

Cluster of the following-mentioned genes were integrated in recombinant yeast at once using the ability of yeast to efficiently recombine free DNA ends which have sequence homology.

In addition, for a better comprehension of following genotypes:
- jlp1, lyp1, sam3, his3, leu2, trp1 and ura3 are insertion sites.
- Lowercase letters mean that the considered gene is inactive, uppercase letters reflect an active gene.
- "::": following a gene name means that the gene is interrupted by what follows (if more than one gene are inserted, they are noted in brackets []). The interruption of the gene is concomitant with an entire deletion of the coding sequence but preserves the promoter. In consequence the gene followed by "::" is inactive and is noted in lowercase. If not specified the transcription of the gene inserted is controlled by the promoter of the disrupted gene.
- "gene.K1" means that the gene originates from *Kluyveromyces lactis.*

More particularly, the coding sequences to be cloned were artificially synthetized. For heterologous sequences (non-yeast), the nucleic sequences were modified in order to obtain a synonymous coding sequence using the yeast codon usage. Using restriction enzyme and classical cloning technology, each synthetic sequence was cloned in between a transcription promoter and a transcription terminator. Each promoter sequence is preceded by a 50 to 200 nucleotide sequence homologous to the sequence of the terminator of the upstream gene. Similarly, the terminator of each gene (a gene comprising the promoter-coding sequence-terminator) is followed by sequences homologous to the gene immediately following. So that each of the unit to be integrated have a 50-200 nucleotide overlap with both the unit upstream and the unit downstream. For the first unit, the promoter is preceded by 50-200 nucleotides homologous to the yeast chromosome nucleotide for the locus in which it will be integrated. Similarly, for the last unit, the terminator is followed by 50-200 nucleotides homologous to the yeast chromosome nucleotide for the locus in which it will be integrated.

Each unit is then PCR amplified from the plasmids constructs, yielding X unit of linear DNA having overlapping sequences. At least one of this gene is an auxotrophic marker, in order to select for recombination event. All the linear fragments are transformed in the yeast at once, and a recombinant yeast cell is selected for the auxotrophy related to the marker used. The integrity of the sequence is then verified by PCR and sequencing.

### Example 2: Comparative examples for the production of Hyaluronic acid

### 1. Production of Hyaluronic acid of less than 50 kDa

A. Firstly, three recombinant strains are obtained: YA5234-1, YA5235-1 and YA5302-2.

Accordingly, these three strains are as follows:
**YA5234-1** : MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pCCW12.sba-HYAL-3.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5235-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pNUP57-HYAL-3.Csa-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5302-2** : MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pCCW12.Sba-HYAL-3.Li-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1-TRP1]x2

HYAL-3 represents a nucleic acid sequence encoding a polypeptide having hyaluronidase activity associated with a secretion signal but no anchoring signal.

HASA, HASA-1, HASA-A, HASA2 and XHASA2 all represent a nucleic acid encoding a polypepyide having hyaluronan synthase activity. They differ from one another in that they are different reencoded versions of a nucleic acid sequence encoding the hyaluronan synthase enzymes. HASA-1 has the sequence SEQ ID NO: 1, HASA-A has the sequence SEQ ID NO: 2, HASA2 has the sequence SEQ ID NO: 7 and XHASA2 has the sequence SEQ ID NO: 6.

HASB and HASB-A represent a nucleic acid sequence encoding a polypeptide having UDP-glucose 6-dehydrogenase activity. They differ from one another in that they are different reencoded versions of a nucleic acid sequence encoding the enzyme. HASB.At has the sequence SEQ ID NO: 12, HASB.Vir has the sequence SEQ ID NO: 13 and HASB-A.Vir has the sequence SEQ ID NO: 14.

All these strains were incubated in 25 ml of SY medium buffered with MES 0,1 M ph 5.5 in a baffled erlenmeyer for 48 hours at 28°C under vigourous agitation.

SY medium comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeC13: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM ; Myo-Inositol (C6H12O6): 555 µM ; Nicotinic acid (C6H5NO2): 29 µM; D-Biotine: 0,82 µM; Ammonium citrate tribasic: 33 mM; and glucose or sucrose 2-30%.

Growth medium was recovered at 48h hours and assayed for hyaluronic acid content and quality. An aliquot of the medium was loaded and ran on a 0.5% agarose gel subsequently colored with "stains all" (sigma Aldrich CAS Number 7423-31-6).

The amount of hyaluronic acid present in the medium was evaluated by a colorimetric determination after treatment with concentrated sulfuric acid and carbazole (Bitter and Muir (1962) analytical biochemistry 4, 330-334).

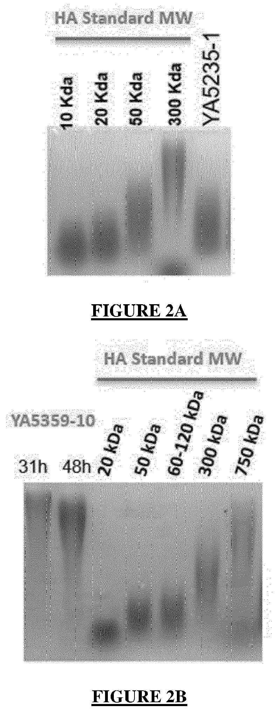

For illustrative purposes, the agarose gel obtained after running an aliquot of the supernatant of strain YA5235-1 and staining it with "stains all" (CAS Number 7423-31-6) is provided as Figure 2A. It is classically run into the gel side-by-side with HA molecular weight (MW) standards (from left to right MW of 10 kDa, 20 kDa, 50 kDa and 300 kDa).

The amounts obtained with these different strains are respectively:
- YA5234-1: 2.4 g.L⁻¹.
- YA5235-1: 4.5 g.L⁻¹.
- YA5302-2: 3.7 g.L⁻¹.

In comparision, a native yeast strain, as for example the CC788-2D strain (Chérest et al. (2000) J Biol. Chem. 275: 14056-14063), does not produce hyaluronic acid.

It results from this experiment that a recombinant strain comprising the modifications according to the invention produces a greater amount of hyarluronic acid when cultured in the same conditions as compared with other recombinant strains not comprising all the genetic modifications according to the invention.

Moreover, these three strains led to the production, after 48 hours, of hyarluronic acid having a molecular weight comprised between 20kDa and 50kDa.

**B.** Three other recombinant strains have also been obtained: YA5110-13 and YA5260-4.

These three strains are as follows:
**YA5110-13:** MAT-α, can1-100, his3::[pSAM1-UGP1-tRPL3, pMET6-QRI1-tIDP1-HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pNUP57-HYAL-3.Hn-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5260-4:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pTEF3-HYAL-31.Hn-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1- TRP1]x2
**YA5569** : MAT-α, his3::[pSAM1-UGP1- tRPL3, pMET6-QRI1-tIDP1, HIS3]x5, jlp1::[LEU2.K1, pCUP1-HASA-1.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pCWP2-HYAL-31.Csa-tRPL15A, pCCW12.Sba-HASB.vir-tRPL3, pCCW12.Sk-HASB-A.Vir-tTEFl.Sba, pCCW12-HASA-1.Vir-tRPL41B, pCCW12.Sm-HASA-A.Vir-tRPL15A.Sba], sam3::[LEU2.Kl, pPDC1-PGM1-tIDP1, pTEF1.Ago-GFA1-tRPL15A, pENO2-UGP1-tRPL3, pCWP2-GNA1-tTPI1, pTEF1-PCM1-tIDP1, pCCW12-XHASA2.X1-tRPL3, pTDH3-QRI1-tIDP1], trp1::[tRPL3-HASB.Vir-pMET6, pMET25-HASA-1.Vir-tIDP1, TRP1]x2

HYAL-31 represents a nucleic acid sequence encoding a polypeptide having hyaluronidase activity associated with both a secretion signal and an anchoring signal.

HASA-1, HASA-A, HASA2 and XHASA2 all represent a nucleic acid sequence encoding a polypeptide having hyaluronan synthase activity. They differ from one another in that they are different reencoded versions of a nucleic acid sequence encoding the hyaluronan synthase enzyme. HASA-1 has the sequence SEQ ID NO: 1, HASA-A has the sequence SEQ ID NO: 2, HASA2 has the sequence SEQ ID NO: 7 and XHASA2 has the sequence SEQ ID NO: 6.

HASB and HASB-A represent a nucleic acid sequence encoding a polypeptide having UDP-glucose 6-dehydrogenase activity. They differ from one another in that they are different reencoded versions of a nucleic acid sequence encoding the enzyme. HASB.At has the sequence SEQ ID NO: 12, HASB.Vir has the sequence SEQ ID NO: 13 and HASB-A.Vir has the sequence SEQ ID NO: 14.

The strains were inoculated in 2 liters of the above defined SY medium at ph 6 in 2% sucrose and 500µM CuSO4 in a bioreactor. They were then grown in fed-batch adding 500 µM CuSO4 at 7h, 24h, 31h and between 130 and 175 g.L-1 of sucrose from 10 to 48 h.

For YA5110-13, pH was maintained between 5.8 and 6.2 using 15% H2SO₄ and 10% NH4OH from t=31 h.

For YA5260-4, pH maintained between 5.8 and 6.2 using 15% H2SO₄ and 10% NH4OH all along the fermentation.

For YA5569, pH maintained between 5.8 and 6.2 using 15% H2SO₄ and 10% NH4OH all along the fermentation.

Growth medium was recovered at 48h hours and assayed for hyaluronic acid content and quality as described above.

The hyaluronic acid amounts obtained with these two strains are respectively:
- YA5110-13: 23 g.L⁻¹.
- YA5260-4: 16 g.L⁻¹.
- YA5569: 32 g.L⁻¹

In comparision, a native strain does not produce hyaluronic acid.

It results from this experiment that a recombinant strain comprising the modifications according to the invention produces a greater amount of hyaluronic acid when cultured in the same conditions as compared with other recombinant strains not comprising all the genetic modifications according to the invention.

Moreover, the hyaluronic acid produced by YA5110-13 and YA5260 strains had a molecular weight of about 20 kDa while the hyaluronic acid produced by YA5569 had a molecular weight of about 20 kDa to 50 kDa.

The exact same experiment was performed using these three recombinant strains while replacing sucrose by glucose. The hyaluronic acid obtained had the same molecular weight of about 20 kDa or of about 20 kDa to about 50 kDa after 48h.
C. Another recombinant strain has been obtained 5672-29A, as follows:
**5672-29A:** MAT-α, his3::[pSAM1-UGP1-tRPL3, pMET6-QRI1-tIDP1, HIS3]x5, jlp1::[LEU2.Sba, pTEF1.Sba-HASB.Vir-tRPL3.Sm, pTDH3.Sk-HASB.Vir-tTEF1.Sba, pTDH3-1.Sba-HASA-1.Vir-tRPL3.Sba, pTDH3.Sar-HASA-1.Vir-tRPL15A.Sba, pTEF1-HYAL-31.Hn-tRPL15A], leu2, sam3::[LEU2.Kl, pPDC1-PGM1-tIDP1, pTEF1.Ago-GFA1-tRPL15A, pENO2-UGP1-tRPL3, pCWP2-GNA1-tTPI1, pTEF1-PCM1-tRPL41B, TRP1.Sba-loxP], trp1

HYAL-31 represents a hyaluronidase associated with an anchoring signal.

1 HASA-1 represents a nucleic acid sequence encoding a polypeptide having hyaluronan synthase activity. HASA-1 has the sequence SEQ ID NO: 1.

The strain was inoculated in 25 ml of the above defined SY medium at ph 5.5 buffered with MES 0.1 M in a baffled erlenmeyer at 28°C under vigourous agitation.

Growth medium was recovered at 48h hours and assayed for hyaluronic acid content and quality as described above.

The hyaluronic acid amount obtained with the strain was 3.7 g.L⁻¹.

### 2. Production of Hyaluronic acid having a molecular weight comprised between 50 kDa and 1000 kDa

**A.** Firstly, three recombinant strains are obtained: YA5233-1, YA5359-10 and YA5381-1.

Accordingly, these three strains are as follows:
**YA5233-1:** MAT-α, can1-100, his3::[pSAM1-UGP1-tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pTEFl-HYAL-3.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5359-10:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pCCW12.sba-HYAL-31.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5381-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pCCW12.Sar-HYAL2-31.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[ LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2

HASA-1, HASA-A, HASA2 and XHASA2 are previously defined.

HASB and HASB-A are previously defined.

All these strains were incubated in 25 ml of the above-defined SY medium buffered with MES 0,1 M ph 5.5 baffled erlenmeyer for 48 hours at 28°C under vigourous agitation.

Growth medium was recovered at 48h hours and assayed for hyaluronic acid content and quality as defined above.

For illustrative purposes, the agarose gel obtained after running an aliquot of the supernatant of strain YA5359-10, after 31h and after 48h, and staining it with "stains all" (CAS Number 7423-31-6) is provided as Figure 2B. It is classically run into the gel side-by-side with HA molecular weight (MW) standards (from left to right MW of 20 kDa, 50 kDa, 60-120 kDa, 300 kDa and 750 kDa).

The amounts obtained with these different strains are respectively:
- YA5233-1: 3.1 g.L⁻¹.
- YA5359-10: 3.4 g.L⁻¹.
- YA5381-1: 3.0 g.L⁻¹.

In comparision, a native strain does not produce hyaluronic acid.

It results from this experiment that a recombinant strain comprising the modifications according to the invention produces a greater amount of hyarluronic acid when cultured in the same conditions as compared with other recombinant strains not comprising all the genetic modifications according to the invention.

Moreover, these three strains led to the production, after 48 hours, of hyaluronic acid having a molecular weight comprised:
- for YA5233-1: between 50 kDa and 500 kDa;
- for YA5359-10: between 100 kDa and 750 kDa; and
- for YA5381-1: between 50 kDa and 250 kDa.

**B.** Six other recombinant strains have also been obtained: YA5262-16, YA5326-3, YA5300-15, YA5358-4, YA5264-1 and YA5331-6.

These six strains are as follows:
**YA5262-16**: MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pCCW10.ago-HYAL-31.Hn-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1- TRP1]x2
**YA5326-3:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pCCW10.ago-HYAL-31.Csa-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5300-15:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pTDH3-HYAL-3.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1-TRP1]x2
**YA5358-4:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tMET25, pCCW12-HASB.At-tRPL15A], leu2, lyp1::[pCCW12.Sar-HYAL-31.Li-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5264-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pTDH3-HYAL-31.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5331-6:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pTEF3-HYAL-31.Li-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[ LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1- TRP1]x2

HASA-1, HASA-A, HASA2 and XHASA2 are previously defined.

HASB and HASB-A are previously defined.

YA5262-16 was inoculated in 2 liters of the above defined SY medium at ph 4 in 2% glucose in a bioreactor and ph was maintained at 4 all along the fermentation using 15% H₂SO₄ and 10% NH₄OH.

YA5326-3 was:
- inoculated in 2 liters of the above defined SY medium at ph 6 in 2% glucose in a bioreactor and ph was maintained at 6 all along the fermentation using 15% H₂SO₄ and 10% NH₄OH; or
- inoculated in 2 liters of the above defined SY medium at ph 6 in 2% sucrose in a bioreactor and ph was maintained at 6 until 34h and at ph 4 from 34h to 48h with 15% H₂SO₄ and 10% NH₄OH.

YA5300-15 was inoculated in 2 liters of the above defined SY medium at ph 6 in 2% sucrose in a bioreactor and ph was maintained at 6 all along the fermentation using 15% H₂SO₄ and 10% NH₄OH.

YA5358-4 was inoculated in 2 liters of the above defined SY medium at ph 6 in 2% glucose in a bioreactor and ph was maintained at 6 all along the fermentation using 15% H₂SO₄ and 10% NH₄OH.

YA5264-1 was inoculated in 2 liters of the above defined SY medium at ph 6 in 2% sucrose in a bioreactor and ph was maintained at 6 all along the fermentation using 15% H₂SO₄ and 10% NH₄OH.

YA5331-6 was inoculated in 2 liters of the above defined SY medium at ph 6 in 2% glucose in a bioreactor and ph was maintained at 6 all along the fermentation using 15% H₂SO₄ and 10% NH₄OH.

Growth medium was recovered at 48h hours and assayed for hyaluronic acid content and quality as described above.

The hyaluronic acid amounts obtained with these two strains are respectively:
- YA5262-16: 19 g.L⁻¹;
- YA5326-3: respectively 13 g.L⁻¹ and 15 g.L⁻¹;
- YA5300-15: 9 g.L⁻¹;
- YA5358-4: 14 g.L⁻¹;
- YA5264-1: 6 g.L⁻¹; and
- YA5331-6: 9 g.L⁻¹.

In comparison, a native strain does not produce hyaluronic acid.

It results from this experiment that a recombinant strain comprising the modifications according to the invention produces a greater amount of hyaluronic acid when cultured in the same conditions as compared with other recombinant strains not comprising all the genetic modifications according to the invention.

Moreover, the hyaluronic acid produced by these strains after 48 hours had a molecular weight:
- YA5262-16: of 50 kDa;
- YA5326-3: of respectively 50 kDa or comprised between 50 kDa and 250 kDa;
- YA5300-15: comprised between 100 kDa and 1000 kDa;
- YA5358-4: comprised between 100 kDa and 1000 kDa;
- YA5264-1: comprised between 100 kDa and 1000 kDa; and
- YA5331-6: comprised between 100 kDa and 1000 kDa.

Furthermore, previously described recombinant strain YA5235-1 was inoculated in a bioreactor at ph 6 in 2% sucrose and ph was maintained at 6 untill 34h and at 4 from 34h to 48h with 15% H₂SO₄ and 10% NH₄OH.

After 48h the medium contains 18 g.L⁻¹ of 100-1000 kDa Hyaluronic acid.

The same experiment was performed using YA5235-1 while using glucose instead of sucrose. The obtained hyaluronic acid also has a molecular weight comprised between 100 and 1000 kDa.

These results show that, in particular, pH regulation along the fermentation process as well as the production of a hyaluronidase coupled to a secretion signal or a secretion signal and an anchoring signal influences, or even allows controling, the size of the hyaluronic acid polymer produced.

### 3. Production of Hyaluronic acid having a molecular weight superior to 1000 kDa

Four recombinant strains are obtained: YA5232-1, YA5303-3, YA5374-4 and YA5382-1.

Accordingly, these four strains are as follows:
**YA5232-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pNUP57-HYAL-3.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5303-2:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pNUP57-HYAL-3.Li-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1-TRP1]x2
**YA5374-3:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pCCW12.Sar-HYAL-3.Ba-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5382-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pTEF1-HYAL2-31.Ba-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.Kl, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2

HASA-1, HASA-A, HASA2 and XHASA2 are previously defined.

HASB and HASB-A are previously defined.

All these strains were incubated in 25 ml of the above-defined SY medium buffered with MES 0,1 M ph 5.5 in a baffled erlenmeyer for 48 hours at 28°C under vigourous agitation.

Growth medium was recovered at 48h hours and assayed for hyaluronic acid content and quality as defined above.

The amounts obtained with these different strains are respectively:
- YA5232-1: 4 g.L⁻¹.
- YA5303-3: 2.7 g.L⁻¹.
- YA5374-3: 2.5 g.L⁻¹.
- YA5382-1: 3.0 g.L⁻¹.

In comparision, a native strain does not produce hyaluronic acid.

It results from this experiment that a recombinant strain comprising the modifications according to the invention produces a greater amount of hyarluronic acid when cultured in the same conditions as compared with other recombinant strains not comprising all the genetic modifications according to the invention.

Moreover, these four strains led to the production, after 48 hours, of hyaluronic acid having a molecular weight:
- for YA5232-1: greater than about 1000 kDa;
- for YA5303-3: of about 1000 kDa;
- for YA5374-3: greater than about 1000 kDa; and
- for YA5382-1: greater than about 1000 kDa.

### SEQUENCES

SEQ ID NO: 1 is a reencoded nucleic acid sequence of hyaluronan synthase (HASA) originating from Chlorella virus PBCV-1
ATGGGTAAGAACATCATTATTATGGTTTCTTGGTACACTATTATTACATCCAATCTCATCGCAGTGGGTGGCGCC
TCACTCATACTAGCCCCAGCTATTACGGGCTATGTCCTTCACTGGAACATTGCCCTTTCAACAATTTGGGGAGTG
TCGGCCTACGGAATTTTCGTGTTTGGTTTCTTTCTTGCCCAGGTATTATTTAGTGAACTCAACCGGAAAAGGCTC
CGGAAGTGGATTTCCCTCCGACCCAAAGGGTGGAATGATGTTAGGTTGGCTGTAATTATTGCCGGCTACCGTGAG
GACCCGTATATGTTCCAAAAGTGTCTTGAAAGTGTGCGTGACTCAGACTATGGGAATGTAGCTAGACTAATATGC
GTTATTGACGGCGATGAAGACGACGACATGAGGATGGCTGCAGTGTACAAGGCTATCTATAACGACAACATCAAG
AAACCTGAGTTTGTCCTCTGTGAGAGTGACGATAAGGAGGGTGAGAGAATAGATAGCGATTTCAGCCGTGATATC
TGCGTGCTGCAACCGCATCGCGGAAAGCGTGAATGTTTGTACACAGGGTTCCAATTGGCAAAGATGGACCCCTCA
GTTAATGCCGTCGTCCTAATCGACAGTGACACTGTGTTAGAAAAGGACGCGATTCTCGAaGTAGTATACCCGCTG
GCATGCGATCCAGAAATACAGGCTGTAGCAGGCGAATGCAAAATATGGAATACTGACACACTGTTGAGTTTGCTG
GTAGCCTGGCGATATTACAGCGCATTTTGCGTAGAGCGTAGCGCCCAATCATTCTTCAGGACAGTACAATGCGTC
GGAGGACCTCTCGGCGCCTACAAGATTGATATAATTAAGGAAATCAAGGACCCATGGATCAGCCAACGTTTCCTT
GGCCAAAAGTGCACATACGGCGACGATAGACGACTCACTAATGAAATACTAATGAGGGGTAAGAAAGTAGTTTTC
ACCCCATTCGCTGTTGGCTGGTCCGACAGCCCGACGAACGTCTTCCGTTACATTGTACAGCAAACACGGTGGTCC
AAGTCGTGGTGTAGGGAGATATGGTATACACTGTTTGCAGCATGGAAGCATGGACTTTCGGGCATTTGGCTCGCA
TTCGAGTGCTTATACCAGATTACTTATTTCTTCCTGGTGATTTACCTATTCTCCCGTTTGGCTGTTGAGGCTGAC
CCACGGGCGCAAACGGCCACCGTCATTGTTTCGACCACAGTTGCGCTTATTAAGTGCGGTTACTTTAGCTTCAGA
GCTAAAGACATTAGGGCCTTCTATTTCGTCCTCTACACGTTCGTTTACTTCTTCTGCATGATTCCGGCACGAATA
ACTGCGATGATGACCCTGTGGGACATAGGTTGGGGAACCAGGGGAGGAAATGAGAAGCCTTCCGTAGGCACCAGA
GTTGCTTTGTGGGCCAAGCAATATTTGATTGCTTACATGTGGTGGGCCGCTGTCGTCGGTGCTGGCGTGTACTCC
ATCGTTCATAATTGGATGTTTGACTGGAATTCACTTTCCTACAGGTTCGCACTGGTAGGCATCTGTTCGTATATA
GTTTTCATTGTAATAGTGCTGGTAGTCTACTTTACAGGAAAGATCACGACCTGGAACTTCACGAAGCTACAGAAA
GAATTGATCGAGGACCGCGTACTGTACGACGCAACGACCAATGCCCAGTCGGTATAA

SEQ ID NO: 2 is a reencoded nucleic acid sequence of hyaluronan synthase (HASA) originating from Chlorella virus PBCV-1
ATGGGCAAGAATATTATTATCATGGTCAGTTGGTATACCATTATTACATCAAACCTAATTGCTGTCGGTGGAGCC
TCACTAATCTTAGCTCCCGCGATTACGGGTTATGTGCTTCATTGGAATATTGCTTTATCCACTATTTGGGGTGTT
AGCGCTTACGGCATCTTTGTGTTCGGTTTCTTCTTGGCTCAGGTGTTATTCTCTGAGCTAAACAGAAAGAGACTC
AGAAAGTGGATTTCGCTGCGGCCGAAGGGATGGAACGATGTTCGGTTAGCGGTGATCATTGCCGGTTATCGTGAA
GATCCTTATATGTTTCAGAAGTGTTTGGAATCTGTTCGAGACTCAGACTACGGTAACGTTGCTAGGCTGATCTGC
GTAATCGATGGAGACGAAGATGACGATATGAGAATGGCCGCGGTTTATAAAGCCATCTATAACGATAACATTAAG
AAGCCAGAGTTTGTTTTATGCGAATCGGATGACAAGGAGGGTGAGAGGATTGATTCTGACTTCTCGCGTGACATC
TGTGTCCTGCAGCCCCACAGGGGAAAGCGAGAGTGCCTCTATACAGGATTCCAATTAGCTAAAATGGACCCAAGC

GTTAATGCTGTCGTCCTTATCGATAGCGACACGGTGTTGGAAAAGGACGCAATATTGGAAGTAGTCTATCCACTC
GCTTGTGATCCAGAGATCCAGGCCGTGGCTGGCGAGTGCAAGATTTGGAATACAGATACGCTTCTGTCATTACTG
GTGGCGTGGCGTTATTATTCAGCATTCTGTGTGGAGAGGAGCGCACAATCGTTCTTTCGGACTGTACAATGCGTA
GGCGGTCCACTAGGAGCATATAAAATTGACATAATCAAAGAGATAAAAGATCCTTGGATTTCGCAGAGATTCCTC
GGTCAAAAGTGTACCTATGGGGACGACAGGCGGTTGACTAACGAGATTCTAATGAGAGGTAAGAAGGTGGTGTTC
ACTCCTTTTGCGGTAGGTTGGAGCGATTCGCCCACAAATGTATTCAGATACATTGTTCAACAAACAAGGTGGTCG
AAATCGTGGTGCCGAGAAATATGGTACACGTTGTTCGCCGCATGGAAACACGGCTTGTCGGGTATATGGCTCGCT
TTCGAGTGCCTTTATCAGATCACTTACTTCTTCCTGGTTATTTACTTGTTTTCAAGACTGGCAGTGGAAGCTGAT
CCAAGGGCTCAGACTGCTACTGTCATCGTTTCAACAACAGTGGCGCTAATAAAGTGTGGTTACTTCAGCTTTCGC
GCGAAGGACATAAGAGCGTTTTACTTTGTTCTTTATACCTTTGTATATTTCTTCTGTATGATCCCGGCCCGGATA
ACAGCTATGATGACATTATGGGACATTGGTTGGGGCACCAGGGGTGGAATGAGAAGCCGTCCGTAGGAACGAGA
GTAGCGCTGTGGGCCAAGCAGTATCTTATCGCATACATGTGGTGGGCAGCTGTCGTCGGGGCAGGTGTATACAGC
ATAGTACATAACTGGATGTTTGACTGGAACTCACTCTCATATCGATTCGCATTGGTCGGGATCTGCTCTTACATC
GTGTTTATTGTTATAGTCTTAGTAGTATATTTCACTGGAAAGATAACAACATGGAATTTCACTAAGCTTCAAAAG
GAATTGATTGAAGACAGGGTGCTCTACGATGCGACAACTAATGCACAAAGCGTATAA

SEQ ID NO: 3 is a reencoded nucleic acid sequence of hyaluronan synthase (HASA) originating from Pasteurella multocida
ATGAACACCCTTTCCCAGGCAATTAAGGCTTATAATAGTAATGATTATCAACTAGCCCTCAAGCTGTTCGAAAAG
TCGGCAGAAATCTACGGCAGAAAGATAGTTGAgTTCCAAATTACGAAGTGCAAGGAGAAACTTTCTGCGCACCCA
TCCGTTAACAGTGCGCATCTCTCGGTTAACAAAGAAGAGAAGGTGAATGTTTGCGACAGTCCATTAGACATAGCC
ACTCAATTATTGTTATCGAATGTGAAAAAACTCGTCCTTAGTGATAGCGAGAAAAATACACTAAAGAATAAATGG
AAGTTGTTGACTGAAAAGAAGTCTGAGAACGCAGAGGTCCGGGCCGTAGCTTTAGTTCCCAAGGACTTTCCGAAA
GATTTAGTGCTTGCGCCGTTACCAGACCATGTGAACGATTTTACGTGGTATAAGAAGCGGAAGAAACGCCTAGGA
ATCAAACCAGAACACCAACACGTTGGCCTCTCCATTATTGTGACAACGTTCAACCGTCCTGCCATCCTCAGCATC
ACCCTGGCTTGCCTCGTCAATCAAAAGACCCATTATCCTTTTGAGGTGATCGTGACCGACGATGGTTCTCAAGAG
GATTTATCGCCTATAATCCGTCAATATGAGAACAAGCTAGACATCCGTTATGTTCGGCAAAAGGACAACGGTTTT
CAGGCATCGGCCGCACGTAATATGGGCCTACGTCTAGCCAAATATGACTTCATTGGGCTCCTTGATTGTGATATG
GCTCCCAACCCTTTGTGGGTACACTCATACGTTGCGGAACTATTAGAGGACGATGATTTGACCATAATAGGCCCC
AGGAAGTACATTGACACGCAACACATAGATCCCAAGGATTTCCTTAATAACGCCTCTCTGTTAGAGTCGTTGCCA
GAGGTTAAGACCAATAATTCCGTCGCGGCTAAGGGCGAGGGGACCGTATCTTTAGACTGGCGTTTGGAACAATTT
GAGAAGACCGAGAACTTGAGGCTATCCGATAGCCCTTTCCGATTCTTCGCAGCTGGGAATGTGGCTTTCGCCAAG
AAGTGGCTTAACAAGTCAGGATTCTTCGACGAGGAATTCAATCACTGGGGAGGTGAAGATGTAGAGTTCGGTTAT
CGTCTGTTTCGGTACGGTTCGTTCTTCAAAACTATAGACGGCATCATGGCCTATCATCAGGAACCGCCAGGTAAA
GAAAACGAAACTGACAGAGAAGCGGGCAAGAACATTACCCTCGATATAATGAGGGAGAAGGTGCCTTACATCTAC
CGTAAACTCCTGCCTATAGAAGACAGTCATATCAACCGAGTACCATTGGTATCAATTTACATCCCGGCCTACAAC
TGTGCTAACTATATTCAAAGATGTGTTGATTCAGCTTTGAATCAAACGGTAGTTGATCTCGAAGTGTGCATTTGC
AACGACGGTAGTACTGACAACACGCTGGAAGTTATTAACAAGCTGTATGGTAATAATCCGCGTGTGCGTATAATG

TCTAAACCCAATGGCGGCATTGCGAGTGCATCCAACGCAGCGGTCAGCTTCGCAAAGGGTTATTACATAGGACAG
TTGGACAGCGACGATTACTTAGAACCCGACGCAGTGGAGTTATGTCTCAAGGAATTTCTTAAGGATAAGACCCTT
GCGTGCGTTTACACCACTAATCGTAACGTCAACCCAGATGGCTCTTTAATAGCCAATGGCTATAACTGGCCAGAG
TTCAGTCGTGAGAAGTTGACTACGGCCATGATTGCTCATCACTTCCGGATGTTTACCATTCGTGCTTGGCATCTG
ACGGATGGGTTCAATGAGAAGATTGAGAACGCTGTTGACTACGACATGTTTCTCAAGCTCAGTGAAGTTGGTAAA
TTTAAGCATCTGAACAAAATATGTTATAATCGGGTGTTACACGGCGATAACACCTCAATCAAGAAgCTTGGCATA
CAAAAGAAGAATCATTTCGTAGTTGTCAATCAGTCTCTAAACCGCCAAGGTATAACTTATTATAACTACGATGAA
TTTGATGATCTCGATGAGAGTCGGAAATACATTTTCAACAAGACTGCAGAGTATCAAGAAGAGATAGATATTCTT
AAAGATATTAAGATCATTCAGAACAAAGACGCCAAAATAGCTGTCTCCATTTTCTATCCGAACACTCTAAACGGG
TTGGTGAAGAAGCTAAATAATATTATAGAGTATAATAAGAATATCTTCGTTATTGTACTTCATGTCGATAAGAAT
CACTTAACCCCAGACATCAAGAAGGAGATATTAGCCTTCTACCATAAGCATCAGGTGAACATCTTATTGAACAAT
GACATCTCCTACTATACATCAAATCGTCTGATTAAGACAGAAGCCCATTTGAGTAACATTAACAAGCTaAGTCAA
TTAAATCTTAACTGCGAATACATTATATTCGACAATCACGATTCCTTATTTGTGAAGAACGATTCCTATGCATAC
ATGAAGAAGTATGATGTTGGAATGAACTTCTCTGCATTGACTCATGATTGGATTGAAAAGATAAACGCTCACCCG
CCATTTAAGAAGCTgATCAAAACTTACTTCAATGACAATGATCTTAAGTCGATGAATGTAAAGGGTGCCTCCCAG
GGAATGTTTATGACATACGCATTAGCCCACGAGTTATTGACGATCATCAAGGAGGTGATAACCTCTTGTCAATCC
ATTGACTCCGTCCCCGAATACAACACAGAAGATATTTGGTTTCAGTTTGCACTTTTAATTCTGGAAAAGAAGACC
GGCCACGTATTCAACAAGACAAGCACTCTCACGTATATGCCATGGGAACGTAAACTGCAGTGGACGAATGAACAA
ATAGAGTCCGCAAAGAGGGGCGAAAACATTCCGGTAAACAAGTTCATCATTAACAGCATTACCCTTTAA

SEQ ID NO: 4 is a reencoded nucleic acid sequence of hyaluronan synthase (HASA) originating from Pasteurella multocida
ATGAACACCCTTTCCCAGGCAATTAAGGCTTATAATAGTAATGATTATCAACTAGCCCTCAAGCTGTTCGAAAAG
TCGGCAGAAATCTACGGCAGAAAGATAGTTGAgTTCCAAATTACGAAGTGCAAGGAGAAACTTTCTGCGCACCCA
TCCGTTAACAGTGCGCATCTCTCGGTTAACAAAGAAGAGAAGGTGAATGTTTGCGACAGTCCATTAGACATAGCC
ACTCAATTATTGTTATCGAATGTGAAAAAACTCGTCCTTAGTGATAGCGAGAAAAATACACTAAAGAATAAATGG
AAGTTGTTGACTGAAAAGAAGTCTGAGAACGCAGAGGTCCGGGCCGTAGCTTTAGTTCCCAAGGACTTTCCGAAA
GATTTAGTGCTTGCGCCGTTACCAGACCATGTGAACGATTTTACGTGGTATAAGAAGCGGAAGAAACGCCTAGGA
ATCAAACCAGAACACCAACACGTTGGCCTCTCCATTATTGTGACAACGTTCAACCGTCCTGCCATCCTCAGCATC
ACCCTGGCTTGCCTCGTCAATCAAAAGACCCATTATCCTTTTGAGGTGATCGTGACCGACGATGGTTCTCAAGAG
GATTTATCGCCTATAATCCGTCAATATGAGAACAAGCTAGACATCCGTTATGTTCGGCAAAAGGACAACGGTTTT
CAGGCATCGGCCGCACGTAATATGGGCCTACGTCTAGCCAAATATGACTTCATTGGGCTCCTTGATTGTGATATG
GCTCCCAACCCTTTGTGGGTACACTCATACGTTGCGGAACTATTAGAGGACGATGATTTGACCATAATAGGCCCC
AGGAAGTACATTGACACGCAACACATAGATCCCAAGGATTTCCTTAATAACGCCTCTCTGTTAGAGTCGTTGCCA
GAGGTTAAGACCAATAATTCCGTCGCGGCTAAGGGCGAGGGGACCGTATCTTTAGACTGGCGTTTGGAACAATTT
GAGAAGACCGAGAACTTGAGGCTATCCGATAGCCCTTTCCGATTCTTCGCAGCTGGGAATGTGGCTTTCGCCAAG
AAGTGGCTTAACAAGTCAGGATTCTTCGACGAGGAATTCAATCACTGGGGAGGTGAAGATGTAGAGTTCGGTTAT
CGTCTGTTTCGGTACGGTTCGTTCTTCAAAACTATAGACGGCATCATGGCCTATCATCAGGAACCGCCAGGTAAA

GAAAACGAAACTGACAGAGAAGCGGGCAAGAACATTACCCTCGATATAATGAGGGAGAAGGTGCCTTACATCTAC
CGTAAACTCCTGCCTATAGAAGACAGTCATATCAACCGAGTACCATTGGTATCAATTTACATCCCGGCCTACAAC
TGTGCTAACTATATTCAAAGATGTGTTGATTCAGCTTTGAATCAAACGGTAGTTGATCTCGAAGTGTGCATTTGC
AACGACGGTAGTACTGACAACACGCTGGAAGTTATTAACAAGCTGTATGGTAATAATCCGCGTGTGCGTATAATG
TCTAAACCCAATGGCGGCATTGCGAGTGCATCCAACGCAGCGGTCAGCTTCGCAAAGGGTTATTACATAGGACAG
TTGGACAGCGACGATTACTTAGAACCCGACGCAGTGGAGTTATGTCTCAAGGAATTTCTTAAGGATAAGACCCTT
GCGTGCGTTTACACCACTAATCGTAACGTCAACCCAGATGGCTCTTTAATAGCCAATGGCTATAACTGGCCAGAG
TTCAGTCGTGAGAAGTTGACTACGGCCATGATTGCTCATCACTTCCGGATGTTTACCATTCGTGCTTGGCATCTG
ACGGATGGGTTCAATGAGAAGATTGAGAACGCTGTTGACTACGACATGTTTCTCAAGCTCAGTGAAGTTGGTAAA
TTTAAGCATCTGAACAAAATATGTTATAATCGGGTGTTACACGGCGATAACACCTCAATCAAGAAgCTTGGCATA
CAAAAGAAGAATCATTTCGTAGTTGTCAATCAGTCTCTAAACCGCCAAGGTATAACTTATTATAACTACGATGAA
TTTGATGATCTCGATGAGAGTCGGAAATACATTTTCAACAAGACTGCAGAGTATCAAGAAGAGATAGATATTCTT
AAAGATATTTAA

SEQ ID NO: 5 is a reencoded nucleic acid sequence of hyaluronan synthase (HASA) originating from Pasteurella multocida
ATGAACACCCTTTCCCAGGCAATTAAGGCTTATAATAGTAATGATTATCAACTAGCCCTCAAGCTGTTCGAAAAG
TCGGCAGAAATCTACGGCAGAAAGATAGTTGAgTTCCAAATTACGAAGTGCAAGGAGAAACTTTCTGCGCACCCA
TCCGTTAACAGTGCGCATCTCTCGGTTAACAAAGAAGAGAAGGTGAATGTTTGCGACAGTCCATTAGACATAGCC
ACTCAATTATTGTTATCGAATGTGAAAAAACTCGTCCTTAGTGATAGCGAGAAAAATACACTAAAGAATAAATGG
AAGTTGTTGACTGAAAAGAAGTCTGAGAACGCAGAGGTCCGGGCCGTAGCTTTAGTTCCCAAGGACTTTCCGAAA
GATTTAGTGCTTGCGCCGTTACCAGACCATGTGAACGATTTTACGTGGTATAAGAAGCGGAAGAAACGCCTAGGA
ATCAAACCAGAACACCAACACGTTGGCCTCTCCATTATTGTGACAACGTTCAACCGTCCTGCCATCCTCAGCATC
ACCCTGGCTTGCCTCGTCAATCAAAAGACCCATTATCCTTTTGAGGTGATCGTGACCGACGATGGTTCTCAAGAG
GATTTATCGCCTATAATCCGTCAATATGAGAACAAGCTAGACATCCGTTATGTTCGGCAAAAGGACAACGGTTTT
CAGGCATCGGCCGCACGTAATATGGGCCTACGTCTAGCCAAATATGACTTCATTGGGCTCCTTGATTGTGATATG
GCTCCCAACCCTTTGTGGGTACACTCATACGTTGCGGAACTATTAGAGGACGATGATTTGACCATAATAGGCCCC
AGGAAGTACATTGACACGCAACACATAGATCCCAAGGATTTCCTTAATAACGCCTCTCTGTTAGAGTCGTTGCCA
GAGGTTAAGACCAATAATTCCGTCGCGGCTAAGGGCGAGGGGACCGTATCTTTAGACTGGCGTTTGGAACAATTT
GAGAAGACCGAGAACTTGAGGCTATCCGATAGCCCTTTCCGATTCTTCGCAGCTGGGAATGTGGCTTTCGCCAAG
AAGTGGCTTAACAAGTCAGGATTCTTCGACGAGGAATTCAATCACTGGGGAGGTGAAGATGTAGAGTTCGGTTAT
CGTCTGTTTCGGTACGGTTCGTTCTTCAAAACTATAGACGGCATCATGGCCTATCATCAGGAACCGCCAGGTAAA
GAAAACGAAACTGACAGAGAAGCGGGCAAGAACATTACCCTCGATATAATGAGGGAGAAGGTGCCTTACATCTAC
CGTAAACTCCTGCCTATAGAAGACAGTCATATCAACCGAGTACCATTGGTATCAATTTACATCCCGGCCTACAAC
TGTGCTAACTATATTCAAAGATGTGTTGATTCAGCTTTGAATCAAACGGTAGTTGATCTCGAAGTGTGCATTTGC
AACGACGGTAGTACTGACAACACGCTGGAAGTTATTAACAAGCTGTATGGTAATAATCCGCGTGTGCGTATAATG
TCTAAACCCAATGGCGGCATTGCGAGTGCATCCAACGCAGCGGTCAGCTTCGCAAAGGGTTATTACATAGGACAG
TTGGACAGCGACGATTACTTAGAACCCGACGCAGTGGAGTTATGTCTCAAGGAATTTCTTAAGGATAAGACCCTT

GCGTGCGTTTACACCACTAATCGTAACGTCAACCCAGATGGCTCTTTAATAGCCAATGGCTATAACTGGCCAGAG
TTCAGTCGTGAGAAGTTGACTACGGCCATGATTGCTCATCACTTCCGGATGTTTACCATTCGTGCTTGGCATCTG
ACGGATGGGTTCAATGAGAAGATTGAGAACGCTGTTGACTACGACATGTTTCTCAAGCTCAGTGAAGTTGGTAAA
TTTAAGCATCTGAACAAAATATGTTATAATCGGGTGTTACACGGCGATAACACCTCAATCAAGAAGCTTGGCATA
CAAAAGAAGAATCATTTCGTAGTTGTCAATCAGTCTCTAAACCGCCAAGGTATAACTTATTATAACTACGATGAA
TTTGATGATCTCGATGAGAGTCGGAAATACATTTTCAACAAGACTGCAGAGTATCAAGAAGAGATAGATATTCTT
AAAGATATTGGATCCGCCATTTCTCAAATCACTGACGGTCAAATCCAAGCTACTACCACTGCTACCACCGAAGCT
ACCACCACTGCTGCCCCATCTTCCACCGTTGAAACTGTTTCTCCATCCAGCACCGAAACTATCTCTCAACAAACT
GAAAATGGTGCTGCTAAGGCCGCTGTCGGTATGGGTGCCGGTGCTCTAGCTGCTGCTGCTATGTTGTTATAA

SEQ ID NO: 6 is a reencoded nucleic acid sequence of hyaluronan synthase (HASA) originating from Xenopus laevis
ATGCACTGTGAACGATTTATTTGTATATTACGAATAATTGGTACGACTTTGTTCGGAGTAAGTCTGCTACTAGGG
ATATCCGCCGCTTATATCGTGGGATACCAATTCATTCAGACGGATAATTACTACTTTTCCTTCGGTCTCTACGGT
GCTATTTTAGCTTTACATTTAATCATTCAGTCTCTCTTCGCATTCCTGGAGCATCGTAAGATGAAGCGGTCACTT
GAAACCCCTATAAAATTAAACAAGAGCGTAGCATTGTGTATCGCTGCATACCAGGAAGACGAAGATTATCTTAGA
AAATGTTTGCTTTCTGTGAAACGGCTCACATACCCCGGTATGAAGGTAATCATGGTGATAGATGGTAACTCTGAT
GACGATCTATACATGATGAACATATTCAGAGAGATCATGGGAAATGATAGCTGCGCCACCTATGTATGGAAGAAT
AATTTTCACATGAAGGGACCAAACGAGACCGACGAGACTCACCGCGAGTCCATGCAGCATGTAACACAGATGGTG
CTTTCTAACAGAAATGTCTGTATCATGCAAAAGTGGAACGGAAACGCGAGGTGATGTATACTGCATTCAAAGCG
CTGGGTCGGTCGGTTGATTACGTCCAAGTGTGCGACTCAGATACAGTCCTTGATCCAGCAAGCTCGGTGGAGATG
GTCAAGGTGTTGGAGGAAGACATTATGGTCGGAGGTGTAGGCGGAGACGTGCAGATACTCAATAAGTACGACTCC
TGGATCAGTTTCTTGAGTTCGGTTCGTTATTGGATGGCTTTCAATATAGAACGAGCATGCCAATCATACTTTGGC
TGTGTGCAGTGTATATCGGGGCCTCTGGGCATGTACAGAAACTCACTCTTACATGAGTTCATTGAAGACTGGTAC
AATCAAGAATTCCTCGGTTCCCAATGCTCTTTCGGTGATGATCGGCATTTGACTAATCGAGTACTATCATTGGGC
TATGCTACTAAATACACTGCGCGCAGTAAGTGTCTGACAGAAACCCCCACAGAATATCTTAGATGGTTGAACCAA
CAAACCAGGTGGAGTAAGTCCTATTTCCGCGAGTGGTTGTACAACTCTTTGTGGTTCCACAAACACCATTTATGG
ATGACTTATGAAGCCGTAATTACGGGTTTCTTCCCCTTCTTCTTAATAGCGACGGTGATTCAGCTCTTCTATCGT
GGTCGAATTTGGAATATCTTACTCTTTCTACTCACAGTTCAATTAGTTGGTCTAATAAAGTCGTCGTTTGCAAGT
GCGTTGCGTGGAAACATCGTTATGGTCTTTATGAGTTTCTACAGTGTTTTATACATGAGTAGCTTGCTGCCAGCA
AAGATGTTTGCAATCGCCACGATAAACAAGGCTGGATGGGGTACTAGTGGTAGAAAGACCATAGTTGTTAATTTT
ATTGGTTTAATCCCTATTACGGTATGGTTCACAATTTTACTAGGGGGAGTATGCTACACTATATGGCGGGAAACC
AAGAAGCCGTTTAGCGAGTCTGAGAAGATCGTTCTTGCGGTCGGTGCTATATTGTATGCTTGTTATTGGGTCATG
TTGCTTACAATGTATGTCAGTCTTGTCATGAAGTGCGGGCGCCGTAGGAAGGAACCACAGCACGACCTTGTTTTA
GCATAA

SEQ ID NO: 7 is a reencoded nucleic acid sequence of hyaluronan synthase (HASA)
originating from Streptococcus zooepidemicus
ATGAGGACGTTAAAGAATCTTATCACTGTTGTAGCGTTCAGTATATTCTGGGTTTTGCTGATCTATGTAAATGTC
TATCTTTTCGGTGCGAAGGGGTCTCTATCAATATATGGATTCTTGCTAATTGCGTATTTGCTTGTGAAGATGAGT
CTTTCATTCTTCTATAAACCATTCAAAGGTCGAGCTGGTCAGTATAAAGTCGCAGCGATTATCCCTTCATACAAC
GAGGATGCAGAATCGCTGTTGGAAACCTTAAAATCTGTTCAACAACAAACCTATCCCCTTGCTGAAATTTATGTA
GTTGATGATGGTTCCGCAGATGAAACCGGTATCAAGCGGATCGAGGATTATGTCAGGGATACAGGCGACTTAAGT
TCAAATGTGATTGTGCATCGATCAGAAAAGAATCAAGGCAAGAGACACGCCCAAGCATGGGCATTTGAAAGATCT
GATGCAGATGTATTTCTAACAGTCGATAGTGATACTTACATTTATCCGGACGCCCTTGAAGAGTTATTGAAAACC
TTCAATGATCCGACAGTATTTGCCGCAACTGGCCACTTGAACGTTCGTAACAGGCAGACTAATCTATTGACCAGA
CTCACTGATATAAGATACGACAATGCTTTTGGCGTCGAACGTGCTGCTCAAAGTGTAACTGGTAATATACTGGTG
TGTTCCGGGCCACTGTCCGTCTATCGCAGAGAAGTAGTAGTCCCGAACATTGACAGGTATATTAATCAGACTTTT
CTGGGTATCCCCGTGTCAATTGGGGACGACCGGTGTTTAACGAACTACGCTACTGATTTGGGCAAGACTGTATAT
CAGTCGACGGCTAAATGTATTACAGACGTCCCAGATAAGATGTCGACTTACCTTAAACAGCAGAATAGATGGAAT
AAGTCATTCTTTAGAGAATCTATCATCAGCGTGAAGAAGATCATGAATAATCCATTCGTGGCGCTTTGGACCATT
TTGGAGGTTTCCATGTTTATGATGCTTGTCTACTCTGTCGTTGATTTCTTTGTAGGTAACGTTCGAGAATTTGAT
TGGCTCAGAGTTCTTGCTTTCTTAGTTATCATCTTCATCGTTGCGCTATGCAGGAATATTCATTACATGCTAAAA
CATCCGCTTAGCTTCTTACTCAGCCCTTTCTACGGCGTTTTGCATCTCTTCGTTTTGCAGCCATTGAAGCTCTAC
TCCTTATTTACCATTCGAAACGCTGATTGGGGTACGCGCAAGAAACTATTAtaa

SEQ ID NO: 8 is an amino acid sequence of hyaluronan synthase (HASA) originating from
Chlorella virus PBCV-1
MGKNIIIMVSWYTIITSNLIAVGGASLILAPAITGYVLHWNIALSTIWGVSAYGIFVFGFFLAQVLFSELNRKRL
RKWISLRPKGWNDVRLAVIIAGYREDPYMFQKCLESVRDSDYGNVARLICVIDGDEDDDMRMAAVYKAIYNDNIK
KPEFVLCESDDKEGERIDSDFSRDICVLQPHRGKRECLYTGFQLAKMDPSVNAVVLIDSDTVLEKDAILEVVYPL
ACDPEIQAVAGECKIWNTDTLLSLLVAWRYYSAFCVERSAQSFFRTVQCVGGPLGAYKIDIIKEIKDPWISQRFL
GQKCTYGDDRRLTNEILMRGKKVVFTPFAVGWSDSPTNVFRYIVQQTRWSKSWCREIWYTLFAAWKHGLSGIWLA
FECLYQITYFFLVIYLFSRLAVEADPRAQTATVIVSTTVALIKCGYFSFRAKDIRAFYFVLYTFVYFFCMIPARI
TAMMTLWDIGWGTRGGNEKPSVGTRVALWAKQYLIAYMWWAAVVGAGVYSIVHNWMFDWNSLSYRFALVGICSYI
VFIVIVLVVYFTGKITTWNFTKLQKELIEDRVLYDATTNAQSV

SEQ ID NO: 9 is an amino acid sequence of hyaluronan synthase (HASA) originating from
Pasteurella multocida
MNTLSQAIKAYNSNDYQLALKLFEKSAEIYGRKIVEFQITKCKEKLSAHPSVNSAHLSVNKEEKVNVCDSPLDIA
TQLLLSNVKKLVLSDSEKNTLKNKWKLLTEKKSENAEVRAVALVPKDFPKDLVLAPLPDHVNDFTWYKKRKKRLG
IKPEHQHVGLSIIVTTFNRPAILSITLACLVNQKTHYPFEVIVTDDGSQEDLSPIIRQYENKLDIRYVRQKDNGF
QASAARNMGLRLAKYDFIGLLDCDMAPNPLWVHSYVAELLEDDDLTIIGPRKYIDTQHIDPKDFLNNASLLESLP

EVKTNNSVAAKGEGTVSLDWRLEQFEKTENLRLSDSPFRFFAAGNVAFAKKWLNKSGFFDEEFNHWGGEDVEFGY
RLFRYGSFFKTIDGIMAYHQEPPGKENETDREAGKNITLDIMREKVPYIYRKLLPIEDSHINRVPLVSIYIPAYN
CANYIQRCVDSALNQTVVDLEVCICNDGSTDNTLEVINKLYGNNPRVRIMSKPNGGIASASNAAVSFAKGYYIGQ
LDSDDYLEPDAVELCLKEFLKDKTLACVYTTNRNVNPDGSLIANGYNWPEFSREKLTTAMIAHHFRMFTIRAWHL
TDGFNEKIENAVDYDMFLKLSEVGKFKHLNKICYNRVLHGDNTSIKKLGIQKKNHFVVVNQSLNRQGITYYNYDE
FDDLDESRKYIFNKTAEYQEEIDILKDIKIIQNKDAKIAVSIFYPNTLNGLVKKLNNIIEYNKNIFVIVLHVDKN
HLTPDIKKEILAFYHKHQVNILLNNDISYYTSNRLIKTEAHLSNINKLSQLNLNCEYIIFDNHDSLFVKNDSYAY
MKKYDVGMNFSALTHDWIEKINAHPPFKKLIKTYFNDNDLKSMNVKGASQGMFMTYALAHELLTIIKEVITSCQS
IDSVPEYNTEDIWFQFALLILEKKTGHVFNKTSTLTYMPWERKLQWTNEQIESAKRGENIPVNKFIINSITL

SEQ ID NO: 10 is an amino acid sequence of hyaluronan synthase (HASA) originating from
Xenopus laevis
MHCERFICILRIIGTTLFGVSLLLGISAAYIVGYQFIQTDNYYFSFGLYGAILALHLIIQSLFAFLEHRKMKRSL
ETPIKLNKSVALCIAAYQEDEDYLRKCLLSVKRLTYPGMKVIMVIDGNSDDDLYMMNIFREIMGNDSCATYVWKN
NFHMKGPNETDETHRESMQHVTQMVLSNRNVCIMQKWNGKREVMYTAFKALGRSVDYVQVCDSDTVLDPASSVEM
VKVLEEDIMVGGVGGDVQILNKYDSWISFLSSVRYWMAFNIERACQSYFGCVQCISGPLGMYRNSLLHEFIEDWY
NQEFLGSQCSFGDDRHLTNRVLSLGYATKYTARSKCLTETPTEYLRWLNQQTRWSKSYFREWLYNSLWFHKHHLW
MTYEAVITGFFPFFLIATVIQLFYRGRIWNILLFLLTVQLVGLIKSSFASALRGNIVMVFMSFYSVLYMSSLLPA
KMFAIATINKAGWGTSGRKTIVVNFIGLIPITVWFTILLGGVCYTIWRETKKPFSESEKIVLAVGAILYACYWVM
LLTMYVSLVMKCGRRRKEPQHDLVLA

SEQ ID NO: 11 is an amino acid sequence of hyaluronan synthase (HASA) originating from
Streptococcus zooepidemicus
MRTLKNLITVVAFSIFWVLLIYVNVYLFGAKGSLSIYGFLLIAYLLVKMSLSFFYKPFKGRAGQYKVAAIIPSYN
EDAESLLETLKSVQQQTYPLAEIYVVDDGSADETGIKRIEDYVRDTGDLSSNVIVHRSEKNQGKRHAQAWAFERS
DADVFLTVDSDTYIYPDALEELLKTFNDPTVFAATGHLNVRNRQTNLLTRLTDIRYDNAFGVERAAQSVTGNILV
CSGPLSVYRREVVVPNIDRYINQTFLGIPVSIGDDRCLTNYATDLGKTVYQSTAKCITDVPDKMSTYLKQQNRWN
KSFFRESIISVKKIMNNPFVALWTILEVSMFMMLVYSVVDFFVGNVREFDWLRVLAFLVIIFIVALCRNIHYMLK
HPLSFLLSPFYGVLHLFVLQPLKLYSLFTIRNADWGTRKKLL

SEQ ID NO: 12 is a nucleic acid sequence of UDP-Glucose dehydrogenase (HASB)
originating from Arabidopsis thaliana
ATGGTCAAAATATGCTGTATCGGGGCTGGATATGTCGGTGGGCCTACAATGGCCGTTATGGCCCTAAAGTGTCCA
GAGATTGAAGTCGTGGTAGTTGATATATCGGAGCCACGAATTAACGCATGGAACTCAGACCGTCTACCAATTTAT
GAGCCAGGGTTAGAGGATGTCGTCAAACAGTGTAGAGGGAAGAATTTGTTCTTCTCTACAGATGTAGAGAAGCAT
GTATTTGAGTCAGATATAGTGTTTGTTTCGGTAAACACTCCTACGAAAACGCAGGGTCTGGGTGCAGGTAAAGCA
GCAGATTTGACATATTGGGAGTCCGCTGCTCGCATGATAGCTGATGTGAGCAAATCGTCGAAAATCGTCGTTGAA

AAGAGTACAGTACCAGTTCGTACAGCCGAGGCTATAGAAAAGATTTTAACGCACAATTCGAAGGGTATCGAATTC
CAGATCTTATCAAATCCAGAATTCTTGGCCGAAGGACGGCGATTAAAGACTTATATAACCCTGATAGAGTTCTA
ATCGGTGGCAGGGACACCGCTGCGGACAAAAGGCCATTAAGGCGTTGCGTGACGTGTATGCCCACTGGGTTCCT
GTTGAGCAAATAATCTGTACTAATTTATGGAGTGCCGAGCTATCAAAGTTGGCTGCGAACGCATTTCTAGCTCAA
AGGATAAGTTCAGTAAACGCAATGTCAGCGCTTTGTGAGGCAACTGGTGCTGACGTAACCCAAGTCGCTCACGCC
GTTGGAACCGACACTAGAATTGGACCGAAGTTTCTTAACGCATCCGTAGGCTTCGGCGGATCTTGCTTTCAGAAA
GACATCCTGAATCTTATTTACATCTGCGAATGCAATGGTCTTCCAGAAGCAGCCAATTATTGGAAACAGGTAGTC
AAGGTAAATGACTACCAAAAGATTAGGTTTGCTAATCGAGTCGTATCTTCTATGTTCAACACCGTCTCCGGTAAG
AAAATTGCTATTTTGGGATTTGCGTTCAAGAAGGACACCGGCGACACGCGTGAAACTCCTGCCATAGATGTGTGT
AATCGCCTCGTGGCTGATAAAGCAAAGCTGTCGATCTATGATCCGCAAGTATTAGAAGAGCAGATCCGCCGTGAT
CTGTCCATGGCCCGATTCGATTGGGACCATCCAGTCCCACTCCAGCAGATCAAAGCTGAAGGTATCTCCGAACAG
GTTAACGTTGTGTCCGACGCCTACGAGGCTACGAAGGATGCTCATGGTTTATGTGTTTTAACCGAATGGGACGAA
TTCAAGTCACTTGATTTTAAGAAGATCTTTGATAATATGCAGAAACCCGCTTTCGTTTTCGACGGAAGAAACGTG
GTCGACGCTGTGAAATTGAGAGAAATTGGATTCATAGTATATTCCATAGGTAAACCTCTGGATAGTTGGCTCAAG
GATATGCCGGCTGTTGCATAA

SEQ ID NO: 13 is a reencoded nucleic acid sequence of UDP-Glucose dehydrogenase
(HASB) originating from Chlorella virus PBCV-1
ATGAGTAGAATTGCTGTCGTTGGATGCGGTTACGTGGGTACGGCCTGCGCCGTACTTTTGGCGCAGAAGAACGAA
GTTATCGTTTTGGATATCTCGGAGGACCGGGTACAACTGATTAAGAATAAGAAGTCACCTATAGAAGATAAGGAA
ATCGAAGAATTCCTGGAGACGAAAGATTTGAATCTAACAGCGACGACGGATAAGGTGCTCGCCTATGAGAATGCT
GAATTCGTTATAATAGCTACACCGACCGATTACGATGTCGTCACTAGATATTTCAACACTAAGTCTGTTGAAAAT
GTGATAGGCGATGTCATTAAGAACACTCAGACACACCCTACTATAGTGATCAAGAGTACTATTCCAATCGGTTTC
GTGGATAAAGTTAGAGAACAGTTCGATTATCAAAATATTATCTTCTCGCCGGAATTCTTGAGAGAAGGACGAGCA
TTATATGATAATCTTTACCCCTCCCGTATCATCGTCGGTGATGACTCCCCAATTGCCTTAAAATTCGCGAATCTC
TTGGTCGAGGGTAGCAAAACTCCACTAGCTCCCGTATTAACTATGGGTACGCGAGAGGCCGAAGCTGTAAAACTA
TTTTCAAATACATATTTGGCTATGAGAGTAGCATACTTCAATGAGCTAGACACATTTGCAATGTCCCATGGTATG
AACGCAAAAGAGATTATCGACGGAGTCACACTAGAACCAAGGATAGGGCAGGGTTATTCGAATCCTTCATTTGGG
TATGGAGCCTATTGTTTCCCAAAAGATACAAAGCAATTGTTGGCTAATTTTGAAGGGGTTCCACAAGATATTATT
GGCGCAATCGTAGAGTCTAACGAAACTAGAAAGGAGGTTATTGTTTCTGAGGTCGAGAACAGATTTCCGACCACA
GTTGGCGTATATAAGTTGGCTGCTAAAGCTGGTTCGGATAATTTCAGGTCTAGTGCGATAGTCGATATCATGGAA
AGGTTGGCTAATAAGGGCTACCACATCAAAATATTTGAACCTACCGTTGAACAATTTGAGAATTTTGAGGTAGAT
AATAATCTCACAACTTTTGCAACCGAGTCTGATGTAATAATTGCGAACAGAGTTCCAGTCGAACATCGCATTCTG
TTTGGGAAGAAGTTAATAACACGGGATGTCTATGGGGATAA

SEQ ID NO: 14 is a reencoded nucleic acid sequence of UDP-Glucose dehydrogenase (HASB) originating from Chlorella virus PBCV-1
ATGTCACGAATCGCAGTAGTTGGCTGTGGGTACGTGGGAACCGCATGCGCTGTACTGCTGGCGCAAAAGAATGAG
GTAATCGTTCTTGATATTAGTGAGGATAGGGTCCAACTAATCAAGAACAAGAAATCCCCGATCGAAGATAAGGAG
ATTGAAGAATTCTTGGAAACAAAAGACCTAAACTTAACTGCAACAACTGACAAAGTTTTAGCCTACGAAAACGCT
GAGTTTGTGATTATAGCGACACCCACAGATTATGACGTAGTTACCAGATACTTTAACACGAAGTCCGTAGAGAAC
GTCATTGGAGATGTTATAAAGAATACTCAGACTCATCCTACGATAGTAATAAAGTCAACCATTCCCATAGGTTTC
GTAGATAAGGTTAGGGAGCAATTCGATTACCAGAACATTATATTTTCGCCAGAATTTCTGAGAGAGGGTCGCGCC
CTGTATGATAATCTATATCCATCACGGATTATAGTGGGCGATGACTCTCCGATTGCACTTAAGTTTGCTAATCTT
TTAGTTGAGGGCTCCAAAACTCCGCTCGCCCCAGTACTTACGATGGGTACACGTGAGGCTGAAGCTGTCAAGCTG
TTTTCAAACACATACCTTGCTATGCGAGTCGCATACTTTAACGAACTAGATACCTTTGCTATGTCGCACGGTATG
AATGCTAAAGAAATCATAGATGGCGTAACGTTGGAGCCTCGGATAGGTCAAGGATATTCCAATCCATCTTTTGGC
TACGGTGCGTATTGTTTCCCAAAGGACACGAAGCAATTATTAGCTAACTTTGAGGGTGTTCCGCAAGATATAATT
GGGGCGATAGTAGAAAGCAATGAAACACGGAAGGAGGTAATCGTGAGTGAAGTGGAAAACCGATTCCCCACTACG
GTCGGCGTTTACAAATTAGCCGCCAAGGCTGGTTCCGACAATTTCCGATCCAGCGCAATAGTAGATATTATGGAA
AGATTAGCTAATAAGGGATACCACATTAAAATCTTTGAACCTACTGTCGAACAGTTCGAGAACTTCGAGGTTGAT
AATAACTTGACGACTTTCGCAACGGAGAGCGATGTAATTATTGCAAACCGCGTACCTGTGGAACATCGAATTTTG
TTCGGAAAGAAGCTGATTACACGCGATGTATATGGCGATAACTAA

SEQ ID NO: 15 is a reencoded nucleic acid sequence of UDP-Glucose dehydrogenase (HASB) originating from Streptococcus zooepidemicus
ATGAAGATATCGGTAGCGGGTTCGGGGTACGTGGGGTTATCCTTGTCAATCTTGCTTGCCCAACATAACGATGTG
ACTGTTGTAGATATAATCGACGAAAAGGTACGGCTAATTAACCAGGGCATATCTCCGATTAAGGATGCGGACATT
GAGGAATATCTGAAGAATGCACCGTTGAATCTTACGGCTACACTAGACGGAGCTTCGGCTTATAGTAATGCTGAT
CTGATTATAATCGCAACGCCAACTAATTACGATTCAGAACGCAATTATTTCGACACCAGACACGTTGAAGAAGTA
ATTGAGCAAGTATTGGATTTAAATGCCTCCGCTACTATAATCATCAAGAGTACCATACCCTTGGGTTTTATTAAA
CACGTAAGAGAGAAATACCAAACAGACAGAATCATCTTTTCTCCAGAGTTCTTAAGAGAGTCAAAGGCATTGTAC
GATAACTTATACCCCTCTCGTATAATAGTCAGTTATGAGAAGGATGACTCTCCAAGAGTTATACAAGCAGCTAAG
GCGTTCGCGGGTTTATTAAAAGAGGGGGCAAAGAGCAAGGATACCCCAGTTCTGTTTATGGGCTCTCAAGAAGCT
GAAGCTGTCAAGCTGTTTGCTAATACTTTTCTCGCCATGAGGGTCAGTTACTTCAACGAGCTTGACACTTATAGC
GAATCAAAAGGACTAGACGCCCAAAGAGTTATAGAAGGCGTCTGCCATGATCAAAGGATAGGTAATCATTACAAT
AATCCATCCTTCGGATATGGCGGTTATTGTTTACCCAAAGACTCAAAGCAACTTTTGGCTAATTATAGAGGCATA
CCTCAGTCTCTAATGTCTGCCATCGTTGAATCGAACAAGATCCGTAAGTCGTATTTAGCTGAACAAATATTAGAT
AGGGCTTCTTCACAAAAGCAGGCTGGTGTACCTTTAACCATAGGATTTTACCGTTTGATTATGAAGTCCAACTCC
GATAACTTTAGAGAATCAGCCATTAAAGATATTATTGACATCATTAATGACTACGGTGTCAATATTGTCATTTAT
GAACCTATGTTGGGAGAAGACATTGGTTATAGAGTCGTTAAAGATTTGGAACAGTTTAAGAACGAAAGTACAATT
ATTGTTAGTAACAGGTTTGAAGATGATTTAGGTGATGTTATTGATAAAGTTTATACACGTGACGTCTTTGGTAG

SEQ ID NO: 16 is an amino acid sequence of UDP-Glucose dehydrogenase (HASB)
originating from Arabidopsis thaliana
MVKICCIGAGYVGGPTMAVMALKCPEIEVVVVDISEPRINAWNSDRLPIYEPGLEDVVKQCRGKNLFFSTDVEKH
VFESDIVFVSVNTPTKTQGLGAGKAADLTYWESAARMIADVSKSSKIVVEKSTVPVRTAEAIEKILTHNSKGIEF
QILSNPEFLAEGTAIKDLYNPDRVLIGGRDTAAGQKAIKALRDVYAHWVPVEQIICTNLWSAELSKLAANAFLAQ
RISSVNAMSALCEATGADVTQVAHAVGTDTRIGPKFLNASVGFGGSCFQKDILNLIYICECNGLPEAANYWKQVV
KVNDYQKIRFANRVVSSMFNTVSGKKIAILGFAFKKDTGDTRETPAIDVCNRLVADKAKLSIYDPQVLEEQIRRD
LSMARFDWDHPVPLQQIKAEGISEQVNVVSDAYEATKDAHGLCVLTEWDEFKSLDFKKIFDNMQKPAFVFDGRNV
VDAVKLREIGFIVYSIGKPLDSWLKDMPAVA

SEQ ID NO: 17 is an amino acid sequence of UDP-Glucose dehydrogenase (HASB)
originating from Chlorella virus PBCV1
MSRIAVVGCGYVGTACAVLLAQKNEVIVLDISEDRVQLIKNKKSPIEDKEIEEFLETKDLNLTATTDKVLAYENA
EFVIIATPTDYDVVTRYFNTKSVENVIGDVIKNTQTHPTIVIKSTIPIGFVDKVREQFDYQNIIFSPEFLREGRA
LYDNLYPSRIIVGDDSPIALKFANLLVEGSKTPLAPVLTMGTREAEAVKLFSNTYLAMRVAYFNELDTFAMSHGM
NAKEIIDGVTLEPRIGQGYSNPSFGYGAYCFPKDTKQLLANFEGVPQDIIGAIVESNETRKEVIVSEVENRFPTT
VGVYKLAAKAGSDNFRSSAIVDIMERLANKGYHIKIFEPTVEQFENFEVDNNLTTFATESDVIIANRVPVEHRIL
FGKKLITRDVYGDN

SEQ ID NO: 18 is an amino acid sequence of UDP-Glucose dehydrogenase (HASB)
originating from Streptococcus zooepidemicus
MKISVAGSGYVGLSLSILLAQHNDVTVVDIIDEKVRLINQGISPIKDADIEEYLKNAPLNLTATLDGASAYSNAD
LIIIATPTNYDSERNYFDTRHVEEVIEQVLDLNASATIIIKSTIPLGFIKHVREKYQTDRIIFSPEFLRESKALY
DNLYPSRIIVSYEKDDSPRVIQAAKAFAGLLKEGAKSKDTPVLFMGSQEAEAVKLFANTFLAMRVSYFNELDTYS
ESKGLDAQRVIEGVCHDQRIGNHYNNPSFGYGGYCLPKDSKQLLANYRGIPQSLMSAIVESNKIRKSYLAEQILD
RASSQKQAGVPLTIGFYRLIMKSNSDNFRESAIKDIIDIINDYGVNIVIYEPMLGEDIGYRVVKDLEQFKNESTI
IVSNRFEDDLGDVIDKVYTRDVFGRD

SEQ ID NO: 19 is a reencoded nucleic acid sequence of hyaluronidase (HYAL) originating
from Bothrops atrox with a N-terminal secretion signal
ATGCAATTTAGCACAGTCGCATCAGTAGCCTTCGTTGCCTTGGCCAACTTCGTGGCAGCACCGATGTATCCGAAC
GAACCGTTCTTAGTCTTTTGGAACGCGCCTACAACTCAGTGTAGACTTCGATATAAGGTTGACCTTGATCTGAAG
ACATTCCATATCGTGACAAATGCTAATGACTCGCTGTCAGGATCGGCTGTCACGATTTTCTATCCCACGCACTTA
GGGGTTTACCCACATATTGATGACAGGGGGCACTTCTTCAATGGCATCATACCCCAAAATGAATCCCTGGTAAAG
CATTTAAACAAATCTAAATCAGATATTAATCGAATGATTCCCTTAAGAACATTCCACGGGCTGGGAGTCATAGAC
TGGGAAAACTGGCGGCCACAGTGGGATAGGAATTGGGGAAGTAAGAACGTTTATAGGAATAGATCAATCCAATTC
GCGCGTGATCTCCACCCAGAGCTTAGTGAGGACAAGATTAAACGCTTGGCAAAACAGGAATTCGAGAAAGCTGCA

AAGAGTTTTATGAGGGATACACTATTATTAGCCGAGGAGATGCGACCAGACGGCTACTGGGGATACTATCTGTAC
CCCGATTGTCACAATTACAATTATAAGACTAAGCCAGATCAGTACACAGGAGAGTGCCCTGACATCGAGATGTCA
CGTAACAATCAACTCTTGTGGCTTTGGCGGATAGCACTGCCCTTTTCCCAATATCTACTTAGAGACTATACTA
AGAAGCTCTGACAATGCCCTGAAGTTTGTGCACCATAGGCTCAAGGAGGCAATGAGGATAGCCTCAATGGCTCGA
AATGACTACGCGTTGCCTTTCTTTGTTTATGCTCGACCATTCTATGCCTATACCTTCGAACCATTGACTCAGGAA
GACCTTGTGACTACGGTCGGAGAGACCGCGGACATGGGCGCCGCTGGGATCGTATTTTGGGGGAGTATGCAGTAC
GCAAGCACGGTTGAATCTTGCGGCAAAGTCAAGGACTACATGAATGGCCCACTGGGGCGTTACATTGTGAATGTT
ACAACTGCCGCCAAAATTTGCTCACGATTCTTGTGCAAACGTCATGGTAGGTGTGTAAGAAAGCACTCAGACTCC
AATGCATTCCTTCACCTATTTCCCGATTCGTTTCGCATAATGGTGCACGGGAATGCAACCGAGAAGAAAGTTATA
GTAAAGGGGAAGCTGGAATTAAAGAATCTTATATTCTTACGTAATAACTTTATGTGCCAGTGTTATCAAGGCTGG
AAAGGTCTATATTGCGAGAAGCATTCGATAAAGGAAATTCGGAAAATCTAA

SEQ ID NO: 20 is a reencoded nucleic acid sequence of hyaluronidase (HYAL) originating from Bothrops atrox with a N-terminal secretion signal and a C-terminal anchoring signal
ATGCAATTTAGCACAGTCGCATCAGTAGCCTTCGTTGCCTTGGCCAACTTCGTGGCAGCACCGATGTATCCGAAC
GAACCGTTCTTAGTCTTTTGGAACGCGCCTACAACTCAGTGTAGACTTCGATATAAGGTTGACCTTGATCTGAAG
ACATTCCATATCGTGACAAATGCTAATGACTCGCTGTCAGGATCGGCTGTCACGATTTTCTATCCCACGCACTTA
GGGGTTTACCCACATATTGATGACAGGGGGCACTTCTTCAATGGCATCATACCCCAAAATGAATCCCTGGTAAAG
CATTTAAACAAATCTAAATCAGATATTAATCGAATGATTCCCTTAAGAACATTCCACGGGCTGGGAGTCATAGAC
TGGGAAAACTGGCGGCCACAGTGGGATAGGAATTGGGGAAGTAAGAACGTTTATAGGAATAGATCAATCCAATTC
GCGCGTGATCTCCACCCAGAGCTTAGTGAGGACAAGATTAAACGCTTGGCAAAACAGGAATTCGAGAAAGCTGCA
AAGAGTTTTATGAGGGATACACTATTATTAGCCGAGGAGATGCGACCAGACGGCTACTGGGGATACTATCTGTAC
CCCGATTGTCACAATTACAATTATAAGACTAAGCCAGATCAGTACACAGGAGAGTGCCCTGACATCGAGATGTCA
CGTAACAATCAACTCTTGTGGCTTTGGCGGGATAGCACTGCCCTTTTCCCCAATATCTACTTAGAGACTATACTA
AGAAGCTCTGACAATGCCCTGAAGTTTGTGCACCATAGGCTCAAGGAGGCAATGAGGATAGCCTCAATGGCTCGA
AATGACTACGCGTTGCCTTTCTTTGTTTATGCTCGACCATTCTATGCCTATACCTTCGAACCATTGACTCAGGAA
GACCTTGTGACTACGGTCGGAGAGACCGCGGACATGGGCGCCGCTGGGATCGTATTTTGGGGGAGTATGCAGTAC
GCAAGCACGGTTGAATCTTGCGGCAAAGTCAAGGACTACATGAATGGCCCACTGGGGCGTTACATTGTGAATGTT
ACAACTGCCGCCAAAATTTGCTCACGATTCTTGTGCAAACGTCATGGTAGGTGTGTAAGAAAGCACTCAGACTCC
AATGCATTCCTTCACCTATTTCCCGATTCGTTTCGCATAATGGTGCACGGGAATGCAACCGAGAAGAAAGTTATA
GTAAAGGGGAAGCTGGAATTAAAGAATCTTATATTCTTACGTAATAACTTTATGTGCCAGTGTTATCAAGGCTGG
AAAGGTCTATATTGCGAGAAGCATTCGATAAAGGAAATTCGGAAAATCGGATCCGCCATTTCTCAAATCACTGAC
GGTCAAATCCAAGCTACTACCACTGCTACCACCGAAGCTACCACCACTGCTGCCCCATCTTCCACCGTTGAAACT
GTTTCTCCATCCAGCACCGAAACTATCTCTCAACAAACTGAAAATGGTGCTGCTAAGGCCGCTGTCGGTATGGGT
GCCGGTGCTCTAGCTGCTGCTGCTATGTTGTTATAA

SEQ ID NO: 21 is a reencoded nucleic acid sequence of hyaluronidase (HYAL) originating
from Cupiennius salei with a N-terminal secretion signal
ATGCAATTCAGCACTGTTGCATCAGTTGCATTTGTCGCCCTGGCGAATTTTGTAGCCGCGTTCAAGATTTACTGG
AACGTCCCAACTTTTCAGTGCACGCATAACTACAAAATCGATTATGTCAAATTGTTGTCCACTTACGGATACAG
GTCAATGATGGCGGTAAGTTTCAAGGAAACCAAGTGACTATCTTTTATGAAACCCAGTTGGGTTTGTATCCACGA
ATCCTAAAATCTGGTAAAATGGAAAACGGCGGAATCCCTCAACGCGGTAACTTTGAGAAACACCTAGAAAAGGCA
AGCACGGACCTCCAGAAAGTGATCCCTTGGAAAGAGTTTAGCGGATTAGGTGTGATAGATTGGGAGGCTTGGAGA
CCCACATGGGAATTTAACTGGGAACCGTTGAGGATATATCAAACCGAATCAATTAAGAGAGCTAAAGAACTACAC
CCTACCGCAAACGATTCCGCAGTAAAAGAAATTGCAGAGCGGCAATGGGAAGATTCAGCCAAGTTATACATGTTA
GAAACACTGCGGCTGGCAAAGAAACTTCGACCTCAAGCGCCTTGGTGTTACTACTTATTTCCTGATTGCTATAAT
TACGTCGGAAAGAAACCAAAAGATTTCCAATGTAGTGCCTCGATACGTAAAGGTAACGATAAGCTAAGCTGGTTG
TGGAAAGATTCTACGGCATTGTGTCCATCGATATACGTATATGAATCACAATTAGACAGGTATTCTTTTGAACAA
AGGACATGGCGCGACAATGAGAAACTTCGGGAAGCGTTGCGTGTAGCCACGAGAACCTCTAAAATATACCCATAC
GTTAACTATTTCGATAAGGAGCTTATACCGGAGCAAGAAGTATGGAGAATGCTTGCGCAGGCAGCTGCTGTCGGT
GGCAGTGGTGCGGTAATTTGGGGCTCATCTGCTGCAGTTGCATCTGAAGAGTTATGTAAATCTTTAAAACAGTAT
ATTATTGAAACGCTTGGGCCGGCGGCAGAGAAGGTGGCTTGGCGTAGTGACTTATGCAGCAAAGAAATTTGTAAT
AATCAGGGTCGCTGCACATTCCCGGACGATGATTATGCAAACGCATGGAAATTATTTACAGATGATACTGTTAAG
TTTTATGCTGGTAATATTACATGTAGGTGCTCCGAGAATTATTCTGGTCGTTTCTGCGAAAAGAAGAATTAA

SEQ ID NO: 22 is a reencoded nucleic acid sequence of hyaluronidase (HYAL) originating
from Cupiennius salei with a N-terminal secretion signal and a C-terminal anchoring signal
ATGCAATTCAGCACTGTTGCATCAGTTGCATTTGTCGCCCTGGCGAATTTTGTAGCCGCGTTCAAGATTTACTGG
AACGTCCCAACTTTTCAGTGCACGCATAACTACAAAATCGATTATGTCAAATTGTTGTCCACTTACGGATACAG
GTCAATGATGGCGGTAAGTTTCAAGGAAACCAAGTGACTATCTTTTATGAAACCCAGTTGGGTTTGTATCCACGA
ATCCTAAAATCTGGTAAAATGGAAAACGGCGGAATCCCTCAACGCGGTAACTTTGAGAAACACCTAGAAAAGGCA
AGCACGGACCTCCAGAAAGTGATCCCTTGGAAAGAGTTTAGCGGATTAGGTGTGATAGATTGGGAGGCTTGGAGA
CCCACATGGGAATTTAACTGGGAACCGTTGAGGATATATCAAACCGAATCAATTAAGAGAGCTAAAGAACTACAC
CCTACCGCAAACGATTCCGCAGTAAAAGAAATTGCAGAGCGGCAATGGGAAGATTCAGCCAAGTTATACATGTTA
GAAACACTGCGGCTGGCAAAGAAACTTCGACCTCAAGCGCCTTGGTGTTACTACTTATTTCCTGATTGCTATAAT
TACGTCGGAAAGAAACCAAAAGATTTCCAATGTAGTGCCTCGATACGTAAAGGTAACGATAAGCTAAGCTGGTTG
TGGAAAGATTCTACGGCATTGTGTCCATCGATATACGTATATGAATCACAATTAGACAGGTATTCTTTTGAACAA
AGGACATGGCGCGACAATGAGAAACTTCGGGAAGCGTTGCGTGTAGCCACGAGAACCTCTAAAATATACCCATAC
GTTAACTATTTCGATAAGGAGCTTATACCGGAGCAAGAAGTATGGAGAATGCTTGCGCAGGCAGCTGCTGTCGGT
GGCAGTGGTGCGGTAATTTGGGGCTCATCTGCTGCAGTTGCATCTGAAGAGTTATGTAAATCTTTAAAACAGTAT
ATTATTGAAACGCTTGGGCCGGCGGCAGAGAAGGTGGCTTGGCGTAGTGACTTATGCAGCAAAGAAATTTGTAAT
AATCAGGGTCGCTGCACATTCCCGGACGATGATTATGCAAACGCATGGAAATTATTTACAGATGATACTGTTAAG
TTTTATGCTGGTAATATTACATGTAGGTGCTCCGAGAATTATTCTGGTCGTTTCTGCGAAAAGAAGAATGGATCC
GCCATTTCTCAAATCACTGACGGTCAAATCCAAGCTACTACCACTGCTACCACCGAAGCTACCACCACTGCTGCC

CCATCTTCCACCGTTGAAACTGTTTCTCCATCCAGCACCGAAACTATCTCTCAACAAACTGAAAATGGTGCTGCT
AAGGCCGCTGTCGGTATGGGTGCCGGTGCTCTAGCTGCTGCTGCTATGTTGTTATAA

SEQ ID NO: 23 is a reencoded nucleic acid sequence of hyaluronidase (HYAL) originating
from Hirudo Nipponia with a N-terminal secretion signal
ATGCAATTCTCTACTGTCGCTTCCGTTGCTTTCGTCGCTTTGGCTAACTTTGTTGCCGCTATGAAGGAGATAGCA
GTTACCATCGATGATAAGAATGTTATAGCGTCTGTCAGTGAAAGCTTCCATGGTGTGGCTTTCGACGCAAGCCTA
TTCTCACCTAAAGGGCTATGGTCGTTTGTTGACATTACTTCACCCAAGTTATTCAAGCTTCTGGAGGGACTCTCC
CCTGGTTATTTCCGTGTCGGCGGCACATTTGCGAACTGGTTATTCTTCGACCTCGATGAAAACAACAAGTGGAAG
GATTACTGGGCTTTTAAGGACAAGACTCCAGAGACCGCCACGATTACCCGACGGTGGCTCTTCAGGAAGCAAAAC
AACCTCAAGAAGGAAACCTTTGATGATTTGGTAAAGCTTACAAAGGGGTCCAAGATGCGGCTGCTATTTGATCTA
AACGCAGAGGTCCGTACAGGATATGAGATAGGCAAGAAGATGACATCAACTTGGGACTCAAGCGAGGCAGAAAAG
TTGTTCAAATATTGCGTTAGCAAGGGATATGGAGATAATATCGATTGGGAGTTAGGCAACGAACCTGATCACACG
TCAGCACACAATTTGACAGAGAAGCAAGTAGGTGAAGATTTTAAGGCCCTACACAAGGTGTTGGAAAAGTATCCA
ACACTTAATAAAGGTAGCTTGGTTGGTCCAGACGTTGGGTGGATGGGAGTGTCGTACGTCAAGGGTCTGGCTGAC
GGGGCTGGAGATCATGTGACCGCTTTTACTCTACATCAGTATTATTTCGATGGAAATACGAGTGATGTTAGTACC
TACTTGGATGCGACGTACTTTAAGAAGTTGCAACAGTTATTCGATAAGGTAAAAGACGTACTCAAGAATTCTCCA
CATAAGGACAAACCCTTGTGGCTAGGGGAAACCTCTTCCGGCTACAACAGTGGGACTAAAGATGTATCCGATAGA
TACGTGTCGGGGTTCTTGACGCTGGACAAGTTGGGGCTTTCGGCGGCAAATAACGTCAAGGTGGTCATCAGACAA
ACGATTTATAATGGTTACTATGGTTTGCTTGACAAGAATACTTTGGAGCCCAATCCGGACTACTGGCTGATGCAC
GTCCACAATTCCTTAGTTGGTAATACCGTTTTCAAGGTCGATGTCAGCGATCCCACAAACAAAGCCCGTGTTTAC
GCTCAGTGTACGAAGACCAATAGTAAACATACCCAGTCACGTTATTATAAGGGGTCCCTAACCATTTTCGCGTTG
AATGTAGGTGATGAAGATGTTACACTCAAGATTGATCAATACAGTGGTAAGAAAATATACTCATATATTCTGACG
CCTGAAGGCGGCCAGTTAACCTCTCAAAAGGTACTGCTTAACGGTAAAGAATTGAAGTTAGTGTCAGATCAACTT
CCAGAACTTAACGCGGACGAAAGTAAAACATCCTTCACATTGTCTCCCAAAACTTTTGGATTCTTTGTGGTCTCG
GATGCCAACGTTGAAGCTTGCAAGAAATAA

SEQ ID NO: 24 is a reencoded nucleic acid sequence of hyaluronidase (HYAL) originating
from Hirudo Nipponia with a N-terminal secretion signal and a C-terminal anchoring signal
ATGCAATTCTCTACTGTCGCTTCCGTTGCTTTCGTCGCTTTGGCTAACTTTGTTGCCGCTATGAAGGAGATAGCA
GTTACCATCGATGATAAGAATGTTATAGCGTCTGTCAGTGAAAGCTTCCATGGTGTGGCTTTCGACGCAAGCCTA
TTCTCACCTAAAGGGCTATGGTCGTTTGTTGACATTACTTCACCCAAGTTATTCAAGCTTCTGGAGGGACTCTCC
CCTGGTTATTTCCGTGTCGGCGGCACATTTGCGAACTGGTTATTCTTCGACCTCGATGAAAACAACAAGTGGAAG
GATTACTGGGCTTTTAAGGACAAGACTCCAGAGACCGCCACGATTACCCGACGGTGGCTCTTCAGGAAGCAAAAC
AACCTCAAGAAGGAAACCTTTGATGATTTGGTAAAGCTTACAAAGGGGTCCAAGATGCGGCTGCTATTTGATCTA
AACGCAGAGGTCCGTACAGGATATGAGATAGGCAAGAAGATGACATCAACTTGGGACTCAAGCGAGGCAGAAAAG
TTGTTCAAATATTGCGTTAGCAAGGGATATGGAGATAATATCGATTGGGAGTTAGGCAACGAACCTGATCACACG
TCAGCACACAATTTGACAGAGAAGCAAGTAGGTGAAGATTTTAAGGCCCTACACAAGGTGTTGGAAAAGTATCCA

ACACTTAATAAAGGTAGCTTGGTTGGTCCAGACGTTGGGTGGATGGGAGTGTCGTACGTCAAGGGTCTGGCTGAC
GGGGCTGGAGATCATGTGACCGCTTTTACTCTACATCAGTATTATTTCGATGGAAATACGAGTGATGTTAGTACC
TACTTGGATGCGACGTACTTTAAGAAGTTGCAACAGTTATTCGATAAGGTAAAAGACGTACTCAAGAATTCTCCA
CATAAGGACAAACCCTTGTGGCTAGGGGAAACCTCTTCCGGCTACAACAGTGGGACTAAAGATGTATCCGATAGA
TACGTGTCGGGGTTCTTGACGCTGGACAAGTTGGGGCTTTCGGCGGCAAATAACGTCAAGGTGGTCATCAGACAA
ACGATTTATAATGGTTACTATGGTTTGCTTGACAAGAATACTTTGGAGCCCAATCCGGACTACTGGCTGATGCAC
GTCCACAATTCCTTAGTTGGTAATACCGTTTTCAAGGTCGATGTCAGCGATCCCACAAACAAAGCCCGTGTTTAC
GCTCAGTGTACGAAGACCAATAGTAAACATACCCAGTCACGTTATTATAAGGGGTCCCTAACCATTTTCGCGTTG
AATGTAGGTGATGAAGATGTTACACTCAAGATTGATCAATACAGTGGTAAGAAAATATACTCATATATTCTGACG
CCTGAAGGCGGCCAGTTAACCTCTCAAAAGGTACTGCTTAACGGTAAAGAATTGAAGTTAGTGTCAGATCAACTT
CCAGAACTTAACGCGGACGAAAGTAAAACATCCTTCACATTGTCTCCCAAAACTTTTGGATTCTTTGTGGTCTCG
GATGCCAACGTTGAAGCTTGCAAGAAAGGATCCGCCATTTCTCAAATCACTGACGGTCAAATCCAAGCTACTACC
ACTGCTACCACCGAAGCTACCACCACTGCTGCCCCATCTTCCACCGTTGAAACTGTTTCTCCATCCAGCACCGAA
ACTATCTCTCAACAAACTGAAAATGGTGCTGCTAAGGCCGCTGTCGGTATGGGTGCCGGTGCTCTAGCTGCTGCT
GCTATGTTGTTATAA

SEQ ID NO: 25 is a reencoded nucleic acid sequence of hyaluronidase (HYAL) originating
from Loxosceles intermedia with a N-terminal secretion signal
ATGCAGTTTTCAACCGTTGCGAGCGTCGCTTTTGTGGCTCTTGCTAATTTCGTTGCCGCCTTCGATGTCTTCTGG
AATGTTCCGTCGCAGCAATGCAAGAAATATGGTATGAAATTCGTTCCGCTCTTAGAGCAATACTCTATCCTAGTC
AACAAAGAAGACAATTTCAAGGGCGACAAAATAACGATCTTTTATGAGTCACAGCTCGGGCTTTACCCACATATT
GGTGCAAACGACGAGTCGTTTAATGGCGGATACCACAATTAGGTGACCTGAAAGCACACTTAGAAAAGTCAGCG
GTTGATATACGACGTGATATTTTGGATAAGTCGGCGACTGGTCTAAGAATTATAGACTGGGAAGCATGGAGACCA
ATATGGGAATTCAACTGGTCTAGCCTACGAAAGTACCAAGATAAAATGAAGAAAGTCGTCCGCCAGTTTAACCCG
ACTGCTCATGAATCCACAGTGGCCAAACTAGCACATAATGAGTGGGAAAATAGTAGTAAGTCTTGGATGCTTTCT
ACATTGCAGTTGGGTAAGCAACTTCGTCCAAACTCTGTATGGTGCTATTATTTATTTCCCGACTGTTATAACTAT
GATGGCAACTCAGTCCAAGAATTTCAGTGTTCTGAAGCTATCCGTAAGGGGAACGATAGGTTGAAATGGTTGTGG
GAAGAATCGACAGCTGTATGCCCATCTATCTACATAAAAGAAGGCCAACTGACCAATTATACCTTGCAAAAGAGA
ATCTGGTTTACCAATGGGAGATTACAGGAAGCCTTGAGAGTAGCTCAACCTAAAGCGCGTATTTATCCATACATA
AATTACTCCATCAAACCCGGAATGATGGTGCCTGAAGTTGAGTTTTGGCGGTTAATCGCTCAGATAGCCTCGCTG
GGTATGGATGGAGCAGTGATTTGGGGATCAAGTGCGAGTGTAGGCAGTAAGAATCATTGTGCGCAATTAATGAAG
TACATTGCAGACGTATTGGGTCCTGCAACTTTGCGCATAAAAGAAAATGTAGCACGGTGCAGTAAACAGGCGTGC
TCTGGTAGGGGTAGATGTACCTGGCCTAAAGATACCTCTGTTATTGCTTGGAAGTTCCTCGTTGAAAAGGAAGAC
TATGACTTTTATCTGGGTGATATAGAGTGTAAATGTGTTGAAGGCTACGAAGGTAGGTACTGTGAACAAAAGACT
AAGTAA

SEQ ID NO: 26 is a reencoded nucleic acid sequence of hyaluronidase (HYAL) originating
from Loxosceles intermedia with a N-terminal secretion signal and a C-terminal anchoring
signal
ATGCAGTTTTCAACCGTTGCGAGCGTCGCTTTTGTGGCTCTTGCTAATTTCGTTGCCGCCTTCGATGTCTTCTGG
AATGTTCCGTCGCAGCAATGCAAGAAATATGGTATGAAATTCGTTCCGCTCTTAGAGCAATACTCTATCCTAGTC
AACAAAGAAGACAATTTCAAGGGCGACAAAATAACGATCTTTTATGAGTCACAGCTCGGGCTTTACCCACATATT
GGTGCAAACGACGAGTCGTTTAATGGCGGATACCACAATTAGGTGACCTGAAAGCACACTTAGAAAAGTCAGCG
GTTGATATACGACGTGATATTTTGGATAAGTCGGCGACTGGTCTAAGAATTATAGACTGGGAAGCATGGAGACCA
ATATGGGAATTCAACTGGTCTAGCCTACGAAAGTACCAAGATAAAATGAAGAAAGTCGTCCGCCAGTTTAACCCG
ACTGCTCATGAATCCACAGTGGCCAAACTAGCACATAATGAGTGGGAAAATAGTAGTAAGTCTTGGATGCTTTCT
ACATTGCAGTTGGGTAAGCAACTTCGTCCAAACTCTGTATGGTGCTATTATTTATTTCCCGACTGTTATAACTAT
GATGGCAACTCAGTCCAAGAATTTCAGTGTTCTGAAGCTATCCGTAAGGGGAACGATAGGTTGAAATGGTTGTGG
GAAGAATCGACAGCTGTATGCCCATCTATCTACATAAAAGAAGGCCAACTGACCAATTATACCTTGCAAAAGAGA
ATCTGGTTTACCAATGGGAGATTACAGGAAGCCTTGAGAGTAGCTCAACCTAAAGCGCGTATTTATCCATACATA
AATTACTCCATCAAACCCGGAATGATGGTGCCTGAAGTTGAGTTTTGGCGGTTAATCGCTCAGATAGCCTCGCTG
GGTATGGATGGAGCAGTGATTTGGGGATCAAGTGCGAGTGTAGGCAGTAAGAATCATTGTGCGCAATTAATGAAG
TACATTGCAGACGTATTGGGTCCTGCAACTTTGCGCATAAAAGAAAATGTAGCACGGTGCAGTAAACAGGCGTGC
TCTGGTAGGGGTAGATGTACCTGGCCTAAAGATACCTCTGTTATTGCTTGGAAGTTCCTCGTTGAAAAGGAAGAC
TATGACTTTTATCTGGGTGATATAGAGTGTAAATGTGTTGAAGGCTACGAAGGTAGGTACTGTGAACAAAAGACT
AAGGGATCCGCCATTTCTCAAATCACTGACGGTCAAATCCAAGCTACTACCACTGCTACCACCGAAGCTACCACC
ACTGCTGCCCCATCTTCCACCGTTGAAACTGTTTCTCCATCCAGCACCGAAACTATCTCTCAACAAACTGAAAAT
GGTGCTGCTAAGGCCGCTGTCGGTATGGGTGCCGGTGCTCTAGCTGCTGCTGCTATGTTGTTATAA

SEQ ID NO: 27 is a reencoded nucleic acid sequence of hyaluronidase (HYAL) originating
from Tityus serrulatus with a N-terminal secretion signal
ATGCAATTCTCTACTGTCGCTTCCGTTGCTTTCGTCGCTTTGGCTAACTTTGTTGCCGCTGCTGATTTTAAAGTT
TACTGGGAAGTGCCTTCCTTCCTTTGTTCTAAACGTTTTAAAATTAATGTAACGGAAGTTTTAACAAGTCACGAG
ATTCTTGTCAATCAGGGTGAGAGTTTCAACGGTGACAAGATAGTAATCTTTTACGAAAACCAATTGGGGAAGTAC
CCGCATATTGACTCAAACAATGTGGAGATCAATGGAGGAATACTTCAAGTAGCCGATTTGGCGAAGCATTTGAAA
GTAGCCAAGGATAATATCACTAAATTCGTCCCGAATCCTAATTTCAACGGTGTCGGAGTGATCGACTGGGAAGCT
TGGCGGCCATCATGGGAATTTAACTGGGGTAAGTTAAAAGTATATAAGAAAAGAGCATTGACTTGGTCAAGTCG
AAACATCCGGAGTGGCCCTCCGACAGGGTTGAAAAGGTTGCTAAAGAGGAGTGGGAGGAGAGTGCCAAAGAATGG
ATGGTGAAGACCCTGAAGTTAGCACAGGAAATGCGACCGAACGCAGTTTGGTGCTATTATCTATTCCCTGACTGC
TACAATTATTTCGGTAAGGATCAACCCTCTCAATTCAGCTGCTCGTCTCGAATTCAGAAGGAAAATTCTCGTCTT
TCTTGGCTCTGGAATCAATCAACAGCCATTTGCCTAAGCATTTATATCCAGGAATCCCATGTTACCAAATATAAT
ATGTCCCAGCGGACATGGTGGATCGATGCGAGATTAAGAGAAGCAATTCGAGTCAGCGAACACAGACCAAACATA
CCCATCTACCCTTACATTAATTATATTCTACCTGGAACTAATCAAACTGTACCAGCAATGGACTTTAAAAGGACA
CTGGGTCAAATAGCTAGCCTCGGCCTAGATGGTGCTTTGTTATGGGGATCTAGCTATCATGTTTTAACAGAATCT

CAATGCAAAATCACTTCTGATTATGTGAAATCAGTGATTGCTCCTACCGTGGCTACTGTCGTTCTCAATACAAAC
AGATGCTCACAGATAATTTGTAAGGGTCGCGGCAACTGTGTTTGGCCTGAAGAACCATTTAGTTCTTGGAAATAC
TTAGTTGACCCCAAAATGCCAGTGTTCAAGCCAACCAACATCCACTGTAAATGTAAAGGTTACCTAGGTAGATAC
TGTGAGATCCCAAAGTAA

SEQ ID NO: 28 is a reencoded nucleic acid sequence of hyaluronidase (HYAL) originating
from Tityus serrulatus with a N-terminal secretion signal and a C-terminal anchoring signal
ATGCAATTCTCTACTGTCGCTTCCGTTGCTTTCGTCGCTTTGGCTAACTTTGTTGCCGCTGCTGATTTTAAAGTT
TACTGGGAAGTGCCTTCCTTCCTTTGTTCTAAACGTTTTAAAATTAATGTAACGGAAGTTTTAACAAGTCACGAG
ATTCTTGTCAATCAGGGTGAGAGTTTCAACGGTGACAAGATAGTAATCTTTTACGAAAACCAATTGGGGAAGTAC
CCGCATATTGACTCAAACAATGTGGAGATCAATGGAGGAATACTTCAAGTAGCCGATTTGGCGAAGCATTTGAAA
GTAGCCAAGGATAATATCACTAAATTCGTCCCGAATCCTAATTTCAACGGTGTCGGAGTGATCGACTGGGAAGCT
TGGCGGCCATCATGGGAATTTAACTGGGGTAAGTTAAAAGTATATAAAGAAAAGAGCATTGACTTGGTCAAGTCG
AAACATCCGGAGTGGCCCTCCGACAGGGTTGAAAAGGTTGCTAAAGAGGAGTGGGAGGAGAGTGCCAAAGAATGG
ATGGTGAAGACCCTGAAGTTAGCACAGGAAATGCGACCGAACGCAGTTTGGTGCTATTATCTATTCCCTGACTGC
TACAATTATTTCGGTAAGGATCAACCCTCTCAATTCAGCTGCTCGTCTCGAATTCAGAAGGAAAATTCTCGTCTT
TCTTGGCTCTGGAATCAATCAACAGCCATTTGCCTAAGCATTTATATCCAGGAATCCCATGTTACCAAATATAAT
ATGTCCCAGCGGACATGGTGGATCGATGCGAGATTAAGAGAAGCAATTCGAGTCAGCGAACACAGACCAAACATA
CCCATCTACCCTTACATTAATTATATTCTACCTGGAACTAATCAAACTGTACCAGCAATGGACTTTAAAAGGACA
CTGGGTCAAATAGCTAGCCTCGGCCTAGATGGTGCTTTGTTATGGGGATCTAGCTATCATGTTTTAACAGAATCT
CAATGCAAAATCACTTCTGATTATGTGAAATCAGTGATTGCTCCTACCGTGGCTACTGTCGTTCTCAATACAAAC
AGATGCTCACAGATAATTTGTAAGGGTCGCGGCAACTGTGTTTGGCCTGAAGAACCATTTAGTTCTTGGAAATAC
TTAGTTGACCCCAAAATGCCAGTGTTCAAGCCAACCAACATCCACTGTAAATGTAAAGGTTACCTAGGTAGATAC
TGTGAGATCCCAAAGGATCCGCCATTTCTCAAATCACTGACGGTCAAATCCAAGCTACTACCACTGCTACCACC
GAAGCTACCACCACTGCTGCCCCATCTTCCACCGTTGAAACTGTTTCTCCATCCAGCACCGAAACTATCTCTCAA
CAAACTGAAAATGGTGCTGCTAAGGCCGCTGTCGGTATGGGTGCCGGTGCTCTAGCTGCTGCTGCTATGTTGTTA
TAA

SEQ ID NO: 29 is a reencoded nucleic acid sequence of hyaluronidase (HYAL) originating
from Vespa magnifica with a N-terminal secretion signal
ATGCAATTTTCTACAGTGGCAAGTGTTGCATTCGTTGCACTAGCCAACTTTGTGGCGGCAGATAGCTGTGGGTCA
AACTGCGAAAAGAGTGAGAGACCGAAAAGGGTCTTCAACATTTACTGGAACGTACCTACATTCATGTGTCACCAG
TACGGACTATACTTTGACGAGGTCACGAATTTTAATATAAAGCACAACAGCAAAGACAATTTTCAAGGGGACAAG
ATCGCGATCTTTTATGACCCCGGGGAGTTTCCCGCTCTGCTGCCACTAAACTATGGTAAGTACAAGATCAGGAAT
GGTGGTGTTCCACAAGAGGGTAACATCACCATCCATCTGCAGAGATTCATAGAGTACCTAGATAAGACCTATCCG
AACCGTAACTTTTCAGGCATCGGTGTGATCGATTTCGAGAGGTGGAGACCAATTTTCAGACAGAATTGGGGTAAT
ATGAAGATTTACAAGAACTTCTCCATCGATCTTGTGCGTAAAGAGCATCCTTTCTGGAATAAGAAAATGATCGAG
TTGGAAGCTTCTAAAAGATTCGAGAAATACGCCCGTCTGTTCATGGAAGAAACATTAAAGTTGGCTAAGAAAACT

AGAAAACAGGCCGATTGGGGCTACTACGGTTACCCCTATTGCTTCAACATGTCTCCTACTAATTTCGTTCCTGAC
TGCGATGTCACAGCTAGGGATGAGAACAACGAGATGTCTTGGTTGTTTAACAACCAGAATGTCCTATTACCAAGT
GTATACATTAGGAGAGAGCTAACTCCTGACCAGAGGATTGGGCTTGTACAGGGGAGAGTGAAGGAAGCTGTGAGA
ATTTCAAATAAACTGAAGCACTCACCTAAAGTCTTCAGCTATTGGTGGTATGTTTACCAAGACGAGACCAACACC
TTCTTAACGGAGACCGACGTCAAGAAGACGTTTCAGGAGATTGTGATCAACGGTGGAGATGGGATTATAATCTGG
GGTTCGTCCTCTGATGTAAACAGCTTGTCCAAGTGTACGAGGTTAAGGGAGTACCTATTGACAGTCTTGGGACCA
ATTGCTGTTAACGTGACTGAAGCAGTAAACTAA

SEQ ID NO: 30 is a reencoded nucleic acid sequence of hyaluronidase (HYAL) originating
from Vespa magnifica with a N-terminal secretion signal and a C-terminal anchoring signal
ATGCAATTTTCTACAGTGGCAAGTGTTGCATTCGTTGCACTAGCCAACTTTGTGGCGGCAGATAGCTGTGGGTCA
AACTGCGAAAAGAGTGAGAGACCGAAAAGGGTCTTCAACATTTACTGGAACGTACCTACATTCATGTGTCACCAG
TACGGACTATACTTTGACGAGGTCACGAATTTTAATATAAAGCACAACAGCAAAGACAATTTTCAAGGGGACAAG
ATCGCGATCTTTTATGACCCCGGGGAGTTTCCCGCTCTGCTGCCACTAAACTATGGTAAGTACAAGATCAGGAAT
GGTGGTGTTCCACAAGAGGGTAACATCACCATCCATCTGCAGAGATTCATAGAGTACCTAGATAAGACCTATCCG
AACCGTAACTTTTCAGGCATCGGTGTGATCGATTTCGAGAGGTGGAGACCAATTTTCAGACAGAATTGGGGTAAT
ATGAAGATTTACAAGAACTTCTCCATCGATCTTGTGCGTAAAGAGCATCCTTTCTGGAATAAGAAAATGATCGAG
TTGGAAGCTTCTAAAAGATTCGAGAAATACGCCCGTCTGTTCATGGAAGAAACATTAAAGTTGGCTAAGAAAACT
AGAAAACAGGCCGATTGGGGCTACTACGGTTACCCCTATTGCTTCAACATGTCTCCTACTAATTTCGTTCCTGAC
TGCGATGTCACAGCTAGGGATGAGAACAACGAGATGTCTTGGTTGTTTAACAACCAGAATGTCCTATTACCAAGT
GTATACATTAGGAGAGAGCTAACTCCTGACCAGAGGATTGGGCTTGTACAGGGGAGAGTGAAGGAAGCTGTGAGA
ATTTCAAATAAACTGAAGCACTCACCTAAAGTCTTCAGCTATTGGTGGTATGTTTACCAAGACGAGACCAACACC
TTCTTAACGGAGACCGACGTCAAGAAGACGTTTCAGGAGATTGTGATCAACGGTGGAGATGGGATTATAATCTGG
GGTTCGTCCTCTGATGTAAACAGCTTGTCCAAGTGTACGAGGTTAAGGGAGTACCTATTGACAGTCTTGGGACCA
ATTGCTGTTAACGTGACTGAAGCAGTAAACGGATCCGCCATTTCTCAAATCACTGACGGTCAAATCCAAGCTACT
ACCACTGCTACCACCGAAGCTACCACCACTGCTGCCCCATCTTCCACCGTTGAAACTGTTTCTCCATCCAGCACC
GAAACTATCTCTCAACAAACTGAAAATGGTGCTGCTAAGGCCGCTGTCGGTATGGGTGCCGGTGCTCTAGCTGCT
GCTGCTATGTTGTTATAA

SEQ ID NO: 31 is an amino acid sequence of hyaluronidase (HYAL) originating from
Bothrops atrox with a N-terminal secretion signal
MQFSTVASVAFVALANFVAAPMYPNEPFLVFWNAPTTQCRLRYKVDLDLKTFHIVTNANDSLSGSAVTIFYPTHL
GVYPHIDDRGHFFNGIIPQNESLVKHLNKSKSDINRMIPLRTFHGLGVIDWENWRPQWDRNWGSKNVYRNRSIQF
ARDLHPELSEDKIKRLAKQEFEKAAKSFMRDTLLLAEEMRPDGYWGYYLYPDCHNYNYKTKPDQYTGECPDIEMS
RNNQLLWLWRDSTALFPNIYLETILRSSDNALKFVHHRLKEAMRIASMARNDYALPFFVYARPFYAYTFEPLTQE
DLVTTVGETADMGAAGIVFWGSMQYASTVESCGKVKDYMNGPLGRYIVNVTTAAKICSRFLCKRHGRCVRKHSDS
NAFLHLFPDSFRIMVHGNATEKKVIVKGKLELKNLIFLRNNFMCQCYQGWKGLYCEKHSIKEIRKI

SEQ ID NO: 32 is an amino acid sequence of hyaluronidase (HYAL) originating from
Bothrops atrox with a N-terminal secretion signal and a C-terminal anchoring signal
MQFSTVASVAFVALANFVAAPMYPNEPFLVFWNAPTTQCRLRYKVDLDLKTFHIVTNANDSLSGSAVTIFYPTHL
GVYPHIDDRGHFFNGIIPQNESLVKHLNKSKSDINRMIPLRTFHGLGVIDWENWRPQWDRNWGSKNVYRNRSIQF
ARDLHPELSEDKIKRLAKQEFEKAAKSFMRDTLLLAEEMRPDGYWGYYLYPDCHNYNYKTKPDQYTGECPDIEMS
RNNQLLWLWRDSTALFPNIYLETILRSSDNALKFVHHRLKEAMRIASMARNDYALPFFVYARPFYAYTFEPLTQE
DLVTTVGETADMGAAGIVFWGSMQYASTVESCGKVKDYMNGPLGRYIVNVTTAAKICSRFLCKRHGRCVRKHSDS
NAFLHLFPDSFRIMVHGNATEKKVIVKGKLELKNLIFLRNNFMCQCYQGWKGLYCEKHSIKEIRKIGSAISQITD
GQIQATTTATTEATTTAAPSSTVETVSPSSTETISQQTENGAAKAAVGMGAGALAAAAMLL

SEQ ID NO: 33 is an amino acid sequence of hyaluronidase (HYAL) originating from
Cupiennius salei with a N-terminal secretion signal
MQFSTVASVAFVALANFVAAFKIYWNVPTFQCTHNYKIDYVKLLSTYGIQVNDGGKFQGNQVTIFYETQLGLYPR
ILKSGKMENGGIPQRGNFEKHLEKASTDLQKVIPWKEFSGLGVIDWEAWRPTWEFNWEPLRIYQTESIKRAKELH
PTANDSAVKEIAERQWEDSAKLYMLETLRLAKKLRPQAPWCYYLFPDCYNYVGKKPKDFQCSASIRKGNDKLSWL
WKDSTALCPSIYVYESQLDRYSFEQRTWRDNEKLREALRVATRTSKIYPYVNYFDKELIPEQEVWRMLAQAAAVG
GSGAVIWGSSAAVASEELCKSLKQYIIETLGPAAEKVAWRSDLCSKEICNNQGRCTFPDDDYANAWKLFTDDTVK
FYAGNITCRCSENYSGRFCEKKN

SEQ ID NO: 34 is an amino acid sequence of hyaluronidase (HYAL) originating from
Cupiennius salei with a N-terminal secretion signal and a C-terminal anchoring signal
MQFSTVASVAFVALANFVAAFKIYWNVPTFQCTHNYKIDYVKLLSTYGIQVNDGGKFQGNQVTIFYETQLGLYPR
ILKSGKMENGGIPQRGNFEKHLEKASTDLQKVIPWKEFSGLGVIDWEAWRPTWEFNWEPLRIYQTESIKRAKELH
PTANDSAVKEIAERQWEDSAKLYMLETLRLAKKLRPQAPWCYYLFPDCYNYVGKKPKDFQCSASIRKGNDKLSWL
WKDSTALCPSIYVYESQLDRYSFEQRTWRDNEKLREALRVATRTSKIYPYVNYFDKELIPEQEVWRMLAQAAAVG
GSGAVIWGSSAAVASEELCKSLKQYIIETLGPAAEKVAWRSDLCSKEICNNQGRCTFPDDDYANAWKLFTDDTVK
FYAGNITCRCSENYSGRFCEKKNGSAISQITDGQIQATTTATTEATTTAAPSSTVETVSPSSTETISQQTENGAA
KAAVGMGAGALAAAAMLL

SEQ ID NO: 35 is an amino acid sequence of hyaluronidase (HYAL) originating from
Hirudo Nipponia with a N-terminal secretion signal
MQFSTVASVAFVALANFVAA~MKEIAVTIDDKNVIASVSESFHGVAFDASLFSPKGLWSFVDITSPKLFKLLEGL
SPGYFRVGGTFANWLFFDLDENNKWKDYWAFKDKTPETATITRRWLFRKQNNLKKETFDDLVKLTKGSKMRLLFD
LNAEVRTGYEIGKKMTSTWDSSEAEKLFKYCVSKGYGDNIDWELGNEPDHTSAHNLTEKQVGEDFKALHKVLEKY
PTLNKGSLVGPDVGWMGVSYVKGLADGAGDHVTAFTLHQYYFDGNTSDVSTYLDATYFKKLQQLFDKVKDVLKNS
PHKDKPLWLGETSSGYNSGTKDVSDRYVSGFLTLDKLGLSAANNVKVVIRQTIYNGYYGLLDKNTLEPNPDYWLM

HVHNSLVGNTVFKVDVSDPTNKARVYAQCTKTNSKHTQSRYYKGSLTIFALNVGDEDVTLKIDQYSGKKIYSYIL

TPEGGQLTSQKVLLNGKELKLVSDQLPELNADESKTSFTLSPKTFGFFVVSDANVEACKKGSAISQITDGQIQAT

TTATTEATTTAAPSSTVETVSPSSTETISQQTENGAAKAAVGMGAGALAAAAMLL

SEQ ID NO: 36 is an amino acid sequence of hyaluronidase (HYAL) originating from
Hirudo Nipponia with a N-terminal secretion signal and a C-terminal anchoring signal
MQFSTVASVAFVALANFVAA~MKEIAVTIDDKNVIASVSESFHGVAFDASLFSPKGLWSFVDITSPKLFKLLEGL
SPGYFRVGGTFANWLFFDLDENNKWKDYWAFKDKTPETATITRRWLFRKQNNLKKETFDDLVKLTKGSKMRLLFD
LNAEVRTGYEIGKKMTSTWDSSEAEKLFKYCVSKGYGDNIDWELGNEPDHTSAHNLTEKQVGEDFKALHKVLEKY
PTLNKGSLVGPDVGWMGVSYVKGLADGAGDHVTAFTLHQYYFDGNTSDVSTYLDATYFKKLQQLFDKVKDVLKNS
PHKDKPLWLGETSSGYNSGTKDVSDRYVSGFLTLDKLGLSAANNVKVVIRQTIYNGYYGLLDKNTLEPNPDYWLM
HVHNSLVGNTVFKVDVSDPTNKARVYAQCTKTNSKHTQSRYYKGSLTIFALNVGDEDVTLKIDQYSGKKIYSYIL
TPEGGQLTSQKVLLNGKELKLVSDQLPELNADESKTSFTLSPKTFGFFVVSDANVEACKK

SEQ ID NO: 37 is an amino acid sequence of hyaluronidase (HYAL) originating from
Loxosceles intermedia with a N-terminal secretion signal
MQFSTVASVAFVALANFVAAFDVFWNVPSQQCKKYGMKFVPLLEQYSILVNKEDNFKGDKITIFYESQLGLYPHI
GANDESFNGGIPQLGDLKAHLEKSAVDIRRDILDKSATGLRIIDWEAWRPIWEFNWSSLRKYQDKMKKVVRQFNP
TAHESTVAKLAHNEWENSSKSWMLSTLQLGKQLRPNSVWCYYLFPDCYNYDGNSVQEFQCSEAIRKGNDRLKWLW
EESTAVCPSIYIKEGQLTNYTLQKRIWFTNGRLQEALRVAQPKARIYPYINYSIKPGMMVPEVEFWRLIAQIASL
GMDGAVIWGSSASVGSKNHCAQLMKYIADVLGPATLRIKENVARCSKQACSGRGRCTWPKDTSVIAWKFLVEKED
YDFYLGDIECKCVEGYEGRYCEQKTK

SEQ ID NO: 38 is an amino acid sequence of hyaluronidase (HYAL) originating from
Loxosceles intermedia with a N-terminal secretion signal and a C-terminal anchoring signal
MQFSTVASVAFVALANFVAAFDVFWNVPSQQCKKYGMKFVPLLEQYSILVNKEDNFKGDKITIFYESQLGLYPHI
GANDESFNGGIPQLGDLKAHLEKSAVDIRRDILDKSATGLRIIDWEAWRPIWEFNWSSLRKYQDKMKKVVRQFNP
TAHESTVAKLAHNEWENSSKSWMLSTLQLGKQLRPNSVWCYYLFPDCYNYDGNSVQEFQCSEAIRKGNDRLKWLW
EESTAVCPSIYIKEGQLTNYTLQKRIWFTNGRLQEALRVAQPKARIYPYINYSIKPGMMVPEVEFWRLIAQIASL
GMDGAVIWGSSASVGSKNHCAQLMKYIADVLGPATLRIKENVARCSKQACSGRGRCTWPKDTSVIAWKFLVEKED
YDFYLGDIECKCVEGYEGRYCEQKTKGSAISQITDGQIQATTTATTEATTTAAPSSTVETVSPSSTETISQQTEN
GAAKAAVGMGAGALAAAAMLL

SEQ ID NO: 39 is an amino acid sequence of hyaluronidase (HYAL) originating from Tityus
serrulatus with a N-terminal secretion signal
MQFSTVASVAFVALANFVAAADFKVYWEVPSFLCSKRFKINVTEVLTSHEILVNQGESFNGDKIVIFYENQLGKY
PHIDSNNVEINGGILQVADLAKHLKVAKDNITKFVPNPNFNGVGVIDWEAWRPSWEFNWGKLKVYKEKSIDLVKS
KHPEWPSDRVEKVAKEEWEESAKEWMVKTLKLAQEMRPNAVWCYYLFPDCYNYFGKDQPSQFSCSSRIQKENSRL
SWLWNQSTAICLSIYIQESHVTKYNMSQRTWWIDARLREAIRVSEHRPNIPIYPYINYILPGTNQTVPAMDFKRT
LGQIASLGLDGALLWGSSYHVLTESQCKITSDYVKSVIAPTVATVVLNTNRCSQIICKGRGNCVWPEEPFSSWKY
LVDPKMPVFKPTNIHCKCKGYLGRYCEIPK

SEQ ID NO: 40 is an amino acid sequence of hyaluronidase (HYAL) originating from Tityus
serrulatus with a N-terminal secretion signal and a C-terminal anchoring signal
MQFSTVASVAFVALANFVAAADFKVYWEVPSFLCSKRFKINVTEVLTSHEILVNQGESFNGDKIVIFYENQLGKY
PHIDSNNVEINGGILQVADLAKHLKVAKDNITKFVPNPNFNGVGVIDWEAWRPSWEFNWGKLKVYKEKSIDLVKS
KHPEWPSDRVEKVAKEEWEESAKEWMVKTLKLAQEMRPNAVWCYYLFPDCYNYFGKDQPSQFSCSSRIQKENSRL
SWLWNQSTAICLSIYIQESHVTKYNMSQRTWWIDARLREAIRVSEHRPNIPIYPYINYILPGTNQTVPAMDFKRT
LGQIASLGLDGALLWGSSYHVLTESQCKITSDYVKSVIAPTVATVVLNTNRCSQIICKGRGNCVWPEEPFSSWKY
LVDPKMPVFKPTNIHCKCKGYLGRYCEIPKGSAISQITDGQIQATTTATTEATTTAAPSSTVETVSPSSTETISQ
QTENGAAKAAVGMGAGALAAAAMLL

SEQ ID NO: 41 is an amino acid sequence of hyaluronidase (HYAL) originating from Vespa magnifica with a N-terminal secretion signal
MQFSTVASVAFVALANFVAADSCGSNCEKSERPKRVFNIYWNVPTFMCHQYGLYFDEVTNFNIKHNSKDNFQGDK
IAIFYDPGEFPALLPLNYGKYKIRNGGVPQEGNITIHLQRFIEYLDKTYPNRNFSGIGVIDFERWRPIFRQNWGN
MKIYKNFSIDLVRKEHPFWNKKMIELEASKRFEKYARLFMEETLKLAKKTRKQADWGYYGYPYCFNMSPTNFVPD
CDVTARDENNEMSWLFNNQNVLLPSVYIRRELTPDQRIGLVQGRVKEAVRISNKLKHSPKVFSYWWYVYQDETNT
FLTETDVKKTFQEIVINGGDGIIIWGSSSDVNSLSKCTRLREYLLTVLGPIAVNVTEAVN

SEQ ID NO: 42 is an amino acid sequence of hyaluronidase (HYAL) originating from Vespa
magnifica with a N-terminal secretion signal and a C-terminal anchoring signal
MQFSTVASVAFVALANFVAADSCGSNCEKSERPKRVFNIYWNVPTFMCHQYGLYFDEVTNFNIKHNSKDNFQGDK
IAIFYDPGEFPALLPLNYGKYKIRNGGVPQEGNITIHLQRFIEYLDKTYPNRNFSGIGVIDFERWRPIFRQNWGN
MKIYKNFSIDLVRKEHPFWNKKMIELEASKRFEKYARLFMEETLKLAKKTRKQADWGYYGYPYCFNMSPTNFVPD
CDVTARDENNEMSWLFNNQNVLLPSVYIRRELTPDQRIGLVQGRVKEAVRISNKLKHSPKVFSYWWYVYQDETNT
FLTETDVKKTFQEIVINGGDGIIIWGSSSDVNSLSKCTRLREYLLTVLGPIAVNVTEAVNAISQITDGQIQATTT
ATTEATTTAAPSSTVETVSPSSTETISQQTENGAAKAAVGMGAGALAAAAMLL

**SEQ ID NO: 43** is a nucleic acid sequence of the secretion sequence added in 5' ATGCAATTTAGCACAGTCGCATCAGTAGCCTTCGTTGCCTTGGCCAACTTCGTGGCAGCA
**SEQ ID NO: 44** is an amino acid sequence of the secretion sequence added in N-ter MQFSTVASVAFVALANFVAA

SEQ ID NO: 45 is a nucleic acid sequence of the anchoring sequence added in 3'
GGATCCGCCATTTCTCAAATCACTGACGGTCAAATCCAAGCTACTACCACTGCTACCACCGAAGCTACCACCACT
GCTGCCCCATCTTCCACCGTTGAAACTGTTTCTCCATCCAGCACCGAAACTATCTCTCAACAAACTGAAAATGGT
GCTGCTAAGGCCGCTGTCGGTATGGGTGCCGGTGCTCTAGCTGCTGCTGCTATGTTGTTATAA

**SEQ ID NO: 46** is an amino acid sequence of the anchoring sequence added in C-ter AISQITDGQIQATTTATTEATTTAAPSSTVETVSPSSTETISQQTENGAAKAAVGMGAGALAAAAMLL

SEQ ID NO: 47 is a nucleic acid sequence of glutamine-fructose-6-phosphate
amidotransferase (GFA1) originating from Saccharomyces cerevisiae
ATGTGTGGTATCTTTGGTTACTGCAATTATCTAGTGGAAAGATCCAGAGGAGAAATTATCGACACCTTAGTGGAT
GGTTTACAAAGATTAGAATATAGAGGCTATGATTCCACCGGTATTGCTATCGATGGTGACGAAGCTGATTCTACT
TTCATCTATAAGCAAATCGGTAAAGTGAGTGCTTTGAAAGAGGAGATTACTAAGCAAAATCCGAACAGAGACGTT
ACTTTTGTCTCTCATTGTGGTATTGCGCATACTAGATGGGCTACTCACGGTCGACCAGAACAAGTTAACTGTCAC
CCTCAAAGATCTGACCCAGAAGACCAATTTGTGGTCGTTCATAATGGTATCATCACAAATTTTAGAGAACTGAAG
ACTCTTTTAATTAACAAAGGTTATAAATTCGAAAGTGATACCGATACCGAGTGTATTGCTAAACTATATTTGCAT
TTATACAATACAAATTTACAAAATGGGCATGACTTAGATTTCCACGAATTAACCAAGCTAGTTCTTTTAGAACTA
GAAGGTTCATACGGGTTATTATGTAAATCTTGTCACTATCCTAATGAGGTTATCGCCACTAGAAAAGGTCCCCT
TTACTGATTGGTGTCAAATCTGAAAAAAACTAAAAGTCGACTTCGTGGATGTGGAATTTCCCGAAGAAAACGCT
GGTCAACCGGAAATTCCATTGAAATCTAACAACAAATCATTTGGCTTGGGCCCAAAGAAAGCTCGTGAATTTGAA
GCTGGTTCCCAAAATGCCAATTTACTACCAATTGCCGCCAATGAATTTAACTTGAGACATTCTCAATCCAGGGCT
TTCCTATCAGAAGATGGATCTCCAACACCGGTGGAATTTTTTGTTTCTTCGGATGCGGCATCTGTTGTTAAACAT
ACCAAGAAGGTGCTATTTTTAGAAGATGACGATTTGGCTCATATTTACGATGGTGAGTTACATATTCATAGATCT
AGAAGAGAAGTAGGCGCATCAATGACAAGGTCCATTCAAACTTTAGAGATGGAGTTAGCTCAGATCATGAAGGGC
CCTTACGACCATTTTATGCAAAAGGAAATCTATGAGCAACCAGAATCTACTTTCAATACTATGAGAGGTAGAATC
GACTATGAAAATAATAAAGTGATATTGGGTGGTTTAAAGGCATGGTTACCAGTTGTCAGAAGAGCACGGAGACTG
ATCATGATCGCATGCGGTACTTCTTATCATTCATGTTTGGCTACTCGTGCTATCTTCGAAGAATTATCAGATATC
CCAGTTAGTGTGGAATTAGCGTCTGACTTTCTGGACAGAAAATGCCCTGTCTTCAGAGACGATGTATGCGTGTTT
GTTTCACAAAGTGGTGAAACTGCGGATACCATGCTGGCTCTAAATTATTGTTTAGAAAGAGGAGCCTTAACTGTC
GGAATTGTTAACAGTGTTGGTTCTTCTATCTCTCGTGTCACCCACTGTGGTGTTCATATTAACGCTGGTCCTGAA
ATTGGTGTTGCCTCTACAAAAGCTTATACTTCCCAGTATATTGCCTTAGTGATGTTTGCTCTATCGCTGTCAGAT

GACCGTGTATCGAAAATAGACAGAAGAATTGAAATCATTCAAGGCTTGAAGTTAATCCCGGGCCAAATTAAGCAG
GTATTAAAGCTGGAACCAAGAATAAAAAAGCTCTGTGCGACTGAATTAAAGGATCAAAAATCTCTATTGTTATTG
GGTAGAGGTTACCAATTTGCTGCTGCTCTGGAAGGTGCTTTGAAGATCAAAGAAATTTCTTATATGCATTCTGAA
GGTGTTTTGGCAGGTGAGTTGAAGCACGGTGTCTTGGCCTTGGTGGACGAAAACTTGCCAATCATTGCTTTTGGT
ACCAGAGACTCTCTATTCCCTAAAGTAGTTTCCTCTATTGAGCAAGTTACTGTAAGAAAGGGCCATCCAATTATT
ATTTGTAACGAAAATGATGAAGTGTGGGCGCAAAAATCTAAATCAATCGACCTGCAAACCTTAGAAGTTCCACAA
ACTGTTGATTGTTTACAAGGTCTAATTAATATTATTCCATTACAACTAATGTCATATTGGTTGGCTGTTAATAAA
GGGATTGATGTTGATTTTCCAAGAAACTTGGCTAAATCTGTTACCGTCGAATAA

SEQ ID NO: 48 is a reencoded nucleic acid sequence of glutamine-fructose-6-phosphate amidotransferase (GFA1) originating from Chlorella virus 1 (PBCV-1)
ATGTGCGGGATCTTCGGTGCTGTGTCGAACAATAATAGCATAGAAGTTTCCATCAAGGGTATACAGAAGCTAGAG
TACCGCGGGTACGATTCGTGTGGAATAGCCTATACAGACGGAGGCGCCATTGAACGGATCAGGTCAATAGATGGG
ATCGACGACTTAAGGAAGAAAACAATAACAGAGTCTTCCCCGGTAGCTATCGCTCATTCAAGGTGGAGTACCACT
GGGATTCCAAGTGTTGTGAACGCCCACCCGCACATCTCTCGGGGCACGTCTGGATGCGAGTCGCGCATTGCAGTC
GTACACAATGGCATTATTGAAAATTACCAGCAGATCCGCAAGTACTTGATAAATCTTGGGTATACTTTCGATTCA
CAGACCGACACGGAAGTCATTGCTCATCTAATAGACTCGCAGTATAACGGGAACATCCTGCACACCGTCCAAATG
GCAGTTAAGCACCTCAAAGGAAGCTACGCCATAGCAGTCATGTGCCATAAGGAGTCCGGGAAGATCGTGGTGGCA
AAACAGAAGAGTCCGTTGGTATTGGGTATAGGAAGCGACGGAGCTTATTATATCGCATCGGACGTATTGGCTCTT
CCCACTAACAAAGTGGTCTACATAAGTGACGGATTCTCTGCTGAGTTAAGTCCTGGATCGATGACTATCTATGAT
CTGGATGGCAATAAGGTAGAATACGAGGTGGAAGACGTTGAAATGGAGCAGACGTCGATGTCTTTAGATAACTTT
GATCACTACATGATCAAAGAGATCAATGAACAGCCGATATCAATTCTCAATACTATTAAGAACAAAGGATTCTAT
GCAGAGATATTTGGTGATCTAGCACATGAAATATTTCAGAAGATCGACAATATATTGATTCTAGCCTGCGGAACT
TCGTATCATGCAGGTCTCGTCGGGAAGCAATGGATAGAGACCATCTCACGCATCCCGGTGGATGTTCATATTGCA
AGCGAGTATGAGCCGACAATTCCACGAGCCAATACACTTGTGATAACGATTAGTCAAAGCGGCGAGACTGCGGAT
ACAATCGCAGCCCTTCAACGAGCGCAAAACGCAGGAATGATATACACTTTGTGTATCTGCAACAGCCCCAAGTCA
ACTCTAGTGCGCGAGTCGATAATGAAATACATAACCAAATGCGGTTCAGAAGTTTCTGTTGCCAGTACTAAAGCA
TTTACGTCGCAACTTGTGGTGTTGTATATGCTTGCGAACGTTCTTGCGAACAAAACTGATGATCTGCTAGGGGAT
CTTCCTCAAGCTATCGAGAGAGTAATCTGTCTTACTAATGACGAAATGAAGCGTTGGGCGGATGAAATTTGTACT
GCCAAAAGTGCCATCTTCTTAGGGAGAGGACTGAACGCACCTGTAGCATTTGAGGGCGCGCTAAAGTTGAAGGAA
ATCTCATATATCCATGCCGAGGGTTTCCTCGGTGGAGAGTTGAAGCATGGGCCGCTGGCTTTGTTGGACGATAAG
ATCCCTGTAATCGTGACTGTTGCTGACCACGCTTATTTGGATCACATTAAGGCTAACATCGACGAGGTATTGGCA
CGAAACGTTACCGTATACGCGATCGTCGATCAGTATGTTAATATTGAACCACAGGAGCGACTGCATGTAGTAAAA
GTGCCGTTTGTGAGTAAAGAGTTTTCTCCCATCATCCACACAATTCCGATGCAATTGTTATCGTATTATGTCGCG
ATCAAGCTGGGCAAGAACGTTGACAAGCCACGTAACCTGGCGAAAAGTGTTACAACATTCtaa

SEQ ID NO: 49 is a reencoded nucleic acid sequence of glutamine-fructose-6-phosphate amidotransferase (GFA1) originating from Chlorella virus 1 (PBCV-1)
ATGTGTGGCATCTTTGGAGCAGTGTCAAACAACAACTCTATCGAGGTGTCAATCAAGGGTATTCAGAAGCTAGAA
TATCGTGGGTATGATTCGTGCGGTATTGCGTATACAGATGGGGGTGCGATTGAGCGTATACGTTCTATTGACGGC
ATTGACGATCTGCGTAAGAAAACAATCACAGAATCATCACCAGTGGCCATTGCTCACTCGCGGTGGAGCACCACT
GGAATTCCATCAGTGGTGAACGCACATCCTCATATTTCTCGCGGAACCAGTGGGTGTGAGTCTCGTATCGCGGTA
GTCCACAACGGTATCATTGAAAACTATCAGCAGATCCGAAAATATCTCATCAATCTTGGTTATACGTTTGATAGT
CAAACGGACACAGAGGTCATTGCGCATTTGATTGATTCTCAGTACAATGGGAATATCTTGCACACCGTCCAAATG
GCTGTCAAGCACCTGAAGGGCTCTTATGCCATTGCAGTTATGTGTCATAAAGAGTCTGGTAAAATAGTCGTGGCG
AAACAGAAGTCACCCCTCGTACTTGGAATCGGCTCAGATGGTGCTTACTACATCGCTTCGGACGTGCTGGCGCTG
CCGACAAATAAAGTTGTTTATATTTCAGACGGTTTCTCCGCAGAACTATCTCCAGGGAGTATGACCATTTACGAT
CTTGATGGAAATAAAGTAGAATATGAAGTAGAGGACGTTGAAATGGAACAAACTAGTATGTCTCTCGATAACTTT
GATCATTACATGATTAAGGAAATTAATGAGCAACCAATCAGTATTCTAAACACTATAAAAAATAAAGGGTTCTAT
GCAGAAATATTCGGTGATTTGGCTCATGAAATCTTCCAAAAAATAGACAACATCCTGATACTGGCTTGTGGTACA
AGTTATCACGCCGGTCTTGTAGGAAAACAGTGGATAGAGACCATCTCtAGAATCCCCGTGGATGTTCACATCGCG
AGTGAATACGAACCTACTATTCCGAGAGCGAACACATTGGTAATCACTATTTCACAGTCGGGTGAAACTGCGGAC
ACGATAGCGGCTTTGCAACGGGCCCAAAACGCCGGGATGATTTATACATTGTGTATTTGCAATTCACCAAAGAGC
ACTCTTGTTCGTGAGAGCATTATGAAGTACATCACGAAATGTGGTTCTGAGGTGTCAGTGGCATCAACGAAGGCG
TTTACTTCTCAGCTCGTAGTACTGTACATGCTGGCAAACGTATTGGCAAATAAAACCGATGATTTGCTGGGAGAC
CTCCCACAGGCAATAGAACGGGTAATTTGTTTGACAAATGACGAAATGAAACGATGGGCCGACGAAATTTGCACT
GCGAAATCCGCGATCTTCCTGGGAAGAGGACTAAACGCACCAGTTGCCTTTGAGGGAGCGTTGAAGCTCAAAGAA
ATCTCTTACATTCATGCAGAGGGCTTCCTGGGAGGTGAGTTGAAACACGGCCCCCTCGCACTCCTTGATGACAAG
ATTCCTGTTATCGTAACCGTAGCAGATCATGCTTATTTGGACCATATCAAAGCAAATATCGACGAAGTGCTTGCG
AGGAACGTTACGGTATACGCCATAGTAGACCAGTATGTGAACATCGAGCCCCAGGAACGCCTTCACGTCGTCAAG
GTTCCGTTTGTATCCAAAGAATTTTCTCCGATAATTCACACTATCCCGATGCAACTGCTTTCGTATTACGTGGCA
ATTAAGCTTGGAAAGAACGTTGACAAACCAAGGAATCTTGCAAAATCCGTGACTACCTTTTAA

SEQ ID NO: 50 is an amino acid sequence of glutamine-fructose-6-phosphate
amidotransferase (GFA1) originating from Saccharomyces cerevisiae
MCGIFGYCNYLVERSRGEIIDTLVDGLQRLEYRGYDSTGIAIDGDEADSTFIYKQIGKVSALKEEITKQNPNRDV
TFVSHCGIAHTRWATHGRPEQVNCHPQRSDPEDQFVVVHNGIITNFRELKTLLINKGYKFESDTDTECIAKLYLH
LYNTNLQNGHDLDFHELTKLVLLELEGSYGLLCKSCHYPNEVIATRKGSPLLIGVKSEKKLKVDFVDVEFPEENA
GQPEIPLKSNNKSFGLGPKKAREFEAGSQNANLLPIAANEFNLRHSQSRAFLSEDGSPTPVEFFVSSDAASVVKH
TKKVLFLEDDDLAHIYDGELHIHRSRREVGASMTRSIQTLEMELAQIMKGPYDHFMQKEIYEQPESTFNTMRGRI
DYENNKVILGGLKAWLPVVRRARRLIMIACGTSYHSCLATRAIFEELSDIPVSVELASDFLDRKCPVFRDDVCVF
VSQSGETADTMLALNYCLERGALTVGIVNSVGSSISRVTHCGVHINAGPEIGVASTKAYTSQYIALVMFALSLSD
DRVSKIDRRIEIIQGLKLIPGQIKQVLKLEPRIKKLCATELKDQKSLLLLGRGYQFAAALEGALKIKEISYMHSE

GVLAGELKHGVLALVDENLPIIAFGTRDSLFPKVVSSIEQVTARKGHPIIICNENDEVWAQKSKSIDLQTLEVPQ

TVDCLQGLINIIPLQLMSYWLAVNKGIDVDFPRNLAKSVTVE

SEQ ID NO: 51 is an amino acid sequence of glutamine-fructose-6-phosphate
amidotransferase (GFA1) originating from Chlorella virus 1 (PBCV-1)
MCGIFGAVSNNNSIEVSIKGIQKLEYRGYDSCGIAYTDGGAIERIRSIDGIDDLRKKTITESSPVAIAHSRWSTT
GIPSVVNAHPHISRGTSGCESRIAVVHNGIIENYQQIRKYLINLGYTFDSQTDTEVIAHLIDSQYNGNILHTVQM
AVKHLKGSYAIAVMCHKESGKIVVAKQKSPLVLGIGSDGAYYIASDVLALPTNKVVYISDGFSAELSPGSMTIYD
LDGNKVEYEVEDVEMEQTSMSLDNFDHYMIKEINEQPISILNTIKNKGFYAEIFGDLAHEIFQKIDNILILACGT
SYHAGLVGKQWIETISRIPVDVHIASEYEPTIPRANTLVITISQSGETADTIAALQRAQNAGMIYTLCICNSPKS
TLVRESIMKYITKCGSEVSVASTKAFTSQLVVLYMLANVLANKTDDLLGDLPQAIERVICLTNDEMKRWADEICT
AKSAIFLGRGLNAPVAFEGALKLKEISYIHAEGFLGGELKHGPLALLDDKIPVIVTVADHAYLDHIKANIDEVLA
RNVTVYAIVDQYVNIEPQERLHVVKVPFVSKEFSPIIHTIPMQLLSYYVAIKLGKNVDKPRNLAKSVTTF

SEQ ID NO: 52 is a nucleic acid sequence of UDP-N-acetylglucosamine pyrophosphorylase
(QRI1) originating from Saccharomyces cerevisiae
ATGACTGACACAAAACAGCTATTCATTGAAGCCGGACAAAGTCAACTTTTCCACAATTGGGAAAGCTTGTCTCGC
AAAGACCAAGAAGAATTGCTTTCAAACCTGGAGCAAATATCTTCCAAGAGGTCCCCTGCAAAACTACTGGAAGAC
TGTCAAAATGCTATTAAATTCTCACTAGCTAACTCTTCTAAGGATACTGGCGTCGAAATTTCACCATTGCCCCCT
ACTTCGTACGAGTCGCTTATTGGCAACAGTAAGAAAGAAAATGAATACTGGCGTTTAGGCCTTGAAGCTATTGGC
AAGGGTGAAGTCGCAGTGATTTTAATGGCTGGCGGACAAGGTACGCGGTTAGGATCCTCTCAACCAAAGGGCTGT
TACGACATTGGATTGCCTTCTAAGAAATCTCTTTTTCAAATTCAAGCTGAAAAGTTGATCAGGTTGCAAGATATG
GTAAAGGACAAAAAGGTAGAAATTCCTTGGTATATTATGACATCAGGCCCCACTAGAGCGGCTACTGAGGCATAC
TTTCAAGAACACAATTATTTTGGCTTGAATAAAGAACAAATTACGTTCTTCAACCAGGGAACCCTGCCTGCCTTT
GATTTAACCGGGAAGCATTTCCTAATGAAAGACCCAGTAAACCTATCTCAATCACCAGATGGAAATGGTGGACTC
TACCGTGCCATCAAGGAAAACAAGTTGAACGAAGACTTTGATAGGAGAGGAATCAAGCATGTTTACATGTACTGT
GTCGATAATGTCCTATCTAAAATCGCAGACCCTGTATTTATTGGTTTTGCCATCAAGCATGGCTTCGAACTGGCC
ACCAAAGCCGTTAGAAAGAGAGATGCGCATGAATCAGTTGGGTTAATTGCTACTAAAAACGAGAAACCATGTGTC
ATAGAATATTCTGAAATTTCCAATGAATTGGCTGAAGCAAAGGATAAAGATGGCTTATTAAAACTACGCGCAGGC
AACATTGTAAATCATTATTACCTAGTGGATTTACTAAAACGTGATTTGGATCAGTGGTGTGAGAATATGCCATAT
CACATTGCGAAGAAGAAAATTCCAGCTTATGATAGTGTTACCGGCAAGTACACTAAGCCTACCGAACCAAACGGT
ATAAAATTAGAGCAATTCATATTTGATGTCTTTGACACTGTACCACTGAACAAGTTTGGGTGCTTAGAAGTAGAT
AGATGCAAAGAATTTTCACCTTTAAAAAACGGTCCTGGTTCTAAGAACGATAATCCTGAGACCAGCAGACTAGCA
TATTTGAAACTAGGAACCTCGTGGTTGGAAGATGCAGGCGCTATTGTAAAAGATGGGGTACTAGTCGAAGTTTCC
AGCAAATTGAGTTATGCAGGTGAAAATCTATCCCAGTTCAAAGGTAAAGTCTTTGACAGAAGTGGTATAGTATTA
GAAAAATAA

SEQ ID NO: 53 is an amino acid sequence of UDP-N-acetylglucosamine pyrophosphorylase
(QRI1) originating from Saccharomyces cerevisiae
MTDTKQLFIEAGQSQLFHNWESLSRKDQEELLSNLEQISSKRSPAKLLEDCQNAIKFSLANSSKDTGVEISPLPP
TSYESLIGNSKKENEYWRLGLEAIGKGEVAVILMAGGQGTRLGSSQPKGCYDIGLPSKKSLFQIQAEKLIRLQDM
VKDKKVEIPWYIMTSGPTRAATEAYFQEHNYFGLNKEQITFFNQGTLPAFDLTGKHFLMKDPVNLSQSPDGNGGL
YRAIKENKLNEDFDRRGIKHVYMYCVDNVLSKIADPVFIGFAIKHGFELATKAVRKRDAHESVGLIATKNEKPCV
IEYSEISNELAEAKDKDGLLKLRAGNIVNHYYLVDLLKRDLDQWCENMPYHIAKKKIPAYDSVTGKYTKPTEPNG
IKLEQFIFDVFDTVPLNKFGCLEVDRCKEFSPLKNGPGSKNDNPETSRLAYLKLGTSWLEDAGAIVKDGVLVEVS
SKLSYAGENLSQFKGKVFDRSGIVLEK

SEQ ID NO: 54 is a nucleic acid sequence of Phosphoglucomutase-1 (PGM1) originating from Saccharomyces cerevisiae
ATGTCACTTCTAATAGATTCTGTACCAACAGTTGCTTATAAGGACCAAAAACCGGGTACTTCAGGTTTACGTAAG
AAGACCAAGGTTTTCATGGATGAGCCTCATTATACTGAGAACTTCATTCAAGCAACAATGCAATCTATCCCTAAT
GGCTCAGAGGGAACCACTTTAGTTGTTGGAGGAGATGGTCGTTTCTACAACGATGTTATCATGAACAAGATTGCC
GCAGTAGGTGCTGCAAACGGTGTCAGAAAGTTAGTCATTGGTCAAGGCGGTTTACTTTCAACACCAGCTGCTTCT
CATATAATTAGAACATACGAGGAAAAGTGTACCGGTGGTGGTATCATATTAACTGCCTCACACAACCCAGGCGGT
CCAGAGAATGATTTAGGTATCAAGTATAATTTACCTAATGGTGGGCCAGCTCCAGAGAGTGTCACTAACGCTATC
TGGGAAGCGTCTAAAAAATTAACTCACTATAAAATTATAAAGAACTTCCCCAAGTTGAATTTGAACAAGCTTGGT
AAAAACCAAAAATATGGCCCATTGTTAGTGGACATAATTGATCCTGCCAAAGCATACGTTCAATTTCTGAAGGAA
ATTTTTGATTTTGACTTAATTAAAAGCTTCTTAGCGAAACAGCGCAAAGACAAAGGGTGGAAGTTGTTGTTTGAC
TCCTTAAATGGTATTACAGGACCATATGGTAAGGCTATATTTGTTGATGAATTTGGTTTACCGGCAGAGGAAGTT
CTTCAAAATTGGCACCCTTTACCTGATTTCGGCGGTTTACATCCCGATCCGAATCTAACCTATGCACGAACTCTT
GTTGACAGGGTTGACCGCGAAAAAATTGCCTTTGGAGCAGCCTCCGATGGTGATGGTGATAGGAATATGATTTAC
GGTTATGGCCCTGCTTTCGTTTCGCCAGGTGATTCTGTTGCCATTATTGCCGAATATGCACCCGAAATTCCATAC
TTCGCCAAACAAGGTATTTATGGCTTGGCACGTTCATTTCCTACATCCTCAGCCATTGATCGTGTTGCAGCAAAA
AAGGGATTAAGATGTTACGAAGTTCCAACCGGCTGGAAATTCTTCTGTGCCTTATTTGATGCTAAAAAGCTATCA
ATCTGTGGTGAAGAATCCTTCGGTACAGGTTCCAATCATATCAGAGAAAAGGACGGTCTATGGGCCATTATTGCT
TGGTTAAATATCTTGGCTATCTACCATAGGCGTAACCCTGAAAAGGAAGCTTCGATCAAAACTATTCAGGACGAA
TTTTGGAACGAGTATGGCCGTACTTTCTTCACAAGATACGATTACGAACATATCGAATGCGAGCAGGCCGAAAAA
GTTGTAGCTCTTTTGAGTGAATTTGTATCAAGGCCAAACGTTTGTGGCTCCCACTTCCCAGCTGATGAGTCTTTA
ACCGTTATCGATTGTGGTGATTTTTCGTATAGAGATCTAGATGGCTCCATCTCTGAAAATCAAGGCCTTTTCGTA
AAGTTTTCGAATGGGACTAAATTTGTTTTGAGGTTATCCGGCACAGGCAGTTCTGGTGCAACAATAAGATTATAC
GTAGAAAAGTATACTGATAAAAAGGAGAACTATGGCCAAACAGCTGACGTCTTCTTGAAACCCGTCATCAACTCC
ATTGTAAAATTCTTAAGATTTAAAGAAATTTAGGAACAGACGAACCAACAGTCCGCACATAG

SEQ ID NO: 55 is an amino acid sequence Phosphoglucomutase-1 (PGM1) originating from
Saccharomyces cerevisiae
MSLLIDSVPTVAYKDQKPGTSGLRKKTKVFMDEPHYTENFIQATMQSIPNGSEGTTLVVGGDGRFYNDVIMNKIA
AVGAANGVRKLVIGQGGLLSTPAASHIIRTYEEKCTGGGIILTASHNPGGPENDLGIKYNLPNGGPAPESVTNAI
WEASKKLTHYKIIKNFPKLNLNKLGKNQKYGPLLVDIIDPAKAYVQFLKEIFDFDLIKSFLAKQRKDKGWKLLFD
SLNGITGPYGKAIFVDEFGLPAEEVLQNWHPLPDFGGLHPDPNLTYARTLVDRVDREKIAFGAASDGDGDRNMIY
GYGPAFVSPGDSVAIIAEYAPEIPYFAKQGIYGLARSFPTSSAIDRVAAKKGLRCYEVPTGWKFFCALFDAKKLS
ICGEESFGTGSNHIREKDGLWAIIAWLNILAIYHRRNPEKEASIKTIQDEFWNEYGRTFFTRYDYEHIECEQAEK
VVALLSEFVSRPNVCGSHFPADESLTVIDCGDFSYRDLDGSISENQGLFVKFSNGTKFVLRLSGTGSSGATIRLY
VEKYTDKKENYGQTADVFLKPVINSIVKFLRFKEILGTDEPTVRT

SEQ ID NO: 56 is a nucleic acid sequence of UTP--glucose-1-phosphate uridylyltransferase
(UGP1) originating from Saccharomyces cerevisiae
ATGTCCACTAAGAAGCACACCAAAACACATTCCACTTATGCATTCGAGAGCAACACAAACAGCGTTGCTGCCTCA
CAAATGAGAAACGCCTTAAACAAGTTGGCGGACTCTAGTAAACTTGACGATGCTGCTCGCGCTAAGTTTGAGAAC
GAACTGGATTCGTTTTTCACGCTTTTCAGGAGATATTTGGTAGAGAAGTCTTCTAGAACCACCTTGGAATGGGAC
AAGATCAAGTCTCCCAACCCGGATGAAGTGGTTAAGTATGAAATTATTTCTCAGCAGCCCGAGAATGTCTCAAAC
CTTTCCAAATTGGCTGTTTTGAAGTTGAACGGTGGGCTGGGTACCTCCATGGGCTGCGTTGGCCCTAAATCTGTT
ATTGAAGTGAGAGAGGGAAACACCTTTTTGGATTTGTCTGTTCGTCAAATTGAATACTTGAACAGACAGTACGAT
AGCGACGTGCCATTGTTATTGATGAATTCTTTCAACACTGACAAGGATACGGAACACTTGATTAAGAAGTATTCC
GCTAACAGAATCAGAATCAGATCTTTCAATCAATCCAGGTTCCCAAGAGTCTACAAGGATTCTTTATTGCCTGTC
CCCACCGAATACGATTCTCCACTGGATGCTTGGTATCCACCAGGTCACGGTGATTTGTTTGAATCTTTACACGTA
TCTGGTGAACTGGATGCCTTAATTGCCCAAGGAAGAGAAATATTATTTGTTTCTAACGGTGACAACTTGGGTGCT
ACCGTCGACTTAAAAATTTTAAACCACATGATCGAGACTGGTGCCGAATATATAATGGAATTGACTGATAAGACC
AGAGCCGATGTTAAAGGTGGTACTTTGATTTCTTACGATGGTCAAGTCCGTTTATTGGAAGTCGCCCAAGTTCCA
AAAGAACACATTGACGAATTCAAAAATATCAGAAAGTTTACCAACTTCAACACGAATAACTTATGGATCAATCTG
AAAGCAGTAAAGAGGTTGATCGAATCGAGCAATTTGGAGATGGAAATCATTCCAAACCAAAAAACTATAACAAGA
GACGGTCATGAAATTAATGTCTTACAATTAGAAACCGCTTGTGGTGCTGCTATCAGGCATTTTGATGGTGCTCAC
GGTGTTGTCGTTCCAAGATCAAGATTCTTGCCTGTCAAGACCTGTTCCGATTTGTTGCTGGTTAAATCAGATCTA
TTCCGTCTGGAACACGGTTCTTTGAAGTTAGACCCATCCCGTTTTGGTCCAAACCCATTAATCAAGTTGGGCTCG
CATTTCAAAAAGGTTTCTGGTTTTAACGCAAGAATCCCTCACATCCCAAAAATCGTCGAGCTAGATCATTTGACC
ATCACTGGTAACGTCTTTTTAGGTAAAGATGTCACTTTGAGGGGTACTGTCATCATCGTTTGCTCCGACGGTCAT
AAAATCGATATTCCAAACGGCTCCATATTGGAAAATGTTGTCGTTACTGGTAATTTGCAAATCTTGGAACATTGA

SEQ ID NO: 57 is an amino acid sequence of UTP--glucose-1-phosphate uridylyltransferase
(UGP1) originating from Saccharomyces cerevisiae
MSTKKHTKTHSTYAFESNTNSVAASQMRNALNKLADSSKLDDAARAKFENELDSFFTLFRRYLVEKSSRTTLEWD
KIKSPNPDEVVKYEIISQQPENVSNLSKLAVLKLNGGLGTSMGCVGPKSVIEVREGNTFLDLSVRQIEYLNRQYD
SDVPLLLMNSFNTDKDTEHLIKKYSANRIRIRSFNQSRFPRVYKDSLLPVPTEYDSPLDAWYPPGHGDLFESLHV
SGELDALIAQGREILFVSNGDNLGATVDLKILNHMIETGAEYIMELTDKTRADVKGGTLISYDGQVRLLEVAQVP
KEHIDEFKNIRKFTNFNTNNLWINLKAVKRLIESSNLEMEIIPNQKTITRDGHEINVLQLETACGAAIRHFDGAH
GVVVPRSRFLPVKTCSDLLLVKSDLFRLEHGSLKLDPSRFGPNPLIKLGSHFKKVSGFNARIPHIPKIVELDHLT
ITGNVFLGKDVTLRGTVIIVCSDGHKIDIPNGSILENVVVTGNLQILEH

SEQ ID NO: 58 is a nucleic acid sequence of Glucosamine 6-phosphate N-acetyltransferase
(GNA1) originating from Saccharomyces cerevisiae
ATGAGCTTACCCGATGGATTTTATATAAGGCGAATGGAAGAGGGGGATTTGGAACAGGTCACTGAGACGCTAAAG
GTTTTGACCACCGTGGGCACTATTACCCCCGAATCCTTCAGCAAACTCATAAAATACTGGAATGAAGCCACAGTA
TGGAATGATAACGAAGATAAAAAAATAATGCAATATAACCCCATGGTGATTGTGGACAAGCGCACCGAGACGGTT
GCCGCTACGGGGAATATCATCATCGAAAGAAAGATCATTCATGAACTGGGGCTATGTGGCCACATCGAGGACATT
GCAGTAAACTCCAAGTATCAGGGCCAAGGTTTGGGCAAGCTCTTGATTGATCAATTGGTAACTATCGGCTTTGAC
TACGGTTGTTATAAGATTATTTTAGATTGCGATGAGAAAAATGTCAAATTCTATGAAAAATGTGGGTTTAGCAAC
GCAGGCGTGGAAATGCAAATTAGAAAATAG

SEQ ID NO: 59 is an amino acid sequence of Glucosamine 6-phosphate N-acetyltransferase
(GNA1) originating from Saccharomyces cerevisiae
MSLPDGFYIRRMEEGDLEQVTETLKVLTTVGTITPESFSKLIKYWNEATVWNDNEDKKIMQYNPMVIVDKRTETV
AATGNIIIERKIIHELGLCGHIEDIAVNSKYQGQGLGKLLIDQLVTIGFDYGCYKIILDCDEKNVKFYEKCGFSN
AGVEMQIRK

SEQ ID NO: 60 is a nucleic acid sequence of phosphoacetylglucosamine mutase (PCM1) originating from Saccharomyces cerevisiae
ATGAAGGTTGATTACGAGCAATTGTGCAAACTCTACGATGACACGTGCCGCACAAAGAATGTGCAGTTCAGTTAC
GGTACGGCCGGATTCAGAACGCTGGCCAAGAATTTGGATACGGTGATGTTCAGTACTGGTATACTGGCGGTTCTC
AGGTCGCTGAAGCTTCAGGGTCAGTATGTGGGGGTGATGATCACGGCGTCGCACAACCCATACCAGGACAACGGG
GTCAAGATCGTGGAACCAGACGGATCGATGCTTTTGGCCACATGGGAGCCATATGCCATGCAGTTGGCCAATGCG
GCCTCTTTTGCCACTAATTTTGAAGAATTTCGTGTTGAGTTGGCCAAGCTGATTGAACACGAAAAGATTGATTTG
AATACAACCGTCGTGCCTCACATCGTGGTTGGAGAGACTCTAGGGAAAGTAGTCCATACTTGCTGCGCTGCTTG
ACTTCCTCCATGGCCAGCGTCTTCCACGCGCAAGTTTTGGACCTAGGCTGTGTCACTACGCCTCAATTGCATTAC
ATTACTGATTTGTCCAACAGGCGGAAACTGGAAGGAGACACAGCGCCAGTTGCCACAGAACAGGACTACTATTCG
TTCTTTATAGGAGCCTTCAACGAGCTCTTCGCCACGTATCAGCTGGAGAAGAGGCTGTCTGTCCCAAAATTGTTC

ATAGACACAGCCAATGGTATCGGTGGTCCACAGTTGAAAAACTACTGGCCTCCGAAGATTGGGACGTGCCAGCG
GAGCAAGTTGAGGTAATCAACGACAGGTCCGATGTTCCAGAACTGTTGAATTTTGAATGCGGTGCGGATTATGTG
AAGACTAACCAGAGATTACCCAAGGGTCTTTCTCCATCCTCGTTTGATTCGCTATATTGCTCCTTTGATGGTGAC
GCAGACAGGGTTGTGTTCTACTATGTCGACTCAGGATCAAAATTTCATTTGTTGGATGGTGACAAAATTTCCACT
TTGTTTGCAAAGTTCTTGTCTAAACAACTAGAATTGGCACACCTAGAACATTCTTTGAAGATTGGTGTTGTGCAA
ACTGCCTATGCAAACGGCAGTTCCACCGCTTACATAAAAAATACGTTGCACTGTCCCGTGTCTTGCACTAAGACA
GGTGTTAAACACTTGCATCATGAAGCTGCCACTCAGTACGATATTGGCATTTATTTCGAAGCAAATGGACATGGT
ACGATTATATTCAGCGAAAAATTTCATCGAACTATCAAATCTGAATTATCCAAGTCCAAGTTAAATGGTGATACG
TTAGCTTTGAGAACTTTGAAGTGTTTCTCTGAATTGATTAATCAGACCGTGGGAGATGCTATTTCAGACATGCTT
GCTGTCCTTGCTACTTTGGCGATTTTGAAAATGTCGCCAATGGATTGGGATGAAGAGTATACTGATTTGCCCAAC
AAGCTGGTTAAGTGCATCGTTCCTGATAGGTCAATTTTCCAAACCACGGACCAGGAAAGAAAATTGCTCAATCCA
GTGGGGTTGCAAGACAAGATAGATCTTGTGGTAGCCAAGTATCCCATGGGAAGAAGCTTTGTCAGAGCCAGTGGT
ACGGAGGATGCGGTGAGGGTTTATGCGGAATGTAAGGACTCCTCTAAGTTAGGTCAATTTTGTGACGAAGTGGTG
GAGCACGTTAAGGCATCTGCTTGA

SEQ ID NO: 61 is an amino acid sequence of phosphoacetylglucosamine mutase (PCM1)
originating from Saccharomyces cerevisiae
MKVDYEQLCKLYDDTCRTKNVQFSYGTAGFRTLAKNLDTVMFSTGILAVLRSLKLQGQYVGVMITASHNPYQDNG
VKIVEPDGSMLLATWEPYAMQLANAASFATNFEEFRVELAKLIEHEKIDLNTTVVPHIVVGRDSRESSPYLLRCL
TSSMASVFHAQVLDLGCVTTPQLHYITDLSNRRKLEGDTAPVATEQDYYSFFIGAFNELFATYQLEKRLSVPKLF
IDTANGIGGPQLKKLLASEDWDVPAEQVEVINDRSDVPELLNFECGADYVKTNQRLPKGLSPSSFDSLYCSFDGD
ADRVVFYYVDSGSKFHLLDGDKISTLFAKFLSKQLELAHLEHSLKIGVVQTAYANGSSTAYIKNTLHCPVSCTKT
GVKHLHHEAATQYDIGIYFEANGHGTIIFSEKFHRTIKSELSKSKLNGDTLALRTLKCFSELINQTVGDAISDML
AVLATLAILKMSPMDWDEEYTDLPNKLVKCIVPDRSIFQTTDQERKLLNPVGLQDKIDLVVAKYPMGRSFVRASG
TEDAVRVYAECKDSSKLGQFCDEVVEHVKASA

SEQ ID NO: 62 is the nucleic acid sequence of promotor pTDH3
CTGCTGTAACCCGTACATGCCCAAAATAGGGGGCGGGTTACACAGAATATATAACATCGTAGGTGTCTGGGTGAA
CAGTTTATTCCTGGCATCCACTAAATATAATGGAGCCCGCTTTTTAAGCTGGCATCCAGAAAAAAAAAGAATCCC
AGCACCAAAATATTGTTTTCTTCACCAACCATCAGTTCATAGGTCCATTCTCTTAGCGCAACTACAGAGAACAGG
GGCACAAACAGGCAAAAAACGGGCACAACCTCAATGGAGTGATGCAACCTGCCTGGAGTAAATGATGACACAAGG
CAATTGACCCACGCATGTATCTATCTCATTTTCTTACACCTTCTATTACCTTCTGCTCTCTCTGATTTGGAAAAA
GCTGAAAAAAAAGGTTGAAACCAGTTCCCTGAAATTATTCCCCTACTTGACTAATAAGTATATAAAGACGGTAGG
TATTGATTGTAATTCTGTAAATCTATTTCTTAAACTTCTTAAATTCTACTTTTATAGTTAGTCTTTTTTTTAGTT
TTAAAACACCAAGAACTTAGTTTCGAATAAACACACATAAACAAACAAA

SEQ ID NO:63 is the nucleic acid sequence of promotor pTDH3.Sk
CAATTCATCGGCCCTTTTAGCGGCTACCCGCGCCATCTAAATGATAGGGCGGGTGACACTATGGTAAATCCCATA
ATTAGGTGTCTGGGTGAGTGGTTCTGATGCCGGCATCCACTAAATATATTGGAGCCCATTTTTTACGCGGGCTTC
CAGAAAAAAAGAGAATCCCAGCACCAAAAGGTGGTTCTCTTCACCAACCATCAGATCATAGGTCCACAACCACAC
ATAACAGGGGCACAAAAAGGCAAAAAACGGACATAACCTCAATGGAGTGATGCAAATTGACTGGAGCAAAAGCTG
ACACAAGGCATTGATTGACCTACGCATGTATCTGTATTCTTTTCTTACACCTTCTATTACCTTCTAACTCTTTGG
GTTGGAAAAAACTGAAAAAAAAGGTTGGGACCTGGTTCCCCCAAGTTGTCCCCCTACTTGGTTATTAAATATATA
AAGACAGCAAGTGTTGATTATAATCTTGTAAATCTATAGTTCTTAATCTATACTTCTATTTATATTTTAAATTAG
TCTTTTTATTTCCAAGTCCCCAAGAACTTAGTTTCGAATAAACACACACAAATAAACACA

SEQ ID NO: 64 is the nucleic acid sequence of promotor pTDH3-1.sba
GCAGCGCTTCTTCCGCTCTAGTTTTATAGTTATTATTACTACCACCTTAAAAATACGTAAATACTCAAAATAGT
AGTGATATTCCCAACCTTATTCATCCAAGGCACATCATCATCATCAGCCATTCATCTTTCACCTGCCATTAGTAA
CCCGTCTTCTCATTGAGCGGGTTACGGCAGCCACAGGCCACATTCCGAATGTCTGGGTGAGCGGTCCCTTTTCCA
GCATCCACTAAATATCTCCGATCCCGCTTTTTAATCTGGCTTCCTGAAAAAAAGAATCCCAGCACCAAAAAAT
GGCTCTCTTCACCAACCATCAGATCATAGGTCCCATTCTCTTACCGCAACCGTACAGAACAGGGGAAAACGGGTA
CAACCTCAATGGAGTGATGCAAACTGACTGGAGCAAAAAGCTGACACAAGGCAATCGACCTACGTGTCTGTCTAT
TTTCTCACACCTTCTATTACCTTCTAACTCTCTGGGTTGGAAAAAACTGAAAAAAAGGTTGAGACCAGTTTCCAC
AAATCATCCCCCTGTTTGATTAATAAATATATAAAGACGACAACTATCGATCATAAACTCATAAAACTATAACTC
CTTTACACTTCTTATTTTATAGTTATTCTATTTTAATTCTTATTGATTTTAAACCCCAAGAACTTAGTTTCGAA
AACACACACACACACAAACAATTAAAA

SEQ ID NO: 65 is the nucleic acid sequence of promotor pTDH3.Sar
GAGCTAAATATCAGCCCTTCGGGTCCTGCCTGCTACCCGGTCCTGTTCGAATAAAAACGCGGGTAACACGACCCA
GTAACACCTGTCGTTGGGTGTCTGGGTCAGAAGTTCTGATACCGGCTTCCACTAAATAGATTGGGTTCCGCTCTT
TACGCTGGCTTCCTGAAAAAAGAGATTCCGGGCACCAAAAAATTGGTCTCTTTGCCAACCATCAGATCATAGGTC
CATTCTCTTACCATAACCACACAGGATAGGGGCACCACAGGCGAAAATGGGCACAAAATCTCAATGGAGTGATGC
AAATTAGCTGGAACAAAAGCTGACACAAGGCAATTAACCTGCGCATGTATCCATCTCCTTTTCTTACACCTTCTC
TTACGTTCTAACTGTTTGGGTTGGAAAAATTAAAAAAAAAAGGTTGAGACCAGTTTCCCCAAATCGTCCCCCTAC
TTGATTCATAAATATATAAAGACGACAACTATTGATTATAATCTTGTAAATCTATAACTCTTTACTTTCTCCTAT
TTATAATTTAACTTAATCTTTTTAGATTTAAAACCCCAAGAACTTAGTTTCGAACAAACACACACAAATAAACAA
AA

SEQ ID NO: 66 is the nucleic acid sequence of promotor pENO2
CGCTCAGCATCTGCTTCTTCCCAAAGATGAACGCGGCGTTATGTCACTAACGACGTGCACCAACTTGCGGAAAGT
GGAATCCCGTTCCAAAACTGGCATCCACTAATTGATACATCTACACACCGCACGCCTTTTTTCTGAAGCCCACTT
TCGTGGACTTTGCCATATGCAAAATTCATGAAGTGTGATACCAAGTCAGCATACACCTCACTAGGGTAGTTTCTT
TGGTTGTATTGATCATTTGGTTCATCGTGGTTCATTAATTTTTTTTCTCCATTGCTTTCTGGCTTTGATCTTACT

ATCATTTGGATTTTTGTCGAAGGTTGTAGAATTGTATGTGACAAGTGGCACCAAGCATATATAAAAAAAAAAAGC
ATTATCTTCCTACCAGAGTTGATTGTTAAAAACGTATTTATAGCAAACGCAATTGTAATTAATTCTTATTTTGTA
TCTTTTCTTCCCTTGTCTCAATCTTTTATTTTTATTTTATTTTTCTTTTCTTAGTTTCTTTCATAACACCAAGCA
ACTAATACTATAACATACAATAATA

SEQ ID NO: 67 is the nucleic acid sequence of promotor pTEF3
GGCTGATAATAGCGTATAAACAATGCATACTTTGTACGTTCAAAATACAATGCAGTAGATATATTTATGCATATT
ACATATAATACATATCACATAGGAAGCAACAGGCGCGTTGGACTTTTAATTTTCGAGGACCGCGAATCCTTACAT
CACACCCAATCCCCCACAAGTGATCCCCCACACACCATAGCTTCAAAATGTTTCTACTCCTTTTTTACTCTTCCA
GATTTTCTCGGACTCCGCGCATCGCCGTACCACTTCAAAACACCCAAGCACAGCATACTAAATTTCCCCTCTTTC
TTCCTCTAGGGTGTCGTTAATTACCCGTACTAAAGGTTTGGAAAAGAAAAAAGAGACCGCCTCGTTTCTTTTTCT
TCGTCGAAAAAGGCAATAAAAATTTTTATCACGTTTCTTTTTCTTGAAAATTTTTTTTTTTGATTTTTTTCTCTT
TCGATGACCTCCCATTGATATTTAAGTTAATAAACGGTCTTCAATTTCTCAAGTTTCAGTTTCATTTTTCTTGTT
CTATTACAACTTTTTTTACTTCTTGCTCATTAGAAAGAAAGCATAGCAATCTAATCTAAGTTTTAATTACAAA

SEQ ID NO: 68 is the nucleic acid sequence of promotor pTEF1
GTTTAGCTTGCCTCGTCCCCGCCGGGTCACCCGGCCAGCGACATGGAGGCCCAGAATACCCTCCTTGACAGTCTT
GACGTGCGCAGCTCAGGGGCATGATGTGACTGTCGCCCGTACATTTAGCCCATACATCCCCATGTATAATCATTT
GCATCCATACATTTTGATGGCCGCACGGCGCGAAGCAAAAATTACGGCTCCTCGCTGCAGACCTGCGAGCAGGGA
AACGCTCCCCTCACAGACGCGTTGAATTGTCCCCACGCCGCGCCCCTGTAGAGAAATATAAAAGGTTAGGATTTG
CCACTGAGGTTCTTCTTTCATATACTTCCTTTTAAAATCTTGCTACGATACAGTTCTCACATCACATCCGAACAT
AAACAACC

SEQ ID NO: 69 is the nucleic acid sequence of promotor pTEF1.ago
GTTTAGCTTGCCTCGTCCCCGCCGGGTCACCCGGCCAGCGACATGGAGGCCCAGAATACCCTCCTTGACAGTCTT
GACGTGCGCAGCTCAGGGGCATGATGTGACTGTCGCCCGTACATTTAGCCCATACATCCCCATGTATAATCATTT
GCATCCATACATTTTGATGGCCGCACGGCGCGAAGCAAAAATTACGGCTCCTCGCTGCAGACCTGCGAGCAGGGA
AACGCTCCCCTCACAGACGCGTTGAATTGTCCCCACGCCGCGCCCCTGTAGAGAAATATAAAAGGTTAGGATTTG
CCACTGAGGTTCTTCTTTCATATACTTCCTTTTAAAATCTTGCTACGATACAGTTCTCACATCACATCCGAACAT
AAACAACC

SEQ ID NO: 70 is the nucleic acid sequence of promotor pTEF1.Sba
CGCCAACAAACCTTCGAACACTTTAATTTTCGAGGACCGCAGATCCTCACATCACACCCACACCCAAGACTGCTT
CCCCCACACACCCTGCATCTGTACACTTTCTTCTGCTCTGTTTTTCTCTCCGGCGTTCTCTCGGGTCGCCCGCAT
CGCCGCGCCGGCTGGAACCGCCCACGCACCGCATATTGCAAATCGCCTGCCCCCTCTTGCTCCTTTTTGAGGGCG
CGCCGTTACCCGCGCCCAGGGTCCGGAAAAAGAAACAAGGCTCTACCGCGTTTCTTTTTCCTTGTCGAAAAAGGC
AAAAATGAAAATTTTTATCACGTTTCTTTTTTTTTGAAAAATTTTTTTTTTGGTTTTTTTCTTTCGATGGCCTC

CCATTGATATTTAAGTTAATAAATGGTTTTCAGTTTTCAAGTTTCAGTTTGTGTTCTTCTTTGCTAACTTTCACT
TACACCTCGAAAGAAAGTATAGCAATCTAATCTTAGTTTTAATTACAA

SEQ ID NO: 71 is the nucleic acid sequence of promotor pPDC1
TTATTTACCTATCTCTAAACTTCAACACCTTATATCATAACTAATATTTCTTGAGATAAGCACACTGCACCCATA
CCTTCCTTAAAAACGTAGCTTCCAGTTTTTGGTGGTTCCGGCTTCCTTCCCGATTCCGCCCGCTAAACGCATATT
TTTGTTGCCTGGTGGCATTTGCAAAATGCATAACCTATGCATTTAAAAGATTATGTATGCTCTTCTGACTTTTCG
TGTGATGAGGCTCGTGGAAAAAATGAATAATTTATGAATTTGAGAACAATTTTGTGTTGTTACGGTATTTTACTA
TGGAATAATCAATCAATTGAGGATTTTATGCAAATATCGTTTGAATATTTTTCCGACCCTTTGAGTACTTTTCTT
CATAATTGCATAATATTGTCCGCTGCCCCTTTTTCTGTTAGACGGTGTCTTGATCTACTTGCTATCGTTCAACAC
CACCTTATTTTCTAACTATTTTTTTTTTAGCTCATTTGAATCAGCTTATGGTGATGGCACATTTTTGCATAAACC
TAGCTGTCCTCGTTGAACATAGGAAAAAAAAATATATAAACAAGGCTCTTTCACTCTCCTTGCAATCAGATTTGG
GTTTGTTCCCTTTATTTTCATATTTCTTGTCATATTCCTTTCTCAATTATTATTTTCTACTCATAACCTCACGCA
AAATAACACAGTCAAATCAATCAAA

SEQ ID NO: 72 is the nucleic acid sequence of promotor pCCW12
aaccagggcaaagcaaaataaaagAAACTTAATACGTTATGCCGTAATGAAGGGCTACCAAAAACGATAATCTCA
ACTGTAAACAGGTACAATGCGGACCCTTTTGCCACAAAACATACATCATTCATTGCCGGAAAAAGAAAGAAGTGA
AGACAGCAGTGCAGCCAGCCATGTTGCGCCAATCTAATTATAGATGCTGGTGCCCTGAGGATGTATCTGGAGCCA
GCCATGGCATCATGCGCTACCGCCGGATGTAAAATCCGACACGCAAAAGAAAACCTTCGAGGTTGCGCACTTCGC
CCACCCATGAACCACACGGTTAGTCCAAAAGGGGCAGTTCAGATTCCAGATGCGGGAATTAGCTTGCTGCCACCC
TCACCTCACTAACGCTGCGGTGTGCGGATACTTCATGCTATTTATAGACGCGCGTGTCGGAATCAGCACGCGCAA
GAACCAAATGGGAAAATCGGAATGGGTCCAGAACTGCTTTGAGTGCTGGCTATTGGCGTCTGATTTCCGTTTTGG
GAATCCTTTGCCGCGCGCCCCTCTCAAAACTCCGCACAAGTCCCAGAAAGCGGGAAAGAAATAAAACGCCACCAA
AAAAAAAAATAAAAGCCAATCCTCGAAGCGTGGGTGGTAGGCCCTGGATTATCCCGTACAAGTATTTCTCAGGAG
TAAAAAAACCGTTTGTTTTGGAATTCCCCATTTCGCGGCCACCTACGCCGCTATCTTTGCAACAACTATCTGCGA
TAACTCAGCAAATTTTGCATATTCGTGTTGCAGTATTGCGATAATGGGAGTCTTACTTCCAACATAACGGCAGAA
AGAAATGTGAGAAAATTTTGCATCCTTTGCCTCCGTTCAAGTATATAAAGTCGGCATGCTTGATAATCTTTCTTT
CCATCCTACATTGTTCTAATTATTCTTATTCTCCTTTATTCTTTCCTAACATACCAAGAAATTAATCTTctgtca
ttcgcttaaacactatatcaata

SEQ ID NO: 73 is the nucleic acid sequence of promotor pCCW12.Sm
CGTTGTGCTGTAGTGAAGGAAGACTAAAAAGGATAATCACAGTTGTAAAGAGGTATAATGCGGACCCTTTTGCCA
CAAAACACACATCTTTCGTTTCCAAAATAAGAAAGAAGAAAGCAAAAAGATTAGCAGCCACGTTGCTGCGATCTA
ATTATAGACGGTGGCGTCATCATTCTCACCCAAGATTGTGTCTTGAACCTGCCACGGGTCCTGCGTTATCGCCGG
ATGTAAAACTAGACATGCAAAAAAAGGACCTTCCAGGTAGCGTGCTCCACACCACCCATGACCACCACAGTTAGT
CCAAAAGAGGCAGCACCACTTCCCGATGGGGGAATTAGATTGCTGCCACCCTCACCTCACTAATGCTGCGGTGTG
CGGATATGCCCTGCTATATATAGCTCCGCGTTTTTGAACCAGCACAGCGCGAGCACCAAAAAGGAAAATCGCATA

GGCCCAGAACTGATTTCAGCACGGGCTATTGGCGTTGGGTTTCCGTTCTGGAAACCTTCGCCGCGTCCCCCTCA
CAAACCTCCGCACAAGTTCGAGCAAGCGGGAAAAACGAAAAACGCCATTAATACTAAATAAAGCAAATCCTCGA
AGCGTGGGTGGCAAGCCCCTGGATTTTTCCGCACAAGTACTCTTCTCAGGAGTAAAAAAACCCGTTTGTTTTGGA
ATTCCCCATTTCGCGGCCATCTACGCCGGTATCTTCGCAATATCTATCAGCGATAACTCAGCAATTTTAATATTC
GTGTTGCAGTGCTGCGATAGCGGGAGTCTTGTTTGTAACATAACGGCAGAAAGGAATGAGAGAAAATTTTCCATT
CTTTGGCCTCCGTTCAAGTATATAAAGCCGGCATGCTTGGTAATCTTTCTCTCTCTTCTGTATTGTTTCTATAAT
ACTTTTATCTTCTAATTATTTCTGAAAAACCAAGAAATTAATCTTCTGTCATTCGCTTAAACACTATATCAAT
A

SEQ ID NO: 74 is the nucleic acid sequence of promotor pCCW12.Sk
TGTAAAATCCTACACGCAAAAAACCTCTTGGGTTGCGCGTTTTCCACCACCCACGACCCGCACAATCAATCCAAA
AGGGGCAACGCCAGTTCCCAATGCGGGAATTAGCTCCTCACCCCCCTCACCCGCTAACGCTGCGGTGTGCGGACA
CGCAGCAGTATTTATAGATCCTCGTGTTGGAACCAGCCCGCGTGAGCACCAAATTGGAAAGTCGCAATGGGCCCA
GAACCGCTTTCAGTACTGGGCCATTGGCGTCTAGTTTCCGTTTTGAGCGTCCTTCGCCGCGTCTCTCTGTGAAAT
CCCCGCACAAGTCTGAGCAGGCAAAAAAAAAAAACGCCACCAAAAATAAATAAAGCCAATCCTCGAAGGATGAGT
AGGAAAGGAAGCCCCTGGTTTTTCCCGCACGAATATTTTTCAGGAGTGAAAAAATCCGTTTGTTTTGGAATTCC
CCATTTCGCGCTCACCTACGCCGGTATCTTTGGAACAACTATCAGCGATAACTCAGCAAAATTTGCATATCCGTG
CTGCAATAGTGCGATAGTGGGATTGGGAGTCTTGTTGCATCATAACGGCAGAAAGGAATGAATAAAAATTTTCCG
TTCTTTGTCTCCGTTCAAGTATATAAAGTCGGCATGCTTGATTATCTTTTCTTCTCTTCTACTACATTTCTATTT
CACTTTCTACTCTATTCTTCCCTGAAAAACCCAAGAAAATAATCTTCTGTCATTCGCTTAAACAATATATCAAAA

SEQ ID NO: 75 is the nucleic acid sequence of promotor pCCW12.Sba
ACCATGCCACGGTGCTGGCCCCACTTCCACCCACGACCCGCACGGTTGGCCCGAAAGGGGCAACACCAGTTCCCA
ATACGGAAATTAGTCCGCCACCACCCTCACCTCGCTAGAGCTGCGGTGTGCGGGCGTGCATCGCTATTTATAGAC
ATGCCTGCTGGCGTCACCGCGCGCGAGCACCAAACAGGAAAATCGCACTGGGCCCAGAACCACGCTATGCGCTGG
GCCGATGGCGTCCGGTTTCCCTTTGGGAGCCCCCTGCCACGTTCGCCTAACAAATCCCCGCACCGGCTTGAGAAA
AAAGCGAAAAGCGAAAAAAAAAAATCAACGCCACCAAAATTAAAAAAAAAGAGCCATCCTCGAAGGGTGAATAGT
AGCCCCTGACTTTTCCCGCACAGACAGACACCTTTCAGGAGTGAACAAAAAAGCAGTTTGTTTTGGAATTCCCCC
ATTTCGCGGTGGCCTGCGCAGGTATCTCTGCGTCAACTATCAGCGATAACTCAGCAAATTTTGCATATTCGTGTT
GCGATACTACGATAATGGGAGTCTGTCGCCTAATAACGGCAACAAGGAATGAGAGAGAAAAATTTTCTTCATTCT
CCAGCTCCCGTTCAAGTATATAAGGTCGGCATGGTCGATTGTCTTTCCTTCTCTTCAGTTACGTCTCTCTATTTA
CATTATTCTTATTTTTATTTAATAAAAACCCCAAGAAATTAATCTTCTGTCATTCGCTTAAACACTATATCAAAA

SEQ ID NO: 76 is the nucleic acid sequence of promotor pCCW12.Sar
AAAAAACAACCTTCTGCCAACCTGCGTGCTTCTCACCACCCATGACCCACACAATTGACCCGAATGGGGCAACTC
CAGTTCCCAATACGGGAATTAACTCGCCACCATATTTACCGCGTTGAAGCTGTGGTGTGCGGACACTCCGTACTA
TTTATAGACCCACGCGGTGGAACCAGCACGCGCGCACTAAACAGGAAAATCGCATTGAGTCCAGAACCGCCAC
CAGCACTTGGCCATTGGCGTCTAATTTCCGTTTTCGGCGCCCCTCACCGCGTCCTTCTAACAAAGCGCGCACAAG

CTTGAGCAAGTGAAAAGAAAATTAAAAATAAAAAACCGCCACCAAAACAAATAAAGCAATCTTCGAAGTGTGGGT
TGGTGGGAAGCCCCTGGCTTTTCCCGCACCAGTCGTTTTCAGGAGTAAAAAAATACCCGTTTGTTTTGGAATTCC
CCATTTCGCGGCGACCTGCGCCGGTATCTTTGCAACAACTATTTGCGATAACTCAGCAAAATTTGCATATTCGTG
TTGGGATATTGCGATAGTGGGAGTCTTGTTGCATAATAACGGTAAAAAGAAGTGAAGGAAAAAAATTTGCATCCT
TTAGTCTCAGTTCAAGTATATAAAGTCGGGATATTCAATTATCTTTCTTTCTCTTGCTCAAAGGTTTCTATATTT
TTTTTATAGTATTTCTTTTGTTATAAAATACCAAGAAATTAATCTTCTGTCATTCGCTTAAATACTACATCAATA

SEQ ID NO: 77 is the nucleic acid sequence of promotor pNUP57
TCATCTGCGCAATGACTATCAAGACCTTCTGCAAGAATTTCAAATCTCACTGAAAATCTTGACCGAAAAGTGTCT
TGAAAACCCATCAAGCCTGCAAAACCTATCTTTGACATTAGTCTCCATTATAAAAACGGCATAGTTGGGAGAAAA
CTTTCCATACTTCAATTGTGGACTGATATAAGTATTTTAGTTTTGCCCGCATGATCATCCCACATGGCTACAGCA
GTTCTCTCATAGGAAAATAGTACAATAGCTACGTGATATAATCTAAATAATTGTTGCCAATGTGTAATTATATCA
TTTTGAACGTACGCGAAATGGATTATTTTCAAAAATTTTGTTTCTTGAAATGAGTAAAAGCAAAAGTCCAACTCT
CCAAGTCGATGTAAACAACTTTTTGCCAAAGGGACTGAAAGACTAAATCGAGGATTATCCCGTTCAAACTATTCC
AGAAACGCTCGTTAGTAACAAAAGACATACCTTGTTGACCAATTGATCAC

SEQ ID NO: 78 is the nucleic acid sequence of promotor pCCW10.ago
GGTACCACGGCAACCTCGTTCGCTGTTCATCCCCTTCGTCACACAGGACGTTGGATGCCGTAAGCAGCGTTGCTT
TTGATCCTCagGATCGGCCGGGTAACCCGCGGCtGCTTCTATTTTAGTATTCATATCTCAAGCACATCCATTCCG
GCCGTTTGGGGGCGCCGCCGCACTCGTGTCCATTCCTACCGTGGCACTTAGGGCTATCCTGTCGGAGCGCCCCGC
CGACCGCCTTATCGGCACCAAAAGTAGAAGCCCCGGCCCCGCGTGGCTCAGACTCACCATCGGTGCTATTTACTT
TTCGATCAGATCGCGGCGCGCGGTGGCCGGCATTTCCGGAAGCGGCCACGGAGCAGAGGTGGCGCATTCGAATCG
CATACGTCTTCGCCACGCCGGAAAAAAATTTTCGGCTATATAAGGAGAGGCGGCCGTCTTGCTGCAGGCAGTTT
CACTTTCTCTAAAACCAAAGAACATCGATTTCTTTAGTCACTCGCTTCCTTACACCGAACTCGAGGCGGCCGC

SEQ ID NO: 79 is the nucleic acid sequence of promotor pCWP2
CTAGCCTCCCCTTTTTATTTTGTGCGGTCACCGCAAGGGACAAAGCTTTTCTTAGAAAACCGTCTGAGAAGCATA
ACGTACGCCATCCCCTAGACATATTAATAATGCTACAGATACTATGCTGCTCGTCTTTTTTTGACGACCCTTTTA
TTGCAATGTGCAACTAATGGCAAACAACCACATAGTATCACAGTATTACATTGCCTCCACCGATGCGGATGTTAG
GGCGCCAAGTCTGTCATGAAGCATGTTCCTGTCATAATCTTGTATGCAAAATACCGCGTTCTGCGCCACTGATAT
GCTAGGCAGCAGCAACCTATGCAGAAGATTGCTTTTCCCACGCCTGTTTTACGTCTCCAGGGCACTTGAAACAAT
GCAGCGATCGCCGCCACAACACGCCAAAGAGAAGCGAAAGTGGGCCTGGGCGGCCTCAGTTTCGGCAGAGGTAAA
CAACACGAACTGAACTGCCTTAGCTCCGAAGGGCAATTCCACAGGCACTCCGCGGGGCCCGGCCAAGGCCCAAAA
GGCGTGGAATATGCGCGTTTTGGGGCCATAACACCCAGTACCACGGCCGGAACGGGCCATATAATAAGTTTTTCA
CTCTCAAGAATGGTAAACGTAAATAGGAACATCCCACTACCCTAGAAATTGCGGAAATTTCGCGCTTATCATTAG
AAAATCTGGAACCGTCCTTTTTCCTCTTTCTTGCATTTCCCTTTCCGTATTATTGCCATTCTTTAACTGCATTTG
GGGAACCGTAGACCAAAAGCCAAACAGAGAAATGTAACGTTCTAAAAAAAAACAACGAAAAAATTGAAAAATAA

GATACAATAATCGTATATAAATCAGGCTTCTTGTTCATCATTTTCAATTCTCTTCTTGCCATCCCTTTTCCTATC
TTTGTTCTTTTCTTCTCATAATCAAGAATAAATAACTTCATCA

SEQ ID NO: 80 is the nucleic acid sequence of promotor pRPLA1
TCAAGTTGGATACTGATCTGATCTCTCCGCCCTACTACCAGGGACCCTCATGATTACCGCTCGAATGCGACGTTT
CCTGCCTCATAAAACTGGCTTGAAAATATTTATTCGCTGAACAGTAGCCTAGCTTATAAAAATTTCATTTAATTA
ATGTAATATGAAAACTCACATGCCTTCTGTTTCTAAAATTGTCACAGCAAGAAATAACATTACCATACGTGATCT
TATTAAACTCTAGTATCTTGTCTAATACTTCATTTAAAAGAAGCCTTAACCCTGTAGCCTCATCTATGTCTGCTA
CATATCGTGAGGTACGAATATCGTAAGATGATACCACGCAACTTTGTAATGATTTTTTTTTTTCATTTTTTAAA
GAATGCCTTTACATGGTATTTGAAAAAAATATCTTTATAAAGTTTGCGATCTCTTCTGTTCTGAATAATTTTTAG
TAAAAGAAATCAAAAGAATAAAGAAATAGTCCGCTTTGTCCAATACAACAGCTTAAACCGATTATCTCTAAAATA
ACAAGAAGAA

SEQ ID NO: 81 is the nucleic acid sequence of promotor pCUP1
CGGCAAACTTCAACGATTTCTATGATGCATTTTATAATTAGTAAGCCGATCCCATTACCGACATTTGGGCGCTAT
ACGTGCATATGTTCATGTATGTATCTGTATTTAAAACACTTTTGTATTATTTTTCCTCATATATGTGTATAGGTT
TATACGGATGATTTAATTATTACTTCACCACCCTTTATTTCAGGCTGATATCTTAGCCTTGTTACTAGTTAGAAA
AAGACATTTTTGCTGTCAGTCACTGTCAAGAGATTCTTTTGCTGGCATTTCTTCTAGAAGCAAAAAGAGCGATGC
GTCTTTTCCGCTGAACCGTTCCAGCAAAAAAGACTACCAACGCAATATGGATTGTCAGAATCATATAAAAGAGAA
GCAAATAACTCCTTGTCTTGTATCAATTGCATTATAATATCTTCTTGTTAGTGCAATATCATATAGAAGTCATCG
AAATAGATATTAAGAAAAACAAACTGTACAATCAATCAATCAATCATCACATAAA

SEQ ID NO: 82 is the nucleic acid sequence of promotor pMET6
CCACAGGAAATATTTCACGTGACTTACAAACAGAGTCGTACGTCAGGACCGGAGTCAGGTGAAAAAATGTGGGCC
GGTAAAGGGAAAAAACCAGAAACGGGACTACTATCGAACTCGTTTAGTCGCGAACGTGCAAAAGGCCAATATTTT
TCGCTAGAGTCATCGCAGTCATGGCAGCTCTTTCGCTCTATCTCCCGGTCGCAAAACTGTGGTAGTCATAGCTCG
TTCTGCTCAATTGAGAACTGTGAATGTGAATATGGAACAAATGCGATAGATGCACTAATTTAAGGGAAGCTAGCT
AGTTTTCCCAACTGCGAAAGAAAAAAAGGAAAGAAAAAAAAATTCTATATAAGTGATAGATATTTCCATCTTTAC
TAGCATTAGTTTCTCTTTTACGTATTCAATATTTTTGTTAAACTCTTCCTTTATCATAAAAAAGCAAGCATCTAA
GAGCATTGACAACACTCTAAGAAACAAAATACCAATATAATTTCAAAGTACATATCAAAA

SEQ ID NO: 83 is the nucleic acid sequence of promotor pMET25
TTACATTATCAATCCTTGCGTTTCAGCTTCCACTAATTTAGATGACTATTTCTCATCATTTGCGTCATCTTCTAA
CACCGTATATGATAATATACTAGTAACGTAAATACTAGTTAGTAGATGATAGTTGATTTTTATTCCAACACTAAG
AAATAATTTCGCCATTTCTTGAATGTATTTAAAGATATTTAATGCTATAATAGACATTTAAATCCAATTCTTCCA
ACATACAATGGGAGTTTGGCCGAGTGGTTTAAGGCGTCAGATTTAGGTGGATTTAACCTCTAAAATCTCTGATAT
CTTCGGATGCAAGGGTTCGAATCCCTTAGCTCTCATTATTTTTTGCTTTTTCTCTTGAGGTCACATGATCGCAAA

ATGGCAAATGGCACGTGAAGCTGTCGATATTGGGGAACTGTGGTGGTTGGCAAATGACTAATTAAGTTAGTCAAG
GCGCCATCCTCATGAAAACTGTGTAACATAATAACCGAAGTGTCGAAAAGGTGGCACCTTGTCCAATTGAACACG
CTCGATGAAAAAAATAAGATATATATAAGGTTAAGTAAAGCGTCTGTTAGAAAGGAAGTTTTTCCTTTTTCTTGC
TCTCTTGTCTTTTCATCTACTATTTCCTTCGTGTAATACAGGGTCGTCAGATACATAGATACAATTCTATTACCC
CCATCCATACA

SEQ ID NO: 84 is the nucleic acid sequence of promotor pSAM1
GAAACGGACGTAAGACGGAAATAGAATTTGAAGATAAAGTTATATATCACTACACACGAATACTTTCTTTTTTTT
TTTTCACAGGAAAACTGTGGTGGCGCCCTTGCCTACTAGTGCATTTCTTTTTTCGGGTTCTTGTCTCGAcGAAAT
TTTAGCCTCATCGTAGTTTTTCACTCTGGTATCGATGAAAAAGGGAAGAGTAAAAAGTTTTCCGTTTAGTACTTA
ATGGGATTGGTTTGGGACGTATATATCGACTGGTGTTGTCTGTTATTCATCGTTGTTTTTCGGTTAGCTTCGAAA
AAAAAATAGAGTAAAAACCAGGAATTTACCCTAAAAACAAGAAAAAATAAGATAAACGAAAAT

SEQ ID NO: 85 is the nucleic acid sequence of terminator tTPI1
GATTAATATAATTATATAAAAATATTATCTTCTTTTCTTTATATCTAGTGTTATGTAAAATAAATTGATGACTAC
GGAAAGCTTTTTTATATTGTTTCTTTTTCATTCTGAGCCACTTAAATTTCGTGAATGTTCTTGTAAGGGACGGTA
GATTTACAAGTGATACAACAAAAAGCAAGGCGCTTTTTCTAATAAAAAGAAGAAAAGCATTTAACAATTGAACAC
CTCTATATCAACGAAGAATATTACTTTGTCTCTAAATCCTTGTAAAATGTGTACGATCTCTATATGGGTTACTCA

SEQ ID NO: 86 is the nucleic acid sequence of terminator tMET25
GTGTGCGTAATGAGTTGTAAAATTATGTATAAACCTACTTTCTCTCACAAGTACTATACTTTTATAAAACGAACT
TTATTGAAATGAATATCCTTTTTTTCCCTTGTTACATGTCGTGACTCGTACTTTGAACCTAAATTGTTCTAACAT
CAAAGAACAGTGTTAATTCGCAGTCGAGAAGAAAAATATGGTGAACAAGACTCATCTACTTCATGAGACTACTTT
ACGCCTCCTATAAAGCTGTCACACTGGATAAATTTATTGTAGGACCAAGTTACAAAAGAGGATGATGGAGGTTT

SEQ ID NO:87 is the nucleic acid sequence of terminator tDIT1

TAAAGTAAGAGCGCTACATTGGTCTACCTTTTTGTTCTTTTACTTAAACATTAGTTAGTTCGTTTTCTTTTTCTC

ATTTTTTTATGTTTCCCCCCCAAAGTTCTGATTTTATAATATTTTATTTCACACAATTCCATTTAACAGAGGGGG

AATAGATTCTTTAGCTTAGAAAATTAGTGATCAATATATATTTGCCTTTCTTTTCATCTTTTCAGTGATATTAAT

GGTTTCGAGACACTGCAATGGCCCTAGTTGTCTAAGAGGATAGATGTTACTGTCAAAGATGATATTTTGAATTTC

SEQ ID NO: 88 is the nucleic acid sequence of terminator tRPL3
GAAGTTTTGTTAGAAAATAAATCATTTTTTAATTGAGCATTCTTATTCCTATTTTATTTAAATAGTTTTATGTAT
TGTTAGCTACATACAACAGTTTAAATCAAATTTTCTTTTTCCCAAGTCCAAAATGGAGGTTTATTTTGATGACCC
GCATGCGATTATGTTTTGAAAGTATAAGACTACATACATGTACATATATTTAAACATGTAAACCCGTCCATTATA
TTGCTTACTTTCTTCTTTTTTGCCGTTTTGACTTGGACCTCTGGTTTGCTATTTCCTTACAATCTTTGCTACAAT

SEQ ID NO: 89 is the nucleic acid sequence of terminator tRPL3.sm
GAAGTTTTTAAAGCATTTTTTAGACACTTCTCATTTTTCTAAGTTTTTTTAAAATAGTTTTATGTATTTACTACG
TATCACAATTTGAAATAATTCATCTTCCCAAAAAACTAAGATTTTATCCTTGTCACGATCCGTAACCAGTTTAT
AATATTTTAGAGCTTATACACGTACGTATACACACGTGTCGGTACATGAGAATTACGTTCAAAATTATTCACTTT
TTTTTTCTCTGCCGTTTTACTTTTGAACTCTGTCTCGCTATTTCCTTACAATCTTCGCTACAATACCACTTGCCC
TTGG

SEQ ID NO: 90 is the nucleic acid sequence of terminator tRPL3.sba

GAAGTTTTCTGACAAAAACATAACGTTTTTTCCAATCATTTCTTATTTTTCCGGTTTATTTAAATAGTTTTTATG

TACTATTATACGTATGACTATTTAACTTAAATTCTTCCTCCCAAGAAATCTCCCAAGTTTTTCATTATCATGGCA

TACACCACTATCAGTTACAAAATGGTAGCTCAACCATATATATATCTCTATATACACATATAAATGCAAACAGGT

CCAAGTCACCGCTCACTGCAGTTTCTTTTGCCGTTTTGACTTCGATCTCTGCTTGGCTATTTTCTCACAATCCT

SEQ ID NO: 91 is the nucleic acid sequence of terminator tRPL41B
GCGGATTGAGAGCAAATCGTTAAGTTCAGGTCAAGTAAAAATTGATTTCGAAAACTAATTTCTCTTATACAATCC
TTTGATTGGACCGTCATCCTTTCGAATATAAGATTTTGTTAAGAATATTTTAGACAGAGATCTACTTTATATTTA
ATATCTAGATATTACATAATTTCCTCTCTAATAAAATATCATTAATAAAATAAAAATGAAGCGATTTGATTTTGT
GTTGTCAACTTAGTTTGCCGCTATGCCTCTTGGGTAATGCTATTATTGAATCGAAGGGCTTTATTATATTACCCT

SEQ ID NO: 92 is the nucleic acid sequence of terminator tRPL15A
GCTGGTTGATGGAAAATATAATTTTATTGGGCAAACTTTTGTTTATCTGATGTGTTTTATACTATTATCTTTTTA
ATTAATGATTCTATATACAAACCTGTATATTTTTCTTTAACCAATTTTTTTTTTATAGACCTAGAGCTGTACT
TTTATTCTGCTATCAAGCAAACCCCTACCCCCTCTTCTCAATCCTCCCCTCAGGCAGAACTTATCTACCTGTATC
AAGGAGCGGACGAGGGAGTCCTAATTGTTCTACGTATACCAATGCTAGCAGCTTACATAGGTGGTGGCACTACCA

SEQ ID NO: 93 is the nucleic acid sequence of terminator tRPL15A.sba
GCTGGCTGATGAAAAATATAGTTCTGTTGGGCAAGCATTTGTTTACCTAGCATCTCTTTTATACTATTATTATCT
TTATATTTGATGATTTTATATACAAGTTGTATACCTTTTCTTTAACCAATTTTTTTTTTCTAATGGTGCACCTA
GAAGTACATTTTTTCTCACCAATAGATAGTCAAGATACTCCCAGCCTCTATGGCGTTACCACGGAGCCGACAAGG
GAAAGCTGCTTATCTTACATATGCAGATGCCAAGGCCCGTTACAGGCCGCCTTATTGATGTTTAGAAATAGCTTC

SEQ ID NO: 94 is the nucleic acid sequence of terminator tIDP1
TCGAATTTACGTAGCCCAATCTACCACTTTTTTTTTTCATTTTTTAAAGTGTTATACTTAGTTATGCTCTAGGAT
AATGAACTACTTTTTTTTTTTTTTTTTACTGTTATCATAAATATATATACCTTATTGTTGTTTGCAACCGTCGG
TTAATTCCTTATCAAGGTTCCCCAAGTTCGGATCATTACCATCAATTTCCAACATTTTCATGAGTTCTTCTTCTT
CATTACCGTGTTTTAGGGGGCTGTTCGCACTTCTAATAGGGCTATCACCAAGCTGTTCTAATTCGTCCAAAAGTT

SEQ ID NO:95 is the nucleic acid sequence of terminator tTEF1.sba
GGAGATTGATAGGACTTTTCTAGTTGCATATCTTTTATTTTTAAATCTTATCTATTAGTTAATTTTTTGTAATTT
ATCCTTATATATATAGTTTGGTTATTCTAAAACATCATTTCAGTATCTAAAACCTCTCTTATTCATTACCTTTTT
ATTTAATGGTTTTTGCTACAGGCAAAAATTTAATGGTTTTTGCTACAGGCAAAAATCCCGCCGTGGACTTATTCC
ACGTTAACTCGGTTACAGGGTCATGAACCATTTTGTCAATTATCGAAATAACTTCTTCAAAAGTCCCTCTTACTT

## Claims

1. A recombinant yeast cell producing hyaluronic acid (HA) wherein the recombinant cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity; and
(c) one or more recombinant nucleic acids encoding a polypeptide having hyaluronidase activity wherein the polypeptide having hyaluronidase activity comprises a secretion signal so that hyaluronic acid, in particular of a desired molecular weight (HAMW) is produced by the recombinant yeast cell,
wherein said recombinant yeast cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.

2. A recombinant host cell producing hyaluronic acid (HA) wherein the recombinant host cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity; and
(c) one or more recombinant nucleic acids encoding a polypeptide having hyaluronidase activity wherein the polypeptide having hyaluronidase activity comprises a secretion signal and an anchoring signal so that hyaluronic acid, in particular of a desired molecular weight (HAMW) is produced by the host cell.

3. The recombinant cell according to claim 1 or 2, wherein the molecular weight of the HA is in the range of less than 50kDa, preferably in the range of about 20kDa to about 50kDa.

4. The recombinant cell according to claim 1 or 2, wherein the molecular weight of the HA is in the range of greater than 50kDa, preferably in the range of about 50kDa to about 250kDa.

5. The recombinant cell according to claim 1 or 2, wherein the molecular weight of the HA is in the range of greater than 100kDa, preferably in the range of about 100kDa to about 1500kDa.

6. The recombinant cell according to any one of claims 1 to 5, wherein the nucleic acid encoding a polypeptide having hyaluronidase activity is obtained or derived from at least one of *Cupiennius salei, Loxosceles intermedia, Hirudo nipponia, Bothrops atrox* or *Tityus serrulatus.*

7. The recombinant cell according to any one of claims 1 to 6, wherein the nucleic acid encoding a polypeptide having hyaluronan synthase activity is obtained or derived from *Streptococcus zooepidemicus, Chlorella virus PBCV1, Xenopus laevis* or *Pasteurella multocida,* and is in particular obtained or derived from at least one of *Streptococcus zooepidemicus, Chlorella virus PBCV1* or *Xenopus laevis.*

8. The recombinant cell according to any one of claims 1 to 7, wherein the nucleic acid encoding a polypeptide having UDP-Glucose dehydrogenase activity is obtained or derived from at least one of *Arabidopsis thaliana, Chlorella virus PBCV1* or *Streptococcus zooepidemicus,* and in particular from *Arabidopsis thaliana* or *Chlorella virus PBCV1.*

9. The recombinant cell according to any one of claims 1 to 8, wherein the recombinant cell comprises a recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

10. The recombinant cell according to any one of claims 1 to 9, wherein the recombinant cell comprises at least one recombinant nucleic acid encoding one or more of:
(i) a polypeptide having Phosphoglucomutase-1 (PGM1) activity; and/or
(ii) a polypeptide having UTP-glucose-1-phosphate uridylyltransferase (UGP1) activity; and/or
(iii) a polypeptide having Glucosamine-6-phosphate N-acetyltransferase (GNA1) activity; and/or
(iv) a polypeptide having phosphoacetylglucosamine mutase (PCM1) activity.

11. The recombinant yeast cell according to any one of claims 1 and 3 to 10, wherein the recombinant yeast cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens Ogataea polymorpha, Yarrowia lypolytica, Debaryomyces hansenii,* and *Ashbya gossypii,* and is preferably *Saccharomyces cerevisiae.*

12. The recombinant host cell according to any one of claims 2 to 10 wherein the recombinant host cell belongs to the *Saccharomycetales* order, and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens Ogataea polymorpha, Yarrowia lypolytica, Debaryomyces hansenii,* and *Ashbya gossypii,* and is preferably *Saccharomyces cerevisiae.*

13. A method of producing hyaluronic acid (HA) of a desired molecular weight (HAMW) comprising:
(a) cultivating a recombinant cell as defined in any one of claims 1 to 12 in a cultivation medium for a time sufficient to produce hyaluronic acid (HA) of the desired molecular weight; and
(b) optionally isolating or recovering the hyaluronic acid (HA) from the recombinant cell and/or from the cultivation medium.

14. The method according to claim 13, wherein the HA has a molecular weight of from about 20kDa to about 50kDA, and preferably from about 20 kDa to about 30 kDa.

15. The method according to claim 14, wherein the HA has a molecular weight of from about 30 kDa to about 50 kDa.

16. The method according to claim 13, wherein the molecular weight of the HA is of from about about 50kDa to about 150 kDa.

17. The method according to claim 13, wherein the molecular weight of the HA is of from about 150kDa to about 1500 kDa.

18. The method according to any one of claims 13 to 17, wherein the recombinant cell comprises at least one recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

19. The method according to any one of claims 13 to 18, wherein the recombinant cell comprises at least one recombinant nucleic acid encoding one or more of:
(i) a polypeptide having Phosphoglucomutase-1 (PGM1) activity;
(ii) a polypeptide having UTP-glucose-1-phosphate uridylyltransferase (UGP1) activity;
(iii) a polypeptide having Glucosamine-6-phosphate N-acetyltransferase (GNA1) activity; and/or
(iv) a polypeptide having phosphoacetylglucosamine mutase (PCM1) activity.

20. The method according to any one of claims 13 to 19, wherein the recombinant cell is a member of the genus *Saccharomyces,* and in particular is *Saccharomyces cerevisiae.*

21. The method according according to any one of claims 13 to 20, wherein the time sufficient to produce hyaluronic acid (HA) of the desired molecular weight is a period of from about 35 hours to about 50 hours, preferably from about 40 hours to about 50 hours, preferably about 48 hours.

22. The method according according to any one of claims 13 to 21, wherein the molecular weight of the hyaluronic acid is controlled by regulating the pH of the cultivation medium.

23. The method according to any one of claims 13 to 22, wherein the method is carried out on an industrial scale, preferably where the cultivation medium is at least about 100L, more preferably in the range of about 1000L to about 3000L, even more preferably about 10,000L, even more preferably about 100,000L, or even about 250,000L.

24. Hyaluronic acid (HA) obtainable from a recombinant cell of any one of claims 1 to 10 or from the method according to any one of claims 13 to 23.

25. A cultivation medium comprising the HA according to claim 24.

26. A composition comprising the Hyaluronic acid (HA) according to claim 24.

27. An industrial product or a consumer product or a consumable comprising (i) the HA according to claim 24, (ii) the cultivation medium of claim 25 or (iii) the composition of claim 26.

28. The industrial product or the consumer product or the consumable of claim 27, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product and/or a dental and/or an oral hygiene composition.

29. Use of a recombinant cell as defined in any one of claims 1 to 12 for the production of hyaluronic acid (HA) having a molecular weight in the range of from about 20kDa to about 50kDa or from about 50kDa to about 1000 kDa.

30. A method for producing hyaluronic acid comprising the steps of:
(a) culturing a recombinant yeast as defined in any one of claims 1 and 3 to 11 in a culture medium; and
(b) recovering the hyaluronic acid from said culture medium,
wherein the hyaluronic acid recovered in step (b) has a molecular weight controlled through the selection of:
- the nature and origin of the nucleic acid encoding the hyaluronidase of the recombinant yeast,
- the nature and origin of the promoter controlling the expression of the nucleic acid encoding the hyaluronidase(s) of the recombinant yeast,
- the presence of an anchoring and/or of a secretion signal associated to the encoded hyaluronidase(s) of the recombinant yeast,
- the pH of the culture medium during the step of culturing the recombinant yeast, and/or
- the duration of the culturing of the recombinant yeast.


SEQUENCE LISTING

<110> GIVAUDAN SA

<120> HYALURONIC ACID-PRODUCING RECOMBINANT CELLS

<130> PR87630/MGG

<160> 95

<170> BiSSAP 1.3.6

<210> 1
<211> 1707
<212> DNA
<213> Paramecium bursaria Chlorella virus 1


<220>
<223> Reencoded nucleic acid sequence of hyaluronan synthase (HASA)
      originating from Chlorella virus PBCV-1

<400> 1
atgggtaaga acatcattat tatggtttct tggtacacta ttattacatc caatctcatc      60

gcagtgggtg gcgcctcact catactagcc ccagctatta cgggctatgt ccttcactgg     120

aacattgccc tttcaacaat ttggggagtg tcggcctacg gaattttcgt gtttggtttc     180

tttcttgccc aggtattatt tagtgaactc aaccggaaaa ggctccggaa gtggatttcc     240

ctccgaccca aagggtggaa tgatgttagg ttggctgtaa ttattgccgg ctaccgtgag     300

gacccgtata tgttccaaaa gtgtcttgaa agtgtgcgtg actcagacta tgggaatgta     360

gctagactaa tatgcgttat tgacggcgat gaagacgacg acatgaggat ggctgcagtg     420

tacaaggcta tctataacga caacatcaag aaacctgagt ttgtcctctg tgagagtgac     480

gataaggagg gtgagagaat agatagcgat ttcagccgtg atatctgcgt gctgcaaccg     540

catcgcggaa agcgtgaatg tttgtacaca gggttccaat tggcaaagat ggacccctca     600

gttaatgccg tcgtcctaat cgacagtgac actgtgttag aaaaggacgc gattctcgaa     660

gtagtatacc cgctggcatg cgatccagaa atacaggctg tagcaggcga atgcaaaata     720

tggaatactg acacactgtt gagtttgctg gtagcctggc gatattacag cgcattttgc     780

gtagagcgta gcgcccaatc attcttcagg acagtacaat gcgtcggagg acctctcggc     840

gcctacaaga ttgatataat taaggaaatc aaggacccat ggatcagcca acgtttcctt     900

ggccaaaagt gcacatacgg cgacgataga cgactcacta atgaaatact aatgaggggt     960

aagaaagtag ttttcacccc attcgctgtt ggctggtccg acagcccgac gaacgtcttc    1020

cgttacattg tacagcaaac acggtggtcc aagtcgtggt gtagggagat atggtataca    1080

ctgtttgcag catggaagca tggactttcg ggcatttggc tcgcattcga gtgcttatac    1140

cagattactt atttcttcct ggtgatttac ctattctccc gtttggctgt tgaggctgac    1200

ccacgggcgc aaacggccac cgtcattgtt tcgaccacag ttgcgcttat taagtgcggt 1260

tactttagct tcagagctaa agacattagg gccttctatt tcgtcctcta cacgttcgtt 1320

tacttcttct gcatgattcc ggcacgaata actgcgatga tgaccctgtg ggacataggt 1380

tggggaacca ggggaggaaa tgagaagcct tccgtaggca ccagagttgc tttgtgggcc 1440

aagcaatatt tgattgctta catgtggtgg gccgctgtcg tcggtgctgg cgtgtactcc 1500

atcgttcata attggatgtt tgactggaat tcactttcct acaggttcgc actggtaggc 1560

atctgttcgt atatagtttt cattgtaata gtgctggtag tctactttac aggaaagatc 1620

acgacctgga acttcacgaa gctacagaaa gaattgatcg aggaccgcgt actgtacgac 1680

gcaacgacca atgcccagtc ggtataa 1707

<210> 2
<211> 1707
<212> DNA
<213> Paramecium bursaria Chlorella virus 1

<220>
<223> Reencoded nucleic acid sequence of hyaluronan synthase (HASA)
      originating from Chlorella virus PBCV-1

<400> 2
atgggcaaga atattattat catggtcagt tggtatacca ttattacatc aaacctaatt 60

gctgtcggtg gagcctcact aatcttagct cccgcgatta cgggttatgt gcttcattgg 120

aatattgctt tatccactat ttggggtgtt agcgcttacg gcatctttgt gttcggtttc 180

ttcttggctc aggtgttatt ctctgagcta aacagaaaga gactcagaaa gtggatttcg 240

ctgcggccga agggatggaa cgatgttcgg ttagcggtga tcattgccgg ttatcgtgaa 300

gatccttata tgtttcagaa gtgtttggaa tctgttcgag actcagacta cggtaacgtt 360

gctaggctga tctgcgtaat cgatggagac gaagatgacg atatgagaat ggccgcggtt 420

tataaagcca tctataacga taacattaag aagccagagt ttgttttatg cgaatcggat 480

gacaaggagg gtgagaggat tgattctgac ttctcgcgtg acatctgtgt cctgcagccc 540

cacaggggaa agcgagagtg cctctataca ggattccaat tagctaaaat ggacccaagc 600

gttaatgctg tcgtccttat cgatagcgac acggtgttgg aaaaggacgc aatattggaa 660

gtagtctatc cactcgcttg tgatccagag atccaggccg tggctggcga gtgcaagatt 720

tggaatacag atacgcttct gtcattactg gtggcgtggc gttattattc agcattctgt 780

gtggagagga gcgcacaatc gttctttcgg actgtacaat gcgtaggcgg tccactagga 840

gcatataaaa ttgacataat caaagagata aaagatcctt ggatttcgca gagattcctc 900

ggtcaaaagt gtacctatgg ggacgacagg cggttgacta acgagattct aatgagaggt 960

aagaaggtgg tgttcactcc ttttgcggta ggttggagcg attcgcccac aaatgtattc 1020

agatacattg ttcaacaaac aaggtggtcg aaatcgtggt gccgagaaat atggtacacg 1080

ttgttcgccg catggaaaca cggcttgtcg ggtatatggc tcgctttcga gtgcctttat 1140

cagatcactt acttcttcct ggttatttac ttgttttcaa gactggcagt ggaagctgat 1200

ccaagggctc agactgctac tgtcatcgtt tcaacaacag tggcgctaat aaagtgtggt 1260

tacttcagct ttcgcgcgaa ggacataaga gcgttttact ttgttcttta tacctttgta 1320

tatttcttct gtatgatccc ggcccggata acagctatga tgacattatg ggacattggt 1380

tggggcacca ggggtgggaa tgagaagccg tccgtaggaa cgagagtagc gctgtgggcc 1440

aagcagtatc ttatcgcata catgtggtgg gcagctgtcg tcggggcagg tgtatacagc 1500

atagtacata actggatgtt tgactggaac tcactctcat atcgattcgc attggtcggg 1560

atctgctctt acatcgtgtt tattgttata gtcttagtag tatatttcac tggaaagata 1620

acaacatgga atttcactaa gcttcaaaag gaattgattg aagacagggt gctctacgat 1680

gcgacaacta atgcacaaag cgtataa 1707

<210> 3
<211> 2919
<212> DNA
<213> Pasteurella multocida

<220>
<223> Reencoded nucleic acid sequence of hyaluronan synthase (HASA)
      originating from Pasteurella multocida

<400> 3
atgaacaccc tttcccaggc aattaaggct tataatagta atgattatca actagccctc 60

aagctgttcg aaaagtcggc agaaatctac ggcagaaaga tagttgagtt ccaaattacg 120

aagtgcaagg agaaactttc tgcgcaccca tccgttaaca gtgcgcatct ctcggttaac 180

aaagaagaga aggtgaatgt ttgcgacagt ccattagaca tagccactca attattgtta 240

tcgaatgtga aaaaactcgt ccttagtgat agcgagaaaa atacactaaa gaataaatgg 300

aagttgttga ctgaaaagaa gtctgagaac gcagaggtcc gggccgtagc tttagttccc 360

aaggactttc cgaaagattt agtgcttgcg ccgttaccag accatgtgaa cgattttacg 420

tggtataaga agcggaagaa acgcctagga atcaaaccag aacaccaaca cgttggcctc 480

tccattattg tgacaacgtt caaccgtcct gccatcctca gcatcaccct ggcttgcctc 540

gtcaatcaaa agacccatta tccttttgag gtgatcgtga ccgacgatgg ttctcaagag 600

gatttatcgc ctataatccg tcaatatgag aacaagctag acatccgtta tgttcggcaa 660

aaggacaacg gttttcaggc atcggccgca cgtaatatgg gcctacgtct agccaaatat 720

gacttcattg ggctccttga ttgtgatatg gctcccaacc ctttgtgggt acactcatac 780

gttgcggaac tattagagga cgatgatttg accataatag gccccaggaa gtacattgac 840

acgcaacaca tagatcccaa ggatttcctt aataacgcct ctctgttaga gtcgttgcca 900

gaggttaaga ccaataattc cgtcgcggct aagggcgagg ggaccgtatc tttagactgg 960

cgtttggaac aatttgagaa gaccgagaac ttgaggctat ccgatagccc tttccgattc 1020

ttcgcagctg ggaatgtggc tttcgccaag aagtggctta acaagtcagg attcttcgac 1080

gaggaattca atcactgggg aggtgaagat gtagagttcg gttatcgtct gtttcggtac 1140

ggttcgttct tcaaaactat agacggcatc atggcctatc atcaggaacc gccaggtaaa 1200

gaaaacgaaa ctgacagaga agcgggcaag aacattaccc tcgatataat gagggagaag 1260

gtgccttaca tctaccgtaa actcctgcct atagaagaca gtcatatcaa ccgagtacca 1320

ttggtatcaa tttacatccc ggcctacaac tgtgctaact atattcaaag atgtgttgat 1380

tcagctttga atcaaacggt agttgatctc gaagtgtgca tttgcaacga cggtagtact 1440

gacaacacgc tggaagttat taacaagctg tatggtaata atccgcgtgt gcgtataatg 1500

tctaaaccca atggcggcat tgcgagtgca tccaacgcag cggtcagctt cgcaaagggt 1560

tattacatag gacagttgga cagcgacgat tacttagaac ccgacgcagt ggagttatgt 1620

ctcaaggaat ttcttaagga taagaccctt gcgtgcgttt acaccactaa tcgtaacgtc 1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatatta agatcattca gaacaaagac gccaaaatag ctgtctccat tttctatccg 2160

aacactctaa acgggttggt gaagaagcta aataatatta tagagtataa taagaatatc 2220

ttcgttattg tacttcatgt cgataagaat cacttaaccc cagacatcaa gaaggagata 2280

ttagccttct accataagca tcaggtgaac atcttattga acaatgacat ctcctactat 2340

acatcaaatc gtctgattaa gacagaagcc catttgagta acattaacaa gctaagtcaa 2400

ttaaatctta actgcgaata cattatattc gacaatcacg attccttatt tgtgaagaac 2460

gattcctatg catacatgaa gaagtatgat gttggaatga acttctctgc attgactcat 2520

gattggattg aaaagataaa cgctcacccg ccatttaaga agctgatcaa aacttacttc 2580

aatgacaatg atcttaagtc gatgaatgta aagggtgcct cccagggaat gtttatgaca 2640

tacgcattag cccacgagtt attgacgatc atcaaggagg tgataacctc ttgtcaatcc 2700

attgactccg tccccgaata caacacagaa gatatttggt ttcagtttgc acttttaatt 2760

ctggaaaaga agaccggcca cgtattcaac aagacaagca ctctcacgta tatgccatgg 2820

gaacgtaaac tgcagtggac gaatgaacaa atagagtccg caaagagggg cgaaaacatt 2880

ccggtaaaca agttcatcat taacagcatt accctttaa 2919

<210> 4
<211> 2112
<212> DNA
<213> Pasteurella multocida

<220>
<223> Reencoded nucleic acid sequence of hyaluronan synthase (HASA)
      originating from Pasteurella multocida

<400> 4
atgaacaccc tttcccaggc aattaaggct tataatagta atgattatca actagccctc 60

aagctgttcg aaaagtcggc agaaatctac ggcagaaaga tagttgagtt ccaaattacg 120

aagtgcaagg agaaactttc tgcgcaccca tccgttaaca gtgcgcatct ctcggttaac 180

aaagaagaga aggtgaatgt ttgcgacagt ccattagaca tagccactca attattgtta 240

tcgaatgtga aaaaactcgt ccttagtgat agcgagaaaa atacactaaa gaataaatgg 300

aagttgttga ctgaaaagaa gtctgagaac gcagaggtcc gggccgtagc tttagttccc 360

aaggactttc cgaaagattt agtgcttgcg ccgttaccag accatgtgaa cgattttacg 420

tggtataaga agcggaagaa acgcctagga atcaaaccag aacaccaaca cgttggcctc 480

tccattattg tgacaacgtt caaccgtcct gccatcctca gcatcaccct ggcttgcctc 540

gtcaatcaaa agacccatta tccttttgag gtgatcgtga ccgacgatgg ttctcaagag 600

gatttatcgc ctataatccg tcaatatgag aacaagctag acatccgtta tgttcggcaa 660

aaggacaacg gttttcaggc atcggccgca cgtaatatgg gcctacgtct agccaaatat 720

gacttcattg ggctccttga ttgtgatatg gctcccaacc ctttgtgggt acactcatac 780

gttgcggaac tattagagga cgatgatttg accataatag gccccaggaa gtacattgac 840

acgcaacaca tagatcccaa ggatttcctt aataacgcct ctctgttaga gtcgttgcca 900

gaggttaaga ccaataattc cgtcgcggct aagggcgagg ggaccgtatc tttagactgg 960

cgtttggaac aatttgagaa gaccgagaac ttgaggctat ccgatagccc tttccgattc 1020

ttcgcagctg ggaatgtggc tttcgccaag aagtggctta acaagtcagg attcttcgac 1080

gaggaattca atcactgggg aggtgaagat gtagagttcg gttatcgtct gtttcggtac 1140

ggttcgttct tcaaaactat agacggcatc atggcctatc atcaggaacc gccaggtaaa 1200

gaaaacgaaa ctgacagaga agcgggcaag aacattaccc tcgatataat gagggagaag 1260

gtgccttaca tctaccgtaa actcctgcct atagaagaca gtcatatcaa ccgagtacca 1320

ttggtatcaa tttacatccc ggcctacaac tgtgctaact atattcaaag atgtgttgat 1380

tcagctttga atcaaacggt agttgatctc gaagtgtgca tttgcaacga cggtagtact 1440

gacaacacgc tggaagttat taacaagctg tatggtaata atccgcgtgt gcgtataatg 1500

tctaaaccca atggcggcat tgcgagtgca tccaacgcag cggtcagctt cgcaaagggt 1560

tattacatag gacagttgga cagcgacgat tacttagaac ccgacgcagt ggagttatgt 1620

ctcaaggaat ttcttaagga taagaccctt gcgtgcgttt acaccactaa tcgtaacgtc 1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatattt aa 2112

<210> 5
<211> 2322
<212> DNA
<213> Pasteurella multocida

<220>
<223> Reencoded nucleic acid sequence of hyaluronan synthase (HASA)
      originating from Pasteurella multocida

<400> 5
atgaacaccc tttcccaggc aattaaggct tataatagta atgattatca actagccctc 60

aagctgttcg aaaagtcggc agaaatctac ggcagaaaga tagttgagtt ccaaattacg 120

aagtgcaagg agaaactttc tgcgcaccca tccgttaaca gtgcgcatct ctcggttaac 180

aaagaagaga aggtgaatgt ttgcgacagt ccattagaca tagccactca attattgtta 240

tcgaatgtga aaaaactcgt ccttagtgat agcgagaaaa atacactaaa gaataaatgg 300

aagttgttga ctgaaaagaa gtctgagaac gcagaggtcc gggccgtagc tttagttccc 360

aaggactttc cgaaagattt agtgcttgcg ccgttaccag accatgtgaa cgattttacg 420

tggtataaga agcggaagaa acgcctagga atcaaaccag aacaccaaca cgttggcctc 480

tccattattg tgacaacgtt caaccgtcct gccatcctca gcatcaccct ggcttgcctc 540

gtcaatcaaa agacccatta tccttttgag gtgatcgtga ccgacgatgg ttctcaagag 600

gatttatcgc ctataatccg tcaatatgag aacaagctag acatccgtta tgttcggcaa 660

aaggacaacg gttttcaggc atcggccgca cgtaatatgg gcctacgtct agccaaatat 720

gacttcattg ggctccttga ttgtgatatg gctcccaacc ctttgtgggt acactcatac 780

gttgcggaac tattagagga cgatgatttg accataatag gccccaggaa gtacattgac 840

acgcaacaca tagatcccaa ggatttcctt aataacgcct ctctgttaga gtcgttgcca 900

gaggttaaga ccaataattc cgtcgcggct aagggcgagg ggaccgtatc tttagactgg 960

cgtttggaac aatttgagaa gaccgagaac ttgaggctat ccgatagccc tttccgattc 1020

ttcgcagctg ggaatgtggc tttcgccaag aagtggctta acaagtcagg attcttcgac 1080

gaggaattca atcactgggg aggtgaagat gtagagttcg gttatcgtct gtttcggtac 1140

ggttcgttct tcaaaactat agacggcatc atggcctatc atcaggaacc gccaggtaaa 1200

gaaaacgaaa ctgacagaga agcgggcaag aacattaccc tcgatataat gagggagaag 1260

gtgccttaca tctaccgtaa actcctgcct atagaagaca gtcatatcaa ccgagtacca 1320

ttggtatcaa tttacatccc ggcctacaac tgtgctaact atattcaaag atgtgttgat 1380

tcagctttga atcaaacggt agttgatctc gaagtgtgca tttgcaacga cggtagtact 1440

gacaacacgc tggaagttat taacaagctg tatggtaata atccgcgtgt gcgtataatg 1500

tctaaaccca atggcggcat tgcgagtgca tccaacgcag cggtcagctt cgcaaagggt 1560

tattacatag gacagttgga cagcgacgat tacttagaac ccgacgcagt ggagttatgt 1620

ctcaaggaat ttcttaagga taagaccctt gcgtgcgttt acaccactaa tcgtaacgtc 1680

aacccagatg gctctttaat agccaatggc tataactggc cagagttcag tcgtgagaag 1740

ttgactacgg ccatgattgc tcatcacttc cggatgttta ccattcgtgc ttggcatctg 1800

acggatgggt tcaatgagaa gattgagaac gctgttgact acgacatgtt tctcaagctc 1860

agtgaagttg gtaaatttaa gcatctgaac aaaatatgtt ataatcgggt gttacacggc 1920

gataacacct caatcaagaa gcttggcata caaaagaaga atcatttcgt agttgtcaat 1980

cagtctctaa accgccaagg tataacttat tataactacg atgaatttga tgatctcgat 2040

gagagtcgga aatacatttt caacaagact gcagagtatc aagaagagat agatattctt 2100

aaagatattg gatccgccat ttctcaaatc actgacggtc aaatccaagc tactaccact 2160

gctaccaccg aagctaccac cactgctgcc ccatcttcca ccgttgaaac tgtttctcca 2220

tccagcaccg aaactatctc tcaacaaact gaaaatggtg ctgctaaggc cgctgtcggt 2280

atgggtgccg gtgctctagc tgctgctgct atgttgttat aa 2322

<210> 6
<211> 1656
<212> DNA
<213> Xenopus laevis

<220>
<223> Reencoded nucleic acid sequence of hyaluronan synthase (HASA)
      originating from Xenopus laevis

<400> 6
atgcactgtg aacgatttat ttgtatatta cgaataattg gtacgacttt gttcggagta      60

agtctgctac tagggatatc cgccgcttat atcgtgggat accaattcat tcagacggat     120

aattactact tttccttcgg tctctacggt gctattttag ctttacattt aatcattcag     180

tctctcttcg cattcctgga gcatcgtaag atgaagcggt cacttgaaac ccctataaaa     240

ttaaacaaga gcgtagcatt gtgtatcgct gcataccagg aagacgaaga ttatcttaga     300

aaatgtttgc tttctgtgaa acggctcaca taccccggta tgaaggtaat catggtgata     360

gatggtaact ctgatgacga tctatacatg atgaacatat tcagagagat catgggaaat     420

gatagctgcg ccacctatgt atggaagaat aattttcaca tgaagggacc aaacgagacc     480

gacgagactc accgcgagtc catgcagcat gtaacacaga tggtgctttc taacagaaat     540

gtctgtatca tgcaaaagtg gaacgggaaa cgcgaggtga tgtatactgc attcaaagcg     600

ctgggtcggt cggttgatta cgtccaagtg tgcgactcag atacagtcct tgatccagca     660

agctcggtgg agatggtcaa ggtgttggag gaagacatta tggtcggagg tgtaggcgga     720

gacgtgcaga tactcaataa gtacgactcc tggatcagtt tcttgagttc ggttcgttat     780

tggatggctt tcaatataga acgagcatgc caatcatact ttggctgtgt gcagtgtata     840

tcggggcctc tgggcatgta cagaaactca ctcttacatg agttcattga agactggtac     900

aatcaagaat tcctcggttc ccaatgctct ttcggtgatg atcggcattt gactaatcga     960

gtactatcat tgggctatgc tactaaatac actgcgcgca gtaagtgtct gacagaaacc    1020

cccacagaat atcttagatg gttgaaccaa caaaccaggt ggagtaagtc ctatttccgc    1080

gagtggttgt acaactcttt gtggttccac aaacaccatt tatggatgac ttatgaagcc    1140

gtaattacgg gtttcttccc cttcttctta atagcgacgg tgattcagct cttctatcgt    1200

ggtcgaattt ggaatatctt actctttcta ctcacagttc aattagttgg tctaataaag    1260

tcgtcgtttg caagtgcgtt gcgtggaaac atcgttatgg tctttatgag tttctacagt    1320

gttttataca tgagtagctt gctgccagca aagatgtttg caatcgccac gataaacaag    1380

gctggatggg gtactagtgg tagaaagacc atagttgtta attttattgg tttaatccct    1440

attacggtat ggttcacaat tttactaggg ggagtatgct acactatatg gcgggaaacc    1500

aagaagccgt ttagcgagtc tgagaagatc gttcttgcgg tcggtgctat attgtatgct    1560

tgttattggg tcatgttgct tacaatgtat gtcagtcttg tcatgaagtg cgggcgccgt    1620

aggaaggaac cacagcacga ccttgtttta gcataa                              1656

<210> 7
<211> 1254
<212> DNA
<213> Streptococcus zooepidemicus

<220>
<223> Reencoded nucleic acid sequence of hyaluronan synthase (HASA)
      originating from Streptococcus zooepidemicus

<400> 7
atgaggacgt taaagaatct tatcactgtt gtagcgttca gtatattctg ggttttgctg      60

atctatgtaa atgtctatct tttcggtgcg aaggggtctc tatcaatata tggattcttg     120

ctaattgcgt atttgcttgt gaagatgagt ctttcattct tctataaacc attcaaaggt     180

cgagctggtc agtataaagt cgcagcgatt atcccttcat acaacgagga tgcagaatcg     240

ctgttggaaa ccttaaaatc tgttcaacaa caaacctatc cccttgctga aatttatgta     300

gttgatgatg gttccgcaga tgaaaccggt atcaagcgga tcgaggatta tgtcagggat     360

acaggcgact taagttcaaa tgtgattgtg catcgatcag aaaagaatca aggcaagaga     420

cacgcccaag catgggcatt tgaaagatct gatgcagatg tatttctaac agtcgatagt     480

gatacttaca tttatccgga cgcccttgaa gagttattga aaaccttcaa tgatccgaca     540

gtatttgccg caactggcca cttgaacgtt cgtaacaggc agactaatct attgaccaga     600

ctcactgata taagatacga caatgctttt ggcgtcgaac gtgctgctca aagtgtaact     660

ggtaatatac tggtgtgttc cgggccactg tccgtctatc gcagagaagt agtagtcccg     720

aacattgaca ggtatattaa tcagactttt ctgggtatcc ccgtgtcaat tggggacgac     780

cggtgtttaa cgaactacgc tactgatttg ggcaagactg tatatcagtc gacggctaaa     840

tgtattacag acgtcccaga taagatgtcg acttacctta aacagcagaa tagatggaat     900

aagtcattct ttagagaatc tatcatcagc gtgaagaaga tcatgaataa tccattcgtg     960

gcgctttgga ccattttgga ggtttccatg tttatgatgc ttgtctactc tgtcgttgat    1020

ttctttgtag gtaacgttcg agaatttgat tggctcagag ttcttgcttt cttagttatc    1080

atcttcatcg ttgcgctatg caggaatatt cattacatgc taaaacatcc gcttagcttc    1140

ttactcagcc ctttctacgg cgttttgcat ctcttcgttt tgcagccatt gaagctctac    1200

tccttattta ccattcgaaa cgctgattgg ggtacgcgca agaaactatt ataa          1254

<210> 8
<211> 568
<212> PRT
<213> Paramecium bursaria Chlorella virus 1

<220>
<223> Amino acid sequence of hyaluronan synthase (HASA) originating
      from Chlorella virus PBCV-1

<400> 8
Met Gly Lys Asn Ile Ile Ile Met Val Ser Trp Tyr Thr Ile Ile Thr
1               5                   10                  15
Ser Asn Leu Ile Ala Val Gly Gly Ala Ser Leu Ile Leu Ala Pro Ala
            20                  25                  30
Ile Thr Gly Tyr Val Leu His Trp Asn Ile Ala Leu Ser Thr Ile Trp
        35                  40                  45
Gly Val Ser Ala Tyr Gly Ile Phe Val Phe Gly Phe Phe Leu Ala Gln
    50                  55                  60
Val Leu Phe Ser Glu Leu Asn Arg Lys Arg Leu Arg Lys Trp Ile Ser
65                  70                  75                  80
Leu Arg Pro Lys Gly Trp Asn Asp Val Arg Leu Ala Val Ile Ile Ala
                85                  90                  95
Gly Tyr Arg Glu Asp Pro Tyr Met Phe Gln Lys Cys Leu Glu Ser Val
            100                 105                 110
Arg Asp Ser Asp Tyr Gly Asn Val Ala Arg Leu Ile Cys Val Ile Asp
        115                 120                 125
Gly Asp Glu Asp Asp Asp Met Arg Met Ala Ala Val Tyr Lys Ala Ile
    130                 135                 140
Tyr Asn Asp Asn Ile Lys Lys Pro Glu Phe Val Leu Cys Glu Ser Asp
145                 150                 155                 160
Asp Lys Glu Gly Glu Arg Ile Asp Ser Asp Phe Ser Arg Asp Ile Cys
                165                 170                 175
Val Leu Gln Pro His Arg Gly Lys Arg Glu Cys Leu Tyr Thr Gly Phe
            180                 185                 190
Gln Leu Ala Lys Met Asp Pro Ser Val Asn Ala Val Val Leu Ile Asp
        195                 200                 205
Ser Asp Thr Val Leu Glu Lys Asp Ala Ile Leu Glu Val Val Tyr Pro
    210                 215                 220
Leu Ala Cys Asp Pro Glu Ile Gln Ala Val Ala Gly Glu Cys Lys Ile
225                 230                 235                 240
Trp Asn Thr Asp Thr Leu Leu Ser Leu Leu Val Ala Trp Arg Tyr Tyr
                245                 250                 255
Ser Ala Phe Cys Val Glu Arg Ser Ala Gln Ser Phe Phe Arg Thr Val
            260                 265                 270
Gln Cys Val Gly Gly Pro Leu Gly Ala Tyr Lys Ile Asp Ile Ile Lys
        275                 280                 285
Glu Ile Lys Asp Pro Trp Ile Ser Gln Arg Phe Leu Gly Gln Lys Cys
    290                 295                 300
Thr Tyr Gly Asp Asp Arg Arg Leu Thr Asn Glu Ile Leu Met Arg Gly
305                 310                 315                 320
Lys Lys Val Val Phe Thr Pro Phe Ala Val Gly Trp Ser Asp Ser Pro
                325                 330                 335
Thr Asn Val Phe Arg Tyr Ile Val Gln Gln Thr Arg Trp Ser Lys Ser
            340                 345                 350
Trp Cys Arg Glu Ile Trp Tyr Thr Leu Phe Ala Ala Trp Lys His Gly
        355                 360                 365
Leu Ser Gly Ile Trp Leu Ala Phe Glu Cys Leu Tyr Gln Ile Thr Tyr
    370                 375                 380
Phe Phe Leu Val Ile Tyr Leu Phe Ser Arg Leu Ala Val Glu Ala Asp
385                 390                 395                 400
Pro Arg Ala Gln Thr Ala Thr Val Ile Val Ser Thr Thr Val Ala Leu
                405                 410                 415
Ile Lys Cys Gly Tyr Phe Ser Phe Arg Ala Lys Asp Ile Arg Ala Phe
            420                 425                 430
Tyr Phe Val Leu Tyr Thr Phe Val Tyr Phe Phe Cys Met Ile Pro Ala
        435                 440                 445
Arg Ile Thr Ala Met Met Thr Leu Trp Asp Ile Gly Trp Gly Thr Arg
    450                 455                 460
Gly Gly Asn Glu Lys Pro Ser Val Gly Thr Arg Val Ala Leu Trp Ala
465                 470                 475                 480
Lys Gln Tyr Leu Ile Ala Tyr Met Trp Trp Ala Ala Val Val Gly Ala

485                     490                     495
Gly Val Tyr Ser Ile Val His Asn Trp Met Phe Asp Trp Asn Ser Leu
            500                     505                     510
Ser Tyr Arg Phe Ala Leu Val Gly Ile Cys Ser Tyr Ile Val Phe Ile
        515                     520                     525
Val Ile Val Leu Val Val Tyr Phe Thr Gly Lys Ile Thr Thr Trp Asn
    530                     535                     540
Phe Thr Lys Leu Gln Lys Glu Leu Ile Glu Asp Arg Val Leu Tyr Asp
545                     550                     555                     560
Ala Thr Thr Asn Ala Gln Ser Val
                565

<210> 9
<211> 972
<212> PRT
<213> Pasteurella multocida

<220>
<223> Amino acid sequence of hyaluronan synthase (HASA) originating
      from Pasteurella multocida

<400> 9
Met Asn Thr Leu Ser Gln Ala Ile Lys Ala Tyr Asn Ser Asn Asp Tyr
1               5                   10                  15
Gln Leu Ala Leu Lys Leu Phe Glu Lys Ser Ala Glu Ile Tyr Gly Arg
            20                  25                  30
Lys Ile Val Glu Phe Gln Ile Thr Lys Cys Lys Glu Lys Leu Ser Ala
        35                  40                  45
His Pro Ser Val Asn Ser Ala His Leu Ser Val Asn Lys Glu Glu Lys
    50                  55                  60
Val Asn Val Cys Asp Ser Pro Leu Asp Ile Ala Thr Gln Leu Leu Leu
65                  70                  75                  80
Ser Asn Val Lys Lys Leu Val Leu Ser Asp Ser Glu Lys Asn Thr Leu
                85                  90                  95
Lys Asn Lys Trp Lys Leu Leu Thr Glu Lys Lys Ser Glu Asn Ala Glu
            100                 105                 110
Val Arg Ala Val Ala Leu Val Pro Lys Asp Phe Pro Lys Asp Leu Val
        115                 120                 125
Leu Ala Pro Leu Pro Asp His Val Asn Asp Phe Thr Trp Tyr Lys Lys
    130                 135                 140
Arg Lys Lys Arg Leu Gly Ile Lys Pro Glu His Gln His Val Gly Leu
145                 150                 155                 160
Ser Ile Ile Val Thr Thr Phe Asn Arg Pro Ala Ile Leu Ser Ile Thr
                165                 170                 175
Leu Ala Cys Leu Val Asn Gln Lys Thr His Tyr Pro Phe Glu Val Ile
            180                 185                 190
Val Thr Asp Asp Gly Ser Gln Glu Asp Leu Ser Pro Ile Ile Arg Gln
        195                 200                 205
Tyr Glu Asn Lys Leu Asp Ile Arg Tyr Val Arg Gln Lys Asp Asn Gly
    210                 215                 220
Phe Gln Ala Ser Ala Ala Arg Asn Met Gly Leu Arg Leu Ala Lys Tyr
225                 230                 235                 240
Asp Phe Ile Gly Leu Leu Asp Cys Asp Met Ala Pro Asn Pro Leu Trp
                245                 250                 255
Val His Ser Tyr Val Ala Glu Leu Leu Glu Asp Asp Asp Leu Thr Ile
            260                 265                 270
Ile Gly Pro Arg Lys Tyr Ile Asp Thr Gln His Ile Asp Pro Lys Asp
        275                 280                 285
Phe Leu Asn Asn Ala Ser Leu Leu Glu Ser Leu Pro Glu Val Lys Thr
    290                 295                 300
Asn Asn Ser Val Ala Ala Lys Gly Glu Gly Thr Val Ser Leu Asp Trp
305                 310                 315                 320

Arg Leu Glu Gln Phe Glu Lys Thr Glu Asn Leu Arg Leu Ser Asp Ser
                325                 330                 335
Pro Phe Arg Phe Phe Ala Ala Gly Asn Val Ala Phe Ala Lys Lys Trp
            340                 345                 350
Leu Asn Lys Ser Gly Phe Phe Asp Glu Glu Phe Asn His Trp Gly Gly
        355                 360                 365
Glu Asp Val Glu Phe Gly Tyr Arg Leu Phe Arg Tyr Gly Ser Phe Phe
    370                 375                 380
Lys Thr Ile Asp Gly Ile Met Ala Tyr His Gln Glu Pro Pro Gly Lys
385                 390                 395                 400
Glu Asn Glu Thr Asp Arg Glu Ala Gly Lys Asn Ile Thr Leu Asp Ile
                405                 410                 415
Met Arg Glu Lys Val Pro Tyr Ile Tyr Arg Lys Leu Leu Pro Ile Glu
            420                 425                 430
Asp Ser His Ile Asn Arg Val Pro Leu Val Ser Ile Tyr Ile Pro Ala
        435                 440                 445
Tyr Asn Cys Ala Asn Tyr Ile Gln Arg Cys Val Asp Ser Ala Leu Asn
    450                 455                 460
Gln Thr Val Val Asp Leu Glu Val Cys Ile Cys Asn Asp Gly Ser Thr
465                 470                 475                 480
Asp Asn Thr Leu Glu Val Ile Asn Lys Leu Tyr Gly Asn Asn Pro Arg
                485                 490                 495
Val Arg Ile Met Ser Lys Pro Asn Gly Gly Ile Ala Ser Ala Ser Asn
            500                 505                 510
Ala Ala Val Ser Phe Ala Lys Gly Tyr Tyr Ile Gly Gln Leu Asp Ser
        515                 520                 525
Asp Asp Tyr Leu Glu Pro Asp Ala Val Glu Leu Cys Leu Lys Glu Phe
    530                 535                 540
Leu Lys Asp Lys Thr Leu Ala Cys Val Tyr Thr Thr Asn Arg Asn Val
545                 550                 555                 560
Asn Pro Asp Gly Ser Leu Ile Ala Asn Gly Tyr Asn Trp Pro Glu Phe
                565                 570                 575
Ser Arg Glu Lys Leu Thr Thr Ala Met Ile Ala His His Phe Arg Met
            580                 585                 590
Phe Thr Ile Arg Ala Trp His Leu Thr Asp Gly Phe Asn Glu Lys Ile
        595                 600                 605
Glu Asn Ala Val Asp Tyr Asp Met Phe Leu Lys Leu Ser Glu Val Gly
    610                 615                 620
Lys Phe Lys His Leu Asn Lys Ile Cys Tyr Asn Arg Val Leu His Gly
625                 630                 635                 640
Asp Asn Thr Ser Ile Lys Lys Leu Gly Ile Gln Lys Lys Asn His Phe
                645                 650                 655
Val Val Val Asn Gln Ser Leu Asn Arg Gln Gly Ile Thr Tyr Tyr Asn
            660                 665                 670
Tyr Asp Glu Phe Asp Asp Leu Asp Glu Ser Arg Lys Tyr Ile Phe Asn
        675                 680                 685
Lys Thr Ala Glu Tyr Gln Glu Glu Ile Asp Ile Leu Lys Asp Ile Lys
    690                 695                 700
Ile Ile Gln Asn Lys Asp Ala Lys Ile Ala Val Ser Ile Phe Tyr Pro
705                 710                 715                 720
Asn Thr Leu Asn Gly Leu Val Lys Lys Leu Asn Asn Ile Ile Glu Tyr
                725                 730                 735
Asn Lys Asn Ile Phe Val Ile Val Leu His Val Asp Lys Asn His Leu
            740                 745                 750
Thr Pro Asp Ile Lys Lys Glu Ile Leu Ala Phe Tyr His Lys His Gln
        755                 760                 765
Val Asn Ile Leu Leu Asn Asn Asp Ile Ser Tyr Tyr Thr Ser Asn Arg
    770                 775                 780
Leu Ile Lys Thr Glu Ala His Leu Ser Asn Ile Asn Lys Leu Ser Gln
785                 790                 795                 800
Leu Asn Leu Asn Cys Glu Tyr Ile Ile Phe Asp Asn His Asp Ser Leu
                805                 810                 815
Phe Val Lys Asn Asp Ser Tyr Ala Tyr Met Lys Lys Tyr Asp Val Gly

                820                 825                 830
Met Asn Phe Ser Ala Leu Thr His Asp Trp Ile Glu Lys Ile Asn Ala
        835                 840                 845
His Pro Pro Phe Lys Lys Leu Ile Lys Thr Tyr Phe Asn Asp Asn Asp
    850                 855                 860
Leu Lys Ser Met Asn Val Lys Gly Ala Ser Gln Gly Met Phe Met Thr
865                 870                 875                 880
Tyr Ala Leu Ala His Glu Leu Leu Thr Ile Ile Lys Glu Val Ile Thr
                885                 890                 895
Ser Cys Gln Ser Ile Asp Ser Val Pro Glu Tyr Asn Thr Glu Asp Ile
            900                 905                 910
Trp Phe Gln Phe Ala Leu Leu Ile Leu Glu Lys Lys Thr Gly His Val
        915                 920                 925
Phe Asn Lys Thr Ser Thr Leu Thr Tyr Met Pro Trp Glu Arg Lys Leu
    930                 935                 940
Gln Trp Thr Asn Glu Gln Ile Glu Ser Ala Lys Arg Gly Glu Asn Ile
945                 950                 955                 960
Pro Val Asn Lys Phe Ile Ile Asn Ser Ile Thr Leu
                965                 970

<210> 10
<211> 551
<212> PRT
<213> Xenopus laevis


<220>
<223> Amino acid sequence of hyaluronan synthase (HASA) originating
      from Xenopus laevis

<400> 10
Met His Cys Glu Arg Phe Ile Cys Ile Leu Arg Ile Ile Gly Thr Thr
1               5                   10                  15
Leu Phe Gly Val Ser Leu Leu Leu Gly Ile Ser Ala Ala Tyr Ile Val
            20                  25                  30
Gly Tyr Gln Phe Ile Gln Thr Asp Asn Tyr Tyr Phe Ser Phe Gly Leu
        35                  40                  45
Tyr Gly Ala Ile Leu Ala Leu His Leu Ile Ile Gln Ser Leu Phe Ala
    50                  55                  60
Phe Leu Glu His Arg Lys Met Lys Arg Ser Leu Glu Thr Pro Ile Lys
65                  70                  75                  80
Leu Asn Lys Ser Val Ala Leu Cys Ile Ala Ala Tyr Gln Glu Asp Glu
                85                  90                  95
Asp Tyr Leu Arg Lys Cys Leu Leu Ser Val Lys Arg Leu Thr Tyr Pro
            100                 105                 110
Gly Met Lys Val Ile Met Val Ile Asp Gly Asn Ser Asp Asp Asp Leu
        115                 120                 125
Tyr Met Met Asn Ile Phe Arg Glu Ile Met Gly Asn Asp Ser Cys Ala
    130                 135                 140
Thr Tyr Val Trp Lys Asn Asn Phe His Met Lys Gly Pro Asn Glu Thr
145                 150                 155                 160
Asp Glu Thr His Arg Glu Ser Met Gln His Val Thr Gln Met Val Leu
                165                 170                 175
Ser Asn Arg Asn Val Cys Ile Met Gln Lys Trp Asn Gly Lys Arg Glu
            180                 185                 190
Val Met Tyr Thr Ala Phe Lys Ala Leu Gly Arg Ser Val Asp Tyr Val
        195                 200                 205
Gln Val Cys Asp Ser Asp Thr Val Leu Asp Pro Ala Ser Ser Val Glu
    210                 215                 220
Met Val Lys Val Leu Glu Glu Asp Ile Met Val Gly Gly Val Gly Gly
225                 230                 235                 240
Asp Val Gln Ile Leu Asn Lys Tyr Asp Ser Trp Ile Ser Phe Leu Ser
                245                 250                 255

Ser Val Arg Tyr Trp Met Ala Phe Asn Ile Glu Arg Ala Cys Gln Ser
                260                 265                 270
Tyr Phe Gly Cys Val Gln Cys Ile Ser Gly Pro Leu Gly Met Tyr Arg
        275                 280                 285
Asn Ser Leu Leu His Glu Phe Ile Glu Asp Trp Tyr Asn Gln Glu Phe
    290                 295                 300
Leu Gly Ser Gln Cys Ser Phe Gly Asp Asp Arg His Leu Thr Asn Arg
305                 310                 315                 320
Val Leu Ser Leu Gly Tyr Ala Thr Lys Tyr Thr Ala Arg Ser Lys Cys
                325                 330                 335
Leu Thr Glu Thr Pro Thr Glu Tyr Leu Arg Trp Leu Asn Gln Gln Thr
            340                 345                 350
Arg Trp Ser Lys Ser Tyr Phe Arg Glu Trp Leu Tyr Asn Ser Leu Trp
        355                 360                 365
Phe His Lys His His Leu Trp Met Thr Tyr Glu Ala Val Ile Thr Gly
    370                 375                 380
Phe Phe Pro Phe Phe Leu Ile Ala Thr Val Ile Gln Leu Phe Tyr Arg
385                 390                 395                 400
Gly Arg Ile Trp Asn Ile Leu Leu Phe Leu Leu Thr Val Gln Leu Val
                405                 410                 415
Gly Leu Ile Lys Ser Ser Phe Ala Ser Ala Leu Arg Gly Asn Ile Val
            420                 425                 430
Met Val Phe Met Ser Phe Tyr Ser Val Leu Tyr Met Ser Ser Leu Leu
        435                 440                 445
Pro Ala Lys Met Phe Ala Ile Ala Thr Ile Asn Lys Ala Gly Trp Gly
    450                 455                 460
Thr Ser Gly Arg Lys Thr Ile Val Val Asn Phe Ile Gly Leu Ile Pro
465                 470                 475                 480
Ile Thr Val Trp Phe Thr Ile Leu Leu Gly Gly Val Cys Tyr Thr Ile
                485                 490                 495
Trp Arg Glu Thr Lys Lys Pro Phe Ser Glu Ser Glu Lys Ile Val Leu
            500                 505                 510
Ala Val Gly Ala Ile Leu Tyr Ala Cys Tyr Trp Val Met Leu Leu Thr
        515                 520                 525
Met Tyr Val Ser Leu Val Met Lys Cys Gly Arg Arg Arg Lys Glu Pro
    530                 535                 540
Gln His Asp Leu Val Leu Ala
545                 550

<210> 11
<211> 417
<212> PRT
<213> Streptococcus zooepidemicus


<220>
<223> Amino acid sequence of hyaluronan synthase (HASA) originating
      from Streptococcus zooepidemicus

<400> 11
Met Arg Thr Leu Lys Asn Leu Ile Thr Val Val Ala Phe Ser Ile Phe
1               5                   10                  15
Trp Val Leu Leu Ile Tyr Val Asn Val Tyr Leu Phe Gly Ala Lys Gly
            20                  25                  30
Ser Leu Ser Ile Tyr Gly Phe Leu Leu Ile Ala Tyr Leu Leu Val Lys
        35                  40                  45
Met Ser Leu Ser Phe Phe Tyr Lys Pro Phe Lys Gly Arg Ala Gly Gln
    50                  55                  60
Tyr Lys Val Ala Ala Ile Ile Pro Ser Tyr Asn Glu Asp Ala Glu Ser
65                  70                  75                  80
Leu Leu Glu Thr Leu Lys Ser Val Gln Gln Gln Thr Tyr Pro Leu Ala
                85                  90                  95
Glu Ile Tyr Val Val Asp Asp Gly Ser Ala Asp Glu Thr Gly Ile Lys

                100                 105                 110
Arg Ile Glu Asp Tyr Val Arg Asp Thr Gly Asp Leu Ser Ser Asn Val
        115                 120                 125
Ile Val His Arg Ser Glu Lys Asn Gln Gly Lys Arg His Ala Gln Ala
    130                 135                 140
Trp Ala Phe Glu Arg Ser Asp Ala Asp Val Phe Leu Thr Val Asp Ser
145                 150                 155                 160
Asp Thr Tyr Ile Tyr Pro Asp Ala Leu Glu Glu Leu Leu Lys Thr Phe
                165                 170                 175
Asn Asp Pro Thr Val Phe Ala Ala Thr Gly His Leu Asn Val Arg Asn
            180                 185                 190
Arg Gln Thr Asn Leu Leu Thr Arg Leu Thr Asp Ile Arg Tyr Asp Asn
        195                 200                 205
Ala Phe Gly Val Glu Arg Ala Ala Gln Ser Val Thr Gly Asn Ile Leu
    210                 215                 220
Val Cys Ser Gly Pro Leu Ser Val Tyr Arg Arg Glu Val Val Val Pro
225                 230                 235                 240
Asn Ile Asp Arg Tyr Ile Asn Gln Thr Phe Leu Gly Ile Pro Val Ser
                245                 250                 255
Ile Gly Asp Asp Arg Cys Leu Thr Asn Tyr Ala Thr Asp Leu Gly Lys
            260                 265                 270
Thr Val Tyr Gln Ser Thr Ala Lys Cys Ile Thr Asp Val Pro Asp Lys
        275                 280                 285
Met Ser Thr Tyr Leu Lys Gln Gln Asn Arg Trp Asn Lys Ser Phe Phe
    290                 295                 300
Arg Glu Ser Ile Ile Ser Val Lys Lys Ile Met Asn Asn Pro Phe Val
305                 310                 315                 320
Ala Leu Trp Thr Ile Leu Glu Val Ser Met Phe Met Met Leu Val Tyr
                325                 330                 335
Ser Val Val Asp Phe Phe Val Gly Asn Val Arg Glu Phe Asp Trp Leu
            340                 345                 350
Arg Val Leu Ala Phe Leu Val Ile Ile Phe Ile Val Ala Leu Cys Arg
        355                 360                 365
Asn Ile His Tyr Met Leu Lys His Pro Leu Ser Phe Leu Leu Ser Pro
    370                 375                 380
Phe Tyr Gly Val Leu His Leu Phe Val Leu Gln Pro Leu Lys Leu Tyr
385                 390                 395                 400
Ser Leu Phe Thr Ile Arg Asn Ala Asp Trp Gly Thr Arg Lys Lys Leu
                405                 410                 415
Leu


<210> 12
<211> 1446
<212> DNA
<213> Arabidopsis thaliana


<220>
<223> Nucleic acid sequence of UDP-Glucose dehydrogenase (HASB)
      originating from Arabidopsis thaliana

<400> 12
atggtcaaaa tatgctgtat cggggctgga tatgtcggtg ggcctacaat ggccgttatg      60

gccctaaagt gtccagagat tgaagtcgtg gtagttgata tatcggagcc acgaattaac     120

gcatggaact cagaccgtct accaatttat gagccagggt tagaggatgt cgtcaaacag     180

tgtagaggga agaatttgtt cttctctaca gatgtagaga agcatgtatt tgagtcagat     240

atagtgtttg tttcggtaaa cactcctacg aaaacgcagg gtctgggtgc aggtaaagca     300

gcagatttga catattggga gtccgctgct cgcatgatag ctgatgtgag caaatcgtcg 360

aaaatcgtcg ttgaaaagag tacagtacca gttcgtacag ccgaggctat agaaaagatt 420

ttaacgcaca attcgaaggg tatcgaattc cagatcttat caaatccaga attcttggcc 480

gaagggacgg cgattaaaga cttatataac cctgatagag ttctaatcgg tggcagggac 540

accgctgcgg gacaaaaggc cattaaggcg ttgcgtgacg tgtatgccca ctgggttcct 600

gttgagcaaa taatctgtac taatttatgg agtgccgagc tatcaaagtt ggctgcgaac 660

gcatttctag ctcaaaggat aagttcagta aacgcaatgt cagcgctttg tgaggcaact 720

ggtgctgacg taacccaagt cgctcacgcc gttggaaccg acactagaat tggaccgaag 780

tttcttaacg catccgtagg cttcggcgga tcttgctttc agaaagacat cctgaatctt 840

atttacatct gcgaatgcaa tggtcttcca gaagcagcca attattggaa acaggtagtc 900

aaggtaaatg actaccaaaa gattaggttt gctaatcgag tcgtatcttc tatgttcaac 960

accgtctccg gtaagaaaat tgctattttg ggatttgcgt tcaagaagga caccggcgac 1020

acgcgtgaaa ctcctgccat agatgtgtgt aatcgcctcg tggctgataa agcaaagctg 1080

tcgatctatg atccgcaagt attagaagag cagatccgcc gtgatctgtc catggcccga 1140

ttcgattggg accatccagt cccactccag cagatcaaag ctgaaggtat ctccgaacag 1200

gttaacgttg tgtccgacgc ctacgaggct acgaaggatg ctcatggttt atgtgtttta 1260

accgaatggg acgaattcaa gtcacttgat tttaagaaga tctttgataa tatgcagaaa 1320

cccgctttcg ttttcgacgg aagaaacgtg gtcgacgctg tgaaattgag agaaattgga 1380

ttcatagtat attccatagg taaacctctg gatagttggc tcaaggatat gccggctgtt 1440

gcataa 1446

<210> 13
<211> 1166
<212> DNA
<213> Paramecium bursaria Chlorella virus 1

<220>
<223> Reencoded nucleic acid sequence of UDP-Glucose dehydrogenase
(HASB) originating from Chlorella virus PBCV-1

<400> 13
atgagtagaa ttgctgtcgt tggatgcggt tacgtgggta cggcctgcgc cgtacttttg 60

gcgcagaaga acgaagttat cgttttggat atctcggagg accgggtaca actgattaag 120

aataagaagt cacctataga agataaggaa atcgaagaat tcctggagac gaaagatttg 180

aatctaacag cgacgacgga taaggtgctc gcctatgaga atgctgaatt cgttataata 240

gctacaccga ccgattacga tgtcgtcact agatatttca acactaagtc tgttgaaaat 300

gtgataggcg atgtcattaa gaacactcag acacacccta ctatagtgat caagagtact 360

attccaatcg gtttcgtgga taaagttaga gaacagttcg attatcaaaa tattatcttc 420

tcgccggaat tcttgagaga aggacgagca ttatatgata atctttaccc ctcccgtatc 480

atcgtcggtg atgactcccc aattgcctta aaattcgcga atctcttggt cgagggtagc 540

aaaactccac tagctcccgt attaactatg ggtacgcgag aggccgaagc tgtaaaacta 600

ttttcaaata catatttggc tatgagagta gcatacttca atgagctaga cacatttgca 660

atgtcccatg gtatgaacgc aaaagagatt atcgacggag tcacactaga accaaggata 720

gggcagggtt attcgaatcc ttcatttggg tatggagcct attgtttccc aaaagataca 780

aagcaattgt tggctaattt tgaaggggtt ccacaagata ttattggcgc aatcgtagag 840

tctaacgaaa ctagaaagga ggttattgtt tctgaggtcg agaacagatt tccgaccaca 900

gttggcgtat ataagttggc tgctaaagct ggttcggata atttcaggtc tagtgcgata 960

gtcgatatca tggaaaggtt ggctaataag ggctaccaca tcaaaatatt tgaacctacc 1020

gttgaacaat ttgagaattt tgaggtagat aataatctca caacttttgc aaccgagtct 1080

gatgtaataa ttgcgaacag agttccagtc gaacatcgca ttctgtttgg gaagaagtta 1140

ataacacggg atgtctatgg ggataa 1166

<210> 14
<211> 1170
<212> DNA
<213> Paramecium bursaria Chlorella virus 1

<220>
<223> Reencoded nucleic acid sequence of UDP-Glucose dehydrogenase
      (HASB) originating from Chlorella virus PBCV-1

<400> 14
atgtcacgaa tcgcagtagt tggctgtggg tacgtgggaa ccgcatgcgc tgtactgctg 60

gcgcaaaaga atgaggtaat cgttcttgat attagtgagg atagggtcca actaatcaag 120

aacaagaaat ccccgatcga agataaggag attgaagaat tcttggaaac aaaagaccta 180

aacttaactg caacaactga caaagtttta gcctacgaaa acgctgagtt tgtgattata 240

gcgacaccca cagattatga cgtagttacc agatacttta acacgaagtc cgtagagaac 300

gtcattggag atgttataaa gaatactcag actcatccta cgatagtaat aaagtcaacc 360

attcccatag gtttcgtaga taaggttagg gagcaattcg attaccagaa cattatattt 420

tcgccagaat ttctgagaga gggtcgcgcc ctgtatgata atctatatcc atcacggatt 480

atagtgggcg atgactctcc gattgcactt aagtttgcta atcttttagt tgagggctcc 540

aaaactccgc tcgccccagt acttacgatg ggtacacgtg aggctgaagc tgtcaagctg 600

ttttcaaaca cataccttgc tatgcgagtc gcatacttta acgaactaga tacctttgct 660

atgtcgcacg gtatgaatgc taaagaaatc atagatggcg taacgttgga gcctcggata 720

ggtcaaggat attccaatcc atcttttggc tacggtgcgt attgtttccc aaaggacacg 780

aagcaattat tagctaactt tgagggtgtt ccgcaagata taattggggc gatagtagaa 840

agcaatgaaa cacggaagga ggtaatcgtg agtgaagtgg aaaaccgatt ccccactacg 900

gtcggcgttt acaaattagc cgccaaggct ggttccgaca atttccgatc cagcgcaata 960

gtagatatta tggaaagatt agctaataag ggataccaca ttaaaatctt tgaacctact 1020

gtcgaacagt tcgagaactt cgaggttgat aataacttga cgactttcgc aacggagagc 1080

gatgtaatta ttgcaaaccg cgtacctgtg gaacatcgaa ttttgttcgg aaagaagctg 1140

attacacgcg atgtatatgg cgataactaa 1170

<210> 15
<211> 1199
<212> DNA
<213> streptococcus zooepidemicus

<220>
<223> Reencoded nucleic acid sequence of UDP-Glucose dehydrogenase
      (HASB) originating from Streptococcus zooepidemicus

<400> 15
atgaagatat cggtagcggg ttcggggtac gtggggttat ccttgtcaat cttgcttgcc 60

caacataacg atgtgactgt tgtagatata atcgacgaaa aggtacggct aattaaccag 120

ggcatatctc cgattaagga tgcggacatt gaggaatatc tgaagaatgc accgttgaat 180

cttacggcta cactagacgg agcttcggct tatagtaatg ctgatctgat tataatcgca 240

acgccaacta attacgattc agaacgcaat tatttcgaca ccagacacgt tgaagaagta 300

attgagcaag tattggattt aaatgcctcc gctactataa tcatcaagag taccataccc 360

ttgggtttta ttaaacacgt aagagagaaa taccaaacag acagaatcat cttttctcca 420

gagttcttaa gagagtcaaa ggcattgtac gataacttat acccctctcg tataatagtc 480

agttatgaga aggatgactc tccaagagtt atacaagcag ctaaggcgtt cgcgggttta 540

ttaaaagagg gggcaaagag caaggatacc ccagttctgt ttatgggctc tcaagaagct 600

gaagctgtca agctgtttgc taatactttt ctcgccatga gggtcagtta cttcaacgag 660

cttgacactt atagcgaatc aaaaggacta gacgcccaaa gagttataga aggcgtctgc 720

catgatcaaa ggataggtaa tcattacaat aatccatcct tcggatatgg cggttattgt 780

ttacccaaag actcaaagca acttttggct aattatagag gcatacctca gtctctaatg 840

tctgccatcg ttgaatcgaa caagatccgt aagtcgtatt tagctgaaca aatattagat 900

agggcttctt cacaaaagca ggctggtgta cctttaacca taggatttta ccgtttgatt 960

atgaagtcca actccgataa ctttagagaa tcagccatta aagatattat tgacatcatt 1020

aatgactacg gtgtcaatat tgtcatttat gaacctatgt tgggagaaga cattggttat    1080

agagtcgtta aagatttgga acagtttaag aacgaaagta caattattgt tagtaacagg    1140

tttgaagatg atttaggtga tgttattgat aaagtttata cacgtgacgt ctttggtag     1199

<210> 16
<211> 481
<212> PRT
<213> Arabidopsis thaliana

<220>
<223> Amino acid sequence of UDP-Glucose dehydrogenase (HASB)
      originating from Arabidopsis thaliana

<400> 16
Met Val Lys Ile Cys Cys Ile Gly Ala Gly Tyr Val Gly Gly Pro Thr
1               5                   10                  15
Met Ala Val Met Ala Leu Lys Cys Pro Glu Ile Glu Val Val Val Val
            20                  25                  30
Asp Ile Ser Glu Pro Arg Ile Asn Ala Trp Asn Ser Asp Arg Leu Pro
        35                  40                  45
Ile Tyr Glu Pro Gly Leu Glu Asp Val Val Lys Gln Cys Arg Gly Lys
    50                  55                  60
Asn Leu Phe Phe Ser Thr Asp Val Glu Lys His Val Phe Glu Ser Asp
65                  70                  75                  80
Ile Val Phe Val Ser Val Asn Thr Pro Thr Lys Thr Gln Gly Leu Gly
                85                  90                  95
Ala Gly Lys Ala Ala Asp Leu Thr Tyr Trp Glu Ser Ala Ala Arg Met
            100                 105                 110
Ile Ala Asp Val Ser Lys Ser Ser Lys Ile Val Val Glu Lys Ser Thr
        115                 120                 125
Val Pro Val Arg Thr Ala Glu Ala Ile Glu Lys Ile Leu Thr His Asn
    130                 135                 140
Ser Lys Gly Ile Glu Phe Gln Ile Leu Ser Asn Pro Glu Phe Leu Ala
145                 150                 155                 160
Glu Gly Thr Ala Ile Lys Asp Leu Tyr Asn Pro Asp Arg Val Leu Ile
                165                 170                 175
Gly Gly Arg Asp Thr Ala Ala Gly Gln Lys Ala Ile Lys Ala Leu Arg
            180                 185                 190
Asp Val Tyr Ala His Trp Val Pro Val Glu Gln Ile Ile Cys Thr Asn
        195                 200                 205
Leu Trp Ser Ala Glu Leu Ser Lys Leu Ala Ala Asn Ala Phe Leu Ala
    210                 215                 220
Gln Arg Ile Ser Ser Val Asn Ala Met Ser Ala Leu Cys Glu Ala Thr
225                 230                 235                 240
Gly Ala Asp Val Thr Gln Val Ala His Ala Val Gly Thr Asp Thr Arg
                245                 250                 255
Ile Gly Pro Lys Phe Leu Asn Ala Ser Val Gly Phe Gly Gly Ser Cys
            260                 265                 270
Phe Gln Lys Asp Ile Leu Asn Leu Ile Tyr Ile Cys Glu Cys Asn Gly
        275                 280                 285
Leu Pro Glu Ala Ala Asn Tyr Trp Lys Gln Val Val Lys Val Asn Asp
    290                 295                 300
Tyr Gln Lys Ile Arg Phe Ala Asn Arg Val Val Ser Ser Met Phe Asn
305                 310                 315                 320
Thr Val Ser Gly Lys Lys Ile Ala Ile Leu Gly Phe Ala Phe Lys Lys
                325                 330                 335
Asp Thr Gly Asp Thr Arg Glu Thr Pro Ala Ile Asp Val Cys Asn Arg
            340                 345                 350

Leu Val Ala Asp Lys Ala Lys Leu Ser Ile Tyr Asp Pro Gln Val Leu
        355                 360                 365
Glu Glu Gln Ile Arg Arg Asp Leu Ser Met Ala Arg Phe Asp Trp Asp
    370                 375                 380
His Pro Val Pro Leu Gln Gln Ile Lys Ala Glu Gly Ile Ser Glu Gln
385                 390                 395                 400
Val Asn Val Val Ser Asp Ala Tyr Glu Ala Thr Lys Asp Ala His Gly
                405                 410                 415
Leu Cys Val Leu Thr Glu Trp Asp Glu Phe Lys Ser Leu Asp Phe Lys
            420                 425                 430
Lys Ile Phe Asp Asn Met Gln Lys Pro Ala Phe Val Phe Asp Gly Arg
        435                 440                 445
Asn Val Val Asp Ala Val Lys Leu Arg Glu Ile Gly Phe Ile Val Tyr
    450                 455                 460
Ser Ile Gly Lys Pro Leu Asp Ser Trp Leu Lys Asp Met Pro Ala Val
465                 470                 475                 480
Ala

<210> 17
<211> 389
<212> PRT
<213> Paramecium bursaria Chlorella virus 1

<220>
<223> Amino acid sequence of UDP-Glucose dehydrogenase (HASB)
      originating from Chlorella virus PBCV1

<400> 17
Met Ser Arg Ile Ala Val Val Gly Cys Gly Tyr Val Gly Thr Ala Cys
1               5                   10                  15
Ala Val Leu Leu Ala Gln Lys Asn Glu Val Ile Val Leu Asp Ile Ser
            20                  25                  30
Glu Asp Arg Val Gln Leu Ile Lys Asn Lys Lys Ser Pro Ile Glu Asp
        35                  40                  45
Lys Glu Ile Glu Glu Phe Leu Glu Thr Lys Asp Leu Asn Leu Thr Ala
    50                  55                  60
Thr Thr Asp Lys Val Leu Ala Tyr Glu Asn Ala Glu Phe Val Ile Ile
65                  70                  75                  80
Ala Thr Pro Thr Asp Tyr Asp Val Val Thr Arg Tyr Phe Asn Thr Lys
                85                  90                  95
Ser Val Glu Asn Val Ile Gly Asp Val Ile Lys Asn Thr Gln Thr His
            100                 105                 110
Pro Thr Ile Val Ile Lys Ser Thr Ile Pro Ile Gly Phe Val Asp Lys
        115                 120                 125
Val Arg Glu Gln Phe Asp Tyr Gln Asn Ile Ile Phe Ser Pro Glu Phe
    130                 135                 140
Leu Arg Glu Gly Arg Ala Leu Tyr Asp Asn Leu Tyr Pro Ser Arg Ile
145                 150                 155                 160
Ile Val Gly Asp Asp Ser Pro Ile Ala Leu Lys Phe Ala Asn Leu Leu
                165                 170                 175
Val Glu Gly Ser Lys Thr Pro Leu Ala Pro Val Leu Thr Met Gly Thr
            180                 185                 190
Arg Glu Ala Glu Ala Val Lys Leu Phe Ser Asn Thr Tyr Leu Ala Met
        195                 200                 205
Arg Val Ala Tyr Phe Asn Glu Leu Asp Thr Phe Ala Met Ser His Gly
    210                 215                 220
Met Asn Ala Lys Glu Ile Ile Asp Gly Val Thr Leu Glu Pro Arg Ile
225                 230                 235                 240
Gly Gln Gly Tyr Ser Asn Pro Ser Phe Gly Tyr Gly Ala Tyr Cys Phe
                245                 250                 255
Pro Lys Asp Thr Lys Gln Leu Leu Ala Asn Phe Glu Gly Val Pro Gln

260                 265                 270
Asp Ile Ile Gly Ala Ile Val Glu Ser Asn Glu Thr Arg Lys Glu Val
        275                 280                 285
Ile Val Ser Glu Val Glu Asn Arg Phe Pro Thr Thr Val Gly Val Tyr
    290                 295                 300
Lys Leu Ala Ala Lys Ala Gly Ser Asp Asn Phe Arg Ser Ser Ala Ile
305                 310                 315                 320
Val Asp Ile Met Glu Arg Leu Ala Asn Lys Gly Tyr His Ile Lys Ile
                325                 330                 335
Phe Glu Pro Thr Val Glu Gln Phe Glu Asn Phe Glu Val Asp Asn Asn
            340                 345                 350
Leu Thr Thr Phe Ala Thr Glu Ser Asp Val Ile Ile Ala Asn Arg Val
        355                 360                 365
Pro Val Glu His Arg Ile Leu Phe Gly Lys Lys Leu Ile Thr Arg Asp
    370                 375                 380
Val Tyr Gly Asp Asn
385

<210> 18
<211> 401
<212> PRT
<213> Streptococcus zooepidemicus


<220>
<223> Amino acid sequence of UDP-Glucose dehydrogenase (HASB)
      originating from Streptococcus zooepidemicus

<400> 18
Met Lys Ile Ser Val Ala Gly Ser Gly Tyr Val Gly Leu Ser Leu Ser
1               5                   10                  15
Ile Leu Leu Ala Gln His Asn Asp Val Thr Val Val Asp Ile Ile Asp
            20                  25                  30
Glu Lys Val Arg Leu Ile Asn Gln Gly Ile Ser Pro Ile Lys Asp Ala
        35                  40                  45
Asp Ile Glu Glu Tyr Leu Lys Asn Ala Pro Leu Asn Leu Thr Ala Thr
    50                  55                  60
Leu Asp Gly Ala Ser Ala Tyr Ser Asn Ala Asp Leu Ile Ile Ile Ala
65                  70                  75                  80
Thr Pro Thr Asn Tyr Asp Ser Glu Arg Asn Tyr Phe Asp Thr Arg His
                85                  90                  95
Val Glu Glu Val Ile Glu Gln Val Leu Asp Leu Asn Ala Ser Ala Thr
            100                 105                 110
Ile Ile Ile Lys Ser Thr Ile Pro Leu Gly Phe Ile Lys His Val Arg
        115                 120                 125
Glu Lys Tyr Gln Thr Asp Arg Ile Ile Phe Ser Pro Glu Phe Leu Arg
    130                 135                 140
Glu Ser Lys Ala Leu Tyr Asp Asn Leu Tyr Pro Ser Arg Ile Ile Val
145                 150                 155                 160
Ser Tyr Glu Lys Asp Asp Ser Pro Arg Val Ile Gln Ala Ala Lys Ala
                165                 170                 175
Phe Ala Gly Leu Leu Lys Glu Gly Ala Lys Ser Lys Asp Thr Pro Val
            180                 185                 190
Leu Phe Met Gly Ser Gln Glu Ala Glu Ala Val Lys Leu Phe Ala Asn
        195                 200                 205
Thr Phe Leu Ala Met Arg Val Ser Tyr Phe Asn Glu Leu Asp Thr Tyr
    210                 215                 220
Ser Glu Ser Lys Gly Leu Asp Ala Gln Arg Val Ile Glu Gly Val Cys
225                 230                 235                 240
His Asp Gln Arg Ile Gly Asn His Tyr Asn Asn Pro Ser Phe Gly Tyr
                245                 250                 255
Gly Gly Tyr Cys Leu Pro Lys Asp Ser Lys Gln Leu Leu Ala Asn Tyr
            260                 265                 270

Arg Gly Ile Pro Gln Ser Leu Met Ser Ala Ile Val Glu Ser Asn Lys
        275                 280                 285
Ile Arg Lys Ser Tyr Leu Ala Glu Gln Ile Leu Asp Arg Ala Ser Ser
    290                 295                 300
Gln Lys Gln Ala Gly Val Pro Leu Thr Ile Gly Phe Tyr Arg Leu Ile
305                 310                 315                 320
Met Lys Ser Asn Ser Asp Asn Phe Arg Glu Ser Ala Ile Lys Asp Ile
                325                 330                 335
Ile Asp Ile Ile Asn Asp Tyr Gly Val Asn Ile Val Ile Tyr Glu Pro
            340                 345                 350
Met Leu Gly Glu Asp Ile Gly Tyr Arg Val Val Lys Asp Leu Glu Gln
        355                 360                 365
Phe Lys Asn Glu Ser Thr Ile Ile Val Ser Asn Arg Phe Glu Asp Asp
    370                 375                 380
Leu Gly Asp Val Ile Asp Lys Val Tyr Thr Arg Asp Val Phe Gly Arg
385                 390                 395                 400
Asp

<210> 19
<211> 1326
<212> DNA
<213> Bothrops atrox

<220>
<223> Reencoded nucleic acid sequence of hyaluronidase (HYAL)
      originating from Bothrops atrox with a N-terminal secretion
      signal

<400> 19
atgcaattta gcacagtcgc atcagtagcc ttcgttgcct tggccaactt cgtggcagca      60

ccgatgtatc cgaacgaacc gttcttagtc ttttggaacg cgcctacaac tcagtgtaga     120

cttcgatata aggttgacct tgatctgaag acattccata tcgtgacaaa tgctaatgac     180

tcgctgtcag gatcggctgt cacgattttc tatcccacgc acttaggggt ttacccacat     240

attgatgaca gggggcactt cttcaatggc atcatacccc aaaatgaatc cctggtaaag     300

catttaaaca aatctaaatc agatattaat cgaatgattc ccttaagaac attccacggg     360

ctgggagtca tagactggga aaactggcgg ccacagtggg ataggaattg ggaagtaaag     420

aacgtttata ggaatagatc aatccaattc gcgcgtgatc tccacccaga gcttagtgag     480

gacaagatta aacgcttggc aaaacaggaa ttcgagaaag ctgcaaagag ttttatgagg     540

gatacactat tattagccga ggagatgcga ccagacggct actggggata ctatctgtac     600

cccgattgtc acaattacaa ttataagact aagccagatc agtacacagg agagtgccct     660

gacatcgaga tgtcacgtaa caatcaactc ttgtggcttt ggcgggatag cactgccctt     720

ttccccaata tctacttaga gactatacta agaagctctg acaatgccct gaagtttgtg     780

caccataggc tcaaggaggc aatgaggata gcctcaatgg ctcgaaatga ctacgcgttg     840

cctttctttg tttatgctcg accattctat gcctatacct tcgaaccatt gactcaggaa     900

gaccttgtga ctacggtcgg agagaccgcg gacatgggcg ccgctgggat cgtattttgg     960

gggagtatgc agtacgcaag cacggttgaa tcttgcggca aagtcaagga ctacatgaat 1020

ggcccactgg ggcgttacat tgtgaatgtt acaactgccg ccaaaatttg ctcacgattc 1080

ttgtgcaaac gtcatggtag gtgtgtaaga aagcactcag actccaatgc attccttcac 1140

ctatttcccg attcgtttcg cataatggtg cacgggaatg caaccgagaa gaaagttata 1200

gtaaagggga agctggaatt aaagaatctt atattcttac gtaataactt tatgtgccag 1260

tgttatcaag gctggaaagg tctatattgc gagaagcatt cgataaagga aattcggaaa 1320

atctaa 1326

<210> 20
<211> 1537
<212> DNA
<213> Bothrops atrox

<220>
<223> Reencoded nucleic acid sequence of hyaluronidase (HYAL)
      originating from Bothrops atrox with a N-terminal secretion
      signal and a C-terminal anchoring signal

<400> 20
atgcaattta gcacagtcgc atcagtagcc ttcgttgcct tggccaactt cgtggcagca 60

ccgatgtatc cgaacgaacc gttcttagtc ttttggaacg cgcctacaac tcagtgtaga 120

cttcgatata aggttgacct tgatctgaag acattccata tcgtgacaaa tgctaatgac 180

tcgctgtcag gatcggctgt cacgattttc tatcccacgc acttaggggt ttacccacat 240

attgatgaca gggggcactt cttcaatggc atcatacccc aaaatgaatc cctggtaaag 300

catttaaaca aatctaaatc agatattaat cgaatgattc ccttaagaac attccacggg 360

ctgggagtca tagactggga aaactggcgg ccacagtggg ataggaattg gggaagtaag 420

aacgtttata ggaatagatc aatccaattc gcgcgtgatc tccacccaga gcttagtgag 480

gacaagatta aacgcttggc aaaacaggaa ttcgagaaag ctgcaaagag ttttatgagg 540

gatacactat tattagccga ggagatgcga ccagacggct actggggata ctatctgtac 600

cccgattgtc acaattacaa ttataagact aagccagatc agtacacagg agagtgccct 660

gacatcgaga tgtcacgtaa caatcaactc ttgtggcttt ggcgggatag cactgccctt 720

ttccccaata tctacttaga gactatacta agaagctctg acaatgccct gaagtttgtg 780

caccataggc tcaaggaggc aatgaggata gcctcaatgg ctcgaaatga ctacgcgttg 840

cctttctttg tttatgctcg accattctat gcctatacct tcgaaccatt gactcaggaa 900

gaccttgtga ctacggtcgg agagaccgcg gacatgggcg ccgctgggat cgtattttgg 960

gggagtatgc agtacgcaag cacggttgaa tcttgcggca aagtcaagga ctacatgaat 1020

ggcccactgg ggcgttacat tgtgaatgtt acaactgccg ccaaaatttg ctcacgattc 1080

ttgtgcaaac gtcatggtag gtgtgtaaga aagcactcag actccaatgc attccttcac 1140

ctatttcccg attcgtttcg cataatggtg cacgggaatg caaccgagaa gaaagttata 1200

gtaaagggga agctggaatt aaagaatctt atattcttac gtaataactt tatgtgccag 1260

tgttatcaag gctggaaagg tctatattgc gagaagcatt cgataaagga aattcggaaa 1320

atcggatccg ccatttctca aatcactgac ggtcaaatcc aagctactac cactgctacc 1380

accgaagcta ccaccactgc tgccccatct tccaccgttg aaactgtttc tccatccagc 1440

accgaaacta tctctcaaca aactgaaaat ggtgctgcta aggccgctgt cggtatgggt 1500

gccggtgctc tagctgctgc tgctatgttg ttataaa 1537

<210> 21
<211> 1197
<212> DNA
<213> Cupiennius salei

<220>
<223> Reencoded nucleic acid sequence of hyaluronidase (HYAL)
      originating from Cupiennius salei with a N-terminal secretion
      signal

<400> 21
atgcaattca gcactgttgc atcagttgca tttgtcgccc tggcgaattt tgtagccgcg 60

ttcaagattt actggaacgt cccaactttt cagtgcacgc ataactacaa aatcgattat 120

gtcaaattgt tgtccactta cgggatacag gtcaatgatg gcggtaagtt tcaaggaaac 180

caagtgacta tcttttatga aacccagttg ggtttgtatc cacgaatcct aaaatctggt 240

aaaatggaaa acggcggaat ccctcaacgc ggtaactttg agaaacacct agaaaaggca 300

agcacggacc tccagaaagt gatcccttgg aaagagttta gcggattagg tgtgatagat 360

tgggaggctt ggagacccac atgggaattt aactgggaac cgttgaggat atatcaaacc 420

gaatcaatta agagagctaa agaactacac cctaccgcaa acgattccgc agtaaaagaa 480

attgcagagc ggcaatggga agattcagcc aagttataca tgttagaaac actgcggctg 540

gcaaagaaac ttcgacctca agcgccttgg tgttactact tatttcctga ttgctataat 600

tacgtcggaa agaaaccaaa agatttccaa tgtagtgcct cgatacgtaa aggtaacgat 660

aagctaagct ggttgtggaa agattctacg gcattgtgtc catcgatata cgtatatgaa 720

tcacaattag acaggtattc ttttgaacaa aggacatggc gcgacaatga gaaacttcgg 780

gaagcgttgc gtgtagccac gagaacctct aaaatatacc catacgttaa ctatttcgat 840

aaggagctta taccggagca agaagtatgg agaatgcttg cgcaggcagc tgctgtcggt 900

ggcagtggtg cggtaatttg ggctcatctg gctgcagttg catctgaaga gttatgtaaa 960

tctttaaaac agtatattat tgaaacgctt gggccggcgg cagagaaggt ggcttggcgt 1020

agtgacttat gcagcaaaga aatttgtaat aatcagggtc gctgcacatt cccggacgat 1080

gattatgcaa acgcatggaa attatttaca gatgatactg ttaagtttta tgctggtaat 1140

attacatgta ggtgctccga gaattattct ggtcgtttct gcgaaaagaa gaattaa 1197

<210> 22
<211> 1407
<212> DNA
<213> Cupiennius salei

<220>
<223> Reencoded nucleic acid sequence of hyaluronidase (HYAL)
      originating from Cupiennius salei with a N-terminal secretion
      signal and a C-terminal anchoring signal

<400> 22
atgcaattca gcactgttgc atcagttgca tttgtcgccc tggcgaattt tgtagccgcg 60

ttcaagattt actggaacgt cccaactttt cagtgcacgc ataactacaa aatcgattat 120

gtcaaattgt tgtccactta cgggatacag gtcaatgatg gcggtaagtt tcaaggaaac 180

caagtgacta tcttttatga aacccagttg ggtttgtatc cacgaatcct aaaatctggt 240

aaaatggaaa acggcggaat ccctcaacgc ggtaactttg agaaacacct agaaaaggca 300

agcacggacc tccagaaagt gatcccttgg aaagagttta gcggattagg tgtgatagat 360

tgggaggctt ggagacccac atgggaattt aactgggaac cgttgaggat atatcaaacc 420

gaatcaatta agagagctaa agaactacac cctaccgcaa acgattccgc agtaaaagaa 480

attgcagagc ggcaatggga agattcagcc aagttataca tgttagaaac actgcggctg 540

gcaaagaaac ttcgacctca agcgccttgg tgttactact tatttcctga ttgctataat 600

tacgtcggaa agaaaccaaa agatttccaa tgtagtgcct cgatacgtaa aggtaacgat 660

aagctaagct ggttgtggaa agattctacg gcattgtgtc catcgatata cgtatatgaa 720

tcacaattag acaggtattc ttttgaacaa aggacatggc gcgacaatga gaaacttcgg 780

gaagcgttgc gtgtagccac gagaacctct aaaatatacc catacgttaa ctatttcgat 840

aaggagctta taccggagca agaagtatgg agaatgcttg cgcaggcagc tgctgtcggt 900

ggcagtggtg cggtaatttg gggctcatct gctgcagttg catctgaaga gttatgtaaa 960

tctttaaaac agtatattat tgaaacgctt gggccggcgg cagagaaggt ggcttggcgt 1020

agtgacttat gcagcaaaga aatttgtaat aatcagggtc gctgcacatt cccggacgat 1080

gattatgcaa acgcatggaa attatttaca gatgatactg ttaagtttta tgctggtaat 1140

attacatgta ggtgctccga gaattattct ggtcgtttct gcgaaaagaa gaatggatcc 1200

gccatttctc aaatcactga cggtcaaatc caagctacta ccactgctac caccgaagct 1260

accaccactg ctgccccatc ttccaccgtt gaaactgttt ctccatccag caccgaaact 1320

atctctcaac aaactgaaaa tggtgctgct aaggccgctg tcggtatggg tgccggtgct 1380

ctagctgctg ctgctatgtt gttataa 1407

<210> 23
<211> 1530
<212> DNA
<213> Hirudo nipponia

<220>
<223> Reencoded nucleic acid sequence of hyaluronidase (HYAL)
      originating from Hirudo Nipponia with a N-terminal secretion
      signal

<400> 23
atgcaattct ctactgtcgc ttccgttgct ttcgtcgctt tggctaactt tgttgccgct 60

atgaaggaga tagcagttac catcgatgat aagaatgtta tagcgtctgt cagtgaaagc 120

ttccatggtg tggctttcga cgcaagccta ttctcaccta aagggctatg gtcgtttgtt 180

gacattactt cacccaagtt attcaagctt ctggagggac tctcccctgg ttatttccgt 240

gtcggcggca catttgcgaa ctggttattc ttcgacctcg atgaaaacaa caagtggaag 300

gattactggg cttttaagga caagactcca gagaccgcca cgattacccg acggtggctc 360

ttcaggaagc aaaacaacct caagaaggaa acctttgatg atttggtaaa gcttacaaag 420

gggtccaaga tgcggctgct atttgatcta aacgcagagg tccgtacagg atatgagata 480

ggcaagaaga tgacatcaac ttgggactca agcgaggcag aaaagttgtt caaatattgc 540

gttagcaagg gatatggaga taatatcgat tgggagttag gcaacgaacc tgatcacacg 600

tcagcacaca atttgacaga gaagcaagta ggtgaagatt ttaaggccct acacaaggtg 660

ttggaaaagt atccaacact taataaaggt agcttggttg gtccagacgt tgggtggatg 720

ggagtgtcgt acgtcaaggg tctggctgac ggggctggag atcatgtgac cgcttttact 780

ctacatcagt attatttcga tggaaatacg agtgatgtta gtacctactt ggatgcgacg 840

tactttaaga agttgcaaca gttattcgat aaggtaaaag acgtactcaa gaattctcca 900

cataaggaca aacccttgtg gctaggggaa acctcttccg gctacaacag tgggactaaa 960

gatgtatccg atagatacgt gtcggggttc ttgacgctgg acaagttggg gctttcggcg 1020

gcaaataacg tcaaggtggt catcagacaa acgatttata atggttacta tggtttgctt 1080

gacaagaata ctttggagcc caatccggac tactggctga tgcacgtcca caattcctta 1140

gttggtaata ccgttttcaa ggtcgatgtc agcgatccca caaacaaagc ccgtgtttac 1200

gctcagtgta cgaagaccaa tagtaaacat acccagtcac gttattataa ggggtcccta 1260

accattttcg cgttgaatgt aggtgatgaa gatgttacac tcaagattga tcaatacagt 1320

ggtaagaaaa tatactcata tattctgacg cctgaaggcg gccagttaac ctctcaaaag 1380

gtactgctta acggtaaaga attgaagtta gtgtcagatc aacttccaga acttaacgcg 1440

gacgaaagta aaacatcctt cacattgtct cccaaaactt ttggattctt tgtggtctcg 1500

gatgccaacg ttgaagcttg caagaaataa 1530

<210> 24
<211> 1740
<212> DNA
<213> Hirudo nipponia

<220>
<223> Reencoded nucleic acid sequence of hyaluronidase (HYAL)
      originating from Hirudo Nipponia with a N-terminal secretion
      signal and a C-terminal anchoring signal

<400> 24
atgcaattct ctactgtcgc ttccgttgct ttcgtcgctt tggctaactt tgttgccgct 60

atgaaggaga tagcagttac catcgatgat aagaatgtta tagcgtctgt cagtgaaagc 120

ttccatggtg tggctttcga cgcaagccta ttctcaccta aagggctatg gtcgtttgtt 180

gacattactt cacccaagtt attcaagctt ctggagggac tctcccctgg ttatttccgt 240

gtcggcggca catttgcgaa ctggttattc ttcgacctcg atgaaaacaa caagtggaag 300

gattactggg cttttaagga caagactcca gagaccgcca cgattacccg acggtggctc 360

ttcaggaagc aaaacaacct caagaaggaa acctttgatg atttggtaaa gcttacaaag 420

gggtccaaga tgcggctgct atttgatcta aacgcagagg tccgtacagg atatgagata 480

ggcaagaaga tgacatcaac ttgggactca agcgaggcag aaaagttgtt caaatattgc 540

gttagcaagg gatatggaga taatatcgat tgggagttag gcaacgaacc tgatcacacg 600

tcagcacaca atttgacaga gaagcaagta ggtgaagatt ttaaggccct acacaaggtg 660

ttggaaaagt atccaacact taataaaggt agcttggttg gtccagacgt tgggtggatg 720

ggagtgtcgt acgtcaaggg tctggctgac ggggctggag atcatgtgac cgcttttact 780

ctacatcagt attatttcga tggaaatacg agtgatgtta gtacctactt ggatgcgacg 840

tactttaaga agttgcaaca gttattcgat aaggtaaaag acgtactcaa gaattctcca 900

cataaggaca aacccttgtg gctaggggaa acctcttccg gctacaacag tgggactaaa 960

gatgtatccg atagatacgt gtcggggttc ttgacgctgg acaagttggg gctttcggcg 1020

gcaaataacg tcaaggtggt catcagacaa acgatttata atggttacta tggtttgctt 1080

gacaagaata ctttggagcc caatccggac tactggctga tgcacgtcca caattcctta 1140

gttggtaata ccgttttcaa ggtcgatgtc agcgatccca caaacaaagc ccgtgtttac 1200

gctcagtgta cgaagaccaa tagtaaacat acccagtcac gttattataa ggggtcccta 1260

accattttcg cgttgaatgt aggtgatgaa gatgttacac tcaagattga tcaatacagt 1320

ggtaagaaaa tatactcata tattctgacg cctgaaggcg gccagttaac ctctcaaaag 1380

gtactgctta acggtaaaga attgaagtta gtgtcagatc aacttccaga acttaacgcg 1440

gacgaaagta aaacatcctt cacattgtct cccaaaactt ttggattctt tgtggtctcg 1500

gatgccaacg ttgaagcttg caagaaagga tccgccattt ctcaaatcac tgacggtcaa 1560

atccaagcta ctaccactgc taccaccgaa gctaccacca ctgctgcccc atcttccacc 1620

gttgaaactg tttctccatc cagcaccgaa actatctctc aacaaactga aaatggtgct 1680

gctaaggccg ctgtcggtat gggtgccggt gctctagctg ctgctgctat gttgttataa 1740

<210> 25
<211> 1206
<212> DNA
<213> Loxosceles intermedia

<220>
<223> Reencoded nucleic acid sequence of hyaluronidase (HYAL)
      originating from Loxosceles intermedia with a N-terminal
      secretion signal

<400> 25
atgcagtttt caaccgttgc gagcgtcgct tttgtggctc ttgctaattt cgttgccgcc 60

ttcgatgtct tctggaatgt tccgtcgcag caatgcaaga aatatggtat gaaattcgtt 120

ccgctcttag agcaatactc tatcctagtc aacaaagaag acaatttcaa gggcgacaaa 180

ataacgatct tttatgagtc acagctcggg ctttacccac atattggtgc aaacgacgag 240

tcgtttaatg gcgggatacc acaattaggt gacctgaaag cacacttaga aaagtcagcg 300

gttgatatac gacgtgatat tttggataag tcggcgactg gtctaagaat tatagactgg 360

gaagcatgga gaccaatatg ggaattcaac tggtctagcc tacgaaagta ccaagataaa 420

atgaagaaag tcgtccgcca gtttaacccg actgctcatg aatccacagt ggccaaacta 480

gcacataatg agtgggaaaa tagtagtaag tcttggatgc tttctacatt gcagttgggt 540

aagcaacttc gtccaaactc tgtatggtgc tattatttat ttcccgactg ttataactat 600

gatggcaact cagtccaaga atttcagtgt tctgaagcta tccgtaaggg gaacgatagg 660

ttgaaatggt tgtgggaaga atcgacagct gtatgcccat ctatctacat aaaagaaggc 720

caactgacca attatacctt gcaaaagaga atctggttta ccaatgggag attacaggaa 780

gccttgagag tagctcaacc taaagcgcgt atttatccat acataaatta ctccatcaaa 840

cccggaatga tggtgcctga agttgagttt tggcggttaa tcgctcagat agcctcgctg 900

ggtatggatg gagcagtgat ttggggatca agtgcgagtg taggcagtaa gaatcattgt 960

gcgcaattaa tgaagtacat tgcagacgta ttgggtcctg caactttgcg cataaaagaa 1020

aatgtagcac ggtgcagtaa acaggcgtgc tctggtaggg gtagatgtac ctggcctaaa 1080

gatacctctg ttattgcttg gaagttcctc gttgaaaagg aagactatga cttttatctg 1140

ggtgatatag agtgtaaatg tgttgaaggc tacgaaggta ggtactgtga acaaaagact 1200

aagtaa 1206

<210> 26
<211> 1416
<212> DNA
<213> Loxosceles intermedia

<220>
<223> Reencoded nucleic acid sequence of hyaluronidase (HYAL)
      originating from Loxosceles intermedia with a N-terminal
      secretion signal and a C-terminal anchoring signal

<400> 26
atgcagtttt caaccgttgc gagcgtcgct tttgtggctc ttgctaattt cgttgccgcc 60

ttcgatgtct tctggaatgt tccgtcgcag caatgcaaga aatatggtat gaaattcgtt 120

ccgctcttag agcaatactc tatcctagtc aacaaagaag acaatttcaa gggcgacaaa 180

ataacgatct tttatgagtc acagctcggg ctttacccac atattggtgc aaacgacgag 240

tcgtttaatg gcgggatacc acaattaggt gacctgaaag cacacttaga aaagtcagcg 300

gttgatatac gacgtgatat tttggataag tcggcgactg gtctaagaat tatagactgg 360

gaagcatgga gaccaatatg ggaattcaac tggtctagcc tacgaaagta ccaagataaa 420

atgaagaaag tcgtccgcca gtttaacccg actgctcatg aatccacagt ggccaaacta 480

gcacataatg agtgggaaaa tagtagtaag tcttggatgc tttctacatt gcagttgggt 540

aagcaacttc gtccaaactc tgtatggtgc tattatttat ttcccgactg ttataactat 600

gatggcaact cagtccaaga atttcagtgt tctgaagcta tccgtaaggg gaacgatagg 660

ttgaaatggt tgtgggaaga atcgacagct gtatgcccat ctatctacat aaaagaaggc 720

caactgacca attatacctt gcaaaagaga atctggttta ccaatgggag attacaggaa 780

gccttgagag tagctcaacc taaagcgcgt atttatccat acataaatta ctccatcaaa 840

cccggaatga tggtgcctga agttgagttt tggcggttaa tcgctcagat agcctcgctg 900

ggtatggatg gagcagtgat ttggggatca agtgcgagtg taggcagtaa gaatcattgt 960

gcgcaattaa tgaagtacat tgcagacgta ttgggtcctg caactttgcg cataaaagaa 1020

aatgtagcac ggtgcagtaa acaggcgtgc tctggtaggg gtagatgtac ctggcctaaa 1080

gatacctctg ttattgcttg gaagttcctc gttgaaaagg aagactatga cttttatctg 1140

ggtgatatag agtgtaaatg tgttgaaggc tacgaaggta ggtactgtga acaaaagact 1200

aagggatccg ccatttctca aatcactgac ggtcaaatcc aagctactac cactgctacc 1260

accgaagcta ccaccactgc tgccccatct tccaccgttg aaactgtttc tccatccagc 1320

accgaaacta tctctcaaca aactgaaaat ggtgctgcta aggccgctgt cggtatgggt 1380

gccggtgctc tagctgctgc tgctatgttg ttataa 1416

<210> 27
<211> 1218
<212> DNA
<213> Tityus serrulatus

<220>
<223> Reencoded nucleic acid sequence of hyaluronidase (HYAL)
      originating from Tityus serrulatus with a N-terminal secretion
      signal

<400> 27
atgcaattct ctactgtcgc ttccgttgct ttcgtcgctt tggctaactt tgttgccgct 60

gctgatttta aagtttactg ggaagtgcct tccttccttt gttctaaacg ttttaaaatt 120

aatgtaacgg aagttttaac aagtcacgag attcttgtca atcagggtga gagtttcaac 180

ggtgacaaga tagtaatctt ttacgaaaac caattgggga agtacccgca tattgactca 240

aacaatgtgg agatcaatgg aggaatactt caagtagccg atttggcgaa gcatttgaaa 300

gtagccaagg ataatatcac taaattcgtc ccgaatccta atttcaacgg tgtcggagtg 360

atcgactggg aagcttggcg gccatcatgg gaatttaact ggggtaagtt aaaagtatat 420

aaagaaaaga gcattgactt ggtcaagtcg aaacatccgg agtggccctc cgacagggtt 480

gaaaaggttg ctaaagagga gtgggaggag agtgccaaag aatggatggt gaagaccctg 540

aagttagcac aggaaatgcg accgaacgca gtttggtgct attatctatt ccctgactgc 600

tacaattatt tcggtaagga tcaaccctct caattcagct gctcgtctcg aattcagaag 660

gaaaattctc gtctttcttg gctctggaat caatcaacag ccatttgcct aagcatttat 720

atccaggaat cccatgttac caaatataat atgtcccagc ggacatggtg gatcgatgcg 780

agattaagag aagcaattcg agtcagcgaa cacagaccaa acatacccat ctacccttac 840

attaattata ttctacctgg aactaatcaa actgtaccag caatggactt taaaaggaca 900

ctgggtcaaa tagctagcct cggcctagat ggtgctttgt tatggggatc tagctatcat 960

gttttaacag aatctcaatg caaaatcact tctgattatg tgaaatcagt gattgctcct 1020

accgtggcta ctgtcgttct caatacaaac agatgctcac agataatttg taagggtcgc 1080

ggcaactgtg tttggcctga agaaccattt agttcttgga aatacttagt tgaccccaaa 1140

atgccagtgt tcaagccaac caacatccac tgtaaatgta aaggttacct aggtagatac 1200

tgtgagatcc caaagtaa 1218

<210> 28
<211> 1428
<212> DNA

<213> Tityus serrulatus

<220>
<223> Reencoded nucleic acid sequence of hyaluronidase (HYAL)
      originating from Tityus serrulatus with a N-terminal secretion
      signal and a C-terminal anchoring signal

<400> 28
atgcaattct ctactgtcgc ttccgttgct ttcgtcgctt tggctaactt tgttgccgct      60

gctgatttta aagtttactg ggaagtgcct tccttccttt gttctaaacg ttttaaaatt     120

aatgtaacgg aagttttaac aagtcacgag attcttgtca atcagggtga gagtttcaac     180

ggtgacaaga tagtaatctt ttacgaaaac caattgggga agtacccgca tattgactca     240

aacaatgtgg agatcaatgg aggaatactt caagtagccg atttggcgaa gcatttgaaa     300

gtagccaagg ataatatcac taaattcgtc ccgaatccta atttcaacgg tgtcggagtg     360

atcgactggg aagcttggcg gccatcatgg gaatttaact ggggtaagtt aaaagtatat     420

aaagaaaaga gcattgactt ggtcaagtcg aaacatccgg agtggccctc cgacagggtt     480

gaaaaggttg ctaaagagga gtgggaggag agtgccaaag aatggatggt gaagaccctg     540

aagttagcac aggaaatgcg accgaacgca gtttggtgct attatctatt ccctgactgc     600

tacaattatt tcggtaagga tcaaccctct caattcagct gctcgtctcg aattcagaag     660

gaaaattctc gtctttcttg gctctggaat caatcaacag ccatttgcct aagcatttat     720

atccaggaat cccatgttac caaatataat atgtcccagc ggacatggtg gatcgatgcg     780

agattaagag aagcaattcg agtcagcgaa cacagaccaa acatacccat ctacccttac     840

attaattata ttctacctgg aactaatcaa actgtaccag caatggactt taaaaggaca     900

ctgggtcaaa tagctagcct cggcctagat ggtgctttgt tatggggatc tagctatcat     960

gttttaacag aatctcaatg caaaatcact tctgattatg tgaaatcagt gattgctcct    1020

accgtggcta ctgtcgttct caatacaaac agatgctcac agataatttg taagggtcgc    1080

ggcaactgtg tttggcctga agaaccattt agttcttgga aatacttagt tgaccccaaa    1140

atgccagtgt tcaagccaac caacatccac tgtaaatgta aaggttacct aggtagatac    1200

tgtgagatcc caaagggatc cgccatttct caaatcactg acggtcaaat ccaagctact    1260

accactgcta ccaccgaagc taccaccact gctgccccat cttccaccgt tgaaactgtt    1320

tctccatcca gcaccgaaac tatctctcaa caaactgaaa atggtgctgc taaggccgct    1380

gtcggtatgg gtgccggtgc tctagctgct gctgctatgt tgttataa                 1428

<210> 29
<211> 1083
<212> DNA
<213> Vespa magnifica

<220>
<223> Reencoded nucleic acid sequence of hyaluronidase (HYAL)
      originating from Vespa magnifica with a N-terminal secretion
      signal

<400> 29
atgcaatttt ctacagtggc aagtgttgca ttcgttgcac tagccaactt tgtggcggca      60

gatagctgtg ggtcaaactg cgaaaagagt gagagaccga aaagggtctt caacatttac     120

tggaacgtac ctacattcat gtgtcaccag tacggactat actttgacga ggtcacgaat     180

tttaatataa agcacaacag caaagacaat tttcaagggg acaagatcgc gatcttttat     240

gaccccgggg agtttcccgc tctgctgcca ctaaactatg gtaagtacaa gatcaggaat     300

ggtggtgttc cacaagaggg taacatcacc atccatctgc agagattcat agagtaccta     360

gataagacct atccgaaccg taacttttca ggcatcggtg tgatcgattt cgagaggtgg     420

agaccaattt tcagacagaa ttggggtaat atgaagattt acaagaactt ctccatcgat     480

cttgtgcgta aagagcatcc tttctggaat aagaaaatga tcgagttgga agcttctaaa     540

agattcgaga aatacgcccg tctgttcatg gaagaaacat taaagttggc taagaaaact     600

agaaaacagg ccgattgggg ctactacggt tacccctatt gcttcaacat gtctcctact     660

aatttcgttc ctgactgcga tgtcacagct agggatgaga acaacgagat gtcttggttg     720

tttaacaacc agaatgtcct attaccaagt gtatacatta ggagagagct aactcctgac     780

cagaggattg ggcttgtaca ggggagagtg aaggaagctg tgagaatttc aaataaactg     840

aagcactcac ctaaagtctt cagctattgg tggtatgttt accaagacga gaccaacacc     900

ttcttaacgg agaccgacgt caagaagacg tttcaggaga ttgtgatcaa cggtggagat     960

gggattataa tctggggttc gtcctctgat gtaaacagct tgtccaagtg tacgaggtta    1020

agggagtacc tattgacagt cttgggacca attgctgtta acgtgactga agcagtaaac    1080

taa                                                                  1083


<210> 30
<211> 1293
<212> DNA
<213> Vespa magnifica


<220>
<223> Reencoded nucleic acid sequence of hyaluronidase (HYAL)
      originating from Vespa magnifica with a N-terminal secretion
      signal and a C-terminal anchoring signal

<400> 30
atgcaatttt ctacagtggc aagtgttgca ttcgttgcac tagccaactt tgtggcggca      60

gatagctgtg ggtcaaactg cgaaaagagt gagagaccga aaagggtctt caacatttac     120

tggaacgtac ctacattcat gtgtcaccag tacggactat actttgacga ggtcacgaat     180

tttaatataa agcacaacag caaagacaat tttcaagggg acaagatcgc gatcttttat 240

gaccccgggg agtttcccgc tctgctgcca ctaaactatg gtaagtacaa gatcaggaat 300

ggtggtgttc cacaagaggg taacatcacc atccatctgc agagattcat agagtaccta 360

gataagacct atccgaaccg taacttttca ggcatcggtg tgatcgattt cgagaggtgg 420

agaccaattt tcagacagaa ttggggtaat atgaagattt acaagaactt ctccatcgat 480

cttgtgcgta aagagcatcc tttctggaat aagaaaatga tcgagttgga agcttctaaa 540

agattcgaga aatacgcccg tctgttcatg gaagaaacat taaagttggc taagaaaact 600

agaaaacagg ccgattgggg ctactacggt tacccctatt gcttcaacat gtctcctact 660

aatttcgttc ctgactgcga tgtcacagct agggatgaga acaacgagat gtcttggttg 720

tttaacaacc agaatgtcct attaccaagt gtatacatta ggagagagct aactcctgac 780

cagaggattg ggcttgtaca ggggagagtg aaggaagctg tgagaatttc aaataaactg 840

aagcactcac ctaaagtctt cagctattgg tggtatgttt accaagacga gaccaacacc 900

ttcttaacgg agaccgacgt caagaagacg tttcaggaga ttgtgatcaa cggtggagat 960

gggattataa tctggggttc gtcctctgat gtaaacagct tgtccaagtg tacgaggtta 1020

agggagtacc tattgacagt cttgggacca attgctgtta acgtgactga agcagtaaac 1080

ggatccgcca tttctcaaat cactgacggt caaatccaag ctactaccac tgctaccacc 1140

gaagctacca ccactgctgc cccatcttcc accgttgaaa ctgtttctcc atccagcacc 1200

gaaactatct ctcaacaaac tgaaaatggt gctgctaagg ccgctgtcgg tatgggtgcc 1260

ggtgctctag ctgctgctgc tatgttgtta taa 1293

<210> 31
<211> 441
<212> PRT
<213> Bothrops atrox

<220>
<223> Amino acid sequence of hyaluronidase (HYAL) originating from
Bothrops atrox with a N-terminal secretion signal

<400> 31
Met Gln Phe Ser Thr Val Ala Ser Val Ala Phe Val Ala Leu Ala Asn
1               5                   10                  15
Phe Val Ala Ala Pro Met Tyr Pro Asn Glu Pro Phe Leu Val Phe Trp
            20                  25                  30
Asn Ala Pro Thr Thr Gln Cys Arg Leu Arg Tyr Lys Val Asp Leu Asp
        35                  40                  45
Leu Lys Thr Phe His Ile Val Thr Asn Ala Asn Asp Ser Leu Ser Gly
    50                  55                  60
Ser Ala Val Thr Ile Phe Tyr Pro Thr His Leu Gly Val Tyr Pro His
65                  70                  75                  80
Ile Asp Asp Arg Gly His Phe Phe Asn Gly Ile Ile Pro Gln Asn Glu
                85                  90                  95

Ser Leu Val Lys His Leu Asn Lys Ser Lys Ser Asp Ile Asn Arg Met
            100                 105                 110
Ile Pro Leu Arg Thr Phe His Gly Leu Gly Val Ile Asp Trp Glu Asn
        115                 120                 125
Trp Arg Pro Gln Trp Asp Arg Asn Trp Gly Ser Lys Asn Val Tyr Arg
    130                 135                 140
Asn Arg Ser Ile Gln Phe Ala Arg Asp Leu His Pro Glu Leu Ser Glu
145                 150                 155                 160
Asp Lys Ile Lys Arg Leu Ala Lys Gln Glu Phe Glu Lys Ala Ala Lys
                165                 170                 175
Ser Phe Met Arg Asp Thr Leu Leu Leu Ala Glu Glu Met Arg Pro Asp
            180                 185                 190
Gly Tyr Trp Gly Tyr Tyr Leu Tyr Pro Asp Cys His Asn Tyr Asn Tyr
        195                 200                 205
Lys Thr Lys Pro Asp Gln Tyr Thr Gly Glu Cys Pro Asp Ile Glu Met
    210                 215                 220
Ser Arg Asn Asn Gln Leu Leu Trp Leu Trp Arg Asp Ser Thr Ala Leu
225                 230                 235                 240
Phe Pro Asn Ile Tyr Leu Glu Thr Ile Leu Arg Ser Ser Asp Asn Ala
                245                 250                 255
Leu Lys Phe Val His His Arg Leu Lys Glu Ala Met Arg Ile Ala Ser
            260                 265                 270
Met Ala Arg Asn Asp Tyr Ala Leu Pro Phe Phe Val Tyr Ala Arg Pro
        275                 280                 285
Phe Tyr Ala Tyr Thr Phe Glu Pro Leu Thr Gln Glu Asp Leu Val Thr
    290                 295                 300
Thr Val Gly Glu Thr Ala Asp Met Gly Ala Ala Gly Ile Val Phe Trp
305                 310                 315                 320
Gly Ser Met Gln Tyr Ala Ser Thr Val Glu Ser Cys Gly Lys Val Lys
                325                 330                 335
Asp Tyr Met Asn Gly Pro Leu Gly Arg Tyr Ile Val Asn Val Thr Thr
            340                 345                 350
Ala Ala Lys Ile Cys Ser Arg Phe Leu Cys Lys Arg His Gly Arg Cys
        355                 360                 365
Val Arg Lys His Ser Asp Ser Asn Ala Phe Leu His Leu Phe Pro Asp
    370                 375                 380
Ser Phe Arg Ile Met Val His Gly Asn Ala Thr Glu Lys Lys Val Ile
385                 390                 395                 400
Val Lys Gly Lys Leu Glu Leu Lys Asn Leu Ile Phe Leu Arg Asn Asn
                405                 410                 415
Phe Met Cys Gln Cys Tyr Gln Gly Trp Lys Gly Leu Tyr Cys Glu Lys
            420                 425                 430
His Ser Ile Lys Glu Ile Arg Lys Ile
        435                 440

<210> 32
<211> 511
<212> PRT
<213> Bothrops atrox

<220>
<223> Amino acid sequence of hyaluronidase (HYAL) originating from
      Bothrops atrox with a N-terminal secretion signal and a
      C-terminal anchoring signal

<400> 32
Met Gln Phe Ser Thr Val Ala Ser Val Ala Phe Val Ala Leu Ala Asn
1               5                   10                  15
Phe Val Ala Ala Pro Met Tyr Pro Asn Glu Pro Phe Leu Val Phe Trp
            20                  25                  30
Asn Ala Pro Thr Thr Gln Cys Arg Leu Arg Tyr Lys Val Asp Leu Asp
        35                  40                  45

Leu Lys Thr Phe His Ile Val Thr Asn Ala Asn Asp Ser Leu Ser Gly
    50                  55                  60
Ser Ala Val Thr Ile Phe Tyr Pro Thr His Leu Gly Val Tyr Pro His
65                  70                  75                  80
Ile Asp Asp Arg Gly His Phe Phe Asn Gly Ile Ile Pro Gln Asn Glu
                85                  90                  95
Ser Leu Val Lys His Leu Asn Lys Ser Lys Ser Asp Ile Asn Arg Met
            100                 105                 110
Ile Pro Leu Arg Thr Phe His Gly Leu Gly Val Ile Asp Trp Glu Asn
        115                 120                 125
Trp Arg Pro Gln Trp Asp Arg Asn Trp Gly Ser Lys Asn Val Tyr Arg
    130                 135                 140
Asn Arg Ser Ile Gln Phe Ala Arg Asp Leu His Pro Glu Leu Ser Glu
145                 150                 155                 160
Asp Lys Ile Lys Arg Leu Ala Lys Gln Glu Phe Glu Lys Ala Ala Lys
                165                 170                 175
Ser Phe Met Arg Asp Thr Leu Leu Leu Ala Glu Glu Met Arg Pro Asp
            180                 185                 190
Gly Tyr Trp Gly Tyr Tyr Leu Tyr Pro Asp Cys His Asn Tyr Asn Tyr
        195                 200                 205
Lys Thr Lys Pro Asp Gln Tyr Thr Gly Glu Cys Pro Asp Ile Glu Met
    210                 215                 220
Ser Arg Asn Asn Gln Leu Leu Trp Leu Trp Arg Asp Ser Thr Ala Leu
225                 230                 235                 240
Phe Pro Asn Ile Tyr Leu Glu Thr Ile Leu Arg Ser Ser Asp Asn Ala
                245                 250                 255
Leu Lys Phe Val His His Arg Leu Lys Glu Ala Met Arg Ile Ala Ser
            260                 265                 270
Met Ala Arg Asn Asp Tyr Ala Leu Pro Phe Phe Val Tyr Ala Arg Pro
        275                 280                 285
Phe Tyr Ala Tyr Thr Phe Glu Pro Leu Thr Gln Glu Asp Leu Val Thr
    290                 295                 300
Thr Val Gly Glu Thr Ala Asp Met Gly Ala Ala Gly Ile Val Phe Trp
305                 310                 315                 320
Gly Ser Met Gln Tyr Ala Ser Thr Val Glu Ser Cys Gly Lys Val Lys
                325                 330                 335
Asp Tyr Met Asn Gly Pro Leu Gly Arg Tyr Ile Val Asn Val Thr Thr
            340                 345                 350
Ala Ala Lys Ile Cys Ser Arg Phe Leu Cys Lys Arg His Gly Arg Cys
        355                 360                 365
Val Arg Lys His Ser Asp Ser Asn Ala Phe Leu His Leu Phe Pro Asp
    370                 375                 380
Ser Phe Arg Ile Met Val His Gly Asn Ala Thr Glu Lys Lys Val Ile
385                 390                 395                 400
Val Lys Gly Lys Leu Glu Leu Lys Asn Leu Ile Phe Leu Arg Asn Asn
                405                 410                 415
Phe Met Cys Gln Cys Tyr Gln Gly Trp Lys Gly Leu Tyr Cys Glu Lys
            420                 425                 430
His Ser Ile Lys Glu Ile Arg Lys Ile Gly Ser Ala Ile Ser Gln Ile
        435                 440                 445
Thr Asp Gly Gln Ile Gln Ala Thr Thr Thr Ala Thr Thr Glu Ala Thr
    450                 455                 460
Thr Thr Ala Ala Pro Ser Ser Thr Val Glu Thr Val Ser Pro Ser Ser
465                 470                 475                 480
Thr Glu Thr Ile Ser Gln Gln Thr Glu Asn Gly Ala Ala Lys Ala Ala
                485                 490                 495
Val Gly Met Gly Ala Gly Ala Leu Ala Ala Ala Ala Met Leu Leu
            500                 505                 510

<210> 33
<211> 398
<212> PRT
<213> Cupiennius salei

<220>
<223> Amino acid sequence of hyaluronidase (HYAL) originating from
      Cupiennius salei with a N-terminal secretion signal

<400> 33
Met Gln Phe Ser Thr Val Ala Ser Val Ala Phe Val Ala Leu Ala Asn
1               5                   10                  15
Phe Val Ala Ala Phe Lys Ile Tyr Trp Asn Val Pro Thr Phe Gln Cys
            20                  25                  30
Thr His Asn Tyr Lys Ile Asp Tyr Val Lys Leu Leu Ser Thr Tyr Gly
        35                  40                  45
Ile Gln Val Asn Asp Gly Gly Lys Phe Gln Gly Asn Gln Val Thr Ile
    50                  55                  60
Phe Tyr Glu Thr Gln Leu Gly Leu Tyr Pro Arg Ile Leu Lys Ser Gly
65                  70                  75                  80
Lys Met Glu Asn Gly Gly Ile Pro Gln Arg Gly Asn Phe Glu Lys His
                85                  90                  95
Leu Glu Lys Ala Ser Thr Asp Leu Gln Lys Val Ile Pro Trp Lys Glu
            100                 105                 110
Phe Ser Gly Leu Gly Val Ile Asp Trp Glu Ala Trp Arg Pro Thr Trp
        115                 120                 125
Glu Phe Asn Trp Glu Pro Leu Arg Ile Tyr Gln Thr Glu Ser Ile Lys
    130                 135                 140
Arg Ala Lys Glu Leu His Pro Thr Ala Asn Asp Ser Ala Val Lys Glu
145                 150                 155                 160
Ile Ala Glu Arg Gln Trp Glu Asp Ser Ala Lys Leu Tyr Met Leu Glu
                165                 170                 175
Thr Leu Arg Leu Ala Lys Lys Leu Arg Pro Gln Ala Pro Trp Cys Tyr
            180                 185                 190
Tyr Leu Phe Pro Asp Cys Tyr Asn Tyr Val Gly Lys Lys Pro Lys Asp
        195                 200                 205
Phe Gln Cys Ser Ala Ser Ile Arg Lys Gly Asn Asp Lys Leu Ser Trp
    210                 215                 220
Leu Trp Lys Asp Ser Thr Ala Leu Cys Pro Ser Ile Tyr Val Tyr Glu
225                 230                 235                 240
Ser Gln Leu Asp Arg Tyr Ser Phe Glu Gln Arg Thr Trp Arg Asp Asn
                245                 250                 255
Glu Lys Leu Arg Glu Ala Leu Arg Val Ala Thr Arg Thr Ser Lys Ile
            260                 265                 270
Tyr Pro Tyr Val Asn Tyr Phe Asp Lys Glu Leu Ile Pro Glu Gln Glu
        275                 280                 285
Val Trp Arg Met Leu Ala Gln Ala Ala Ala Val Gly Gly Ser Gly Ala
    290                 295                 300
Val Ile Trp Gly Ser Ser Ala Ala Val Ala Ser Glu Glu Leu Cys Lys
305                 310                 315                 320
Ser Leu Lys Gln Tyr Ile Ile Glu Thr Leu Gly Pro Ala Ala Glu Lys
                325                 330                 335
Val Ala Trp Arg Ser Asp Leu Cys Ser Lys Glu Ile Cys Asn Asn Gln
            340                 345                 350
Gly Arg Cys Thr Phe Pro Asp Asp Asp Tyr Ala Asn Ala Trp Lys Leu
        355                 360                 365
Phe Thr Asp Asp Thr Val Lys Phe Tyr Ala Gly Asn Ile Thr Cys Arg
    370                 375                 380
Cys Ser Glu Asn Tyr Ser Gly Arg Phe Cys Glu Lys Lys Asn
385                 390                 395

<210> 34
<211> 468
<212> PRT
<213> Cupiennius salei

<220>
<223> Amino acid sequence of hyaluronidase (HYAL) originating from
      Cupiennius salei with a N-terminal secretion signal and a
      C-terminal anchoring signal

<400> 34
Met Gln Phe Ser Thr Val Ala Ser Val Ala Phe Val Ala Leu Ala Asn
1               5                   10                  15
Phe Val Ala Ala Phe Lys Ile Tyr Trp Asn Val Pro Thr Phe Gln Cys
            20                  25                  30
Thr His Asn Tyr Lys Ile Asp Tyr Val Lys Leu Leu Ser Thr Tyr Gly
        35                  40                  45
Ile Gln Val Asn Asp Gly Gly Lys Phe Gln Gly Asn Gln Val Thr Ile
    50                  55                  60
Phe Tyr Glu Thr Gln Leu Gly Leu Tyr Pro Arg Ile Leu Lys Ser Gly
65                  70                  75                  80
Lys Met Glu Asn Gly Gly Ile Pro Gln Arg Gly Asn Phe Glu Lys His
                85                  90                  95
Leu Glu Lys Ala Ser Thr Asp Leu Gln Lys Val Ile Pro Trp Lys Glu
            100                 105                 110
Phe Ser Gly Leu Gly Val Ile Asp Trp Glu Ala Trp Arg Pro Thr Trp
        115                 120                 125
Glu Phe Asn Trp Glu Pro Leu Arg Ile Tyr Gln Thr Glu Ser Ile Lys
    130                 135                 140
Arg Ala Lys Glu Leu His Pro Thr Ala Asn Asp Ser Ala Val Lys Glu
145                 150                 155                 160
Ile Ala Glu Arg Gln Trp Glu Asp Ser Ala Lys Leu Tyr Met Leu Glu
                165                 170                 175
Thr Leu Arg Leu Ala Lys Lys Leu Arg Pro Gln Ala Pro Trp Cys Tyr
            180                 185                 190
Tyr Leu Phe Pro Asp Cys Tyr Asn Tyr Val Gly Lys Lys Pro Lys Asp
        195                 200                 205
Phe Gln Cys Ser Ala Ser Ile Arg Lys Gly Asn Asp Lys Leu Ser Trp
    210                 215                 220
Leu Trp Lys Asp Ser Thr Ala Leu Cys Pro Ser Ile Tyr Val Tyr Glu
225                 230                 235                 240
Ser Gln Leu Asp Arg Tyr Ser Phe Glu Gln Arg Thr Trp Arg Asp Asn
                245                 250                 255
Glu Lys Leu Arg Glu Ala Leu Arg Val Ala Thr Arg Thr Ser Lys Ile
            260                 265                 270
Tyr Pro Tyr Val Asn Tyr Phe Asp Lys Glu Leu Ile Pro Glu Gln Glu
        275                 280                 285
Val Trp Arg Met Leu Ala Gln Ala Ala Ala Val Gly Gly Ser Gly Ala
    290                 295                 300
Val Ile Trp Gly Ser Ser Ala Ala Val Ala Ser Glu Glu Leu Cys Lys
305                 310                 315                 320
Ser Leu Lys Gln Tyr Ile Ile Glu Thr Leu Gly Pro Ala Ala Glu Lys
                325                 330                 335
Val Ala Trp Arg Ser Asp Leu Cys Ser Lys Glu Ile Cys Asn Asn Gln
            340                 345                 350
Gly Arg Cys Thr Phe Pro Asp Asp Asp Tyr Ala Asn Ala Trp Lys Leu
        355                 360                 365
Phe Thr Asp Asp Thr Val Lys Phe Tyr Ala Gly Asn Ile Thr Cys Arg
    370                 375                 380
Cys Ser Glu Asn Tyr Ser Gly Arg Phe Cys Glu Lys Lys Asn Gly Ser
385                 390                 395                 400
Ala Ile Ser Gln Ile Thr Asp Gly Gln Ile Gln Ala Thr Thr Thr Ala
                405                 410                 415
Thr Thr Glu Ala Thr Thr Thr Ala Ala Pro Ser Ser Thr Val Glu Thr
            420                 425                 430
Val Ser Pro Ser Ser Thr Glu Thr Ile Ser Gln Gln Thr Glu Asn Gly
        435                 440                 445

Ala Ala Lys Ala Ala Val Gly Met Gly Ala Gly Ala Leu Ala Ala Ala
    450                 455                 460
Ala Met Leu Leu
465

<210> 35
<211> 579
<212> PRT
<213> Hirudo nipponia


<220>
<223> Amino acid sequence of hyaluronidase (HYAL) originating from
      Hirudo Nipponia with a N-terminal secretion signal

<400> 35
Met Gln Phe Ser Thr Val Ala Ser Val Ala Phe Val Ala Leu Ala Asn
1               5                   10                  15
Phe Val Ala Ala Met Lys Glu Ile Ala Val Thr Ile Asp Asp Lys Asn
            20                  25                  30
Val Ile Ala Ser Val Ser Glu Ser Phe His Gly Val Ala Phe Asp Ala
        35                  40                  45
Ser Leu Phe Ser Pro Lys Gly Leu Trp Ser Phe Val Asp Ile Thr Ser
    50                  55                  60
Pro Lys Leu Phe Lys Leu Leu Glu Gly Leu Ser Pro Gly Tyr Phe Arg
65                  70                  75                  80
Val Gly Gly Thr Phe Ala Asn Trp Leu Phe Phe Asp Leu Asp Glu Asn
                85                  90                  95
Asn Lys Trp Lys Asp Tyr Trp Ala Phe Lys Asp Lys Thr Pro Glu Thr
            100                 105                 110
Ala Thr Ile Thr Arg Arg Trp Leu Phe Arg Lys Gln Asn Asn Leu Lys
        115                 120                 125
Lys Glu Thr Phe Asp Asp Leu Val Lys Leu Thr Lys Gly Ser Lys Met
    130                 135                 140
Arg Leu Leu Phe Asp Leu Asn Ala Glu Val Arg Thr Gly Tyr Glu Ile
145                 150                 155                 160
Gly Lys Lys Met Thr Ser Thr Trp Asp Ser Ser Glu Ala Glu Lys Leu
                165                 170                 175
Phe Lys Tyr Cys Val Ser Lys Gly Tyr Gly Asp Asn Ile Asp Trp Glu
            180                 185                 190
Leu Gly Asn Glu Pro Asp His Thr Ser Ala His Asn Leu Thr Glu Lys
        195                 200                 205
Gln Val Gly Glu Asp Phe Lys Ala Leu His Lys Val Leu Glu Lys Tyr
    210                 215                 220
Pro Thr Leu Asn Lys Gly Ser Leu Val Gly Pro Asp Val Gly Trp Met
225                 230                 235                 240
Gly Val Ser Tyr Val Lys Gly Leu Ala Asp Gly Ala Gly Asp His Val
                245                 250                 255
Thr Ala Phe Thr Leu His Gln Tyr Tyr Phe Asp Gly Asn Thr Ser Asp
            260                 265                 270
Val Ser Thr Tyr Leu Asp Ala Thr Tyr Phe Lys Lys Leu Gln Gln Leu
        275                 280                 285
Phe Asp Lys Val Lys Asp Val Leu Lys Asn Ser Pro His Lys Asp Lys
    290                 295                 300
Pro Leu Trp Leu Gly Glu Thr Ser Ser Gly Tyr Asn Ser Gly Thr Lys
305                 310                 315                 320
Asp Val Ser Asp Arg Tyr Val Ser Gly Phe Leu Thr Leu Asp Lys Leu
                325                 330                 335
Gly Leu Ser Ala Ala Asn Asn Val Lys Val Val Ile Arg Gln Thr Ile
            340                 345                 350
Tyr Asn Gly Tyr Tyr Gly Leu Leu Asp Lys Asn Thr Leu Glu Pro Asn
        355                 360                 365
Pro Asp Tyr Trp Leu Met His Val His Asn Ser Leu Val Gly Asn Thr

370                 375                 380
Val Phe Lys Val Asp Val Ser Asp Pro Thr Asn Lys Ala Arg Val Tyr
385                 390                 395                 400
Ala Gln Cys Thr Lys Thr Asn Ser Lys His Thr Gln Ser Arg Tyr Tyr
                405                 410                 415
Lys Gly Ser Leu Thr Ile Phe Ala Leu Asn Val Gly Asp Glu Asp Val
            420                 425                 430
Thr Leu Lys Ile Asp Gln Tyr Ser Gly Lys Lys Ile Tyr Ser Tyr Ile
        435                 440                 445
Leu Thr Pro Glu Gly Gly Gln Leu Thr Ser Gln Lys Val Leu Leu Asn
    450                 455                 460
Gly Lys Glu Leu Lys Leu Val Ser Asp Gln Leu Pro Glu Leu Asn Ala
465                 470                 475                 480
Asp Glu Ser Lys Thr Ser Phe Thr Leu Ser Pro Lys Thr Phe Gly Phe
                485                 490                 495
Phe Val Val Ser Asp Ala Asn Val Glu Ala Cys Lys Lys Gly Ser Ala
            500                 505                 510
Ile Ser Gln Ile Thr Asp Gly Gln Ile Gln Ala Thr Thr Thr Ala Thr
        515                 520                 525
Thr Glu Ala Thr Thr Thr Ala Ala Pro Ser Ser Thr Val Glu Thr Val
    530                 535                 540
Ser Pro Ser Ser Thr Glu Thr Ile Ser Gln Gln Thr Glu Asn Gly Ala
545                 550                 555                 560
Ala Lys Ala Ala Val Gly Met Gly Ala Gly Ala Leu Ala Ala Ala Ala
                565                 570                 575
Met Leu Leu


<210> 36
<211> 509
<212> PRT
<213> Hirudo nipponia


<220>
<223> Amino acid sequence of hyaluronidase (HYAL) originating from
      Hirudo Nipponia with a N-terminal secretion signal and a
      C-terminal anchoring signal

<400> 36
Met Gln Phe Ser Thr Val Ala Ser Val Ala Phe Val Ala Leu Ala Asn
1               5                   10                  15
Phe Val Ala Ala Met Lys Glu Ile Ala Val Thr Ile Asp Asp Lys Asn
            20                  25                  30
Val Ile Ala Ser Val Ser Glu Ser Phe His Gly Val Ala Phe Asp Ala
        35                  40                  45
Ser Leu Phe Ser Pro Lys Gly Leu Trp Ser Phe Val Asp Ile Thr Ser
    50                  55                  60
Pro Lys Leu Phe Lys Leu Leu Glu Gly Leu Ser Pro Gly Tyr Phe Arg
65                  70                  75                  80
Val Gly Gly Thr Phe Ala Asn Trp Leu Phe Phe Asp Leu Asp Glu Asn
                85                  90                  95
Asn Lys Trp Lys Asp Tyr Trp Ala Phe Lys Asp Lys Thr Pro Glu Thr
            100                 105                 110
Ala Thr Ile Thr Arg Arg Trp Leu Phe Arg Lys Gln Asn Asn Leu Lys
        115                 120                 125
Lys Glu Thr Phe Asp Asp Leu Val Lys Leu Thr Lys Gly Ser Lys Met
    130                 135                 140
Arg Leu Leu Phe Asp Leu Asn Ala Glu Val Arg Thr Gly Tyr Glu Ile
145                 150                 155                 160
Gly Lys Lys Met Thr Ser Thr Trp Asp Ser Ser Glu Ala Glu Lys Leu
                165                 170                 175
Phe Lys Tyr Cys Val Ser Lys Gly Tyr Gly Asp Asn Ile Asp Trp Glu

180                     185                     190
Leu Gly Asn Glu Pro Asp His Thr Ser Ala His Asn Leu Thr Glu Lys
        195                     200                     205
Gln Val Gly Glu Asp Phe Lys Ala Leu His Lys Val Leu Glu Lys Tyr
    210                     215                     220
Pro Thr Leu Asn Lys Gly Ser Leu Val Gly Pro Asp Val Gly Trp Met
225                     230                     235                     240
Gly Val Ser Tyr Val Lys Gly Leu Ala Asp Gly Ala Gly Asp His Val
                245                     250                     255
Thr Ala Phe Thr Leu His Gln Tyr Tyr Phe Asp Gly Asn Thr Ser Asp
            260                     265                     270
Val Ser Thr Tyr Leu Asp Ala Thr Tyr Phe Lys Lys Leu Gln Gln Leu
        275                     280                     285
Phe Asp Lys Val Lys Asp Val Leu Lys Asn Ser Pro His Lys Asp Lys
    290                     295                     300
Pro Leu Trp Leu Gly Glu Thr Ser Ser Gly Tyr Asn Ser Gly Thr Lys
305                     310                     315                     320
Asp Val Ser Asp Arg Tyr Val Ser Gly Phe Leu Thr Leu Asp Lys Leu
                325                     330                     335
Gly Leu Ser Ala Ala Asn Asn Val Lys Val Val Ile Arg Gln Thr Ile
            340                     345                     350
Tyr Asn Gly Tyr Tyr Gly Leu Leu Asp Lys Asn Thr Leu Glu Pro Asn
        355                     360                     365
Pro Asp Tyr Trp Leu Met His Val His Asn Ser Leu Val Gly Asn Thr
    370                     375                     380
Val Phe Lys Val Asp Val Ser Asp Pro Thr Asn Lys Ala Arg Val Tyr
385                     390                     395                     400
Ala Gln Cys Thr Lys Thr Asn Ser Lys His Thr Gln Ser Arg Tyr Tyr
                405                     410                     415
Lys Gly Ser Leu Thr Ile Phe Ala Leu Asn Val Gly Asp Glu Asp Val
            420                     425                     430
Thr Leu Lys Ile Asp Gln Tyr Ser Gly Lys Lys Ile Tyr Ser Tyr Ile
        435                     440                     445
Leu Thr Pro Glu Gly Gly Gln Leu Thr Ser Gln Lys Val Leu Leu Asn
    450                     455                     460
Gly Lys Glu Leu Lys Leu Val Ser Asp Gln Leu Pro Glu Leu Asn Ala
465                     470                     475                     480
Asp Glu Ser Lys Thr Ser Phe Thr Leu Ser Pro Lys Thr Phe Gly Phe
                485                     490                     495
Phe Val Val Ser Asp Ala Asn Val Glu Ala Cys Lys Lys
            500                     505

<210> 37
<211> 401
<212> PRT
<213> Loxosceles intermedia


<220>
<223> Amino acid sequence of hyaluronidase (HYAL) originating from
      Loxosceles intermedia with a N-terminal secretion signal

<400> 37
Met Gln Phe Ser Thr Val Ala Ser Val Ala Phe Val Ala Leu Ala Asn
1               5                   10                  15
Phe Val Ala Ala Phe Asp Val Phe Trp Asn Val Pro Ser Gln Gln Cys
            20                  25                  30
Lys Lys Tyr Gly Met Lys Phe Val Pro Leu Leu Glu Gln Tyr Ser Ile
        35                  40                  45
Leu Val Asn Lys Glu Asp Asn Phe Lys Gly Asp Lys Ile Thr Ile Phe
    50                  55                  60
Tyr Glu Ser Gln Leu Gly Leu Tyr Pro His Ile Gly Ala Asn Asp Glu
65                  70                  75                  80

Ser Phe Asn Gly Gly Ile Pro Gln Leu Gly Asp Leu Lys Ala His Leu
                85                  90                  95
Glu Lys Ser Ala Val Asp Ile Arg Arg Asp Ile Leu Asp Lys Ser Ala
            100                 105                 110
Thr Gly Leu Arg Ile Ile Asp Trp Glu Ala Trp Arg Pro Ile Trp Glu
        115                 120                 125
Phe Asn Trp Ser Ser Leu Arg Lys Tyr Gln Asp Lys Met Lys Lys Val
    130                 135                 140
Val Arg Gln Phe Asn Pro Thr Ala His Glu Ser Thr Val Ala Lys Leu
145                 150                 155                 160
Ala His Asn Glu Trp Glu Asn Ser Ser Lys Ser Trp Met Leu Ser Thr
                165                 170                 175
Leu Gln Leu Gly Lys Gln Leu Arg Pro Asn Ser Val Trp Cys Tyr Tyr
            180                 185                 190
Leu Phe Pro Asp Cys Tyr Asn Tyr Asp Gly Asn Ser Val Gln Glu Phe
        195                 200                 205
Gln Cys Ser Glu Ala Ile Arg Lys Gly Asn Asp Arg Leu Lys Trp Leu
    210                 215                 220
Trp Glu Glu Ser Thr Ala Val Cys Pro Ser Ile Tyr Ile Lys Glu Gly
225                 230                 235                 240
Gln Leu Thr Asn Tyr Thr Leu Gln Lys Arg Ile Trp Phe Thr Asn Gly
                245                 250                 255
Arg Leu Gln Glu Ala Leu Arg Val Ala Gln Pro Lys Ala Arg Ile Tyr
            260                 265                 270
Pro Tyr Ile Asn Tyr Ser Ile Lys Pro Gly Met Met Val Pro Glu Val
        275                 280                 285
Glu Phe Trp Arg Leu Ile Ala Gln Ile Ala Ser Leu Gly Met Asp Gly
    290                 295                 300
Ala Val Ile Trp Gly Ser Ser Ala Ser Val Gly Ser Lys Asn His Cys
305                 310                 315                 320
Ala Gln Leu Met Lys Tyr Ile Ala Asp Val Leu Gly Pro Ala Thr Leu
                325                 330                 335
Arg Ile Lys Glu Asn Val Ala Arg Cys Ser Lys Gln Ala Cys Ser Gly
            340                 345                 350
Arg Gly Arg Cys Thr Trp Pro Lys Asp Thr Ser Val Ile Ala Trp Lys
        355                 360                 365
Phe Leu Val Glu Lys Glu Asp Tyr Asp Phe Tyr Leu Gly Asp Ile Glu
    370                 375                 380
Cys Lys Cys Val Glu Gly Tyr Glu Gly Arg Tyr Cys Glu Gln Lys Thr
385                 390                 395                 400
Lys


<210>  38
<211>  471
<212>  PRT
<213>  Loxosceles intermedia


<220>
<223>  Amino acid sequence of hyaluronidase (HYAL) originating from
       Loxosceles intermedia with a N-terminal secretion signal and a
       C-terminal anchoring signal

<400>  38
Met Gln Phe Ser Thr Val Ala Ser Val Ala Phe Val Ala Leu Ala Asn
1               5                   10                  15
Phe Val Ala Ala Phe Asp Val Phe Trp Asn Val Pro Ser Gln Gln Cys
            20                  25                  30
Lys Lys Tyr Gly Met Lys Phe Val Pro Leu Leu Glu Gln Tyr Ser Ile
        35                  40                  45
Leu Val Asn Lys Glu Asp Asn Phe Lys Gly Asp Lys Ile Thr Ile Phe
    50                  55                  60

Tyr Glu Ser Gln Leu Gly Leu Tyr Pro His Ile Gly Ala Asn Asp Glu
65                  70                  75                  80
Ser Phe Asn Gly Gly Ile Pro Gln Leu Gly Asp Leu Lys Ala His Leu
                85                  90                  95
Glu Lys Ser Ala Val Asp Ile Arg Arg Asp Ile Leu Asp Lys Ser Ala
            100                 105                 110
Thr Gly Leu Arg Ile Ile Asp Trp Glu Ala Trp Arg Pro Ile Trp Glu
        115                 120                 125
Phe Asn Trp Ser Ser Leu Arg Lys Tyr Gln Asp Lys Met Lys Lys Val
    130                 135                 140
Val Arg Gln Phe Asn Pro Thr Ala His Glu Ser Thr Val Ala Lys Leu
145                 150                 155                 160
Ala His Asn Glu Trp Glu Asn Ser Ser Lys Ser Trp Met Leu Ser Thr
                165                 170                 175
Leu Gln Leu Gly Lys Gln Leu Arg Pro Asn Ser Val Trp Cys Tyr Tyr
            180                 185                 190
Leu Phe Pro Asp Cys Tyr Asn Tyr Asp Gly Asn Ser Val Gln Glu Phe
        195                 200                 205
Gln Cys Ser Glu Ala Ile Arg Lys Gly Asn Asp Arg Leu Lys Trp Leu
    210                 215                 220
Trp Glu Glu Ser Thr Ala Val Cys Pro Ser Ile Tyr Ile Lys Glu Gly
225                 230                 235                 240
Gln Leu Thr Asn Tyr Thr Leu Gln Lys Arg Ile Trp Phe Thr Asn Gly
                245                 250                 255
Arg Leu Gln Glu Ala Leu Arg Val Ala Gln Pro Lys Ala Arg Ile Tyr
            260                 265                 270
Pro Tyr Ile Asn Tyr Ser Ile Lys Pro Gly Met Met Val Pro Glu Val
        275                 280                 285
Glu Phe Trp Arg Leu Ile Ala Gln Ile Ala Ser Leu Gly Met Asp Gly
    290                 295                 300
Ala Val Ile Trp Gly Ser Ser Ala Ser Val Gly Ser Lys Asn His Cys
305                 310                 315                 320
Ala Gln Leu Met Lys Tyr Ile Ala Asp Val Leu Gly Pro Ala Thr Leu
                325                 330                 335
Arg Ile Lys Glu Asn Val Ala Arg Cys Ser Lys Gln Ala Cys Ser Gly
            340                 345                 350
Arg Gly Arg Cys Thr Trp Pro Lys Asp Thr Ser Val Ile Ala Trp Lys
        355                 360                 365
Phe Leu Val Glu Lys Glu Asp Tyr Asp Phe Tyr Leu Gly Asp Ile Glu
    370                 375                 380
Cys Lys Cys Val Glu Gly Tyr Glu Gly Arg Tyr Cys Glu Gln Lys Thr
385                 390                 395                 400
Lys Gly Ser Ala Ile Ser Gln Ile Thr Asp Gly Gln Ile Gln Ala Thr
                405                 410                 415
Thr Thr Ala Thr Thr Glu Ala Thr Thr Thr Ala Ala Pro Ser Ser Thr
            420                 425                 430
Val Glu Thr Val Ser Pro Ser Ser Thr Glu Thr Ile Ser Gln Gln Thr
        435                 440                 445
Glu Asn Gly Ala Ala Lys Ala Ala Val Gly Met Gly Ala Gly Ala Leu
    450                 455                 460
Ala Ala Ala Ala Met Leu Leu
465                 470

<210> 39
<211> 405
<212> PRT
<213> Tityus serrulatus

<220>
<223> Amino acid sequence of hyaluronidase (HYAL) originating from
      Tityus serrulatus with a N-terminal secretion signal

<400> 39
Met Gln Phe Ser Thr Val Ala Ser Val Ala Phe Val Ala Leu Ala Asn
1               5                   10                  15
Phe Val Ala Ala Ala Asp Phe Lys Val Tyr Trp Glu Val Pro Ser Phe
            20                  25                  30
Leu Cys Ser Lys Arg Phe Lys Ile Asn Val Thr Glu Val Leu Thr Ser
        35                  40                  45
His Glu Ile Leu Val Asn Gln Gly Glu Ser Phe Asn Gly Asp Lys Ile
    50                  55                  60
Val Ile Phe Tyr Glu Asn Gln Leu Gly Lys Tyr Pro His Ile Asp Ser
65                  70                  75                  80
Asn Asn Val Glu Ile Asn Gly Gly Ile Leu Gln Val Ala Asp Leu Ala
                85                  90                  95
Lys His Leu Lys Val Ala Lys Asp Asn Ile Thr Lys Phe Val Pro Asn
            100                 105                 110
Pro Asn Phe Asn Gly Val Gly Val Ile Asp Trp Glu Ala Trp Arg Pro
        115                 120                 125
Ser Trp Glu Phe Asn Trp Gly Lys Leu Lys Val Tyr Lys Glu Lys Ser
    130                 135                 140
Ile Asp Leu Val Lys Ser Lys His Pro Glu Trp Pro Ser Asp Arg Val
145                 150                 155                 160
Glu Lys Val Ala Lys Glu Glu Trp Glu Glu Ser Ala Lys Glu Trp Met
                165                 170                 175
Val Lys Thr Leu Lys Leu Ala Gln Glu Met Arg Pro Asn Ala Val Trp
            180                 185                 190
Cys Tyr Tyr Leu Phe Pro Asp Cys Tyr Asn Tyr Phe Gly Lys Asp Gln
        195                 200                 205
Pro Ser Gln Phe Ser Cys Ser Ser Arg Ile Gln Lys Glu Asn Ser Arg
    210                 215                 220
Leu Ser Trp Leu Trp Asn Gln Ser Thr Ala Ile Cys Leu Ser Ile Tyr
225                 230                 235                 240
Ile Gln Glu Ser His Val Thr Lys Tyr Asn Met Ser Gln Arg Thr Trp
                245                 250                 255
Trp Ile Asp Ala Arg Leu Arg Glu Ala Ile Arg Val Ser Glu His Arg
            260                 265                 270
Pro Asn Ile Pro Ile Tyr Pro Tyr Ile Asn Tyr Ile Leu Pro Gly Thr
        275                 280                 285
Asn Gln Thr Val Pro Ala Met Asp Phe Lys Arg Thr Leu Gly Gln Ile
    290                 295                 300
Ala Ser Leu Gly Leu Asp Gly Ala Leu Leu Trp Gly Ser Ser Tyr His
305                 310                 315                 320
Val Leu Thr Glu Ser Gln Cys Lys Ile Thr Ser Asp Tyr Val Lys Ser
                325                 330                 335
Val Ile Ala Pro Thr Val Ala Thr Val Val Leu Asn Thr Asn Arg Cys
            340                 345                 350
Ser Gln Ile Ile Cys Lys Gly Arg Gly Asn Cys Val Trp Pro Glu Glu
        355                 360                 365
Pro Phe Ser Ser Trp Lys Tyr Leu Val Asp Pro Lys Met Pro Val Phe
    370                 375                 380
Lys Pro Thr Asn Ile His Cys Lys Cys Lys Gly Tyr Leu Gly Arg Tyr
385                 390                 395                 400
Cys Glu Ile Pro Lys
                405

<210> 40
<211> 475
<212> PRT
<213> Tityus serrulatus

<220>
<223> Amino acid sequence of hyaluronidase (HYAL) originating from
      Tityus serrulatus with a N-terminal secretion signal and a

C-terminal anchoring signal

<400> 40
Met Gln Phe Ser Thr Val Ala Ser Val Ala Phe Val Ala Leu Ala Asn
1               5                   10                  15
Phe Val Ala Ala Ala Asp Phe Lys Val Tyr Trp Glu Val Pro Ser Phe
            20                  25                  30
Leu Cys Ser Lys Arg Phe Lys Ile Asn Val Thr Glu Val Leu Thr Ser
        35                  40                  45
His Glu Ile Leu Val Asn Gln Gly Glu Ser Phe Asn Gly Asp Lys Ile
    50                  55                  60
Val Ile Phe Tyr Glu Asn Gln Leu Gly Lys Tyr Pro His Ile Asp Ser
65                  70                  75                  80
Asn Asn Val Glu Ile Asn Gly Gly Ile Leu Gln Val Ala Asp Leu Ala
                85                  90                  95
Lys His Leu Lys Val Ala Lys Asp Asn Ile Thr Lys Phe Val Pro Asn
            100                 105                 110
Pro Asn Phe Asn Gly Val Gly Val Ile Asp Trp Glu Ala Trp Arg Pro
        115                 120                 125
Ser Trp Glu Phe Asn Trp Gly Lys Leu Lys Val Tyr Lys Glu Lys Ser
    130                 135                 140
Ile Asp Leu Val Lys Ser Lys His Pro Glu Trp Pro Ser Asp Arg Val
145                 150                 155                 160
Glu Lys Val Ala Lys Glu Glu Trp Glu Glu Ser Ala Lys Glu Trp Met
                165                 170                 175
Val Lys Thr Leu Lys Leu Ala Gln Glu Met Arg Pro Asn Ala Val Trp
            180                 185                 190
Cys Tyr Tyr Leu Phe Pro Asp Cys Tyr Asn Tyr Phe Gly Lys Asp Gln
        195                 200                 205
Pro Ser Gln Phe Ser Cys Ser Ser Arg Ile Gln Lys Glu Asn Ser Arg
    210                 215                 220
Leu Ser Trp Leu Trp Asn Gln Ser Thr Ala Ile Cys Leu Ser Ile Tyr
225                 230                 235                 240
Ile Gln Glu Ser His Val Thr Lys Tyr Asn Met Ser Gln Arg Thr Trp
                245                 250                 255
Trp Ile Asp Ala Arg Leu Arg Glu Ala Ile Arg Val Ser Glu His Arg
            260                 265                 270
Pro Asn Ile Pro Ile Tyr Pro Tyr Ile Asn Tyr Ile Leu Pro Gly Thr
        275                 280                 285
Asn Gln Thr Val Pro Ala Met Asp Phe Lys Arg Thr Leu Gly Gln Ile
    290                 295                 300
Ala Ser Leu Gly Leu Asp Gly Ala Leu Leu Trp Gly Ser Ser Tyr His
305                 310                 315                 320
Val Leu Thr Glu Ser Gln Cys Lys Ile Thr Ser Asp Tyr Val Lys Ser
                325                 330                 335
Val Ile Ala Pro Thr Val Ala Thr Val Val Leu Asn Thr Asn Arg Cys
            340                 345                 350
Ser Gln Ile Ile Cys Lys Gly Arg Gly Asn Cys Val Trp Pro Glu Glu
        355                 360                 365
Pro Phe Ser Ser Trp Lys Tyr Leu Val Asp Pro Lys Met Pro Val Phe
    370                 375                 380
Lys Pro Thr Asn Ile His Cys Lys Cys Lys Gly Tyr Leu Gly Arg Tyr
385                 390                 395                 400
Cys Glu Ile Pro Lys Gly Ser Ala Ile Ser Gln Ile Thr Asp Gly Gln
                405                 410                 415
Ile Gln Ala Thr Thr Thr Ala Thr Thr Glu Ala Thr Thr Thr Ala Ala
            420                 425                 430
Pro Ser Ser Thr Val Glu Thr Val Ser Pro Ser Ser Thr Glu Thr Ile
        435                 440                 445
Ser Gln Gln Thr Glu Asn Gly Ala Ala Lys Ala Ala Val Gly Met Gly
    450                 455                 460
Ala Gly Ala Leu Ala Ala Ala Ala Met Leu Leu
465                 470                 475

<210> 41
<211> 360
<212> PRT
<213> Vespa magnifica

<220>
<223> Amino acid sequence of hyaluronidase (HYAL) originating from
      Vespa magnifica with a N-terminal secretion signal

<400> 41
Met Gln Phe Ser Thr Val Ala Ser Val Ala Phe Val Ala Leu Ala Asn
1               5                   10                  15
Phe Val Ala Ala Asp Ser Cys Gly Ser Asn Cys Glu Lys Ser Glu Arg
            20                  25                  30
Pro Lys Arg Val Phe Asn Ile Tyr Trp Asn Val Pro Thr Phe Met Cys
        35                  40                  45
His Gln Tyr Gly Leu Tyr Phe Asp Glu Val Thr Asn Phe Asn Ile Lys
    50                  55                  60
His Asn Ser Lys Asp Asn Phe Gln Gly Asp Lys Ile Ala Ile Phe Tyr
65                  70                  75                  80
Asp Pro Gly Glu Phe Pro Ala Leu Leu Pro Leu Asn Tyr Gly Lys Tyr
                85                  90                  95
Lys Ile Arg Asn Gly Gly Val Pro Gln Glu Gly Asn Ile Thr Ile His
            100                 105                 110
Leu Gln Arg Phe Ile Glu Tyr Leu Asp Lys Thr Tyr Pro Asn Arg Asn
        115                 120                 125
Phe Ser Gly Ile Gly Val Ile Asp Phe Glu Arg Trp Arg Pro Ile Phe
    130                 135                 140
Arg Gln Asn Trp Gly Asn Met Lys Ile Tyr Lys Asn Phe Ser Ile Asp
145                 150                 155                 160
Leu Val Arg Lys Glu His Pro Phe Trp Asn Lys Lys Met Ile Glu Leu
                165                 170                 175
Glu Ala Ser Lys Arg Phe Glu Lys Tyr Ala Arg Leu Phe Met Glu Glu
            180                 185                 190
Thr Leu Lys Leu Ala Lys Lys Thr Arg Lys Gln Ala Asp Trp Gly Tyr
        195                 200                 205
Tyr Gly Tyr Pro Tyr Cys Phe Asn Met Ser Pro Thr Asn Phe Val Pro
    210                 215                 220
Asp Cys Asp Val Thr Ala Arg Asp Glu Asn Asn Glu Met Ser Trp Leu
225                 230                 235                 240
Phe Asn Asn Gln Asn Val Leu Leu Pro Ser Val Tyr Ile Arg Arg Glu
                245                 250                 255
Leu Thr Pro Asp Gln Arg Ile Gly Leu Val Gln Gly Arg Val Lys Glu
            260                 265                 270
Ala Val Arg Ile Ser Asn Lys Leu Lys His Ser Pro Lys Val Phe Ser
        275                 280                 285
Tyr Trp Trp Tyr Val Tyr Gln Asp Glu Thr Asn Thr Phe Leu Thr Glu
    290                 295                 300
Thr Asp Val Lys Lys Thr Phe Gln Glu Ile Val Ile Asn Gly Gly Asp
305                 310                 315                 320
Gly Ile Ile Ile Trp Gly Ser Ser Ser Asp Val Asn Ser Leu Ser Lys
                325                 330                 335
Cys Thr Arg Leu Arg Glu Tyr Leu Leu Thr Val Leu Gly Pro Ile Ala
            340                 345                 350
Val Asn Val Thr Glu Ala Val Asn
        355                 360

<210> 42
<211> 428
<212> PRT
<213> Vespa magnifica

<220>
<223> Amino acid sequence of hyaluronidase (HYAL) originating from
      Vespa magnifica with a N-terminal secretion signal and a
      C-terminal anchoring signal

<400> 42
Met Gln Phe Ser Thr Val Ala Ser Val Ala Phe Val Ala Leu Ala Asn
1               5                   10                  15
Phe Val Ala Ala Asp Ser Cys Gly Ser Asn Cys Glu Lys Ser Glu Arg
            20                  25                  30
Pro Lys Arg Val Phe Asn Ile Tyr Trp Asn Val Pro Thr Phe Met Cys
        35                  40                  45
His Gln Tyr Gly Leu Tyr Phe Asp Glu Val Thr Asn Phe Asn Ile Lys
    50                  55                  60
His Asn Ser Lys Asp Asn Phe Gln Gly Asp Lys Ile Ala Ile Phe Tyr
65                  70                  75                  80
Asp Pro Gly Glu Phe Pro Ala Leu Leu Pro Leu Asn Tyr Gly Lys Tyr
                85                  90                  95
Lys Ile Arg Asn Gly Gly Val Pro Gln Glu Gly Asn Ile Thr Ile His
            100                 105                 110
Leu Gln Arg Phe Ile Glu Tyr Leu Asp Lys Thr Tyr Pro Asn Arg Asn
        115                 120                 125
Phe Ser Gly Ile Gly Val Ile Asp Phe Glu Arg Trp Arg Pro Ile Phe
    130                 135                 140
Arg Gln Asn Trp Gly Asn Met Lys Ile Tyr Lys Asn Phe Ser Ile Asp
145                 150                 155                 160
Leu Val Arg Lys Glu His Pro Phe Trp Asn Lys Lys Met Ile Glu Leu
                165                 170                 175
Glu Ala Ser Lys Arg Phe Glu Lys Tyr Ala Arg Leu Phe Met Glu Glu
            180                 185                 190
Thr Leu Lys Leu Ala Lys Lys Thr Arg Lys Gln Ala Asp Trp Gly Tyr
        195                 200                 205
Tyr Gly Tyr Pro Tyr Cys Phe Asn Met Ser Pro Thr Asn Phe Val Pro
    210                 215                 220
Asp Cys Asp Val Thr Ala Arg Asp Glu Asn Asn Glu Met Ser Trp Leu
225                 230                 235                 240
Phe Asn Asn Gln Asn Val Leu Leu Pro Ser Val Tyr Ile Arg Arg Glu
                245                 250                 255
Leu Thr Pro Asp Gln Arg Ile Gly Leu Val Gln Gly Arg Val Lys Glu
            260                 265                 270
Ala Val Arg Ile Ser Asn Lys Leu Lys His Ser Pro Lys Val Phe Ser
        275                 280                 285
Tyr Trp Trp Tyr Val Tyr Gln Asp Glu Thr Asn Thr Phe Leu Thr Glu
    290                 295                 300
Thr Asp Val Lys Lys Thr Phe Gln Glu Ile Val Ile Asn Gly Gly Asp
305                 310                 315                 320
Gly Ile Ile Ile Trp Gly Ser Ser Ser Asp Val Asn Ser Leu Ser Lys
                325                 330                 335
Cys Thr Arg Leu Arg Glu Tyr Leu Leu Thr Val Leu Gly Pro Ile Ala
            340                 345                 350
Val Asn Val Thr Glu Ala Val Asn Ala Ile Ser Gln Ile Thr Asp Gly
        355                 360                 365
Gln Ile Gln Ala Thr Thr Thr Ala Thr Thr Glu Ala Thr Thr Thr Ala
    370                 375                 380
Ala Pro Ser Ser Thr Val Glu Thr Val Ser Pro Ser Ser Thr Glu Thr
385                 390                 395                 400
Ile Ser Gln Gln Thr Glu Asn Gly Ala Ala Lys Ala Ala Val Gly Met
                405                 410                 415
Gly Ala Gly Ala Leu Ala Ala Ala Ala Met Leu Leu
            420                 425

<210> 43
<211> 60
<212> DNA
<213> Artificial Sequence

<220>
<223> Nucleic acid sequence of the secretion sequence added in 5â\200\231

<400> 43
atgcaattta gcacagtcgc atcagtagcc ttcgttgcct tggccaactt cgtggcagca     60

<210> 44
<211> 20
<212> PRT
<213> Artificial Sequence

<220>
<223> Amino acid sequence of the secretion sequence added in N-ter

<400> 44
Met Gln Phe Ser Thr Val Ala Ser Val Ala Phe Val Ala Leu Ala Asn
1               5                   10                  15
Phe Val Ala Ala
            20

<210> 45
<211> 213
<212> DNA
<213> Artificial Sequence

<220>
<223> Nucleic acid sequence of the anchoring sequence added in 3â\200\231

<400> 45
ggatccgcca tttctcaaat cactgacggt caaatccaag ctactaccac tgctaccacc     60

gaagctacca ccactgctgc cccatcttcc accgttgaaa ctgtttctcc atccagcacc    120

gaaactatct ctcaacaaac tgaaaatggt gctgctaagg ccgctgtcgg tatgggtgcc    180

ggtgctctag ctgctgctgc tatgttgtta taa                                 213

<210> 46
<211> 68
<212> PRT
<213> Artificial Sequence

<220>
<223> Amino acid sequence of the anchoring sequence added in C-ter

<400> 46
Ala Ile Ser Gln Ile Thr Asp Gly Gln Ile Gln Ala Thr Thr Thr Ala
1               5                   10                  15
Thr Thr Glu Ala Thr Thr Thr Ala Ala Pro Ser Ser Thr Val Glu Thr
            20                  25                  30
Val Ser Pro Ser Ser Thr Glu Thr Ile Ser Gln Gln Thr Glu Asn Gly
        35                  40                  45

Ala Ala Lys Ala Ala Val Gly Met Gly Ala Gly Ala Leu Ala Ala Ala
    50                  55                  60
Ala Met Leu Leu
65

<210> 47
<211> 2154
<212> DNA
<213> Saccharomyces cerevisiae


<220>
<223> Nucleic acid sequence of glutamine-fructose-6-phosphate
      amidotransferase (GFA1) originating from

<400> 47
atgtgtggta tctttggtta ctgcaattat ctagtggaaa gatccagagg agaaattatc      60

gacaccttag tggatggttt acaaagatta gaatatagag gctatgattc caccggtatt     120

gctatcgatg gtgacgaagc tgattctact ttcatctata agcaaatcgg taaagtgagt     180

gctttgaaag aggagattac taagcaaaat ccgaacagag acgttacttt tgtctctcat     240

tgtggtattg cgcatactag atgggctact cacggtcgac cagaacaagt taactgtcac     300

cctcaaagat ctgacccaga agaccaattt gtggtcgttc ataatggtat catcacaaat     360

tttagagaac tgaagactct tttaattaac aaaggttata aattcgaaag tgataccgat     420

accgagtgta ttgctaaact atatttgcat ttatacaata caaatttaca aaatgggcat     480

gacttagatt tccacgaatt aaccaagcta gttcttttag aactagaagg ttcatacggg     540

ttattatgta aatcttgtca ctatcctaat gaggttatcg ccactagaaa agggtcccct     600

ttactgattg gtgtcaaatc tgaaaaaaaa ctaaaagtcg acttcgtgga tgtggaattt     660

cccgaagaaa acgctggtca accggaaatt ccattgaaat ctaacaacaa atcatttggc     720

ttgggcccaa agaaagctcg tgaatttgaa gctggttccc aaaatgccaa tttactacca     780

attgccgcca atgaatttaa cttgagacat tctcaatcca gggctttcct atcagaagat     840

ggatctccaa caccggtgga attttttgtt tcttcggatg cggcatctgt tgttaaacat     900

accaagaagg tgctattttt agaagatgac gatttggctc atatttacga tggtgagtta     960

catattcata gatctagaag agaagtaggc gcatcaatga caaggtccat tcaaacttta    1020

gagatggagt tagctcagat catgaagggc ccttacgacc attttatgca aaaggaaatc    1080

tatgagcaac cagaatctac tttcaatact atgagaggta gaatcgacta tgaaaataat    1140

aaagtgatat tgggtggttt aaaggcatgg ttaccagttg tcagaagagc acggagactg    1200

atcatgatcg catgcggtac ttcttatcat tcatgtttgg ctactcgtgc tatcttcgaa    1260

gaattatcag atatcccagt tagtgtggaa ttagcgtctg actttctgga cagaaaatgc    1320

cctgtcttca gagacgatgt atgcgtgttt gtttcacaaa gtggtgaaac tgcggatacc    1380

atgctggctc taaattattg tttagaaaga ggagccttaa ctgtcggaat tgttaacagt    1440

gttggttctt ctatctctcg tgtcacccac tgtggtgttc atattaacgc tggtcctgaa 1500

attggtgttg cctctacaaa agcttatact tcccagtata ttgccttagt gatgtttgct 1560

ctatcgctgt cagatgaccg tgtatcgaaa atagacagaa gaattgaaat cattcaaggc 1620

ttgaagttaa tcccgggcca aattaagcag gtattaaagc tggaaccaag aataaaaaag 1680

ctctgtgcga ctgaattaaa ggatcaaaaa tctctattgt tattgggtag aggttaccaa 1740

tttgctgctg ctctggaagg tgctttgaag atcaaagaaa tttcttatat gcattctgaa 1800

ggtgttttgg caggtgagtt gaagcacggt gtcttggcct tggtggacga aaacttgcca 1860

atcattgctt ttggtaccag agactctcta ttccctaaag tagtttcctc tattgagcaa 1920

gttactgtaa gaaagggcca tccaattatt atttgtaacg aaaatgatga agtgtgggcg 1980

caaaaatcta aatcaatcga cctgcaaacc ttagaagttc cacaaactgt tgattgttta 2040

caaggtctaa ttaatattat tccattacaa ctaatgtcat attggttggc tgttaataaa 2100

gggattgatg ttgattttcc aagaaacttg gctaaatctg ttaccgtcga ataa 2154

<210> 48
<211> 1788
<212> DNA
<213> Paramecium bursaria Chlorella virus 1

<220>
<223> Reencoded nucleic acid sequence of glutamine-fructose-6-phosphate
      amidotransferase (GFA1) originating from Chlorella virus 1
      (PBCV-1)

<400> 48
atgtgcggga tcttcggtgc tgtgtcgaac aataatagca tagaagtttc catcaagggt 60

atacagaagc tagagtaccg cgggtacgat tcgtgtggaa tagcctatac agacggaggc 120

gccattgaac ggatcaggtc aatagatggg atcgacgact taaggaagaa aacaataaca 180

gagtcttccc cggtagctat cgctcattca aggtggagta ccactgggat tccaagtgtt 240

gtgaacgccc acccgcacat ctctcggggc acgtctggat gcgagtcgcg cattgcagtc 300

gtacacaatg gcattattga aaattaccag cagatccgca agtacttgat aaatcttggg 360

tatactttcg attcacagac cgacacggaa gtcattgctc atctaataga ctcgcagtat 420

aacgggaaca tcctgcacac cgtccaaatg gcagttaagc acctcaaagg aagctacgcc 480

atagcagtca tgtgccataa ggagtccggg aagatcgtgg tggcaaaaca gaagagtccg 540

ttggtattgg gtataggaag cgacggagct tattatatcg catcggacgt attggctctt 600

cccactaaca aagtggtcta cataagtgac ggattctctg ctgagttaag tcctggatcg 660

atgactatct atgatctgga tggcaataag gtagaatacg aggtggaaga cgttgaaatg 720

gagcagacgt cgatgtcttt agataacttt gatcactaca tgatcaaaga gatcaatgaa 780

cagccgatat caattctcaa tactattaag aacaaaggat tctatgcaga gatatttggt 840

gatctagcac atgaaatatt tcagaagatc gacaatatat tgattctagc ctgcggaact 900

tcgtatcatg caggtctcgt cgggaagcaa tggatagaga ccatctcacg catcccggtg 960

gatgttcata ttgcaagcga gtatgagccg acaattccac gagccaatac acttgtgata 1020

acgattagtc aaagcggcga gactgcggat acaatcgcag cccttcaacg agcgcaaaac 1080

gcaggaatga tatacacttt gtgtatctgc aacagcccca agtcaactct agtgcgcgag 1140

tcgataatga aatacataac caaatgcggt tcagaagttt ctgttgccag tactaaagca 1200

tttacgtcgc aacttgtggt gttgtatatg cttgcgaacg ttcttgcgaa caaaactgat 1260

gatctgctag gggatcttcc tcaagctatc gagagagtaa tctgtcttac taatgacgaa 1320

atgaagcgtt gggcggatga aatttgtact gccaaaagtg ccatcttctt agggagagga 1380

ctgaacgcac ctgtagcatt tgagggcgcg ctaaagttga aggaaatctc atatatccat 1440

gccgagggtt tcctcggtgg agagttgaag catgggccgc tggctttgtt ggacgataag 1500

atccctgtaa tcgtgactgt tgctgaccac gcttatttgg atcacattaa ggctaacatc 1560

gacgaggtat tggcacgaaa cgttaccgta tacgcgatcg tcgatcagta tgttaatatt 1620

gaaccacagg agcgactgca tgtagtaaaa gtgccgtttg tgagtaaaga gttttctccc 1680

atcatccaca caattccgat gcaattgtta tcgtattatg tcgcgatcaa gctgggcaag 1740

aacgttgaca agccacgtaa cctggcgaaa agtgttacaa cattctaa 1788

<210> 49
<211> 1788
<212> DNA
<213> Paramecium bursaria Chlorella virus 1

<220>
<223> Reencoded nucleic acid sequence of glutamine-fructose-6-phosphate
      amidotransferase (GFA1) originating from Chlorella virus 1
      (PBCV-1)

<400> 49
   atgtgtggca tctttggagc agtgtcaaac aacaactcta tcgaggtgtc aatcaagggt 60
   attcagaagc tagaatatcg tgggtatgat tcgtgcggta ttgcgtatac agatgggggt 120
   gcgattgagc gtatacgttc tattgacggc attgacgatc tgcgtaagaa aacaatcaca 180
   gaatcatcac cagtggccat tgctcactcg cggtggagca ccactggaat tccatcagtg 240
   gtgaacgcac atcctcatat ttctcgcgga accagtgggt gtgagtctcg tatcgcggta 300
   gtccacaacg gtatcattga aaactatcag cagatccgaa aatatctcat caatcttggt 360
   tatacgtttg atagtcaaac ggacacagag gtcattgcgc atttgattga ttctcagtac 420
   aatgggaata tcttgcacac cgtccaaatg gctgtcaagc acctgaaggg ctcttatgcc 480
   attgcagtta tgtgtcataa agagtctggt aaaatagtcg tggcgaaaca gaagtcaccc 540
   ctcgtacttg gaatcggctc agatggtgct tactacatcg cttcggacgt gctggcgctg 600
   ccgacaaata aagttgttta tatttcagac ggtttctccg cagaactatc tccagggagt 660
   atgaccattt acgatcttga tggaaataaa gtagaatatg aagtagagga cgttgaaatg 720
   gaacaaacta gtatgtctct cgataacttt gatcattaca tgattaagga aattaatgag 780
   caaccaatca gtattctaaa cactataaaa aataaagggt tctatgcaga aatattcggt 840
   gatttggctc atgaaatctt ccaaaaaata gacaacatcc tgatactggc ttgtggtaca 900
   agttatcacg ccggtcttgt aggaaaacag tggatagaga ccatctctag aatccccgtg 960
   gatgttcaca tcgcgagtga atacgaacct actattccga gagcgaacac attggtaatc 1020
   actatttcac agtcgggtga aactgcggac acgatagcgg ctttgcaacg ggcccaaaac 1080
   gccgggatga tttatacatt gtgtatttgc aattcaccaa agagcactct tgttcgtgag 1140
   agcattatga agtacatcac gaaatgtggt tctgaggtgt cagtggcatc aacgaaggcg 1200
   tttacttctc agctcgtagt actgtacatg ctggcaaacg tattggcaaa taaaaccgat 1260
   gatttgctgg gagacctccc acaggcaata gaacgggtaa tttgtttgac aaatgacgaa 1320
   atgaaacgat gggccgacga aatttgcact gcgaaatccg cgatcttcct gggaagagga 1380
   ctaaacgcac cagttgcctt tgagggagcg ttgaagctca aagaaatctc ttacattcat 1440
   gcagagggct tcctgggagg tgagttgaaa cacggccccc tcgcactcct tgatgacaag 1500
   attcctgtta tcgtaaccgt agcagatcat gcttatttgg accatatcaa agcaaatatc 1560
   gacgaagtgc ttgcgaggaa cgttacggta tacgccatag tagaccagta tgtgaacatc 1620
   gagccccagg aacgccttca cgtcgtcaag gttccgtttg tatccaaaga attttctccg 1680
   ataattcaca ctatcccgat gcaactgctt tcgtattacg tggcaattaa gcttggaaag 1740
   aacgttgaca aaccaaggaa tcttgcaaaa tccgtgacta ccttttaa 1788

<210> 50
<211> 717
<212> PRT
<213> Saccharomyces cerevisiae

<220>
<223> Amino acid sequence of glutamine-fructose-6-phosphate
      amidotransferase (GFA1) originating from Saccharomyces cerevisiae

<400> 50
Met Cys Gly Ile Phe Gly Tyr Cys Asn Tyr Leu Val Glu Arg Ser Arg
1               5                   10                  15
Gly Glu Ile Ile Asp Thr Leu Val Asp Gly Leu Gln Arg Leu Glu Tyr
            20                  25                  30
Arg Gly Tyr Asp Ser Thr Gly Ile Ala Ile Asp Gly Asp Glu Ala Asp
        35                  40                  45

Ser Thr Phe Ile Tyr Lys Gln Ile Gly Lys Val Ser Ala Leu Lys Glu
    50                  55                  60
Glu Ile Thr Lys Gln Asn Pro Asn Arg Asp Val Thr Phe Val Ser His
65                  70                  75                  80
Cys Gly Ile Ala His Thr Arg Trp Ala Thr His Gly Arg Pro Glu Gln
                85                  90                  95
Val Asn Cys His Pro Gln Arg Ser Asp Pro Glu Asp Gln Phe Val Val
            100                 105                 110
Val His Asn Gly Ile Ile Thr Asn Phe Arg Glu Leu Lys Thr Leu Leu
        115                 120                 125
Ile Asn Lys Gly Tyr Lys Phe Glu Ser Asp Thr Asp Thr Glu Cys Ile
    130                 135                 140
Ala Lys Leu Tyr Leu His Leu Tyr Asn Thr Asn Leu Gln Asn Gly His
145                 150                 155                 160
Asp Leu Asp Phe His Glu Leu Thr Lys Leu Val Leu Leu Glu Leu Glu
                165                 170                 175
Gly Ser Tyr Gly Leu Leu Cys Lys Ser Cys His Tyr Pro Asn Glu Val
            180                 185                 190
Ile Ala Thr Arg Lys Gly Ser Pro Leu Leu Ile Gly Val Lys Ser Glu
        195                 200                 205
Lys Lys Leu Lys Val Asp Phe Val Asp Val Glu Phe Pro Glu Glu Asn
    210                 215                 220
Ala Gly Gln Pro Glu Ile Pro Leu Lys Ser Asn Asn Lys Ser Phe Gly
225                 230                 235                 240
Leu Gly Pro Lys Lys Ala Arg Glu Phe Glu Ala Gly Ser Gln Asn Ala
                245                 250                 255
Asn Leu Leu Pro Ile Ala Ala Asn Glu Phe Asn Leu Arg His Ser Gln
            260                 265                 270
Ser Arg Ala Phe Leu Ser Glu Asp Gly Ser Pro Thr Pro Val Glu Phe
        275                 280                 285
Phe Val Ser Ser Asp Ala Ala Ser Val Val Lys His Thr Lys Lys Val
    290                 295                 300
Leu Phe Leu Glu Asp Asp Asp Leu Ala His Ile Tyr Asp Gly Glu Leu
305                 310                 315                 320
His Ile His Arg Ser Arg Arg Glu Val Gly Ala Ser Met Thr Arg Ser
                325                 330                 335
Ile Gln Thr Leu Glu Met Glu Leu Ala Gln Ile Met Lys Gly Pro Tyr
            340                 345                 350
Asp His Phe Met Gln Lys Glu Ile Tyr Glu Gln Pro Glu Ser Thr Phe
        355                 360                 365
Asn Thr Met Arg Gly Arg Ile Asp Tyr Glu Asn Asn Lys Val Ile Leu
    370                 375                 380
Gly Gly Leu Lys Ala Trp Leu Pro Val Val Arg Arg Ala Arg Arg Leu
385                 390                 395                 400
Ile Met Ile Ala Cys Gly Thr Ser Tyr His Ser Cys Leu Ala Thr Arg
                405                 410                 415
Ala Ile Phe Glu Glu Leu Ser Asp Ile Pro Val Ser Val Glu Leu Ala
            420                 425                 430
Ser Asp Phe Leu Asp Arg Lys Cys Pro Val Phe Arg Asp Asp Val Cys
        435                 440                 445
Val Phe Val Ser Gln Ser Gly Glu Thr Ala Asp Thr Met Leu Ala Leu
    450                 455                 460
Asn Tyr Cys Leu Glu Arg Gly Ala Leu Thr Val Gly Ile Val Asn Ser
465                 470                 475                 480
Val Gly Ser Ser Ile Ser Arg Val Thr His Cys Gly Val His Ile Asn
                485                 490                 495
Ala Gly Pro Glu Ile Gly Val Ala Ser Thr Lys Ala Tyr Thr Ser Gln
            500                 505                 510
Tyr Ile Ala Leu Val Met Phe Ala Leu Ser Leu Ser Asp Asp Arg Val
        515                 520                 525
Ser Lys Ile Asp Arg Arg Ile Glu Ile Ile Gln Gly Leu Lys Leu Ile
    530                 535                 540
Pro Gly Gln Ile Lys Gln Val Leu Lys Leu Glu Pro Arg Ile Lys Lys

545                 550                 555                 560
Leu Cys Ala Thr Glu Leu Lys Asp Gln Lys Ser Leu Leu Leu Leu Gly
                565                 570                 575
Arg Gly Tyr Gln Phe Ala Ala Ala Leu Glu Gly Ala Leu Lys Ile Lys
            580                 585                 590
Glu Ile Ser Tyr Met His Ser Glu Gly Val Leu Ala Gly Glu Leu Lys
        595                 600                 605
His Gly Val Leu Ala Leu Val Asp Glu Asn Leu Pro Ile Ile Ala Phe
    610                 615                 620
Gly Thr Arg Asp Ser Leu Phe Pro Lys Val Val Ser Ser Ile Glu Gln
625                 630                 635                 640
Val Thr Ala Arg Lys Gly His Pro Ile Ile Ile Cys Asn Glu Asn Asp
                645                 650                 655
Glu Val Trp Ala Gln Lys Ser Lys Ser Ile Asp Leu Gln Thr Leu Glu
            660                 665                 670
Val Pro Gln Thr Val Asp Cys Leu Gln Gly Leu Ile Asn Ile Ile Pro
        675                 680                 685
Leu Gln Leu Met Ser Tyr Trp Leu Ala Val Asn Lys Gly Ile Asp Val
    690                 695                 700
Asp Phe Pro Arg Asn Leu Ala Lys Ser Val Thr Val Glu
705                 710                 715

<210> 51
<211> 595
<212> PRT
<213> Paramecium bursaria Chlorella virus 1

<220>
<223> Amino acid sequence of glutamine-fructose-6-phosphate
      amidotransferase (GFA1) originating from Chlorella virus 1
      (PBCV-1)

<400> 51
Met Cys Gly Ile Phe Gly Ala Val Ser Asn Asn Asn Ser Ile Glu Val
1               5                   10                  15
Ser Ile Lys Gly Ile Gln Lys Leu Glu Tyr Arg Gly Tyr Asp Ser Cys
            20                  25                  30
Gly Ile Ala Tyr Thr Asp Gly Gly Ala Ile Glu Arg Ile Arg Ser Ile
        35                  40                  45
Asp Gly Ile Asp Asp Leu Arg Lys Lys Thr Ile Thr Glu Ser Ser Pro
    50                  55                  60
Val Ala Ile Ala His Ser Arg Trp Ser Thr Thr Gly Ile Pro Ser Val
65                  70                  75                  80
Val Asn Ala His Pro His Ile Ser Arg Gly Thr Ser Gly Cys Glu Ser
                85                  90                  95
Arg Ile Ala Val Val His Asn Gly Ile Ile Glu Asn Tyr Gln Gln Ile
            100                 105                 110
Arg Lys Tyr Leu Ile Asn Leu Gly Tyr Thr Phe Asp Ser Gln Thr Asp
        115                 120                 125
Thr Glu Val Ile Ala His Leu Ile Asp Ser Gln Tyr Asn Gly Asn Ile
    130                 135                 140
Leu His Thr Val Gln Met Ala Val Lys His Leu Lys Gly Ser Tyr Ala
145                 150                 155                 160
Ile Ala Val Met Cys His Lys Glu Ser Gly Lys Ile Val Val Ala Lys
                165                 170                 175
Gln Lys Ser Pro Leu Val Leu Gly Ile Gly Ser Asp Gly Ala Tyr Tyr
            180                 185                 190
Ile Ala Ser Asp Val Leu Ala Leu Pro Thr Asn Lys Val Val Tyr Ile
        195                 200                 205
Ser Asp Gly Phe Ser Ala Glu Leu Ser Pro Gly Ser Met Thr Ile Tyr
    210                 215                 220
Asp Leu Asp Gly Asn Lys Val Glu Tyr Glu Val Glu Asp Val Glu Met

225                 230                 235                 240
Glu Gln Thr Ser Met Ser Leu Asp Asn Phe Asp His Tyr Met Ile Lys
                245                 250                 255
Glu Ile Asn Glu Gln Pro Ile Ser Ile Leu Asn Thr Ile Lys Asn Lys
            260                 265                 270
Gly Phe Tyr Ala Glu Ile Phe Gly Asp Leu Ala His Glu Ile Phe Gln
        275                 280                 285
Lys Ile Asp Asn Ile Leu Ile Leu Ala Cys Gly Thr Ser Tyr His Ala
    290                 295                 300
Gly Leu Val Gly Lys Gln Trp Ile Glu Thr Ile Ser Arg Ile Pro Val
305                 310                 315                 320
Asp Val His Ile Ala Ser Glu Tyr Glu Pro Thr Ile Pro Arg Ala Asn
                325                 330                 335
Thr Leu Val Ile Thr Ile Ser Gln Ser Gly Glu Thr Ala Asp Thr Ile
            340                 345                 350
Ala Ala Leu Gln Arg Ala Gln Asn Ala Gly Met Ile Tyr Thr Leu Cys
        355                 360                 365
Ile Cys Asn Ser Pro Lys Ser Thr Leu Val Arg Glu Ser Ile Met Lys
    370                 375                 380
Tyr Ile Thr Lys Cys Gly Ser Glu Val Ser Val Ala Ser Thr Lys Ala
385                 390                 395                 400
Phe Thr Ser Gln Leu Val Val Leu Tyr Met Leu Ala Asn Val Leu Ala
                405                 410                 415
Asn Lys Thr Asp Asp Leu Leu Gly Asp Leu Pro Gln Ala Ile Glu Arg
            420                 425                 430
Val Ile Cys Leu Thr Asn Asp Glu Met Lys Arg Trp Ala Asp Glu Ile
        435                 440                 445
Cys Thr Ala Lys Ser Ala Ile Phe Leu Gly Arg Gly Leu Asn Ala Pro
    450                 455                 460
Val Ala Phe Glu Gly Ala Leu Lys Leu Lys Glu Ile Ser Tyr Ile His
465                 470                 475                 480
Ala Glu Gly Phe Leu Gly Gly Glu Leu Lys His Gly Pro Leu Ala Leu
                485                 490                 495
Leu Asp Asp Lys Ile Pro Val Ile Val Thr Val Ala Asp His Ala Tyr
            500                 505                 510
Leu Asp His Ile Lys Ala Asn Ile Asp Glu Val Leu Ala Arg Asn Val
        515                 520                 525
Thr Val Tyr Ala Ile Val Asp Gln Tyr Val Asn Ile Glu Pro Gln Glu
    530                 535                 540
Arg Leu His Val Val Lys Val Pro Phe Val Ser Lys Glu Phe Ser Pro
545                 550                 555                 560
Ile Ile His Thr Ile Pro Met Gln Leu Leu Ser Tyr Tyr Val Ala Ile
                565                 570                 575
Lys Leu Gly Lys Asn Val Asp Lys Pro Arg Asn Leu Ala Lys Ser Val
            580                 585                 590
Thr Thr Phe
        595

<210> 52
<211> 1434
<212> DNA
<213> Saccharomyces cerevisiae

<220>
<223> Nucleic acid sequence of UDP-N-acetylglucosamine
      pyrophosphorylase (QRI1) originating from Saccharomyces
      cerevisiae

<400> 52
atgactgaca caaaacagct attcattgaa gccggacaaa gtcaactttt ccacaattgg      60

gaaagcttgt ctcgcaaaga ccaagaagaa ttgctttcaa acctggagca aatatcttcc     120

aagaggtccc ctgcaaaact actggaagac tgtcaaaatg ctattaaatt ctcactagct 180

aactcttcta aggatactgg cgtcgaaatt tcaccattgc cccctacttc gtacgagtcg 240

cttattggca acagtaagaa agaaaatgaa tactggcgtt taggccttga agctattggc 300

aagggtgaag tcgcagtgat tttaatggct ggcggacaag gtacgcggtt aggatcctct 360

caaccaaagg gctgttacga cattggattg ccttctaaga aatctctttt tcaaattcaa 420

gctgaaaagt tgatcaggtt gcaagatatg gtaaaggaca aaaaggtaga aattccttgg 480

tatattatga catcaggccc cactagagcg gctactgagg catactttca agaacacaat 540

tattttggct tgaataaaga acaaattacg ttcttcaacc agggaaccct gcctgccttt 600

gatttaaccg ggaagcattt cctaatgaaa gacccagtaa acctatctca atcaccagat 660

ggaaatggtg gactctaccg tgccatcaag gaaaacaagt tgaacgaaga ctttgatagg 720

agaggaatca agcatgttta catgtactgt gtcgataatg tcctatctaa aatcgcagac 780

cctgtattta ttggttttgc catcaagcat ggcttcgaac tggccaccaa agccgttaga 840

aagagagatg cgcatgaatc agttgggtta attgctacta aaaacgagaa accatgtgtc 900

atagaatatt ctgaaatttc caatgaattg gctgaagcaa aggataaaga tggcttatta 960

aaactacgcg caggcaacat tgtaaatcat tattacctag tggatttact aaaacgtgat 1020

ttggatcagt ggtgtgagaa tatgccatat cacattgcga agaagaaaat tccagcttat 1080

gatagtgtta ccggcaagta cactaagcct accgaaccaa acggtataaa attagagcaa 1140

ttcatatttg atgtctttga cactgtacca ctgaacaagt ttgggtgctt agaagtagat 1200

agatgcaaag aattttcacc tttaaaaaac ggtcctggtt ctaagaacga taatcctgag 1260

accagcagac tagcatattt gaaactagga acctcgtggt tggaagatgc aggcgctatt 1320

gtaaaagatg gggtactagt cgaagtttcc agcaaattga gttatgcagg tgaaaatcta 1380

tcccagttca aaggtaaagt ctttgacaga agtggtatag tattagaaaa ataa 1434

<210> 53
<211> 477
<212> PRT
<213> Saccharomyces cerevisiae

<220>
<223> Amino acid sequence of UDP-N-acetylglucosamine pyrophosphorylase
      (QRI1) originating from Saccharomyces cerevisiae

<400> 53
Met Thr Asp Thr Lys Gln Leu Phe Ile Glu Ala Gly Gln Ser Gln Leu
1               5                   10                  15
Phe His Asn Trp Glu Ser Leu Ser Arg Lys Asp Gln Glu Glu Leu Leu
            20                  25                  30
Ser Asn Leu Glu Gln Ile Ser Ser Lys Arg Ser Pro Ala Lys Leu Leu
        35                  40                  45

Glu Asp Cys Gln Asn Ala Ile Lys Phe Ser Leu Ala Asn Ser Ser Lys
    50                  55                  60
Asp Thr Gly Val Glu Ile Ser Pro Leu Pro Pro Thr Ser Tyr Glu Ser
65                  70                  75                  80
Leu Ile Gly Asn Ser Lys Lys Glu Asn Glu Tyr Trp Arg Leu Gly Leu
                85                  90                  95
Glu Ala Ile Gly Lys Gly Glu Val Ala Val Ile Leu Met Ala Gly Gly
            100                 105                 110
Gln Gly Thr Arg Leu Gly Ser Ser Gln Pro Lys Gly Cys Tyr Asp Ile
        115                 120                 125
Gly Leu Pro Ser Lys Lys Ser Leu Phe Gln Ile Gln Ala Glu Lys Leu
    130                 135                 140
Ile Arg Leu Gln Asp Met Val Lys Asp Lys Lys Val Glu Ile Pro Trp
145                 150                 155                 160
Tyr Ile Met Thr Ser Gly Pro Thr Arg Ala Ala Thr Glu Ala Tyr Phe
                165                 170                 175
Gln Glu His Asn Tyr Phe Gly Leu Asn Lys Glu Gln Ile Thr Phe Phe
            180                 185                 190
Asn Gln Gly Thr Leu Pro Ala Phe Asp Leu Thr Gly Lys His Phe Leu
        195                 200                 205
Met Lys Asp Pro Val Asn Leu Ser Gln Ser Pro Asp Gly Asn Gly Gly
    210                 215                 220
Leu Tyr Arg Ala Ile Lys Glu Asn Lys Leu Asn Glu Asp Phe Asp Arg
225                 230                 235                 240
Arg Gly Ile Lys His Val Tyr Met Tyr Cys Val Asp Asn Val Leu Ser
                245                 250                 255
Lys Ile Ala Asp Pro Val Phe Ile Gly Phe Ala Ile Lys His Gly Phe
            260                 265                 270
Glu Leu Ala Thr Lys Ala Val Arg Lys Arg Asp Ala His Glu Ser Val
        275                 280                 285
Gly Leu Ile Ala Thr Lys Asn Glu Lys Pro Cys Val Ile Glu Tyr Ser
    290                 295                 300
Glu Ile Ser Asn Glu Leu Ala Glu Ala Lys Asp Lys Asp Gly Leu Leu
305                 310                 315                 320
Lys Leu Arg Ala Gly Asn Ile Val Asn His Tyr Tyr Leu Val Asp Leu
                325                 330                 335
Leu Lys Arg Asp Leu Asp Gln Trp Cys Glu Asn Met Pro Tyr His Ile
            340                 345                 350
Ala Lys Lys Lys Ile Pro Ala Tyr Asp Ser Val Thr Gly Lys Tyr Thr
        355                 360                 365
Lys Pro Thr Glu Pro Asn Gly Ile Lys Leu Glu Gln Phe Ile Phe Asp
    370                 375                 380
Val Phe Asp Thr Val Pro Leu Asn Lys Phe Gly Cys Leu Glu Val Asp
385                 390                 395                 400
Arg Cys Lys Glu Phe Ser Pro Leu Lys Asn Gly Pro Gly Ser Lys Asn
                405                 410                 415
Asp Asn Pro Glu Thr Ser Arg Leu Ala Tyr Leu Lys Leu Gly Thr Ser
            420                 425                 430
Trp Leu Glu Asp Ala Gly Ala Ile Val Lys Asp Gly Val Leu Val Glu
        435                 440                 445
Val Ser Ser Lys Leu Ser Tyr Ala Gly Glu Asn Leu Ser Gln Phe Lys
    450                 455                 460
Gly Lys Val Phe Asp Arg Ser Gly Ile Val Leu Glu Lys
465                 470                 475

<210> 54
<211> 1713
<212> DNA
<213> Saccharomyces cerevisiae

<220>
<223> Nucleic acid sequence of Phosphoglucomutase-1 (PGM1) originating

from Saccharomyces cerevisiae

<400> 54
atgtcacttc taatagattc tgtaccaaca gttgcttata aggaccaaaa accgggtact 60

tcaggtttac gtaagaagac caaggttttc atggatgagc ctcattatac tgagaacttc 120

attcaagcaa caatgcaatc tatccctaat ggctcagagg gaaccacttt agttgttgga 180

ggagatggtc gtttctacaa cgatgttatc atgaacaaga ttgccgcagt aggtgctgca 240

aacggtgtca gaaagttagt cattggtcaa ggcggtttac tttcaacacc agctgcttct 300

catataatta gaacatacga ggaaaagtgt accggtggtg gtatcatatt aactgcctca 360

cacaacccag gcggtccaga gaatgattta ggtatcaagt ataatttacc taatggtggg 420

ccagctccag agagtgtcac taacgctatc tgggaagcgt ctaaaaaatt aactcactat 480

aaaattataa agaacttccc caagttgaat ttgaacaagc ttggtaaaaa ccaaaaatat 540

ggcccattgt tagtggacat aattgatcct gccaaagcat acgttcaatt tctgaaggaa 600

atttttgatt ttgacttaat taaaagcttc ttagcgaaac agcgcaaaga caaagggtgg 660

aagttgttgt ttgactcctt aaatggtatt acaggaccat atggtaaggc tatatttgtt 720

gatgaatttg gtttaccggc agaggaagtt cttcaaaatt ggcacccttt acctgatttc 780

ggcggtttac atcccgatcc gaatctaacc tatgcacgaa ctcttgttga cagggttgac 840

cgcgaaaaaa ttgcctttgg agcagcctcc gatggtgatg gtgataggaa tatgatttac 900

ggttatggcc ctgctttcgt ttcgccaggt gattctgttg ccattattgc cgaatatgca 960

cccgaaattc catacttcgc caaacaaggt atttatggct tggcacgttc atttcctaca 1020

tcctcagcca ttgatcgtgt tgcagcaaaa aagggattaa gatgttacga agttccaacc 1080

ggctggaaat tcttctgtgc cttatttgat gctaaaaagc tatcaatctg tggtgaagaa 1140

tccttcggta caggttccaa tcatatcaga gaaaaggacg gtctatgggc cattattgct 1200

tggttaaata tcttggctat ctaccatagg cgtaaccctg aaaaggaagc ttcgatcaaa 1260

actattcagg acgaattttg gaacgagtat ggccgtactt tcttcacaag atacgattac 1320

gaacatatcg aatgcgagca ggccgaaaaa gttgtagctc ttttgagtga atttgtatca 1380

aggccaaacg tttgtggctc ccacttccca gctgatgagt ctttaaccgt tatcgattgt 1440

ggtgattttt cgtatagaga tctagatggc tccatctctg aaaatcaagg ccttttcgta 1500

aagttttcga atgggactaa atttgttttg aggttatccg gcacaggcag ttctggtgca 1560

acaataagat tatacgtaga aaagtatact gataaaaagg agaactatgg ccaaacagct 1620

gacgtcttct tgaaacccgt catcaactcc attgtaaaat tcttaagatt taaagaaatt 1680

ttaggaacag acgaaccaac agtccgcaca tag 1713

<210> 55

<211> 570
<212> PRT
<213> Saccharomyces cerevisiae

<220>
<223> Amino acid sequence Phosphoglucomutase-1 (PGM1) originating from
      Saccharomyces cerevisiae

<400> 55
Met Ser Leu Leu Ile Asp Ser Val Pro Thr Val Ala Tyr Lys Asp Gln
1               5                   10                  15
Lys Pro Gly Thr Ser Gly Leu Arg Lys Lys Thr Lys Val Phe Met Asp
            20                  25                  30
Glu Pro His Tyr Thr Glu Asn Phe Ile Gln Ala Thr Met Gln Ser Ile
        35                  40                  45
Pro Asn Gly Ser Glu Gly Thr Thr Leu Val Val Gly Gly Asp Gly Arg
    50                  55                  60
Phe Tyr Asn Asp Val Ile Met Asn Lys Ile Ala Ala Val Gly Ala Ala
65                  70                  75                  80
Asn Gly Val Arg Lys Leu Val Ile Gly Gln Gly Gly Leu Leu Ser Thr
                85                  90                  95
Pro Ala Ala Ser His Ile Ile Arg Thr Tyr Glu Glu Lys Cys Thr Gly
            100                 105                 110
Gly Gly Ile Ile Leu Thr Ala Ser His Asn Pro Gly Gly Pro Glu Asn
        115                 120                 125
Asp Leu Gly Ile Lys Tyr Asn Leu Pro Asn Gly Gly Pro Ala Pro Glu
    130                 135                 140
Ser Val Thr Asn Ala Ile Trp Glu Ala Ser Lys Lys Leu Thr His Tyr
145                 150                 155                 160
Lys Ile Ile Lys Asn Phe Pro Lys Leu Asn Leu Asn Lys Leu Gly Lys
                165                 170                 175
Asn Gln Lys Tyr Gly Pro Leu Leu Val Asp Ile Ile Asp Pro Ala Lys
            180                 185                 190
Ala Tyr Val Gln Phe Leu Lys Glu Ile Phe Asp Phe Asp Leu Ile Lys
        195                 200                 205
Ser Phe Leu Ala Lys Gln Arg Lys Asp Lys Gly Trp Lys Leu Leu Phe
    210                 215                 220
Asp Ser Leu Asn Gly Ile Thr Gly Pro Tyr Gly Lys Ala Ile Phe Val
225                 230                 235                 240
Asp Glu Phe Gly Leu Pro Ala Glu Glu Val Leu Gln Asn Trp His Pro
                245                 250                 255
Leu Pro Asp Phe Gly Gly Leu His Pro Asp Pro Asn Leu Thr Tyr Ala
            260                 265                 270
Arg Thr Leu Val Asp Arg Val Asp Arg Glu Lys Ile Ala Phe Gly Ala
        275                 280                 285
Ala Ser Asp Gly Asp Gly Asp Arg Asn Met Ile Tyr Gly Tyr Gly Pro
    290                 295                 300
Ala Phe Val Ser Pro Gly Asp Ser Val Ala Ile Ile Ala Glu Tyr Ala
305                 310                 315                 320
Pro Glu Ile Pro Tyr Phe Ala Lys Gln Gly Ile Tyr Gly Leu Ala Arg
                325                 330                 335
Ser Phe Pro Thr Ser Ser Ala Ile Asp Arg Val Ala Ala Lys Lys Gly
            340                 345                 350
Leu Arg Cys Tyr Glu Val Pro Thr Gly Trp Lys Phe Phe Cys Ala Leu
        355                 360                 365
Phe Asp Ala Lys Lys Leu Ser Ile Cys Gly Glu Glu Ser Phe Gly Thr
    370                 375                 380
Gly Ser Asn His Ile Arg Glu Lys Asp Gly Leu Trp Ala Ile Ile Ala
385                 390                 395                 400
Trp Leu Asn Ile Leu Ala Ile Tyr His Arg Arg Asn Pro Glu Lys Glu
                405                 410                 415
Ala Ser Ile Lys Thr Ile Gln Asp Glu Phe Trp Asn Glu Tyr Gly Arg

                420                 425                 430
Thr Phe Phe Thr Arg Tyr Asp Tyr Glu His Ile Glu Cys Glu Gln Ala
        435                 440                 445
Glu Lys Val Val Ala Leu Leu Ser Glu Phe Val Ser Arg Pro Asn Val
    450                 455                 460
Cys Gly Ser His Phe Pro Ala Asp Glu Ser Leu Thr Val Ile Asp Cys
465                 470                 475                 480
Gly Asp Phe Ser Tyr Arg Asp Leu Asp Gly Ser Ile Ser Glu Asn Gln
                485                 490                 495
Gly Leu Phe Val Lys Phe Ser Asn Gly Thr Lys Phe Val Leu Arg Leu
            500                 505                 510
Ser Gly Thr Gly Ser Ser Gly Ala Thr Ile Arg Leu Tyr Val Glu Lys
        515                 520                 525
Tyr Thr Asp Lys Lys Glu Asn Tyr Gly Gln Thr Ala Asp Val Phe Leu
    530                 535                 540
Lys Pro Val Ile Asn Ser Ile Val Lys Phe Leu Arg Phe Lys Glu Ile
545                 550                 555                 560
Leu Gly Thr Asp Glu Pro Thr Val Arg Thr
                565                 570

<210> 56
<211> 1500
<212> DNA
<213> Saccharomyces cerevisiae

<220>
<223> Nucleic acid sequence of UTP--glucose-1-phosphate
      uridylyltransferase (UGP1) originating from

<400> 56
atgtccacta agaagcacac caaaacacat tccacttatg cattcgagag caacacaaac      60

agcgttgctg cctcacaaat gagaaacgcc ttaaacaagt tggcggactc tagtaaactt     120

gacgatgctg ctcgcgctaa gtttgagaac gaactggatt cgtttttcac gcttttcagg     180

agatatttgg tagagaagtc ttctagaacc accttggaat gggacaagat caagtctccc     240

aacccggatg aagtggttaa gtatgaaatt atttctcagc agcccgagaa tgtctcaaac     300

ctttccaaat tggctgtttt gaagttgaac ggtgggctgg gtacctccat gggctgcgtt     360

ggccctaaat ctgttattga agtgagagag ggaaacacct ttttggattt gtctgttcgt     420

caaattgaat acttgaacag acagtacgat agcgacgtgc cattgttatt gatgaattct     480

ttcaacactg acaaggatac ggaacacttg attaagaagt attccgctaa cagaatcaga     540

atcagatctt tcaatcaatc caggttccca agagtctaca aggattcttt attgcctgtc     600

cccaccgaat acgattctcc actggatgct tggtatccac caggtcacgg tgatttgttt     660

gaatctttac acgtatctgg tgaactggat gccttaattg cccaaggaag agaaatatta     720

tttgtttcta acggtgacaa cttgggtgct accgtcgact taaaaatttt aaaccacatg     780

atcgagactg gtgccgaata tataatggaa ttgactgata agaccagagc cgatgttaaa     840

ggtggtactt tgatttctta cgatggtcaa gtccgtttat tggaagtcgc ccaagttcca     900

aaagaacaca ttgacgaatt caaaaatatc agaaagttta ccaacttcaa cacgaataac     960

ttatggatca atctgaaagc agtaaagagg ttgatcgaat cgagcaattt ggagatggaa 1020

atcattccaa accaaaaaac tataacaaga gacggtcatg aaattaatgt cttacaatta 1080

gaaaccgctt gtggtgctgc tatcaggcat tttgatggtg ctcacggtgt tgtcgttcca 1140

agatcaagat tcttgcctgt caagacctgt tccgatttgt tgctggttaa atcagatcta 1200

ttccgtctgg aacacggttc tttgaagtta gacccatccc gttttggtcc aaacccatta 1260

atcaagttgg gctcgcattt caaaaaggtt tctggtttta acgcaagaat ccctcacatc 1320

ccaaaaatcg tcgagctaga tcatttgacc atcactggta acgtcttttt aggtaaagat 1380

gtcactttga ggggtactgt catcatcgtt tgctccgacg gtcataaaat cgatattcca 1440

aacggctcca tattggaaaa tgttgtcgtt actggtaatt tgcaaatctt ggaacattga 1500

<210> 57
<211> 499
<212> PRT
<213> Saccharomyces cerevisiae

<220>
<223> Amino acid sequence of UTP--glucose-1-phosphate
      uridylyltransferase (UGP1) originating from Saccharomyces
      cerevisiae

<400> 57
Met Ser Thr Lys Lys His Thr Lys Thr His Ser Thr Tyr Ala Phe Glu
1               5                   10                  15
Ser Asn Thr Asn Ser Val Ala Ala Ser Gln Met Arg Asn Ala Leu Asn
            20                  25                  30
Lys Leu Ala Asp Ser Ser Lys Leu Asp Asp Ala Ala Arg Ala Lys Phe
        35                  40                  45
Glu Asn Glu Leu Asp Ser Phe Phe Thr Leu Phe Arg Arg Tyr Leu Val
    50                  55                  60
Glu Lys Ser Ser Arg Thr Thr Leu Glu Trp Asp Lys Ile Lys Ser Pro
65                  70                  75                  80
Asn Pro Asp Glu Val Val Lys Tyr Glu Ile Ile Ser Gln Gln Pro Glu
                85                  90                  95
Asn Val Ser Asn Leu Ser Lys Leu Ala Val Leu Lys Leu Asn Gly Gly
            100                 105                 110
Leu Gly Thr Ser Met Gly Cys Val Gly Pro Lys Ser Val Ile Glu Val
        115                 120                 125
Arg Glu Gly Asn Thr Phe Leu Asp Leu Ser Val Arg Gln Ile Glu Tyr
    130                 135                 140
Leu Asn Arg Gln Tyr Asp Ser Asp Val Pro Leu Leu Leu Met Asn Ser
145                 150                 155                 160
Phe Asn Thr Asp Lys Asp Thr Glu His Leu Ile Lys Lys Tyr Ser Ala
                165                 170                 175
Asn Arg Ile Arg Ile Arg Ser Phe Asn Gln Ser Arg Phe Pro Arg Val
            180                 185                 190
Tyr Lys Asp Ser Leu Leu Pro Val Pro Thr Glu Tyr Asp Ser Pro Leu
        195                 200                 205
Asp Ala Trp Tyr Pro Pro Gly His Gly Asp Leu Phe Glu Ser Leu His
    210                 215                 220
Val Ser Gly Glu Leu Asp Ala Leu Ile Ala Gln Gly Arg Glu Ile Leu
225                 230                 235                 240
Phe Val Ser Asn Gly Asp Asn Leu Gly Ala Thr Val Asp Leu Lys Ile
                245                 250                 255

Leu Asn His Met Ile Glu Thr Gly Ala Glu Tyr Ile Met Glu Leu Thr
            260                 265                 270
Asp Lys Thr Arg Ala Asp Val Lys Gly Gly Thr Leu Ile Ser Tyr Asp
        275                 280                 285
Gly Gln Val Arg Leu Leu Glu Val Ala Gln Val Pro Lys Glu His Ile
    290                 295                 300
Asp Glu Phe Lys Asn Ile Arg Lys Phe Thr Asn Phe Asn Thr Asn Asn
305                 310                 315                 320
Leu Trp Ile Asn Leu Lys Ala Val Lys Arg Leu Ile Glu Ser Ser Asn
                325                 330                 335
Leu Glu Met Glu Ile Ile Pro Asn Gln Lys Thr Ile Thr Arg Asp Gly
            340                 345                 350
His Glu Ile Asn Val Leu Gln Leu Glu Thr Ala Cys Gly Ala Ala Ile
        355                 360                 365
Arg His Phe Asp Gly Ala His Gly Val Val Val Pro Arg Ser Arg Phe
    370                 375                 380
Leu Pro Val Lys Thr Cys Ser Asp Leu Leu Leu Val Lys Ser Asp Leu
385                 390                 395                 400
Phe Arg Leu Glu His Gly Ser Leu Lys Leu Asp Pro Ser Arg Phe Gly
                405                 410                 415
Pro Asn Pro Leu Ile Lys Leu Gly Ser His Phe Lys Lys Val Ser Gly
            420                 425                 430
Phe Asn Ala Arg Ile Pro His Ile Pro Lys Ile Val Glu Leu Asp His
        435                 440                 445
Leu Thr Ile Thr Gly Asn Val Phe Leu Gly Lys Asp Val Thr Leu Arg
    450                 455                 460
Gly Thr Val Ile Ile Val Cys Ser Asp Gly His Lys Ile Asp Ile Pro
465                 470                 475                 480
Asn Gly Ser Ile Leu Glu Asn Val Val Val Thr Gly Asn Leu Gln Ile
                485                 490                 495
Leu Glu His


<210> 58
<211> 480
<212> DNA
<213> Saccharomyces cerevisiae


<220>
<223> Nucleic acid sequence of Glucosamine 6-phosphate
      N-acetyltransferase (GNA1) originating from Saccharomyces
      cerevisiae

<400> 58
atgagcttac ccgatggatt ttatataagg cgaatggaag agggggattt ggaacaggtc      60

actgagacgc taaaggtttt gaccaccgtg ggcactatta cccccgaatc cttcagcaaa     120

ctcataaaat actggaatga agccacagta tggaatgata acgaagataa aaaaataatg     180

caatataacc ccatggtgat tgtggacaag cgcaccgaga cggttgccgc tacggggaat     240

atcatcatcg aaagaaagat cattcatgaa ctggggctat gtggccacat cgaggacatt     300

gcagtaaact ccaagtatca gggccaaggt ttgggcaagc tcttgattga tcaattggta     360

actatcggct ttgactacgg ttgttataag attattttag attgcgatga gaaaaatgtc     420

aaattctatg aaaaatgtgg gtttagcaac gcaggcgtgg aaatgcaaat tagaaaatag     480


<210> 59

<211> 159
<212> PRT
<213> Saccharomyces cerevisiae

<220>
<223> Amino acid sequence of Glucosamine 6-phosphate
      N-acetyltransferase (GNA1) originating from Saccharomyces
      cerevisiae

<400> 59
Met Ser Leu Pro Asp Gly Phe Tyr Ile Arg Arg Met Glu Glu Gly Asp
1               5                   10                  15
Leu Glu Gln Val Thr Glu Thr Leu Lys Val Leu Thr Thr Val Gly Thr
            20                  25                  30
Ile Thr Pro Glu Ser Phe Ser Lys Leu Ile Lys Tyr Trp Asn Glu Ala
        35                  40                  45
Thr Val Trp Asn Asp Asn Glu Asp Lys Lys Ile Met Gln Tyr Asn Pro
    50                  55                  60
Met Val Ile Val Asp Lys Arg Thr Glu Thr Val Ala Ala Thr Gly Asn
65                  70                  75                  80
Ile Ile Ile Glu Arg Lys Ile Ile His Glu Leu Gly Leu Cys Gly His
                85                  90                  95
Ile Glu Asp Ile Ala Val Asn Ser Lys Tyr Gln Gly Gln Gly Leu Gly
            100                 105                 110
Lys Leu Leu Ile Asp Gln Leu Val Thr Ile Gly Phe Asp Tyr Gly Cys
        115                 120                 125
Tyr Lys Ile Ile Leu Asp Cys Asp Glu Lys Asn Val Lys Phe Tyr Glu
    130                 135                 140
Lys Cys Gly Phe Ser Asn Ala Gly Val Glu Met Gln Ile Arg Lys
145                 150                 155

<210> 60
<211> 1674
<212> DNA
<213> Saccharomyces cerevisiae

<220>
<223> Nucleic acid sequence of phosphoacetylglucosamine mutase (PCM1)
      originating from Saccharomyces cerevisiae

<400> 60
atgaaggttg attacgagca attgtgcaaa ctctacgatg acacgtgccg cacaaagaat      60

gtgcagttca gttacggtac ggccggattc agaacgctgg ccaagaattt ggatacggtg     120

atgttcagta ctggtatact ggcggttctc aggtcgctga agcttcaggg tcagtatgtg     180

ggggtgatga tcacggcgtc gcacaaccca taccaggaca acggggtcaa gatcgtggaa     240

ccagacggat cgatgctttt ggccacatgg gagccatatg ccatgcagtt ggccaatgcg     300

gcctcttttg ccactaattt tgaagaattt cgtgttgagt tggccaagct gattgaacac     360

gaaaagattg atttgaatac aaccgtcgtg cctcacatcg tggttgggag agactctagg     420

gaaagtagtc catacttgct gcgctgcttg acttcctcca tggccagcgt cttccacgcg     480

caagttttgg acctaggctg tgtcactacg cctcaattgc attacattac tgatttgtcc     540

aacaggcgga aactggaagg agacacagcg ccagttgcca cagaacagga ctactattcg     600

ttctttatag gagccttcaa cgagctcttc gccacgtatc agctggagaa gaggctgtct 660

gtcccaaaat tgttcataga cacagccaat ggtatcggtg gtccacagtt gaaaaaacta 720

ctggcctccg aagattggga cgtgccagcg gagcaagttg aggtaatcaa cgacaggtcc 780

gatgttccag aactgttgaa ttttgaatgc ggtgcggatt atgtgaagac taaccagaga 840

ttacccaagg gtctttctcc atcctcgttt gattcgctat attgctcctt tgatggtgac 900

gcagacaggg ttgtgttcta ctatgtcgac tcaggatcaa aatttcattt gttggatggt 960

gacaaaattt ccactttgtt tgcaaagttc ttgtctaaac aactagaatt ggcacaccta 1020

gaacattctt tgaagattgg tgttgtgcaa actgcctatg caaacggcag ttccaccgct 1080

tacataaaaa atacgttgca ctgtcccgtg tcttgcacta agacaggtgt taaacacttg 1140

catcatgaag ctgccactca gtacgatatt ggcatttatt tcgaagcaaa tggacatggt 1200

acgattatat tcagcgaaaa atttcatcga actatcaaat ctgaattatc caagtccaag 1260

ttaaatggtg atacgttagc tttgagaact ttgaagtgtt tctctgaatt gattaatcag 1320

accgtgggag atgctatttc agacatgctt gctgtccttg ctactttggc gattttgaaa 1380

atgtcgccaa tggattggga tgaagagtat actgatttgc ccaacaagct ggttaagtgc 1440

atcgttcctg ataggtcaat tttccaaacc acggaccagg aaagaaaatt gctcaatcca 1500

gtggggttgc aagacaagat agatcttgtg gtagccaagt atcccatggg aagaagcttt 1560

gtcagagcca gtggtacgga ggatgcggtg agggtttatg cggaatgtaa ggactcctct 1620

aagttaggtc aatttgtgga cgaagtggtg gagcacgtta aggcatctgc ttga 1674

<210> 61
<211> 557
<212> PRT
<213> Saccharomyces cerevisiae

<220>
<223> Amino acid sequence of phosphoacetylglucosamine mutase (PCM1)
      originating from Saccharomyces cerevisiae

<400> 61
Met Lys Val Asp Tyr Glu Gln Leu Cys Lys Leu Tyr Asp Asp Thr Cys
1               5                   10                  15
Arg Thr Lys Asn Val Gln Phe Ser Tyr Gly Thr Ala Gly Phe Arg Thr
            20                  25                  30
Leu Ala Lys Asn Leu Asp Thr Val Met Phe Ser Thr Gly Ile Leu Ala
        35                  40                  45
Val Leu Arg Ser Leu Lys Leu Gln Gly Gln Tyr Val Gly Val Met Ile
    50                  55                  60
Thr Ala Ser His Asn Pro Tyr Gln Asp Asn Gly Val Lys Ile Val Glu
65                  70                  75                  80
Pro Asp Gly Ser Met Leu Leu Ala Thr Trp Glu Pro Tyr Ala Met Gln
                85                  90                  95
Leu Ala Asn Ala Ala Ser Phe Ala Thr Asn Phe Glu Glu Phe Arg Val
            100                 105                 110
Glu Leu Ala Lys Leu Ile Glu His Glu Lys Ile Asp Leu Asn Thr Thr

115                 120                 125
Val Val Pro His Ile Val Val Gly Arg Asp Ser Arg Glu Ser Ser Pro
    130                 135                 140
Tyr Leu Leu Arg Cys Leu Thr Ser Ser Met Ala Ser Val Phe His Ala
145                 150                 155                 160
Gln Val Leu Asp Leu Gly Cys Val Thr Thr Pro Gln Leu His Tyr Ile
                165                 170                 175
Thr Asp Leu Ser Asn Arg Arg Lys Leu Glu Gly Asp Thr Ala Pro Val
            180                 185                 190
Ala Thr Glu Gln Asp Tyr Tyr Ser Phe Phe Ile Gly Ala Phe Asn Glu
        195                 200                 205
Leu Phe Ala Thr Tyr Gln Leu Glu Lys Arg Leu Ser Val Pro Lys Leu
    210                 215                 220
Phe Ile Asp Thr Ala Asn Gly Ile Gly Gly Pro Gln Leu Lys Lys Leu
225                 230                 235                 240
Leu Ala Ser Glu Asp Trp Asp Val Pro Ala Glu Gln Val Glu Val Ile
                245                 250                 255
Asn Asp Arg Ser Asp Val Pro Glu Leu Leu Asn Phe Glu Cys Gly Ala
            260                 265                 270
Asp Tyr Val Lys Thr Asn Gln Arg Leu Pro Lys Gly Leu Ser Pro Ser
        275                 280                 285
Ser Phe Asp Ser Leu Tyr Cys Ser Phe Asp Gly Asp Ala Asp Arg Val
    290                 295                 300
Val Phe Tyr Tyr Val Asp Ser Gly Ser Lys Phe His Leu Leu Asp Gly
305                 310                 315                 320
Asp Lys Ile Ser Thr Leu Phe Ala Lys Phe Leu Ser Lys Gln Leu Glu
                325                 330                 335
Leu Ala His Leu Glu His Ser Leu Lys Ile Gly Val Val Gln Thr Ala
            340                 345                 350
Tyr Ala Asn Gly Ser Ser Thr Ala Tyr Ile Lys Asn Thr Leu His Cys
        355                 360                 365
Pro Val Ser Cys Thr Lys Thr Gly Val Lys His Leu His His Glu Ala
    370                 375                 380
Ala Thr Gln Tyr Asp Ile Gly Ile Tyr Phe Glu Ala Asn Gly His Gly
385                 390                 395                 400
Thr Ile Ile Phe Ser Glu Lys Phe His Arg Thr Ile Lys Ser Glu Leu
                405                 410                 415
Ser Lys Ser Lys Leu Asn Gly Asp Thr Leu Ala Leu Arg Thr Leu Lys
            420                 425                 430
Cys Phe Ser Glu Leu Ile Asn Gln Thr Val Gly Asp Ala Ile Ser Asp
        435                 440                 445
Met Leu Ala Val Leu Ala Thr Leu Ala Ile Leu Lys Met Ser Pro Met
    450                 455                 460
Asp Trp Asp Glu Glu Tyr Thr Asp Leu Pro Asn Lys Leu Val Lys Cys
465                 470                 475                 480
Ile Val Pro Asp Arg Ser Ile Phe Gln Thr Thr Asp Gln Glu Arg Lys
                485                 490                 495
Leu Leu Asn Pro Val Gly Leu Gln Asp Lys Ile Asp Leu Val Val Ala
            500                 505                 510
Lys Tyr Pro Met Gly Arg Ser Phe Val Arg Ala Ser Gly Thr Glu Asp
        515                 520                 525
Ala Val Arg Val Tyr Ala Glu Cys Lys Asp Ser Ser Lys Leu Gly Gln
    530                 535                 540
Phe Cys Asp Glu Val Val Glu His Val Lys Ala Ser Ala
545                 550                 555

<210> 62
<211> 574
<212> DNA
<213> Artificial Sequence


<220>

<223> Nucleic acid sequence of promotor pTDH3

<400> 62
ctgctgtaac ccgtacatgc ccaaaatagg gggcgggtta cacagaatat ataacatcgt 60

aggtgtctgg gtgaacagtt tattcctggc atccactaaa tataatggag cccgcttttt 120

aagctggcat ccagaaaaaa aaagaatccc agcaccaaaa tattgttttc ttcaccaacc 180

atcagttcat aggtccattc tcttagcgca actacagaga acaggggcac aaacaggcaa 240

aaaacgggca caacctcaat ggagtgatgc aacctgcctg gagtaaatga tgacacaagg 300

caattgaccc acgcatgtat ctatctcatt ttcttacacc ttctattacc ttctgctctc 360

tctgatttgg aaaaagctga aaaaaaaggt tgaaaccagt tccctgaaat tattccccta 420

cttgactaat aagtatataa agacggtagg tattgattgt aattctgtaa atctatttct 480

taaacttctt aaattctact tttatagtta gtcttttttt tagttttaaa acaccaagaa 540

cttagtttcg aataaacaca cataaacaaa caaa 574

<210> 63
<211> 585
<212> DNA
<213> Saccharomyces kudriavzevii

<220>
<223> Nucleic acid sequence of promotor pTDH3.Sk

<400> 63
caattcatcg gcccttttag cggctacccg cgccatctaa atgatagggc gggtgacact 60

atggtaaatc ccataattag gtgtctgggt gagtggttct gatgccggca tccactaaat 120

atattggagc ccatttttta cgcgggcttc cagaaaaaaa gagaatccca gcaccaaaag 180

gtggttctct tcaccaacca tcagatcata ggtccacaac cacacataac aggggcacaa 240

aaaggcaaaa aacggacata acctcaatgg agtgatgcaa attgactgga gcaaaagctg 300

acacaaggca ttgattgacc tacgcatgta tctgtattct tttcttacac cttctattac 360

cttctaactc tttgggttgg aaaaaactga aaaaaaaggt tgggacctgg ttcccccaag 420

ttgtccccct acttggttat taaatatata aagacagcaa gtgttgatta taatcttgta 480

aatctatagt tcttaatcta tacttctatt tatattttaa attagtcttt ttatttccaa 540

gtccccaaga acttagtttc gaataaacac acacaaataa acaca 585

<210> 64
<211> 700
<212> DNA
<213> Saccharomyces bayanus

<220>
<223> Nucleic acid sequence of promotor pTDH3-1.sba

<400> 64
gcagcgcttc ttccgctcta gtttttatag ttattattac taccacctta aaaatacgta 60

aatactcaaa atagtagtga tattcccaac cttattcatc caaggcacat catcatcatc 120

agccattcat ctttcacctg ccattagtaa cccgtcttct cattgagcgg gttacggcag 180

ccacaggcca cattccgaat gtctgggtga gcggtccctt ttccagcatc cactaaatat 240

ctccgatccc gctttttaat ctggcttcct gaaaaaaaga gaatcccagc accaaaaaat 300

ggctctcttc accaaccatc agatcatagg tcccattctc ttaccgcaac cgtacagaac 360

aggggaaaac gggtacaacc tcaatggagt gatgcaaact gactggagca aaaagctgac 420

acaaggcaat cgacctacgt gtctgtctat tttctcacac cttctattac cttctaactc 480

tctgggttgg aaaaaactga aaaaaaggtt gagaccagtt tccacaaatc atccccctgt 540

ttgattaata aatatataaa gacgacaact atcgatcata aactcataaa actataactc 600

ctttacactt cttattttat agttattcta ttttaattct tattgatttt aaaaccccaa 660

gaacttagtt tcgaaaacac acacacacaa acaattaaaa 700

<210> 65
<211> 602
<212> DNA
<213> Saccharomyces arboricola

<220>
<223> Nucleic acid sequence of promotor pTDH3.Sar

<400> 65
gagctaaata tcagcccttc gggtcctgcc tgctacccgg tcctgttcga ataaaaacgc 60

gggtaacacg acccagtaac acctgtcgtt gggtgtctgg gtcagaagtt ctgataccgg 120

cttccactaa atagattggg ttccgctctt tacgctggct tcctgaaaaa agagattccg 180

ggcaccaaaa aattggtctc tttgccaacc atcagatcat aggtccattc tcttaccata 240

accacacagg ataggggcac cacaggcgaa aatgggcaca aaatctcaat ggagtgatgc 300

aaattagctg gaacaaaagc tgacacaagg caattaacct gcgcatgtat ccatctcctt 360

ttcttacacc ttctcttacg ttctaactgt ttgggttgga aaaattaaaa aaaaaaggtt 420

gagaccagtt tccccaaatc gtccccctac ttgattcata aatatataaa gacgacaact 480

attgattata atcttgtaaa tctataactc tttactttct cctatttata atttaactta 540

atctttttag atttaaaacc ccaagaactt agtttcgaac aaacacacac aaataaacaa 600

aa 602

<210> 66
<211> 550
<212> DNA

<213> Artificial Sequence

<220>
<223> Nucleic acid sequence of promotor pENO2

<400> 66
cgctcagcat ctgcttcttc ccaaagatga acgcggcgtt atgtcactaa cgacgtgcac 60

caacttgcgg aaagtggaat cccgttccaa aactggcatc cactaattga tacatctaca 120

caccgcacgc cttttttctg aagcccactt tcgtggactt tgccatatgc aaaattcatg 180

aagtgtgata ccaagtcagc atacacctca ctagggtagt ttctttggtt gtattgatca 240

tttggttcat cgtggttcat taattttttt tctccattgc tttctggctt tgatcttact 300

atcatttgga tttttgtcga aggttgtaga attgtatgtg acaagtggca ccaagcatat 360

ataaaaaaaa aaagcattat cttcctacca gagttgattg ttaaaaacgt atttatagca 420

aacgcaattg taattaattc ttattttgta tcttttcttc ccttgtctca atcttttatt 480

tttattttat ttttcttttc ttagtttctt tcataacacc aagcaactaa tactataaca 540

tacaataata 550

<210> 67
<211> 598
<212> DNA
<213> Artificial Sequence

<220>
<223> Nucleic acid sequence of promotor pTEF3

<400> 67
ggctgataat agcgtataaa caatgcatac tttgtacgtt caaaatacaa tgcagtagat 60

atatttatgc atattacata taatacatat cacataggaa gcaacaggcg cgttggactt 120

ttaattttcg aggaccgcga atccttacat cacacccaat cccccacaag tgatccccca 180

cacaccatag cttcaaaatg tttctactcc ttttttactc ttccagattt tctcggactc 240

cgcgcatcgc cgtaccactt caaaacaccc aagcacagca tactaaattt cccctctttc 300

ttcctctagg gtgtcgttaa ttacccgtac taaaggtttg gaaaagaaaa aagagaccgc 360

ctcgtttctt tttcttcgtc gaaaaaggca ataaaaattt ttatcacgtt tctttttctt 420

gaaaattttt ttttttgatt tttttctctt tcgatgacct cccattgata tttaagttaa 480

taaacggtct tcaatttctc aagtttcagt ttcatttttc ttgttctatt acaacttttt 540

ttacttcttg ctcattagaa agaaagcata gcaatctaat ctaagtttta attacaaa 598

<210> 68
<211> 383
<212> DNA
<213> Artificial Sequence

<220>
<223> Nucleic acid sequence of promotor pTEF1

<400> 68
gtttagcttg cctcgtcccc gccgggtcac ccggccagcg acatggaggc ccagaatacc 60

ctccttgaca gtcttgacgt gcgcagctca ggggcatgat gtgactgtcg cccgtacatt 120

tagcccatac atccccatgt ataatcattt gcatccatac attttgatgg ccgcacggcg 180

cgaagcaaaa attacggctc ctcgctgcag acctgcgagc agggaaacgc tcccctcaca 240

gacgcgttga attgtcccca cgccgcgccc ctgtagagaa atataaaagg ttaggatttg 300

ccactgaggt tcttctttca tatacttcct tttaaaatct tgctacgata cagttctcac 360

atcacatccg aacataaaca acc 383


<210> 69
<211> 383
<212> DNA
<213> Ashbya gossypii


<220>
<223> Nucleic acid sequence of promotor pTEF1.ago

<400> 69
gtttagcttg cctcgtcccc gccgggtcac ccggccagcg acatggaggc ccagaatacc 60

ctccttgaca gtcttgacgt gcgcagctca ggggcatgat gtgactgtcg cccgtacatt 120

tagcccatac atccccatgt ataatcattt gcatccatac attttgatgg ccgcacggcg 180

cgaagcaaaa attacggctc ctcgctgcag acctgcgagc agggaaacgc tcccctcaca 240

gacgcgttga attgtcccca cgccgcgccc ctgtagagaa atataaaagg ttaggatttg 300

ccactgaggt tcttctttca tatacttcct tttaaaatct tgctacgata cagttctcac 360

atcacatccg aacataaaca acc 383


<210> 70
<211> 498
<212> DNA
<213> Saccharomyces bayanus


<220>
<223> Nucleic acid sequence of promotor pTEF1.Sba

<400> 70
cgccaacaaa ccttcgaaca ctttaatttt cgaggaccgc agatcctcac atcacaccca 60

cacccaagac tgcttccccc acacaccctg catctgtaca ctttcttctg ctctgttttt 120

ctctccggcg ttctctcggg tcgcccgcat cgccgcgccg gctggaaccg cccacgcacc 180

gcatattgca aatcgcctgc cccctcttgc tcctttttga gggcgcgccg ttacccgcgc 240

ccagggtccg gaaaaagaaa caaggctcta ccgcgtttct ttttccttgt cgaaaaaggc 300

aaaaatgaaa atttttatca cgtttctttt tttttgaaaa attttttttt tggttttttt 360

tctttcgatg gcctcccatt gatatttaag ttaataaatg gttttcagtt ttcaagtttc 420

agtttgtgtt cttctttgct aactttcact tacacctcga aagaaagtat agcaatctaa 480

tcttagtttt aattacaa 498

<210> 71
<211> 76
<212> DNA
<213> Artificial Sequence

<220>
<223> Nucleic acid sequence of promotor pPDC1

<400> 71
ttatttacct atctctaaac ttcaacacct tatatcataa ctaatatttc ttgagataag 60

cacactgcac ccatac 76

<210> 72
<211> 998
<212> DNA
<213> Artificial Sequence

<220>
<223> Nucleic acid sequence of promotor pCCW12

<400> 72
aaccagggca aagcaaaata aaagaaactt aatacgttat gccgtaatga agggctacca 60

aaaacgataa tctcaactgt aaacaggtac aatgcggacc cttttgccac aaaacataca 120

tcattcattg ccggaaaaag aaagaagtga agacagcagt gcagccagcc atgttgcgcc 180

aatctaatta tagatgctgg tgccctgagg atgtatctgg agccagccat ggcatcatgc 240

gctaccgccg gatgtaaaat ccgacacgca aaagaaaacc ttcgaggttg cgcacttcgc 300

ccacccatga accacacggt tagtccaaaa ggggcagttc agattccaga tgcgggaatt 360

agcttgctgc caccctcacc tcactaacgc tgcggtgtgc ggatacttca tgctatttat 420

agacgcgcgt gtcggaatca gcacgcgcaa gaaccaaatg ggaaaatcgg aatgggtcca 480

gaactgcttt gagtgctggc tattggcgtc tgatttccgt tttgggaatc ctttgccgcg 540

cgcccctctc aaaactccgc acaagtccca gaaagcggga aagaaataaa acgccaccaa 600

aaaaaaaaat aaaagccaat cctcgaagcg tgggtggtag gccctggatt atcccgtaca 660

agtatttctc aggagtaaaa aaaccgtttg ttttggaatt ccccatttcg cggccaccta 720

cgccgctatc tttgcaacaa ctatctgcga taactcagca aattttgcat attcgtgttg 780

cagtattgcg ataatgggag tcttacttcc aacataacgg cagaaagaaa tgtgagaaaa 840

ttttgcatcc tttgcctccg ttcaagtata taaagtcggc atgcttgata atctttcttt 900

ccatcctaca ttgttctaat tattcttatt ctcctttatt ctttcctaac ataccaagaa 960

attaatcttc tgtcattcgc ttaaacacta tatcaata 998

<210> 73
<211> 976
<212> DNA
<213> Saccharomyces mikatae

<220>
<223> Nucleic acid sequence of promotor pCCW12.Sm

<400> 73
cgttgtgctg tagtgaagga agactaaaaa ggataatcac agttgtaaag aggtataatg 60

cggacccttt tgccacaaaa cacacatctt tcgtttccaa aataagaaag aagaaagcaa 120

aaagattagc agccacgttg ctgcgatcta attatagacg gtggcgtcat cattctcacc 180

caagattgtg tcttgaacct gccacgggtc ctgcgttatc gccggatgta aaactagaca 240

tgcaaaaaaa ggaccttcca ggtagcgtgc tccacaccac ccatgaccac cacagttagt 300

ccaaaagagg cagcaccact tcccgatggg ggaattagat tgctgccacc ctcacctcac 360

taatgctgcg gtgtgcggat atgccctgct atatatagct ccgcgttttt gaaccagcac 420

agcgcgagca ccaaaaagga aaatcgcata ggcccagaac tgatttcagc acgggctatt 480

ggcgttgggt ttccgttctg ggaaaccttc gccgcgtccc cctcacaaac ctccgcacaa 540

gttcgagcaa gcgggaaaaa acgaaaaacg ccattaatac taaataaagc aaatcctcga 600

agcgtgggtg gcaagcccct ggatttttcc gcacaagtac tcttctcagg agtaaaaaaa 660

cccgtttgtt ttggaattcc ccatttcgcg gccatctacg ccggtatctt cgcaatatct 720

atcagcgata actcagcaat tttaatattc gtgttgcagt gctgcgatag cgggagtctt 780

gtttgtaaca taacggcaga aaggaatgag agaaaatttt ccattctttg gcctccgttc 840

aagtatataa agccggcatg cttggtaatc tttctctctc ttctgtattg tttctataat 900

acttttatct tctaattatt ttctgaaaaa accaagaaat taatcttctg tcattcgctt 960

aaacactata tcaata 976

<210> 74
<211> 750
<212> DNA
<213> Saccharomyces kudriavzevii

<220>
<223> Nucleic acid sequence of promotor pCCW12.Sk

<400> 74
tgtaaaatcc tacacgcaaa aaacctcttg ggttgcgcgt tttccaccac ccacgacccg 60

cacaatcaat ccaaaagggg caacgccagt tcccaatgcg ggaattagct cctcaccccc 120

ctcacccgct aacgctgcgg tgtgcggaca cgcagcagta tttatagatc ctcgtgttgg 180

aaccagcccg cgtgagcacc aaattggaaa gtcgcaatgg gcccagaacc gctttcagta 240

ctgggccatt ggcgtctagt ttccgttttg agcgtccttc gccgcgtctc tctgtgaaat 300

ccccgcacaa gtctgagcag gcaaaaaaaa aaaacgccac caaaaataaa taaagccaat 360

cctcgaagga tgagtaggaa aggaagcccc tggttttttc ccgcacgaat atttttcagg 420

agtgaaaaaa tccgtttgtt ttggaattcc ccatttcgcg ctcacctacg ccggtatctt 480

tggaacaact atcagcgata actcagcaaa atttgcatat ccgtgctgca atagtgcgat 540

agtgggattg ggagtcttgt tgcatcataa cggcagaaag gaatgaataa aaattttccg 600

ttctttgtct ccgttcaagt atataaagtc ggcatgcttg attatctttt cttctcttct 660

actacatttc tatttcactt tctactctat tcttccctga aaaacccaag aaaataatct 720

tctgtcattc gcttaaacaa tatatcaaaa 750

<210> 75
<211> 750
<212> DNA
<213> Saccharomyces bayanus

<220>
<223> Nucleic acid sequence of promotor pCCW12.Sba

<400> 75
accatgccac ggtgctggcc ccacttccac ccacgacccg cacggttggc ccgaaagggg 60

caacaccagt tcccaatacg gaaattagtc cgccaccacc ctcacctcgc tagagctgcg 120

gtgtgcgggc gtgcatcgct atttatagac atgcctgctg gcgtcaccgc gcgcgagcac 180

caaacaggaa aatcgcactg ggcccagaac cacgctatgc gctgggccga tggcgtccgg 240

tttccctttg ggagccccct gccacgttcg cctaacaaat ccccgcaccg gcttgagaaa 300

aaagcgaaaa gcgaaaaaaa aaaatcaacg ccaccaaaat taaaaaaaaa gagccatcct 360

cgaagggtga atagtagccc ctgacttttc ccgcacagac agacaccttt caggagtgaa 420

caaaaaagca gtttgttttg gaattccccc atttcgcggt ggcctgcgca ggtatctctg 480

cgtcaactat cagcgataac tcagcaaatt ttgcatattc gtgttgcgat actacgataa 540

tgggagtctg tcgcctaata acggcaacaa ggaatgagag agaaaaattt tcttcattct 600

ccagctcccg ttcaagtata taaggtcggc atggtcgatt gtctttcctt ctcttcagtt 660

acgtctctct atttacatta ttcttatttt tatttaataa aaaccccaag aaattaatct 720

tctgtcattc gcttaaacac tatatcaaaa 750

<210> 76
<211> 750
<212> DNA
<213> Saccharomyces arboricola

<220>
<223> Nucleic acid sequence of promotor pCCW12.Sar

<400> 76
aaaaaacaac cttctgccaa cctgcgtgct tctcaccacc catgacccac acaattgacc 60

cgaatggggc aactccagtt cccaatacgg gaattaactc gccaccatat ttaccgcgtt 120

gaagctgtgg tgtgcggaca ctccgtacta tttatagacc cacgcggtgg aaccagcacg 180

cgcgcgcact aaacaggaaa atcgcattga gtccagaacc gccaccagca cttggccatt 240

ggcgtctaat ttccgttttc ggcgcccctc accgcgtcct tctaacaaag cgcgcacaag 300

cttgagcaag tgaaaagaaa attaaaaata aaaaaccgcc accaaaacaa ataaagcaat 360

cttcgaagtg tgggttggtg ggaagcccct ggcttttccc gcaccagtcg ttttcaggag 420

taaaaaaata cccgtttgtt ttggaattcc ccatttcgcg gcgacctgcg ccggtatctt 480

tgcaacaact atttgcgata actcagcaaa atttgcatat tcgtgttggg atattgcgat 540

agtgggagtc ttgttgcata ataacggtaa aaagaagtga aggaaaaaaa tttgcatcct 600

ttagtctcag ttcaagtata taaagtcggg atattcaatt atctttcttt ctcttgctca 660

aaggtttcta tatttttttt atagtatttc ttttgttata aaataccaag aaattaatct 720

tctgtcattc gcttaaatac tacatcaata 750

<210> 77
<211> 500
<212> DNA
<213> Artificial Sequence

<220>
<223> Nucleic acid sequence of promotor pNUP57

<400> 77
tcatctgcgc aatgactatc aagaccttct gcaagaattt caaatctcac tgaaaatctt 60

gaccgaaaag tgtcttgaaa acccatcaag cctgcaaaac ctatctttga cattagtctc 120

cattataaaa acggcatagt tgggagaaaa ctttccatac ttcaattgtg gactgatata 180

agtattttag ttttgcccgc atgatcatcc cacatggcta cagcagttct ctcataggaa 240

aatagtacaa tagctacgtg atataatcta aataattgtt gccaatgtgt aattatatca 300

ttttgaacgt acgcgaaatg gattattttc aaaaattttg tttcttgaaa tgagtaaaag 360

caaaagtcca actctccaag tcgatgtaaa caactttttg ccaaagggac tgaaagacta 420

aatcgaggat tatcccgttc aaactattcc agaaacgctc gttagtaaca aaagacatac 480

cttgttgacc aattgatcac 500

<210> 78
<211> 523
<212> DNA
<213> Ashbya gossypii

<220>
<223> Nucleic acid sequence of promotor pCCW10.ago

<400> 78
ggtaccacgg caacctcgtt cgctgttcat ccccttcgtc acacaggacg ttggatgccg 60

taagcagcgt tgcttttgat cctcaggatc ggccgggtaa cccgcggctg cttctatttt 120

agtattcata tctcaagcac atccattccg gccgtttggg ggcgccgccg cactcgtgtc 180

cattcctacc gtggcactta gggctatcct gtcggagcgc cccgccgacc gccttatcgg 240

caccaaaagt agaagccccg gccccgcgtg gctcagactc accatcggtg ctatttactt 300

ttcgatcaga tcgcggcgcg cggtggccgg catttccgga agcggccacg gagcagaggt 360

ggcgcattcg aatcgcatac gtcttcgcca cgccggaaaa aaaattttcg gctatataag 420

gagaggcggc cgtcttgctg caggcagttt cactttctct aaaaccaaag aacatcgatt 480

tctttagtca ctcgcttcct tacaccgaac tcgaggcggc cgc 523

<210> 79
<211> 943
<212> DNA
<213> Artificial Sequence

<220>
<223> Nucleic acid sequence of promotor pCWP2

<400> 79
ctagcctccc ctttttattt tgtgcggtca ccgcaaggga caaagctttt cttagaaaac 60

cgtctgagaa gcataacgta cgccatcccc tagacatatt aataatgcta cagatactat 120

gctgctcgtc tttttttgac gaccctttta ttgcaatgtg caactaatgg caaacaacca 180

catagtatca cagtattaca ttgcctccac cgatgcggat gttagggcgc caagtctgtc 240

atgaagcatg ttcctgtcat aatcttgtat gcaaaatacc gcgttctgcg ccactgatat 300

gctaggcagc agcaacctat gcagaagatt gcttttccca cgcctgtttt acgtctccag 360

ggcacttgaa acaatgcagc gatcgccgcc acaacacgcc aaagagaagc gaaagtgggc 420

ctgggcggcc tcagtttcgg cagaggtaaa caacacgaac tgaactgcct tagctccgaa 480

gggcaattcc acaggcactc cgcggggccc ggccaaggcc caaaaggcgt ggaatatgcg 540

cgttttgggg ccataacacc cagtaccacg gccggaacgg gccatataat aagtttttca 600

ctctcaagaa tggtaaacgt aaataggaac atcccactac cctagaaatt gcggaaattt 660

cgcgcttatc attagaaaat ctggaaccgt cctttttcct ctttcttgca tttccctttc 720

cgtattattg ccattcttta actgcatttg gggaaccgta gaccaaaagc caaacagaga 780

aatgtaacgt tctaaaaaaa aaacaacgaa aaaattgaaa aataagatac aataatcgta 840

tataaatcag gcttcttgtt catcattttc aattctcttc ttgccatccc ttttcctatc 900

tttgttcttt tcttctcata atcaagaata aataacttca tca 943

<210> 80
<211> 535
<212> DNA
<213> Artificial Sequence

<220>
<223> Nucleic acid sequence of promotor pRPLA1

<400> 80
tcaagttgga tactgatctg atctctccgc cctactacca gggaccctca tgattaccgc 60

tcgaatgcga cgtttcctgc ctcataaaac tggcttgaaa atatttattc gctgaacagt 120

agcctagctt ataaaaattt catttaatta atgtaatatg aaaactcaca tgccttctgt 180

ttctaaaatt gtcacagcaa gaaataacat taccatacgt gatcttatta aactctagta 240

tcttgtctaa tacttcattt aaaagaagcc ttaaccctgt agcctcatct atgtctgcta 300

catatcgtga ggtacgaata tcgtaagatg ataccacgca actttgtaat gatttttttt 360

ttttcatttt ttaaagaatg cctttacatg gtatttgaaa aaaatatctt tataaagttt 420

gcgatctctt ctgttctgaa taatttttag taaaagaaat caaaagaata aagaaatagt 480

ccgctttgtc caatacaaca gcttaaaccg attatctcta aaataacaag aagaa 535

<210> 81
<211> 505
<212> DNA
<213> Artificial Sequence

<220>
<223> Nucleic acid sequence of promotor pCUP1

<400> 81
cggcaaactt caacgatttc tatgatgcat tttataatta gtaagccgat cccattaccg 60

acatttgggc gctatacgtg catatgttca tgtatgtatc tgtatttaaa acacttttgt 120

attatttttc ctcatatatg tgtataggtt tatacggatg atttaattat tacttcacca 180

ccctttattt caggctgata tcttagcctt gttactagtt agaaaaagac atttttgctg 240

tcagtcactg tcaagagatt cttttgctgg catttcttct agaagcaaaa agagcgatgc 300

gtcttttccg ctgaaccgtt ccagcaaaaa agactaccaa cgcaatatgg attgtcagaa 360

tcatataaaa gagaagcaaa taactccttg tcttgtatca attgcattat aatatcttct 420

tgttagtgca atatcatata gaagtcatcg aaatagatat taagaaaaac aaactgtaca 480

atcaatcaat caatcatcac ataaa 505

<210> 82
<211> 510
<212> DNA
<213> Artificial Sequence

<220>
<223> Nucleic acid sequence of promotor pMET6

<400> 82
ccacaggaaa tatttcacgt gacttacaaa cagagtcgta cgtcaggacc ggagtcaggt 60

gaaaaaatgt gggccggtaa agggaaaaaa ccagaaacgg gactactatc gaactcgttt 120

agtcgcgaac gtgcaaaagg ccaatatttt tcgctagagt catcgcagtc atggcagctc 180

tttcgctcta tctcccggtc gcaaaactgt ggtagtcata gctcgttctg ctcaattgag 240

aactgtgaat gtgaatatgg aacaaatgcg atagatgcac taatttaagg gaagctagct 300

agttttccca actgcgaaag aaaaaaagga aagaaaaaaa aattctatat aagtgataga 360

tatttccatc tttactagca ttagtttctc ttttacgtat tcaatatttt tgttaaactc 420

ttcctttatc ataaaaaagc aagcatctaa gagcattgac aacactctaa gaaacaaaat 480

accaatataa tttcaaagta catatcaaaa 510

<210> 83
<211> 686
<212> DNA
<213> Artificial Sequence

<220>
<223> Nucleic acid sequence of promotor pMET25

<400> 83
ttacattatc aatccttgcg tttcagcttc cactaattta gatgactatt tctcatcatt 60

tgcgtcatct tctaacaccg tatatgataa tatactagta acgtaaatac tagttagtag 120

atgatagttg atttttattc caacactaag aaataatttc gccatttctt gaatgtattt 180

aaagatattt aatgctataa tagacattta aatccaattc ttccaacata caatgggagt 240

ttggccgagt ggtttaaggc gtcagattta ggtggattta acctctaaaa tctctgatat 300

cttcggatgc aagggttcga atcccttagc tctcattatt ttttgctttt tctcttgagg 360

tcacatgatc gcaaaatggc aaatggcacg tgaagctgtc gatattgggg aactgtggtg 420

gttggcaaat gactaattaa gttagtcaag gcgccatcct catgaaaact gtgtaacata 480

ataaccgaag tgtcgaaaag gtggcacctt gtccaattga acacgctcga tgaaaaaaat 540

aagatatata taaggttaag taaagcgtct gttagaaagg aagtttttcc tttttcttgc 600

tctcttgtct tttcatctac tatttccttc gtgtaataca gggtcgtcag atacatagat 660

acaattctat tacccccatc cataca 686

<210> 84
<211> 363
<212> DNA
<213> Artificial Sequence

<220>
<223> Nucleic acid sequence of promotor pSAM1

<400> 84
gaaacggacg taagacggaa atagaatttg aagataaagt tatatatcac tacacacgaa 60

tactttcttt tttttttttc acaggaaaac tgtggtggcg cccttgccta ctagtgcatt 120

tcttttttcg ggttcttgtc tcgacgaaat tttagcctca tcgtagtttt tcactctggt 180

atcgatgaaa aagggaagag taaaaagttt tccgtttagt acttaatggg attggtttgg 240

gacgtatata tcgactggtg ttgtctgtta ttcatcgttg tttttcggtt agcttcgaaa 300

aaaaaataga gtaaaaacca ggaatttacc ctaaaaacaa gaaaaaataa gataaacgaa 360

aat 363

<210> 85
<211> 300
<212> DNA
<213> Artificial Sequence

<220>
<223> Nucleic acid sequence of terminator tTPI1

<400> 85
gattaatata attatataaa aatattatct tcttttcttt atatctagtg ttatgtaaaa 60

taaattgatg actacggaaa gcttttttat attgtttctt tttcattctg agccacttaa 120

atttcgtgaa tgttcttgta agggacggta gatttacaag tgatacaaca aaaagcaagg 180

cgctttttct aataaaaaga agaaaagcat ttaacaattg aacacctcta tatcaacgaa 240

gaatattact ttgtctctaa atccttgtaa aatgtgtacg atctctatat gggttactca 300

<210> 86
<211> 299
<212> DNA
<213> Artificial Sequence

<220>

<223> Nucleic acid sequence of terminator tMET25

<400> 86
gtgtgcgtaa tgagttgtaa aattatgtat aaacctactt tctctcacaa gtactatact      60

tttataaaac gaactttatt gaaatgaata tccttttttt cccttgttac atgtcgtgac     120

tcgtactttg aacctaaatt gttctaacat caaagaacag tgttaattcg cagtcgagaa     180

gaaaaatatg gtgaacaaga ctcatctact tcatgagact actttacgcc tcctataaag     240

ctgtcacact ggataaattt attgtaggac caagttacaa aagaggatga tggaggttt      299


<210> 87
<211> 300
<212> DNA
<213> Artificial Sequence


<220>
<223> Nucleic acid sequence of terminator tDIT1

<400> 87
taaagtaaga gcgctacatt ggtctacctt tttgttcttt tacttaaaca ttagttagtt      60

cgttttcttt ttctcatttt tttatgtttc ccccccaaag ttctgatttt ataatatttt     120

atttcacaca attccattta acagaggggg aatagattct ttagcttaga aaattagtga     180

tcaatatata tttgcctttc ttttcatctt ttcagtgata ttaatggttt cgagacactg     240

caatggccct agttgtctaa gaggatagat gttactgtca aagatgatat tttgaatttc     300


<210> 88
<211> 300
<212> DNA
<213> Artificial Sequence


<220>
<223> Nucleic acid sequence of terminator tRPL3

<400> 88
gaagttttgt tagaaaataa atcatttttt aattgagcat tcttattcct attttattta      60

aatagtttta tgtattgtta gctacataca acagtttaaa tcaaattttc tttttcccaa     120

gtccaaaatg gaggtttatt ttgatgaccc gcatgcgatt atgttttgaa agtataagac     180

tacatacatg tacatatatt taaacatgta aacccgtcca ttatattgct tactttcttc     240

ttttttgccg ttttgacttg gacctctggt ttgctatttc cttacaatct ttgctacaat     300


<210> 89
<211> 304
<212> DNA
<213> Saccharomyces mikatae


<220>

<223> Nucleic acid sequence of terminator tRPL3.sm

<400> 89
gaagttttta aagcattttt tagacacttc tcatttttct aagttttttt aaaatagttt 60

tatgtattta ctacgtatca caatttgaaa taattcatct tcccaaaaaa ctaagatttt 120

tatccttgtc acgatccgta accagtttat aatattttag agcttataca cgtacgtata 180

cacacgtgtc ggtacatgag aattacgttc aaaattattc actttttttt tctctgccgt 240

tttacttttg aactctgtct cgctatttcc ttacaatctt cgctacaata ccacttgccc 300

ttgg 304

<210> 90
<211> 299
<212> DNA
<213> Saccharomyces bayanus

<220>
<223> Nucleic acid sequence of terminator tRPL3.sba

<400> 90
gaagttttct gacaaaaaca taacgttttt tccaatcatt tcttattttt ccggtttatt 60

taaatagttt ttatgtacta ttatacgtat gactatttaa cttaaattct tcctcccaag 120

aaatctccca agtttttcat tatcatggca tacaccacta tcagttacaa aatggtagct 180

caaccatata tatatctcta tatacacata taaatgcaaa caggtccaag tcaccgctca 240

ctgcagtttc ttttgccgtt ttgacttcga tctctgcttg gctattttct cacaatcct 299

<210> 91
<211> 300
<212> DNA
<213> Artificial Sequence

<220>
<223> Nucleic acid sequence of terminator tRPL41B

<400> 91
gcggattgag agcaaatcgt taagttcagg tcaagtaaaa attgatttcg aaaactaatt 60

tctcttatac aatcctttga ttggaccgtc atcctttcga atataagatt ttgttaagaa 120

tattttagac agagatctac tttatattta atatctagat attacataat ttcctctcta 180

ataaaatatc attaataaaa taaaaatgaa gcgatttgat tttgtgttgt caacttagtt 240

tgccgctatg cctcttgggt aatgctatta ttgaatcgaa gggctttatt atattaccct 300

<210> 92
<211> 300
<212> DNA
<213> Artificial Sequence

<220>
<223> Nucleic acid sequence of terminator tRPL15A

<400> 92
gctggttgat ggaaaatata attttattgg gcaaactttt gtttatctga tgtgttttat      60

actattatct ttttaattaa tgattctata tacaaacctg tatatttttt ctttaaccaa     120

tttttttttt tatagaccta gagctgtact tttattctgc tatcaagcaa acccctaccc     180

cctcttctca atcctcccct caggcagaac ttatctacct gtatcaagga gcggacgagg     240

gagtcctaat tgttctacgt ataccaatgc tagcagctta cataggtggt ggcactacca     300


<210> 93
<211> 300
<212> DNA
<213> Saccharomyces bayanus


<220>
<223> Nucleic acid sequence of terminator tRPL15A.sba

<400> 93
gctggctgat gaaaaatata gttctgttgg gcaagcattt gtttacctag catctctttt      60

atactattat tatctttata tttgatgatt ttatatacaa gttgtatacc ttttctttaa     120

ccaatttttt tttttctaat ggtgcaccta gaagtacatt ttttctcacc aatagatagt     180

caagatactc ccagcctcta tggcgttacc acggagccga caagggaaag ctgcttatct     240

tacatatgca gatgccaagg cccgttacag gccgccttat tgatgtttag aaatagcttc     300


<210> 94
<211> 300
<212> DNA
<213> Artificial Sequence


<220>
<223> Nucleic acid sequence of terminator tIDP1

<400> 94
tcgaatttac gtagcccaat ctaccacttt tttttttcat tttttaaagt gttatactta      60

gttatgctct aggataatga actacttttt tttttttttt tttactgtta tcataaatat     120

atatacctta ttgttgtttg caaccgtcgg ttaattcctt atcaaggttc cccaagttcg     180

gatcattacc atcaatttcc aacattttca tgagttcttc ttcttcatta ccgtgtttta     240

gggggctgtt cgcacttcta atagggctat caccaagctg ttctaattcg tccaaaagtt     300


<210> 95
<211> 300
<212> DNA
<213> Saccharomyces bayanus

<220>
<223> Nucleic acid sequence of terminator tTEF1.sba

<400> 95
ggagattgat aggacttttc tagttgcata tcttttattt ttaaatctta tctattagtt      60

aattttttgt aatttatcct tatatatata gtttggttat tctaaaacat catttcagta     120

tctaaaacct ctcttattca ttaccttttt atttaatggt ttttgctaca ggcaaaaatt     180

taatggtttt tgctacaggc aaaaatcccg ccgtggactt attccacgtt aactcggtta     240

cagggtcatg aaccattttg tcaattatcg aaataacttc ttcaaaagtc cctcttactt     300